(19) Europäisches Patentamt / European Patent Office / Office européen des brevets

(11) EP 1 862 554 B1

(12) **EUROPEAN PATENT SPECIFICATION**

(45) Date of publication and mention of the grant of the patent:
**24.03.2010 Bulletin 2010/12**

(51) Int Cl.:
***C12P 7/64*** *(2006.01)*

(21) Application number: **07017066.7**

(22) Date of filing: **23.12.2004**

(54) **ENZYMATIC REMOVAL OF DIGLYCERIDES FROM EDIBLE OILS**

ENZYMATISCHE ENTFERNUNG VON DIGLYCERIDEN AUS SPEISEÖLEN

ÉLIMINATION ENZYMATIQUE DE DIGLYCÉRIDES D'HUILES ALIMENTAIRES

(84) Designated Contracting States:
**AT BE BG CH CY CZ DE DK EE ES FI FR GB GR HU IE IS IT LI LT LU MC NL PL PT RO SE SI SK TR**

(30) Priority:
**24.12.2003 GB 0330016**
**15.01.2004 PCT/IB2004/000655**
**16.07.2004 GB 0416023**
**26.07.2004 US 898775**

(43) Date of publication of application:
**05.12.2007 Bulletin 2007/49**

(83) **Declaration under Rule 32(1) EPC (expert solution)**

(62) Document number(s) of the earlier application(s) in accordance with Art. 76 EPC:
**04806534.6 / 1 704 240**

(73) Proprietor: **DANISCO A/S**
**1001 Copenhagen K. (DK)**

(72) Inventors:
- **Kristensen, Anna Cecilie**
  **8000 Arhus C. (DK)**
- **Wassell, Paul**
  **EAST Sussex, TN40 2PF (GB)**
- **Mikkelsen, Jörn Dalgaard**
  **2650 Hividovre (DK)**
- **Söe, Jörn Borch**
  **8381 Tilst (DK)**

(74) Representative: **Williams, Aylsa et al**
**D Young & Co**
**120 Holborn**
**London EC1N 2DY (GB)**

(56) References cited:
**US-A- 5 219 744**

- **UPTON ET AL: "A new family of lipolytic enzymes ?" TRENDS IN BIOCHEMICAL SCIENCES, vol. 20, May 1995 (1995-05), pages 178-179, XP004222260**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 862 554 B1

**Description**

FIELD OF INVENTION

The present invention relates to a novel method for the enzymatic removal and/or reduction of diglyceride (preferably 1, 2-diacylglyceride) from an edible oil.

TECHNICAL BACKGROUND

Oils and fats consist of complex mixtures of triacylglycerols (TAGs), diacylglycerols (DAGs), free fatty acids and other minor components. The crystallisation of these mixtures depends on the characteristics of the TAGs (structure, chain length, saturation compared with unsaturation, and the like) and the interaction of these TAGs with each other. Regarding the presence of DAGs, previous studies have shown that they have significant effect on the physical properties of oils and fats. These vary from rate of crystallisation, polymorphism changes, melting point, crystal size and habits (Siew, 2001).

In most oils that are extracted from oilseeds, the effect of DAGs is less pronounced, as DAGs are only present in small quantities. In primarily palm oil and olive oil, which are oils containing high natural amounts of DAGs, however, the quality of these oils suffers if DAGs are present therein.

Palm oil obtained from oil palm (*Elaeis guineensis*) is commercially important edible oil. Palm oil has been a prominent fat and oil resource for the food industry due to several advantageous properties, such as high productivity, low price, high thermal and oxidative stability and plasticity at room temperature, In addition, compared with other vegetable oils, palm oil is a rich source of the anti-oxidant vitamin E.

A typical chemical composition of refined palm oil is about 93% triglycerides, 6% diglycerides and 1% monoglycerides (MAGs) (Okiy, 1977).

When palm oil crystallizes, a complex 3-dimensional network of the present components is formed. In the theory it is described that the bigger the diversity of the building blocks (TAGs, DAGs and MAGs) in the network, the more complicated the network will be and the slower the crystallization will happen (Jacobsberg & Ho, 1976). This theory was confirmed by Drozdowski (1994). Furthermore, his studies showed that the more the fatty acid composition variation in the triacylglycerol molecule, the more difficult was the transition between the different crystal phases.

As previously mentioned, a high content of diglycerides in palm oil affects its crystallization properties (Okiy *et al.,* 1978, Okiy, 1978).

The presence of diglycerides in such oils is disadvantageous. In particular, diglycerides in edible oils (in particular palm oil) can lead to a low quality oil.

The problems relating to the diglyceride content in palm oil and other edible oils and fat have been the' subject of many studies and different solutions to attempt to overcome the problem of too much diglyceride can be found in the literature.

The Japanese enzyme producer Amano on their home page (Amano Enzyme Inc., 2004), recommend an enzymatic process to remove diglyceride in fats and oils. This process is based on the use of an enzyme LIPASE G "AMANO" 50 which is able to degrade diglycerides to free fatty acids and glycerol This enzyme is a diglyceride (DAG) and/or monoglyceride (MAG) hydrolyzing. The free fatty acids produced are removed by vacuum distillation or fractional crystallisation.

EP 0 55 8 112 describes a process for the enzymatic hydrolysis of residual diglycerides in triglyceride preparations in emulsions. The process is based on the hydrolysis of diglyceride with Lipase G from Amano, Japan (*supra*). The process was enhanced by making the enzymatic reaction in an emulsion for the degradation of diglyceride to fatty acids and glyceroL The water phase is separated after reaction and the enzyme is partly reused.

JP 62061590 teaches a hard butter containing low amounts of diglyceride, which is manufactured by treating oils or fats with a partially glyceride-specific enzyme (e.g., a lipase) in the presence of a catalytic amount of water and by a lipase that is a 1,3-specific enzyme in the presence of fatty acids, fatty acid esters, or other glyceride oils or fats. The product is hard butter especially suitable for use as a cacao butter substitute. Thus, lipase G and *Rhizopus deremer* lipase (1,3-specific enzyme) were mixed with diatomaceous earth and granulated. The granules was mixed with palm medium melting point fraction (5.7% diglyceride, acid value 0.25) and water (10% with regard to the partial glyceride-specific enzyme). The mixture was stirred at room temp. for 1 h, and the enzymes and water removed to give a hard butter containing 1.2% diglyceride (acid value 10.5).

The prior art thus teaches ways to reduce or remove the content of diglyceride in palm oil and other edible oils by enzymatic reactions. These processes rely on the hydrolysis of diglyceride with a specific diglyceride hydrolysing lipase during formation of free fatty acids and glycerol. The free fatty acids can then be removed by means of different processes like vacuum distillation or fractionation.

The disadvantage of using a specific diglyceride hydrolysing enzyme is the disadvantageous formation of free

fatty acids. These free fatty acids have to be removed from the palm oil. Thus, the formation of free fatty acids is often considered as loss of product.

To overcome the problems with the removal of free fatty acid and the loss of product caused by the free fatty acid formation we have found a new method to overcome the problems with high diglyceride content in palm oil and other vegetable oils.

Enzymatic removal of diglycerides from palm oil has been taught by use of lipases, which are typically 1,3 specific triacylglycerol hydrolyzing enzymes (E.C. 3.1.1.3) (for example see JP 62061590 or EP 0 652 289). WO00/05396 teaches *inter alia* treatment of a food material which may comprise glycerol with a lipase to effect glycerolysis in a low water environment.

However, both 1,3 specific triacylglycerol hydrolyzing enzymes (lipases) and DAG/MAG hydrolyzing enzymes result in a significant increase in free fatty acid in the oil, and also result in the hydrolysis of monoglycerides.

However, in some vegetable oils for some applications for example it may be desirable to increase the monoglyceride content of the oil as this provides emulsifier functionality. Thus, in one aspect it is preferable to reduce diglyceride content without decreasing the monoglyceride content. In another aspects it may preferably to reduce both the diglyceride and monoglyceride content.

Lipase enzymes can also result in a detrimental increase in DAG due to the hydrolysis of triacylglycerol (TAG), the bulk lipid present in food oils.

In WO2000/36114, US2003/0028923 and US2003/0074695 the transformation of a plant with a nucleic acid having a sequence which encodes a diacylglycerol acyltransferase (DGAT) enzyme or an antisense sequence thereto is taught. The DGAT enzyme taught in these documents catalyses the final step in the "Kennedy pathway" where a diacylglycerol (DAG) is combined with the acyl groups of acyl CoA to form a triglyceride (TAG). Thus, these documents teach the production of transgenic plants with modified TAG compositions and/or contents. The DGAT enzymes taught in these documents are not lipid acyl transferases and/or diglyceride:glycerol acyltransferases in accordance with the present invention.

In particular, DGAT's require the presence of acyl CoA or fatty acid CoA to function. Acyl CoA is not suitably for use commercially for treating edible oils as it is prohibitively expensive. However, these enzymes will not work without the present of acyl CoA. In addition enzyme reactions relying on fatty acid-CoA are very difficult to control industrially.

In addition, all of these documents teach that it is often desirable to reduce the expression of DGAT enzymes in a plant, thus to reduce the amount of TAG in the plant This contrasts sharply with the present invention which ultimately requires the preservation and/or production of TAG whilst reducing diglycerides (DAG) in an edible oil.

WO03/100044 teaches a phospholipids:diacylglycerol acyltransferase (PDAT) which catalyses the formation oftriglycerides (TAGs) by an acyltransfer from phospholipids (lecithin) to *inter alia* diacylglycerols (DAGs). PDATs require phospholipid as the acyl donor. This contrasts sharply with the present invention where the acyl donor is DAG. This document does not teach the removal of DAG from edible oils using a lipid acyltransferase in accordance with the present invention.

## SUMMARY OF THE INVENTION

It has been found that fatty-acid CoA independent diglyceride:glycerol acyltransferases of E.C.2.3.1.x, wherein x is any subgroup, but which is not an enzyme classified as E.C.2.3.1.20 or E.C.2.3.1.158, results in the selective reduction and/or removal of diglycerides (preferably 1,2-diglycerides) from edible oils.

The term "selective" as used herein means that in an edible oil environment the enzyme utilizes diglycerides (DAGs), preferably 1,2-diglycerides, as a substrate preferentially to either triacylglycerides (TAGs) or monoglycerides (MAGs). Thus, diglycerides can be removed and/or reduced from the edible oil whilst leaving the amount of triglyceride in the oil unchanged (or substantially unchanged). The amount of monoglycerides in the oil either remains unchanged (or substantially unchanged) or may increase. In some applications, the amount of monoglyceride in the oil may be reduced.

In a further aspect of the present invention there is provided a method of reducing and/or removing diglyceride from an edible oil, comprising a) admixing an edible oil with an acyl acceptor substrate and a fatty-acid CoA independent diglyceride:glycerol acyltransferase of E.C.2.3.1.x, wherein x is any subgroup, but which is not an enzyme classified as E.C.2.3.1.20 or E.C.2.3.1.158, wherein the fatty-acid CoA independent diglyceride:glycerol acyltransferase is characterized as an enzyme which in an edible oil can transfer an acyl group from a diglyceride to glycerol.

Suitably, the method according to the present invention may further comprise adding the treated edible oil or a portion thereof to one or more food constituents to formulate a foodstuff, such as margarine or spread for example.

The present invention yet further provides the use of a fatty-acid CoA independent diglyceride:glycerol acyltransferase as defined above, in the manufacture of a foodstuff, for reducing and/or removing (preferably selectively reducing and/or removing) diglyceride from said foodstuff.

In a further aspect, the present invention yet further provides the use of a fatty-acid CoA independent diglyceride:

glycerol acyltransferase as defined above, in the manufacture of a foodstuff, for improving, the crystallization properties of the foodstuff.

In another aspect the present invention provides the use of a fatty-acid CoA independent diglyceride:glycerol acyltransferase as defined above , in the manufacture of an edible oil, for reducing and/or removing (preferably selectively reducing and/or removing) diglyceride from said edible oil.

In another aspect the present invention provides the use of a fatty-acid CoA independent diglyceride:glycerol acyltransferase as defined above , in the manufacture of an edible oil, for improving the crystallization properties of the edible oil.

## DETAILED DISCLOSURE OF INVENTION

The terms "fatty-acid CoA independent lipid acyltransferase" and "fatty-acid CoA independent diglyceride: glycerol acyltransferase" as used herein means an enzyme which has acyltransferase activity (generally classified as E.C. 2.3.1.x in accordance with the Enzyme Nomenclature Recommendations (1992) of the Nomenclature Committee of the International Union of Biochemistry and Molecular Biology), whereby the enzyme is capable of transferring an acyl group from a diglyceride to one or more acceptor substrates).

Thus, the "fatty-acid CoA independent lipid acyltransferase" or "fatty-acid CoA independent diglyceride:glycerol acyltransferase" is an enzyme which has acyltransferase activity (classified as E.C. 2.3.1.x), but which is not a diacylglycerol acyltransferase (DGAT) or a phospholipid:diacylglycerol acyltransferase (PDAT). DGATs are classified as E.C. 2.3.1.20. PDATs are classified as E.C. 2.3.1.158. Thus, the fatty-acid CoA independent lipid acyltransferase or fatty-acid CoA independent diglyceride:glycerol acyltransferase is an enzyme which has acyltransferase activity, but which is not any enzyme classified as E.C. 2.3.1.20 or E.C. 2.3.1.158.

The fatty-acid CoA independent lipid acyltransferase enzyme or fatty-acid CoA independent diglyceride:glycerol acyltransferase enzyme according to the present invention is one which is capable in an edible oil of transferring an acyl group from DAG to glycerol. Thus, the reaction catalysed by the enzyme is:

$$\text{Diglyceride (DAG) + glycerol} \rightarrow \text{2 monoglycerides (MAGs)}$$

This contrasts sharply with enzymes known as diacylglycerol acyltransferases (or diacylglycerol O-acyltransferase) (DGATs) (such enzymes are classified as E.C. 2.3.1.20) which catalyse the final step in the Kennedy process, namely:

$$\text{1,2-DAG + acyl CoA} \rightarrow \text{CoA + triacylglycerol (TAG)}$$

For the avoidance of doubt, DGATs are not fatty-acid CoA independent lipid acyltransferases or fatty-acid CoA independent diglyceride:glycerol acyltransferases in accordance with the present invention.

The reaction catalysed by the enzyme according to the present invention also contrasts sharply with that catalysed by enzymes known as phospholipids:diacylglycerol acyltransferase (PDAT), namely:

$$\text{Phospholipids (such as lecithin) + 1,2-DAG} \rightarrow \text{triacylglycerol (TAG) + lysophospholipid}$$

For the avoidance of doubt, PDATs are not fatty-acid CoA independent lipid acyltransferases or fatty-acid CoA independent diglyceride:glycerol acyltransferases in accordance with the present invention.

Preferably, the fatty-acid CoA independent lipid acyltransferase or fatty-acid CoA independent diglyceride: glycerol acyltransferase according to the present invention is an enzyme classified as E.C. 23.1.73.

Suitably, the fatty-acid CoA independent diglyceride:glycerol acyltransferase may be membrane independent, i.e, may be a protein that is not, in its natural environment, associated with a membrane by the presence of a membrane anchor or membrane spanning domain.

For the avoidance of doubt, the fatty-acid CoA independent lipid acyltransferase according to the present invention is not an enzyme taught in any one of WO03/100044, WO2000/36114 US2003/0028923 or US2003/0074695.

As well as having acyltransferase activity the enzyme may have lipase activity, e.g. phospholipase activity.

The fatty-acid CoA independent lipid acyltransferase in accordance with the present invention is a fatty-acid CoA independent diglyceride:glycerol acyltransferase. These terms may be used interchangeably herein.

Preferably the fatty-acid CoA independent diglyceride:glycerol acyltransferase according to the present invention is an acyltransferase which comprises the amino acid sequence motif GDSX, wherein X is one or more of the following amino acid residues L, A, V, I, F, Y, H, Q, T, N, M or S.

The term "diglyceride:glycerol acyltransferase" as used herein is synonymous with the term "fatty-acid CoA independent diglyceride:glycerol acyltransferase".

For the avoidance of doubt, the term "fatty-acid CoA" is the same as the terms "Acyl-CoA", "fatty-acid enzyme. CoA" and "Acyl-Co enzyme A". These terms may be used interchangeably herein.

The edible oil used in a process or use according to the present invention may be in the form of a crude oil or may be a refined oil.

In one embodiment, preferably the amount of diglyceride is reduced rather than completely removed.

The term "reduced" as used herein means that the amount of diglyceride in an edible oil treated with the enzyme in accordance with the present invention is less than the amount of diglyceride in the edible oil before enzyme treatment

Preferably, diglycerides are not completely removed from the edible oil.

In some applications, such as the use of the treated oil in margarines and/or shortening, the amount of diglycerides, particularly 1,2 diglycerides, should be reduced to a point where the crystallisation speed of the fat blend produces small beta-crystals. The amount of 1,2-diglyceride to the amount of total diglyceride in the palm oil depends on the age and storage conditions of the oil. For commercial oils the ratio of 1,3 diglyceride: 1,2 diglyceride is 1.8:3.3. The removal of 1,2 diglycerides will have the most impact on the crystallisation properties.

As will be readily apparent to the skilled person the reduction in the amount of diglyceride can be controlled by the reaction time and temperature of the reaction. In a flow reactor with an immobilised enzyme the reaction may be controlled by the flow rate.

Preferably, the lipid acyltransferase for use in the methods and/or uses of the present invention is capable of transferring an acyl group from a diglyceride to an acyl acceptor, wherein the acyl acceptor is any compound comprising a hydroxy group (-OH).

Suitably, the term "diglyceride" as used herein means one or more of 1,2-diglyceride or 1,3-diglyceride. Preferably, the diglyceride is a 1,2-diglyceride.

The terms "diglyceride" and "diacylglycerol" are used herein interchangeably.

The term "diglyceride" does not encompass digalactosyldiglyceride (DGDG) and/or lecithin, e.g. phosphatidylcholine.

Preferably the acyl acceptor is one which is soluble in an edible oil.

Suitably, the acyl acceptor may be an alcohol such as for example ethanol or polyvalent alcohols, including glycerol and mixtures and derivatives thereof. Suitably, the acyl acceptor may be one or more of a sterol, a stanol, a hydroxy acid, sorbitol, sorbitan or other carbohydrate.

In one preferred embodiment the acyl acceptor is glycerol.

Thus, in one embodiment the present invention provides a method of reducing and/or removing diglyceride from an edible oil, comprising a) admixing an edible oil with both glycerol and a fatty-acid CoA independent diglyceride: glycerol acyltransferase, wherein the fatty-acid CoA independent diglyceride:glycerol acyltransferase is characterized as an enzyme which possesses acyl transferase activity and which comprises the amino acid sequence motif GDSX, wherein X is one or more of the following amino acid residues L, A, V, I, F, Y, H, Q. T, N, M or S.

Preferably, the acyl acceptor according to the present invention is not water.

Preferably the acyl acceptor is not a monoglyceride and/or a diglyceride.

Preferably, the fatty-acid CoA independent lipid acyltransferase according to the present invention is capable of cleaving the acyl bond between a fatty acid residue(s) and a glycerol backbone of a lipid substrate; wherein preferably the lipid substrate is a diglyceride, preferably 1,2-diglyceride.

Preferably, the fatty-acid CoA independent lipid acyltransferase according to the present invention does not act on triglycerides and/or monoglycerides. In other words, preferably the fatty-acid CoA independent lipid acyltransferase is selective for diglycerides, preferably selective for 1,2-diglycerides. This lipid substrate may be referred to herein as the "lipid acyl donor".

Thus in accordance with the present invention, one or more of the following advantageous properties can be achieved: reduction in diglyceride content of an edible oil; a reduction in the diglyceride content of an edible oil without a reduction in the triglyceride content of the edible oil; a reduction in the diglyceride content of the edible oil without an increase in the monoglyceride content; a reduction in the diglyceride content of the edible oil with an increase in the monoglyceride content; a reduction of diglyceride and a reduction in monoglyceride content of an edible oil; a reduction in the diglyceride content of the edible oil without a significant increase in the fatty acid content in the edible oil.

Preferably, the fatty-acid CoA independent lipid acyltransferase in accordance with the present invention performs an alcoholysis reaction (preferably glycerolysis) by transferring a fatty acid acyl group from the diglyceride (preferably the 1,2-DAG) to an alcohol (preferably glycerol) thereby producing two monoglyceride molecules, i.e. one from the diglyceride and one from the glycerol together with the accepted acyl group.

Preferably, X of the GDSX motif is L. Thus, preferably the enzyme according to the present invention comprises the amino acid sequence motif GSDL.

The GDSX motif is comprised of four conserved amino acids. Preferably, the serine within the motif is a catalytic serine of the lipid acyltransferase enzyme. Suitably, the serine of the GDSX motif may be in a position corresponding to Ser-16 in *Aeromonas hydrophila* lipolytic enzyme taught in Brumlik & Buckley (Journal of Bacteriology. Apr. 1996,

Vol. 178, No. 7, p 2060-2064).

To determine if a protein has the GDSX motif according to the present invention, the sequence is preferably compared with the hidden markov model profiles (HMM profiles) of the pfam database.

Pfam is a database of protein domain families. Pfam contains curated multiple sequence alignments for each family as well as profile hidden Markov models (profile HMMs) for identifying these domains in new sequences. An introduction to Pfam can be found in Bateman A et al. (2002) Nucleic Acids Res. 30; 276-280. Hidden Markov models are used in a number of databases that aim at classifying proteins, for review see Bateman A and Haft DH (2002) Brief Bioinform 3; 236-245.

http://www.ncbi.nlm.nih.goy/entrez/query.fcgi?cmd=Retrieve&db=PubMed&list uids =12230032&dopt=Abstract

http:/www.ncbi.nlm.nih.gov/entrez/query.fcgi?cmd=Retrieve&db=PubMed&list uids =11752314&dopt=Abstract

For a detailed explanation of hidden Markov models and how they are applied in the Pfam database see Durbin R, Eddy S, and Krogh A (1998) Biological sequence analysis; probabilistic models of proteins and nucleic acids. Cambridge University Press, ISBN 0-521-62041-4. The Hammer software package can be obtained from Washington University, St Louis, USA.

Alternatively, the GDSX motif can be identified using the Hammer software package, the instructions are provided in Durbin R, Eddy S, and Krogh A (1998) Biological sequence analysis; probabilistic models of proteins and nucleic acids. Cambridge University Press, ISBN 0-521-62041-4 and the references therein, and the HAMMER2 profile provided within this specification.

The PFAM database can be accessed, for example, through several servers which are currently located at the following websites.

http:/www.sanger.ac.uk/Software/Pfam/index.shtml

http://pfam.wustl.edu/

http://pfam.jouy.inra-fr/

http://pfam.cgb.ki.se/

The database offers a search facility where one can enter a protein sequence. Using the default parameters of the database the protein sequence will then be analysed for the presence of Pfam domains. The GDSX domain is an established domain in the database and as such its presence in any query sequence will be recognised . The database will return the alignment of the Pfam00657 consensus sequence to the query sequence.

A multiple alignment, including *Aeromonas salmonicida* or *Aeromonas hydrophila* can be obtained by:

a) manual
obtain an alignment of the protein of interest with the Pfam00657 consensus sequence and obtain an alignment of P10480 with the Pfam00657 consensus sequence following the procedure described above;
or

b) through the database
After identification of the Pfam00657 consensus sequence the database offers the option to show an alignment of the query sequence to the seed alignment of the Pfam00657 consensus sequence. P10480 is part of this seed alignment and is indicated by GCAT_AERHY. Both the query sequence and P10480 will be displayed in the same window.

The *Aeromonas hydrophila* reference sequence:

The residues of *Aeromonas hydrophila* GDSX lipase are numbered in the NCBI file P 10480, the numbers in this text refer to the numbers given in that file which in the present invention is used to determine specific amino acids residues which, in a preferred embodiment are present in the lipid acyltransferase enzymes of the invention.

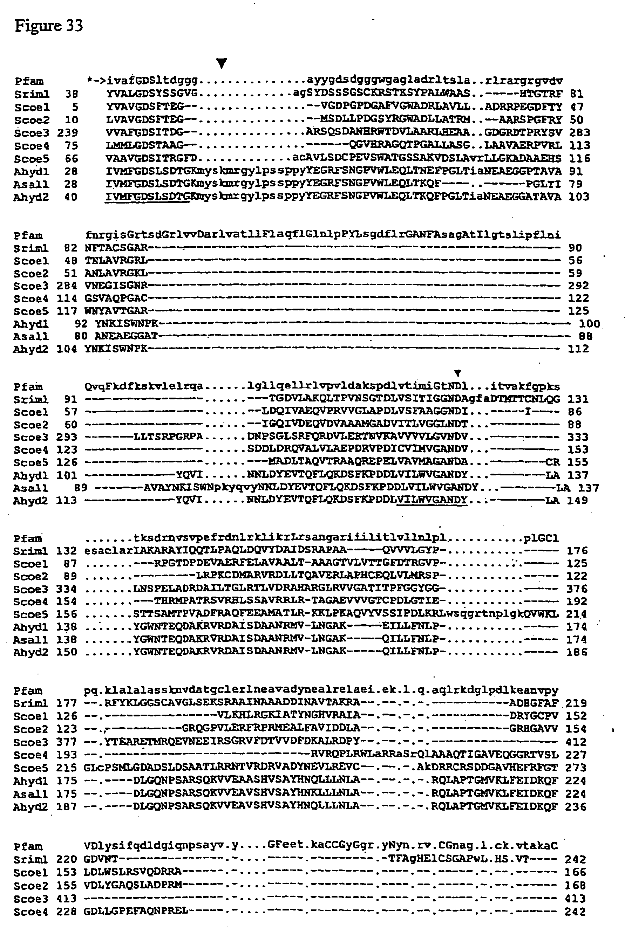

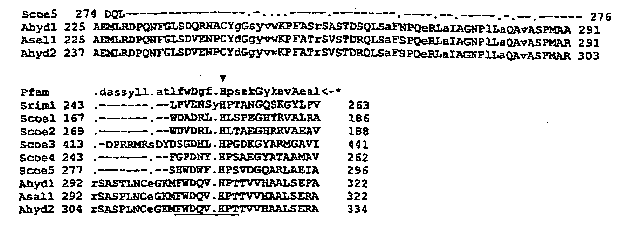

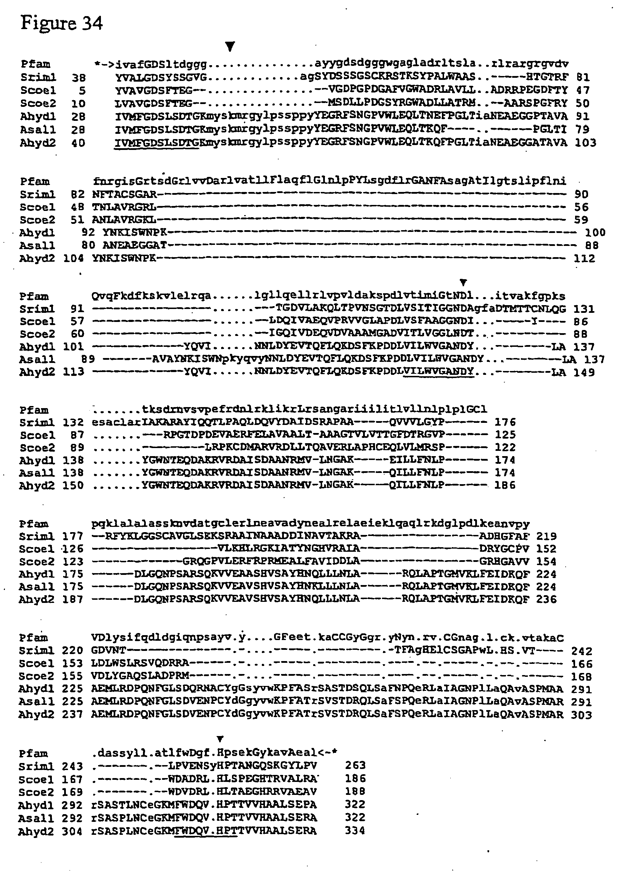

**<u>The Pfam alignment was performed (Figure 33 and 34):</u>**

The following conserved residues can be recognised and in a preferable embodiment may be present in the enzymes for use in the compositions and methods of the invention;

**Block 1 - GDSX block**

| | | | | | | | |
|---|---|---|---|---|---|---|---|
| hid | hid | hid | hid | Gly | Asp | Ser | hid |
| 28 | 29 | 30 | 31 | 32 | 33 | 34 | 35 |

Block 2 - GANDY block

hid Gly hid Asn Asp hid

130 131 132 133 134 135

Block 3 - HPT block

His

309

Where 'hid' means a hydrophobic residue selected from Met, Ile, Leu, Val, Ala, Gly, Cys, His, Lys, Trp, Tyr, Phe.

Preferably the lipid acyltransferase enzyme for use in the compositions/methods of the invention can be aligned using the Pfam00657 consensus sequence.

Preferably, a positive match with the hidden markov model profile (HMM profile) of the pfam00657 domain family indicates the presence of the GDSL or GDSX domain according to the present invention.

Preferably when aligned with the Pfam00657 consensus sequence the lipid acyltransferase for use in the compositions/methods of the invention have at least one, preferably more than one, preferably more than two, of the following, a GDSx block, a GANDY block, a HPT block. Suitably, the lipid acyltransferase may have a GDSx block and a GANDY block. Alternatively, the enzyme may have a GDSx block and a HPT block. Preferably the enzyme comprises at least a GDSx block.

Preferably, when aligned with the Pfam00657 consensus sequence the enzyme for use in the compositions/methods of the invention have at least one, preferably more than one, preferably more than two, preferably more than three, preferably more than four, preferably more than five, preferably more than six, preferably more than seven, preferably more than eight, preferably more than nine, preferably more than ten, preferably more than eleven, preferably more than twelve, preferably more than thirteen, preferably more than fourteen, of the following amino acid residues when compared to the reference *A.hydrophilia* polypeptide sequence, namely SEQ ID No. 32: 28hid, 29hid 30hid, 31hid, 32gly, 33Asp, 34Ser, 35hid, 130hid, 131 Gly, 132Hid, 133Asn, 134Asp, 135hid, 309His

The pfam00657 GDSX domain is a unique identifier which distinguishes proteins possessing this domain from other enzymes.

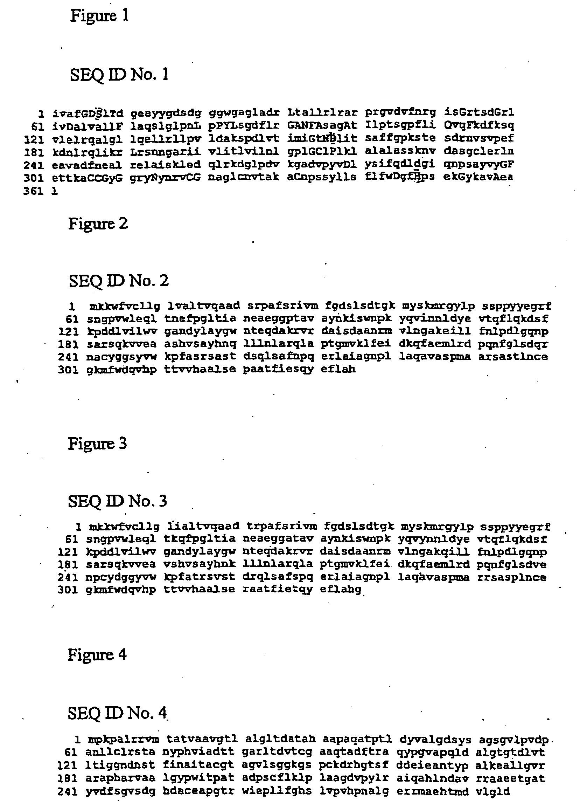

The pfam00657 consensus sequence is presented in Figure 1 as SEQ ID No. 1. This is derived from the identification of the pfam family 00657, database version 6, which may also be referred to as pfam00657.6 herein.

The consensus sequence may be updated by using further releases of the pfam database.

For example, Figures 33 and 34 show the pfam alignment of family 00657, from database version 11, which may also be referred to as pfam00657.11 herein.

The presence of the GDSx, GANDY and HPT blocks are found in the pfam family 00657 from both releases of the database. Future releases of the pfam database can be used to identify the pfam family 00657.

Preferably, the fatty-acid CoA independent lipid acyltransferase enzyme according to the present invention may be characterised using the following criteria:

(i) the enzyme possesses acyl transferase activity which may be defined as ester transfer activity whereby the acyl part of an original ester bond of a lipid acyl donor is transferred to acyl acceptor to form a new ester,

(ii) the enzyme comprises the amino acid sequence motifs GDSX, wherein X is one or more of the following amino acid residues L, A, V, I, F, Y, H, Q, T, N, M or S.;

(iii) the enzyme comprises His-309 or comprises a histidine residue at a position corresponding to His-309 in the *Aeromonas hydrophila* lipolytic enzyme shown in Figure 2 (SEQ ID No. 2 or SEQ ID No. 32).

Preferably, the amino acid residue of the GDSX motif is L.

In SEQ ID No. 2 or SEQ ID No. 32 the first 18 amino acid residues form a signal sequence. His-309 of the full length sequence, that is the protein including the signal sequence, equates to His-291 of the mature part of the protein, i.e. the sequence without the signal sequence.

Preferably, the fatty-acid CoA independent lipid acyltransferase enzyme according to the present invention comprises the following catalytic triad: Ser-34, Asp-134 and His-309 or comprises a serine residue, an aspartic acid residue and a histidine residue, respectively:, at positions corresponding to Ser-34, Asp-134 and His-309 in the *Aerom-*

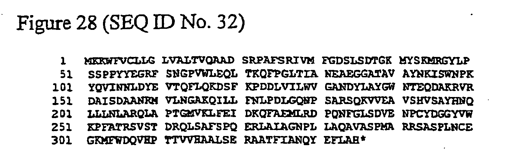

*onas hydrophila* lipolytic enzyme shown in Figure 2 (SEQ ID No. 2) or Figure 28 (SEQ ID No. 32). As stated above, in the sequence shown in SEQ ID No. 2 or SEQ ID No. 32 the first 18 amino acid residues form a signal sequence. Ser-34, Asp-134 and His-309 of the full length sequence, that is the protein including the signal sequence, equate to Ser-16, Asp-116 and His-291 of the mature part of the protein, i.e. the sequence without the signal sequence. In the pfam00657 consensus sequence, as given in Figure 1 (SEQ ID No. 1) the active site residues correspond to Ser-7, Asp-157 and His-348.

Preferably, the fatty-acid CoA independent lipid acyltransferase enzyme according to the present invention may be characterised using the following criteria:

(i) the enzyme possesses acyl transferase activity which may be defined as ester transfer activity whereby the acyl part of an original ester bond of a first lipid acyl donor is transferred to an acyl acceptor to form a new ester, and
(ii) the enzyme comprises at least Gly-32, Asp-33, Ser-34, Asp-134 and His-309 or comprises glycine, aspartic acid, serine; aspartic acid and histidine residues at positions corresponding to Gly-32, Asp-33, Ser-34, Asp-134 and His-309, respectively, in the *Aeromonas hydrophila* lipolytic enzyme shown in Figure 2 (SEQ ID No. 2) or Figure 28 (SEQ ID No. 32).

Suitably, the fatty-acid CoA independent lipid acyltransferase enzyme may be obtainable, preferably obtained, from organisms from one or more of the following genera: *Aeromonas, Streptomyces, Saccharomyces, Lactococcus, Mycobacterium, Streptococcus, Lactobacillus, Desulfitobacterium, Bacillus, Campylobacter, Vibrionaceae, Xylella, Sulfolobus, Aspergillus, Schizosaccharomyces, Listeria, Neisseria, Mesorhizobium, Ralstonia, Xanthomonas* and *Candida.*

Suitably, the fatty-acid CoA independent lipid acyltransferase enzyme according to the present invention may be obtainable, preferably obtained, from one or more of the following organisms: *Aeromonas hydrophila, Aeromonas salmonicida, Streptomyces coelicolor, Streptomyces rimosus, Mycobacterium. Streptococcus pyogenes, Lactococcus lactis, Streptococcus pyogenes, Streptococcus thermophilus, Lactobacillus helveticus, Desulfitobacterium dehalogenans, Bacillus sp, Campylobacter jejuni, Vibrionaceae, Xylella fastidiosa, Sulfolobus solfataricus, Saccharomyces cerevisiae, Aspergillus terreus, Schizosaccharomyces pombe, Listeria innocua, Listeria monocytogenes, Neisseria meningitidis, Mesorhizobium loti, Ralstonia solanacearum, Xanthomonas campestris, Xanthomonas axonopodis* and *Candida parapsilosis.*

In one aspect, preferably the fatty-acid CoA independent lipid acyltransferase enzyme is obtainable, preferably obtained, from one or more of *Aeromonas hydrophila* or *Aeromonas salmonicida.*

In one aspect, the fatty-acid CoA independent lipid acyltransferase may be a lecithin:cholesterol acyltransferases (LCAT) or variant thereof (for example a variant made by molecular evolution)

Suitable LCATs are known in the art and may be obtainable from one or more of the following organisms for example: mammals, rat, mice, chickens, *Drosophilia melanogaster,* plants, including *Arabidopsis* and *Oryza sativa,* nematodes, fungi and yeast.

In one embodiment the fatty-acid CoA independent lipid acyltransferase enzyme according to the present invention may be the lipid acyltransferase obtainable, preferably obtained, from the *E. coli* strains TOP 10 harbouring pPet12aAhydro and pPet12aASalmo deposited by Danisco A/S of Langebrogade 1, DK-1001 Copenhagen K, Denmark under the Budapest Treaty on the International Recognition of the Deposit of Microorganisms for the purposes of Patent Procedure at the National Collection of Industrial, Marine and Food Bacteria (NCIMB) 23 St Machar Street. Aberdeen Scotland, GB on 22 December 2003 under accession numbers NICMB 41204 and NCIMB 41205, respectively.

Preferably, when carrying out a method according to the present invention the product is produced without increasing or substantially increasing the free fatty acids in the foodstuff.

The term "transferase" as used herein is interchangeable with the term "lipid acyltransferase".

Suitably, the fatty-acid CoA independent lipid acyltransferase as defined herein catalyses one or more of the following reactions: interesterification, transesterification, alcoholysis, hydrolysis.

The term "interesterification" refers to the enzymatic catalysed transfer of acyl groups between a lipid donor and lipid acceptor, wherein the lipid donor is not a free acyl group.

The term "transesterification" as used herein means the enzymatic catalysed transfer of an acyl group from a lipid donor (other than a free fatty acid) to an acyl acceptor (other than water).

As used herein, the term "alcoholysis" refers to the enzymatic cleavage of a covalent bond of an acid derivative by reaction with an alcohol ROH so that one of the products combines with the H of the alcohol and the other product combines with the OR group of the alcohol.

As used herein, the term "alcohol" refers to an alkyl compound containing a hydroxyl group.

As used herein, the term "hydrolysis" refers to the enzymatic catalysed transfer of an acyl group from a lipid to the OH group of a water molecule. Acyl transfer which results from hydrolysis requires the separation of the water molecule.

The term "without increasing or without substantially increasing the fee fatty acids" as used herein means that

preferably the lipid acyl transferase according to the present invention has 100% transferase activity (i.e. transfers 100% of the acyl groups from an acyl donor onto the acyl acceptor, with no hydrolytic activity) in an edible oil environment; however, the enzyme may transfer less than 100% of the acyl groups present in the lipid acyl donor to the acyl acceptor. In which case, preferably the acyltransferase activity accounts for at least 30%, more preferably at least 40%, more preferably at least 50%, more preferably at least 70%, more preferably at least 80%, more preferably at least 90% and more preferably at least 98% of the total enzyme activity. The % transferase activity (i.e. the transferase activity as a percentage of the total enzymatic activity) may be determined by the following protocol:

*Protocol for the determination of % acyltransferase activity:*

An edible oil to which a fatty-acid CoA independent lipid acyltransferase according to the present method has been added may be extracted following the enzymatic reaction with $CHCl_3$:$CH_3OH$ 2:1 and the organic phase containing the lipid material is isolated and analysed by GLC according to the procedure detailed herein below. From the GLC analyses the amount of free fatty acids and diglycerides are determined. A control edible oil to which no enzyme according to the present method has been added, is analysed in the same way.

*Calculation:*

From the results of the GLC analyses the increase in free fatty acids and the decrease in diglycerides can be calculated:

Δ % fatty acid = % Fatty acid(enzyme) - % fatty acid(control);
Mv Fa = average molecular weight of the fatty acids;
Δ % diglyceride = % Diglyceride(control) - % Diglyceride(enzyme);
Mv Di= average molecular weight of diglyceride;

The transferase activity is calculated as a percentage of the total enzymatic activity:

$$\% \text{ transferase activity} = \frac{((\Delta \% \text{ Di}/(\text{Mv Di}) - \Delta \% \text{ Fa}/(\text{Mv FA})) \times 100}{\Delta \% \text{ Di}/(\text{Mv Di}) - \Delta \% \text{ Fa}/(\text{Mv Fa}) + \Delta \% \text{ Fa}/(\text{Mv Fa})}$$

$$\% \text{ transferase activity} = \; = \frac{((\Delta \% \text{ Di}/(\text{Mv Di}) - \Delta \% \text{ Fa}/(\text{Mv FA})) \times 100}{\Delta \% \text{ Di}/(\text{Mv Di})}$$

If the free fatty acids are increased in the edible oil they are preferably not increased substantially, i.e. to a significant degree. By this we mean, that the increase in free fatty acid does not adversely affect the quality of the edible oil.
In some aspects of the present invention, the term "without substantially increasing free fatty acids" as used herein means that the amount of free fatty acid in an edible oil treated with an lipid acyltransferase is less than the amount of free fatty acid produced in an edible oil or composition when an enzyme other than a lipid acyltransferase according to the present method had been used, such as for example as compared with the amount of free fatty acid produced when a conventional lipase e.g. *Pseudomonas cepacia* lipase (Lipase PS, Amano Japan) or *Rhizopus oryzae* lipase (LipaseF, Amano Japan).
Suitably, any glycerol remaining in the edible oil after the reaction has taken place may be removed, e.g. by centrifugation or vacuum distillation.
Optionally, the enzyme may be removed from the edible oil after the enzymatic reaction has taken place. Alternatively, the enzyme may simply be deactivated and left in the edible oil. Suitably, the enzyme may be deactivated by heating for example.
In one embodiment the fatty-acid CoA independent lipid acyltransferase for use in the methods of the present invention may be immobilised. When it is the case that the enzyme is immobilised an admixture comprising an acyl acceptor and the edible oil may be passed through a column for example comprising the immobilised enzyme. By immobilising the enzyme it is possible to easily reuse it.
Suitably the immobilised enzyme may be used in a flow reactor or in a batch reactor containing a reaction mixture which comprises an acyl acceptor and an edible oil as a two-phase system. The reaction mixture may be optionally stirred or sonicated Once the reaction has reached equilibrium for example, the reaction mixture and the

immobilised enzyme may be separated. Suitably, excess acyl acceptor (such as excess glycerol) may be removed after the reaction, e.g. by centrifugation or vacuum distillation.

Immobilized lipid acyltransferase can be prepared using immobilisation techniques known in the art. There are numerous methods of preparing immobilised enzymes, which will be apparent to a person skilled in the art (for example the techniques referred to in EP 0 746 608; or Balcao V.M., Paiva A.L., Malcata F.X., Enzyme Microb Technol. 1996 May 1;18(6):392-416; or Retz M.T., Jaeger K.E. Chem Phys Lipids. 1998 Jun;93(1-2):3-14; Bornscheuer U.T., Bessler C, Srinivas R, Krishna S.H. Trends BiotechnoL 2002 Oct; 20(10):433-7; Plou et al, J. Biotechnology 92 (2002) 55-66; Warmuth et al., 1992. Bio Forum 9, 282-283; Ferrer et al., 2000. J. Chem. Technol. Biotechnol. 75, 1-8; or Christensen et al., 1998. Nachwachsende Rohstoff 10, 98-105; Petersen and Christenen, 2000, Applied Biocatalysis. Harwood Academic Publishers, Amsterdam. . Techniques which may be used herein include covalent coupling to Eupergit C, adsorption on polypropylene and silica-granulation for example.

Preferably, the edible oil is any edible oil containing a diglyceride, preferably a significant amount of diglyceride.

Preferably, the edible oil is one or more of the following oils: oils extracted from or derived from palm oil, palm olein, palm stearin, palm mid fraction or any palm oil fraction or olive oil.

More preferably, the edible oil is palm oil and/or palm olein and/or palm stearin.

With regard to the admixing of the edible oil, the acyl acceptor substrate and the lipid acyltransferase, as a person skilled in the art would readily appreciate this can be done in any combination and/or order. By way of example only, the acyl acceptor substrate may be admixed with the edible oil followed by addition of the lipid acyltransferase. Alternatively, the edible oil may be admixed with the lipid acyltransferase followed by addition of the acyl acceptor substrate. Alternatively, the lipid acyltransferase and acyl acceptor substrate may be admixed followed by admixing of the enzyme/substrate mixture with the edible oil. Alternatively, of course, all three substances (namely the edible oil, the acyl acceptor substrate and the lipid acyltransferase) may be admixed simultaneously.

Preferably the method is carried out at a temperature above the melting point of the edible oil.

Suitably the method may be carried out at a temperature of between 30-50°C, preferably between 35-45°C, preferably between 40-45°C.

Preferably, the enzyme is added to crude or refined edible oil. Preferably the present invention does not encompass the enzyme treatment of an edible oil when admixed with water containing constituents. Thus, the present invention envisages the treatment of an edible oil *per se,* i.e. in a low water environment.

Although water may be added to the oil prior or during the method of the present invention, in a most preferable embodiment no water is added. If water is present in the edible oil, preferably there is less than 10% water present, more preferably less than 7.5%, less than 5%, less than 1%, less than 0.5%, less than 0.4%, less than 0.3%, less than 0.2% or less than 0.1 % water present Suitably, between 0.1. and 1% water may be present in the edible oil.

In one embodiment the edible oil used in a process according to the present invention may be supplemented with one or more acyl donors and/or may be supplemented with one or more acyl acceptors and/or may be supplemented with both one or more acyl acceptors and one or more acyl donors. By "supplemented with" means that either the acyl donor and/or the acyl acceptor is not naturally present in the edible oil and is added at the same time, immediately after and/or immediately before bringing the edible into contact with the lipid acyltransferase in accordance with the present method.

Suitably, the supplementary acyl donor may be other than a DAG. Suitably the supplemental acyl donor may be for example a phospholipid (e.g. lecithin).

Suitably, the supplementary acyl acceptor may be glycerol. Suitably, however it may be an acyl acceptor other than glycerol, for example a plant sterol and/or plant stanol for instance. Suitably, the supplementary acyl acceptor may be a combination of glycerol and one or more further acyl acceptors.

The use of an oil supplemented with a phospholipid allows for an additional emulsifier (lyso-phospholipid) to be produced in the oil. The use of an oil supplemented with a plant sterol and/or plant stanol allows for a plant sterol ester and/or a plant stanol ester to be produced in the edible both. Both plant sterol esters and plant stanol esters have been reported to have blood serum cholesterol reducing effects when incorporated into the diet.

Enzymatic interesterification using immobilised lipases, such as Lipozyrne® TL IM, a 1,3 specific lipase (Novozymes, Denmark), is used to reduce the amount of trans fatty acids in oils for dietary use and/or for modifying the melting characteristics of edible oils and fats.

During interesterification, one or two of the polyunsaturated fatty acids in the triglycerides of the food oil can be replaced with a fatty acid from another oil low in trans fatty acids, such as palm oil. This transfer of fatty acids from palm oil allows the modification of the melting point of the food oil without introduction of the trans fatty acids. The immobilisation of lipases is described in US5776741, US4798793 and US5156963. US6284501 describes the interesterification of phospholipids.

An immobilised transferase can be produced using the same technology as used for lipases.

In one embodiment, the fatty-acid CoA independent lipid acyl transferase as described herein can be used in combination with a interesterification lipase. The lipase interesterification and the acyl transferase steps are preferably

carried out separately.

In a further aspect, the fatty-acid CoA independent lipid acyl transferase as described herein can be used in combination with conventional crystallisation inhibitors.

In a further aspect, the present invention provides a method of improving the crystallization properties in a foodstuff comprising an edible oil, comprising admixing an edible oil with an acyl acceptor substrate and a fatty-acid CoA independent diglyceride:glycerol acyltransferase as described herein and optionally a further crystallization inhibitor.

In one embodiment the present invention provides a fatty-acid CoA independent diglyceride:glycerol acyltransferase in the preparation of a foodstuff comprising an edible oil for improving the crystallization properties of the foodstuff.

Suitably one or more further crystallisation inhibitors may optionally be added.

The present invention yet further provides the use of a fatty-acid CoA independent diglyceride:glycerol acyltransferase as described herein in the manufacture of a foodstuff comprising an edible oil for improving the crystallization properties of said foodstuff.

In another aspect, the present invention provides the use of a fatty-acid CoA independent diglyceride:glycerol acyltransferase as described herein in combination with a further crystallization inhibitor in the manufacture of a foodstuff comprising an edible oil for improving the crystallization properties of said foodstuff

The further crystallization inhibitor may be any conventional crystallisation inhibitor such as one or more of sorbitan tristearates, lecithins, PGE or polysorbates for example.

ADVANTAGES

In the present invention by use of the method taught herein, and in particular by selection of the enzymes taught herein for use in the claimed method, for removing or reducing diglyceride from or in an edible oil, a selective reduction in diglyceride over mono- and di-glyceride can be effected, without a decrease in triglycerides and/or a significant increase in free fatty acids (FFAs).

The fact that the present inventive method can be carried out without substantially increasing free fatty acid content of the edible oil overcomes the problem of loss of product.

If a crude palm oil is treated with a conventional lipase it is necessary to remove the free fatty acid during the oil refining, but if a refined palm oil is treated with the lipid acyltransferase according to the present invention (thus negating the need to treat the crude palm oil with a conventional lipase) it is not necessary to remove the fatty acids because the content of free fatty acid does not increase substantially.

In addition or alternatively, the present invention results in the removal and/or reduction of diglycerides from or in an edible oil, with no significant decrease in monoglyceride levels.

In addition or alternatively, the present invention results in the removal and/or reduction of diglycerides from or in an edible oil, whilst increasing monoglyceride levels in the edible oil. This contrasts prior art enzymes which utilise monoglyceride as a substrate and therefore reduce the amount of monoglyceride in the edible oil.

The present invention is advantageous as it does not require the addition of fatty acid CoA. This contrasts sharply with the acyl CoA dependent DGATs. Rather the enzyme according to the present invention relies on the presence (and addition if necessary) of glycerol - which is cheap commodity.

COMMERCIAL RELEVANCE OF REMOVING/REDUCING DIGLYCERIDE IN PALM OIL

Diglycerides retard the crystallisation in palm based margarines and shortenings

For many years, we have seen a steady increase in the growth of palm oil consumption, partially because of localised availability, partially because of economics, and primarily because of performance. Presence of Palm oil in Margarine / shortening type products is found to enhance the β' quality of an oil blend. However, the use of palm oil was previously restricted to the use of palm stearine, and palm olein, with some use of palm kernels and its fractions. Today, this is more diverse, because of confectionery and food processors wanting very specific melting profiles.

With customers trying to increase the use of palm oil in formulations, the industry is seeing more crystallization issues than ever before. Where it is necessary to add "pro-crystal" promoters to initiate crystallization in low trans or no-trans formulations thereby preventing or delaying post crystal formation before point of use. Also increased interest in anti-crystallisers, such as Sorbitan Tristearates, lecithins, PGE, Polysorbates, which will slow, delay post-crystal growth has been seen. This is a symptom of high diglyceride presence.

The commercial benefits of the present invention may be one or more of the following.

a) reduces the need to add additional monoglyceride to the edible oil during processing and/or later during use. Monoglyceride is a widely used emulsifier in food systems.

b) decreases in diglyceride from about 6 - 8% to about 4-5%, and even more.

c) increases the flexibility on plant (factory) capacity - hence reduction in production overheads.

d) allows for major decreases in post-crystallisation issues.

e) in some formulations, it would be possible to remove some fully saturated oil triglycerides, because we reduce the need for crystal promotion. In this regard manufacturers conventionally add fully saturated triglycerides to formulations in order to "promote" crystal formation and overcome undesirable delayed crystal formation in the product post-sale. Such crystal formation is a symptom of having a significant amount of palm oil or its components in a formulation, which would contain concentrations of diglycerides sufficient to delay crystal growth to the desired crystal type (i.e. preferably beta-prime in the case of margarines and shortenings) during manufacture. However, by use of palm oil treated in accordance with the present invention, the diglyceride content is sufficiently reduced within a given blend or formulation so that the need for additional fully saturated triglyceride addition to aid crystal development becomes less important.

f) allows greater diversification towards palm based blends, with increased liquid oil costs, and without major process changes.

g) allows for replacement of anti-crystallisers and/or partial replacement of anti-crystallisers.

B Trans fatty acid

Today, we see legislation and public opinion against the use of trans acids in food products (Fødevareministeriet, Denmark 2003). So, this has led to performance problems. Manufacturers where possible may wish to switch to palm based solutions because of the high levels ofC:16:0 and C:18:0 isomers found within this oil type. The result is that we are beginning to see increased consumption of palm in economies that previously did not use palm.

C Crystallisation inhibition of diglycerides in confectionery products

Cocoa Butter Equivalents (CBEs) are formulated from speciality fats such as fractionated palm oil, in particular palm mid fractions (PMF), fractionated shea fat as well as many other exotic fats. For cost reasons it is favourable to include as much as possible PMF in the CBE. In fact many CBEs may only contain palm fractions. The most delicate aspect of chocolate manufacture is the crystallisation of the fat. Diglycerides will have a negative impact on the crystallisation, for example demoulding can be a serious problem (Siew, 2001).

D Crystal quality

Triglyceride quality greatly affects performance, and this is evident, from examples of a typical trans acid containing oil formula for standard 82% fat table margarine, against trans free version containing a typical interesterified palm stearine fraction and palm kernel as hard stock, both having similar SFC profile. Reduced speed and quality of crystallization can result in a symptom known as "oiling out." In turn this calls for the need of crystal promoters, such as hard MAGs. Further, delay in crystal stabilized crystal formation leads to symptom known as "sandiness" whereby transformation of desired crystal type eventually reverts to the more stable Beta form, and so gives sandy texture. This symptom is particular problem for Industrial type products.

SOME USES

**Fat Modification**

During the chemical production of fats for use in high fat solid food products such as margarines, spreads, confectionery/chocolates, trans fatty acids are introduced into the food oil during a process of hydrogenation (hardening). This allows for the modification of the melting temperature of the food oil to ensure a suitable consistency of the final food product, e.g. a table margarine, which is solid but spreadable at room temperature, or a chocolate which is solid during storage, but melts in the mouth.

However, chemical production uses large quantities of solvents such as hexane which are considered hazardous to the environment and health and requires complete removal of the solvent prior to use of the modified oil/fat as a food ingredient .

In many countries the use of partial hydrogenation for food-production is being limited, by legislation and regulatory controls, and many major food producer are now switching to alternative low-trans alternatives.

The oil prepared by the process of the invention can be used as an ingredient for margarine and/or spreads.

**Cocoa Butter Replacements/Equivalents**

Cocoa butter contains a unique composition which provides a solid confectionery which melts in the mouth. In order to substitute cocoa butter using cheaper alternatives, plant oils have been hydrogenated to produce trans fatty acids, thereby raising the melting point of the food oil to provide a similar solid confectionery which melts at body temperature. Such modified plant oils are known as cocoa butter equivalents (CBEs) or, in the case where the modified fats enhance the characteristics of the chocolate product, cocoa butter replacements (CBRs). One major problem with CBEs and CBRs is that the hydrogenation of plant oils produces trans fats which are considered to be detrimental to health and temperature. This has lead to the use of low trans plant oils, or fractions thereof, which have a higher melting temperature. Palm oil and palm oil fractions are considered advantageous in this respect as a major component low in trans CBRs/CBEs.

Cocoa butter replacement (CBR) fats are used to provide preferable characteristics to chocolate products such as non-tempering, reduced post hardening, stability, bloom resistant. It is particularly desirable to use non-lauric/non-trans fats such as palm oil. The crystallisation properties of the fats used in CBRs play a key role in ensuring a suitable balance between the product melting in the mouth whilst retaining the above preferable characteristics. The use of low-trans fats, such as palm oil, in CBR blends is particularly desirable for health reasons. The use of palm oil in CBRs is described in EP0293194.

The oil prepared in accordance with a process of the present invention can be used as an ingredient for chocolate, for example :within a fat blend as a cocoa butter replacement and/or equivalent

SEQUENCES OF SOME DIGLYCERIDE:GLYCEROL ACYLTRANSFERASE ENZYMES FOR USE IN ACCORDANCE WITH THE PRESENT INVENTION

Suitable diglyceride:glycerol acyltransferase enzymes for use in accordance with the present invention and/or in the methods of the present invention may comprise any one of the following amino acid sequences and/or be encoded by the following nucleotide sequences.

Termobifida\fusca GDSx 548 aa

SEQ ID No. 58

ZP_00058717

```
  1 mlphpagerg evgaffallv gtpqdrrlrl echetrplrg rcgcgerrvp pltlpgdgvl
 61 cttsstrdae tvwrkhlqpr pdggfrphlg vgcllagqgs pgvlwcgreg crfevcrrdt
121 pglsrtrngd ssppfragws lppkcgeisq sarktpavpr ysllrtdrpd gprgrfvgsg
181 praatrrrlf lgipalvlvt altlvlavpt gretlwrmwc eatqdwclgv pvdsrgqpae
241 dgefllispv qaatwgnyya lgdsyssgdg ardyypgtav kggcwrsana ypelvaeayd
301 faghlsflac sgqrgyamld aidevgsqld wnsphtslvt igiggndlgf stvlktcmvr
361 vplldskact dqedairkrm akfettfeel isevrtrapd arilvvgypr ifpeeptgay
421 ytltasnqrw lnetiqefnq qlaeavavhd eeiaasggvg svefvdvyha ldgheigsde
481 pwvngvqlrd latgvtvdrs tfhpnaaghr avgervieqi etgpgrplya tfavvagatv
541 dtlagevg
```

SEQ ID No. 59
nt

1 ggtggtgaac cagaacaccc ggtcgtcggc gtgggcgtcc aggtgcaggt gcaggttctt
61 caactgctcc agcaggatgc cgccgtggcc gtgcacgatg gccttgggca ggcctgtggt
121 ccccgacgag tacagcaccc atagcggatg gtcgaacggc agcggggtga actccagttc
181 cgcgccttcg cccgcggctt cgaactccgc ccaggacagg gtgtcggcga cagggccgca
241 gcccaggtac ggcaggacga cggtgtgctg caggctgggc atgccgtcgc gcagggcttt
301 gagcacgtca cggcggtcga agtccttacc gccgtagcgg tagccgtcca cggccagcag
361 cactttcggt tcgatctgcg cgaaccggtc gaggacgctg cgcacccccga agtcggggga
421 acaggacgac caggtcgcac cgatcgcggc gcaggcgagg aatgcggccg tcgcctcggc
481 gatgttcggc aggtaggcca cgacccggtc gccggggccc accccgaggc tgcggagggc
541 cgcagcgatc gcggcggtgc gggtccgcag ttctccccag gtccactcgg tcaacggccg
601 gagttcggac gcgtgccgga tcgccacggc tgatgggtca cggtcgcgga agatgtgctc
661 ggcgtagttg agggtggcgc cggggaacca gacggcgccg ggcatggcgt cggaggcgag
721 cactgtggtg tacggggtgg cggcgcgcac ccggtagtac tcccagatcg cggaccagaa
781 tccttcgagg tcggttaccg accagcgcca cagtgcctcg tagtccggtg cgtccacacc
841 gcggtgctcc cgcacccagc gggtgaacgc ggtgaggttg gcgcgttctt tgcgctcctc
901 gtcgggactc cacaggatcg gcggctgcgg cttgagtgtc atgaaacgcg acccccttcgt
961 ggacggtgcg gatgcggtga gcgtcgggtg cctcccctaa cgctccccgg tgacggagtg
1021 ttgtgcacca catctagcac gcgggacgcg gaaaccgtat ggagaaaaca cctacaaccc
1081 cggccggacg gtgggtttcg gccacactta ggggtcgggt gcctgcttgc cgggcagggc
1141 agtcccgggg tgctgtggtg cgggcgggag ggctgtcgct tcgaggtgtg ccggcgggac
1201 actccgggcc tcagccgtac ccgcaacggg gacagttctc ctcccttccg ggctggatgg
1261 tcccttcccc cgaaatgcgg cgagatctcc cagtcagccc ggaaaacacc cgctgtgccc
1321 aggtactctt tgcttcgaac agacaggccg gacggtccac gggggaggtt tgtgggcagc
1381 ggaccacgtg cggcgaccag acgacggttg ttcctcggta tccccgctct tgtacttgtg
1441 acagcgctca cgctggtctt ggctgtcccg acggggcgcg agacgctgtg gcgcatgtgg
1501 tgtgaggcca cccaggactg gtgcctgggg gtgccggtcg actcccgcgg acagcctgcg
1561 gaggacggcg agtttctgct gctttctccg gtccaggcag cgacctgggg gaactattac
1621 gcgctcgggg attcgtactc ttcgggggac ggggcccgcg actactatcc cggcaccgcg
1681 gtgaagggcg gttgctggcg gtccgctaac gcctatccgg agctggtcgc cgaagcctac
1741 gacttcgccg gacacttgtc gttcctggcc tgcagcggcc agcgcggcta cgccatgctt
1801 gacgctatcg acgaggtcgg ctcgcagctg gactggaact cccctcacac gtcgctggtg
1861 acgatcggga tcggcggcaa cgatctgggg ttctccacgg ttttgaagac ctgcatggtg
1921 cgggtgccgc tgctggacag caaggcgtgc acggaccagg aggacgctat ccgcaagcgg
1981 atggcgaaat tcgagacgac gtttgaagag ctcatcagcg aagtgcgcac ccgcgcgccg
2041 gacgcccgga tccttgtcgt gggctacccc cggatttttc cggaggaacc gaccggcgcc
2101 tactacacgc tgaccgcgag caaccagcgg tggctcaacg aaaccattca ggagttcaac
2161 cagcagctcg ccgaggctgt cgcggtccac gacgaggaga ttgccgcgtc gggcggggtg
2221 ggcagcgtgg agttcgtgga cgtctaccac gcgttggacg gccacgagat cggctcggac

2281 gagccgtggg tgaacggggt gcagttgcgg gacctcgcca ccggggtgac tgtggaccgc
2341 agtaccttcc accccaacgc cgctgggcac cgggcggtcg gtgagcgggt catcgagcag
2401 atcgaaaccg gcccgggccg tccgctctat gccactttcg cggtggtggc gggggcgacc
2461 gtggacactc tcgcgggcga ggtggggtga cccggcttac cgtccggccc gcaggtctgc
2521 gagcactgcg gcgatctggt ccactgccca gtgcagttcg tcttcggtga tgaccagcgg
2581 cggggagagc cggatcgttg agccgtgcgt gtctttgacg agcacacccc gctgcaggag
2641 ccgttcgcac agttctcttc cggtggccag agtcgggtcg acgtcgatcc cagcccacag
2701 gccgatgctg cgggccgcga ccacgccgtt gccgaccagt tggtcgaggc gggcgcgcag
2761 cacgggggcg agggcgcgga catggtccag gtaagggccg tcgcggacga ggctcaccac
2821 ggcagtgccg accgcgcagg cgagggcgtt gccgccgaag gtgctgccgt gctggccggg
2881 gcggatcacg tcgaagactt ccgcgtcgcc taccgccgcc gccacgggca ggatgccgcc
2941 gcccagcgct ttgccgaaca ggtagatatc ggcgtcgact ccgctgtggt cgcaggcccg
//

Termobifida\fusca\ - GDSx

SEQ ID No. 60

1 vgsgpraatr rrlflgipal vlvtaltlvl avptgretlw rmwceatqdw clgvpvdsrg
61 qpaedgefll lspvqaatwg nyyalgdsys sgdgardyyp gtavkggcwr sanaypelva
121 eaydfaghls flacsgqrgy amldaidevg sqldwnspht slvtigiggn dlgfstvlkt
181 crnvrvplldsf kactdqedai rkrmakfett feelisevrt rapdarilvv gyprifpeep
241 tgayytltas nqrwlnetiq efnqqlaeav avhdeeiaas ggvgsvefvd vyhaldghei
301 gsdepwvngv qlrdlatgvt vdrstfhpna aghravgerv ieqietgpgr plyatfavva
361 gatvdtlage vg

Corynebacterium\effciens\ GDSx 300 aa

SEQ ID No. 61

1 mrttviaasa llllagcadg areetagapp gessggiree gaeastsitd vyialgdsya
61 amggrdqplr gepfclrssg nypellhaev tdltcqgavt gdlleprtlg ertlpaqvda
121 ltedttlvtl siggndlgfg evagcireri agenaddcvd llgetigeql dqlppqldrv
181 heairdragd aqvvvtgylp lvsagdcpel gdvseadrrw aveltgqine tvreaaerhd
241 alfvlpddad ehtscappqq rwadiqgqqt dayplhptsa gheamaaavr dalglepvqp
//

SEQ ID No. 62
nt

1 ttctggggtg ttatggggtt gttatcggct cgtcctgggt ggatcccgcc aggtggggta
61 ttcacggggg acttttgtgt ccaacagccg agaatgagtg ccctgagcgg tgggaatgag
121 gtgggcgggg ctgtgtcgcc atgagggggc ggcgggctct gtggtgcccc gcgacccccg
181 gccccggtga gcggtgaatg aaatccggct gtaatcagca tcccgtgccc accccgtcgg
241 ggaggtcagc gcccggagtg tctacgcagt cggatcctct cggactcggc catgctgtcg
301 gcagcatcgc gctcccgggt cttggcgtcc ctcggctgtt ctgcctgctg tccctggaag
361 gcgaaatgat caccggggag tgatacaccg gtggtctcat cccggatgcc cacttcggcg
421 ccatccggca attcgggcag ctccgggtgg aagtaggtgg catccgatgc gtcggtgacg
481 ccatagtggg cgaagatctc atcctgctcg agggtgctca ggccactctc cggatcgata
541 tcgggggcgt ccttgatggc gtccttgctg aaaccgaggt gcagcttgtg ggcttccaat
601 ttcgcaccac ggagcgggac gaggctggaa tgacggccga agagcccgtg gtggacctca

661 acgaaggtgg gtagtcccgt gtcatcattg aggaacacgc cctccaccgc acccagcttg
721 tggccggagt tgtcgtaggc gctggcatcc agaagggaaa cgatctcata tttgtcggtg
781 tgctcagaca tgatcttcct ttgctgtcgg tgtctggtac taccacggta gggctgaatg
841 caactgttat ttttctgtta ttttaggaat tggtccatat cccacaggct ggctgtggtc
901 aaatcgtcat caagtaatcc ctgtcacaca aaatgggtgg tgggagccct ggtcgcggtt
961 ccgtgggagg cgccgtgccc cgcaggatcg tcggcatcgg cggatctggc cggtaccccg
1021 cggtgaataa aatcattctg taaccttcat cacggttggt tttaggtatc cgcccctttc
1081 gtcctgaccc cgtccccggc gcgcgggagc ccgcgggttg cggtagacag ggagacgtg
1141 gacaccatga ggacaacggt catcgcagca agcgcattac tccttctcgc cggatgcgcg
1201 gatggggccc gggaggagac cgccggtgca ccgccgggtg agtcctccgg gggcatccgg
1261 gaggaggggg cggaggcgtc gacaagcatc accgacgtct acatcgccct cggggattcc
1321 tatgcggcga tgggcgggcg ggatcagccg ttacggggtg agccgttctg cctgcgctcg
1381 tccggtaatt acccggaact cctccacgca gaggtcaccg atctcacctg ccaggggggcg
1441 gtgaccgggg atctgctcga acccaggacg ctgggggagc gcacgctgcc ggcgcaggtg
1501 gatgcgctga cggaggacac caccctggtc accctctcca tcgggggcaa tgacctcgga
1561 ttcggggagg tggcgggatg catccgggaa cggatcgccg gggagaacgc tgatgattgc
1621 gtggacctgc tgggggaaac catcggggag cagctcgatc agcttccccc gcagctggac
1681 cgcgtgcacg aggctatccg ggaccgcgcc ggggacgcgc aggttgtggt caccggttac
1741 ctgccgctcg tgtctgccgg ggactgcccc gaactggggg atgtctccga ggcggatcgt
1801 cgttgggcgg ttgagctgac cgggcagatc aacgagaccg tgcgcgaggc ggccgaacga
1861 cacgatgccc tctttgtcct gcccgacgat gccgatgagc acaccagttg tgcaccccca
1921 cagcagcgct gggcggatat ccagggccaa cagaccgatg cctatccgct gcacccgacc
1981 tccgccggcc atgaggcgat ggccgccgcc gtccgggacg cgctgggcct ggaaccggtc
2041 cagccgtagc gccgggcgcg cgcttgtcga cgaccaaccc atgccaggct gcagtcacat
2101 ccgcacatag cgcgcgcggg cgatggagta cgcaccatag aggatgagcc cgatgccgac
2161 gatgatgagc agcacactgc cgaagggttg ttccccgagg gtgcgcagag ccgagtccag
2221 acctgcggcc tgctccggat catgggccca accggcgatg acgatcaaca cccccaggat
2281 cccgaaggcg ataccacggg cgacataacc ggctgttccg gtgatgatga tcgcggtccc
2341 gacctgccct gaccccgcac ccgcctccag atcctcccgg aaatcccggg tggccccctt
2401 ccagaggttg tagacacccg cccccagtac caccagcccg gcgaccacaa ccagcaccac
2461 accccaggt tgggatagga cggtggcggt gacatcggtg gcggtctccc catcggaggt
2521 gctgccgccc cgggcgaagg tggaggtggt caccgccagg gagaagtaga ccatggccat
2581 gaccgccccc ttggcccttt ccttgaggtc ctcgcccgcc agcagctggc tcaattgcca
2641 gagtcccagg gccgccaggg cgatgacggc aacccacagg aggaactgcc cacccggagc
2701 ctccgcgatg gtggccaggg cacctgaatt cgaggcctca tcacccgaac cgccggatcc
2761 agtggcgatg cgcaccgcga tccacccgat gaggatgtgc agtatgccca ggacaatgaa
2821 accacctctg gccagggtgg tcagcgcggg gtggtcctcg gcctggtcgg cagcccgttc
2881 gatcgtccgt ttcgcggatc tggtgtcgcc cttatccata gctcccattg aaccgccttg
2941 aggggtgggc ggccactgtc agggcggatt gtgatctgaa ctgtgatgtt ccatcaaccc

//

Novosphingobium\aromaticivorans\ GDSx 284 aa

SEQ ID No. 63

ZP 00094165

1 mgqvkdfarr capvllalag lapaatvare aplaegaryv algssfaagp gvgpnapgsp
61 ercgrgtlny phllaealkl dlvdatcsga tthhvlgpwn evppqidsvn gdtrlvtlti
121 ggndvsfvgn ifaaacekma spdprcgkwr eiteeewqad eermrsivrq iharaplarv
181 vvvdyitvlp psgtcaamai spdrlaqsrs aakrlarita rvareegasl lkfshisrrh
241 hpcsakpwsn glsapaddgi pvhpnrlgha eaaaalvklv klmk

//

SEQ ID No. 64

1 tgccggaact caagcggcgt ctagccgaac tcatgcccga aagcgcgtgg cactatcccg
61 aagaccaggt ctcggacgcc agcgagcgcc tgatggccgc cgaaatcacg cgcgaacagc
121 tctaccgcca gctccacgac gagctgccct atgacagtac cgtacgtccc gagaagtacc
181 tccatcgcaa ggacggttcg atcgagatcc accagcagat cgtgattgcc cgcgagacac
241 agcgtccgat cgtgctgggc aagggtggcg cgaagatcaa ggcgatcgga gaggccgcac
301 gcaaggaact ttcgcaattg ctcgacacca aggtgcacct gttcctgcat gtgaaggtcg
361 acgagcgctg ggccgacgcc aaggaaatct acgaggaaat cggcctcgaa tgggtcaagt
421 gaagctcttc gcgcgccgct gcgccccagt acttctcgcc cttgccgggc tggctccggc
481 ggctacggtc gcgcgggaag caccgctggc cgaaggcgcg cgttacgttg cgctgggaag
541 ctccttcgcc gcaggtccgg gcgtggggcc caacgcgccc ggatcgcccg aacgctgcgg
601 ccggggcacg ctcaactacc cgcacctgct cgccgaggcg ctcaagctcg atctcgtcga
661 tgcgacctgc agcggcgcga cgacccacca cgtgctgggc ccctggaacg aggttccccc
721 tcagatcgac agcgtgaatg gcgacacccg cctcgtcacc ctgaccatcg gcggaaacga
781 tgtgtcgttc gtcggcaaca tcttcgccgc cgcttgcgag aagatggcgt cgcccgatcc
841 gcgctgcggc aagtggcggg agatcaccga ggaagagtgg caggccgacg aggagcggat
901 gcgctccatc gtacgccaga tccacgcccg cgcgcctctc gcccgggtgg tggtggtcga
961 ttacatcacg gtcctgccgc catcaggcac ttgcgctgcc atggcgattt cgccggaccg
1021 gctggcccag agccgcagcg ccgcgaaacg gcttgcccgg attaccgcac gggtcgcgcg
1081 agaagagggt gcatcgctgc tcaagttctc gcatatctcg cgccggcacc atccatgctc
1141 tgccaagccc tggagcaacg gcctttccgc cccggccgac gacggcatcc cggtccatcc
1201 gaaccggctc ggacatgctg aagcggcagc ggcgctggtc aagcttgtga aattgatgaa
1261 gtagctactg cactgatttc aaatagtatt gcctgtcagc tttccagccc ggattgttgc
1321 agcgcaacag aaacttgtcc gtaatggatt gatggtttat gtcgctcgca aattgccgtc
1381 gaagggaacg ggcgcgtcgc tcgttaacgt cctgggtgca gcagtgacgg agcgcgtgga
1441 tgagtgatac tggcggtgtc atcggtgtac gcgccgccat tcccatgcct gtacgcgccg

//

S.coelicolor\ GDSx 268 aa

SEQ ID No. 65

NP_625998.

1 mrrfrlvgfl sslvlaagaa ltgaataqaa qpaaadgyva lgdsyssgvg agsyissgd
61 ckrstkahpy lwaaahspst fdftacsgar tgdvlsgqlg plssgtglvs isiggndagf
121 adtmttcvlq sesscslsria taeayvdstl pgkldgvysa isdkapnahv vvigyprfyk
181 lgttciglse tkrtainkas dhlntvlaqr aaahgftfgd vrttftghel csgspwlhsv
241 nwlnigesyh ptaagqsggy lpvlngaa

//

SEQ ID No. 66

1 cccggcggcc cgtgcaggag cagcagccgg cccgcgatgt cctcgggcgt cgtcttcatc
61 aggccgtcca tcgcgtcggc gaccggcgcc gtgtagttgg cccggacctc gtcccaggtg
121 cccgcggcga tctggcgggt ggtgcggtgc gggccgcgcc gaggggagac gtaccagaag
181 cccatcgtca cgttctccgg ctgcggttcg ggctcgtccg ccgctccgtc cgtcgcctcg
241 ccgagcacct tctcggcgag gtcggcgctg gtcgccgtca ccgtgacgtc ggcgccccgg
301 ctccagcgcg agatcagcag cgtccagccg tcgccctccg ccagcgtcgc gctgcggtcg
361 tcgtcgcggg cgatccgcag cacgcgcgcg ccgggcggca gcagcgtggc gccggaccgt
421 acgcggtcga tgttcgccgc gtgcgagtac ggctgctcac ccgtggcgaa acggccgagg
481 aacagcgcgt cgacgacgtc ggacggggag tcgctgtcgt ccacgttgag ccggatcggc
541 agggcttcgt gcgggttcac ggacatgtcg ccatgatcgg gcacccggcc gccgcgtgca
601 cccgctttcc cgggcacgca cgacaggggc tttctcgccg tcttccgtcc gaacttgaac

661 gagtgtcagc catttcttgg catggacact tccagtcaac gcgcgtagct gctaccacgg
721 ttgtggcagc aatcctgcta agggaggttc catgagacgt ttccgacttg tcggcttcct
781 gagttcgctc gtcctcgccg ccggcgccgc cctcaccggg gcagcgaccg cccaggcggc
841 ccaacccgcc gccgccgacg gctatgtggc cctcggcgac tcctactcct ccggggtcgg
901 agcgggcagc tacatcagct cgagcggcga ctgcaagcgc agcacgaagg cccatcccta
961 cctgtgggcg gccgcccact cgccctccac gttcgacttc accgcctgtt ccggcgcccg
1021 tacgggtgat gttctctccg gacagctcgg cccgctcagc tccggcaccg gcctcgtctc
1081 gatcagcatc ggcggcaacg acgccggttt cgccgacacc atgacgacct gtgtgctcca
1141 gtccgagagc tcctgcctgt cgcggatcgc caccgccgag gcgtacgtcg actcgacgct
1201 gcccggcaag ctcgacggcg tctactcggc aatcagcgac aaggcgccga acgcccacgt
1261 cgtcgtcatc ggctacccgc gcttctacaa gctcggcacc acctgcatcg gcctgtccga
1321 gaccaagcgg acggcgatca acaaggcctc cgaccacctc aacaccgtcc tcgcccagcg
1381 cgccgccgcc cacggcttca ccttcggcga cgtacgcacc accttcaccg gccacgagct
1441 gtgctccggc agcccctggc tgcacagcgt caactggctg aacatcggcg agtcgtacca
1501 ccccaccgcg gccggccagt ccggtggcta cctgccggtc ctcaacggcg ccgcctgacc
1561 tcaggcggaa ggagaagaag aaggagcgga gggagacgag gagtgggagg ccccgcccga
1621 cggggtcccc gtccccgtct ccgtctccgt cccggtcccg caagtcaccg agaacgccac
1681 cgcgtcggac gtggcccgca ccggactccg cacctccacg cgcacggcac tctcgaacgc
1741 gccggtgtcg tcgtgcgtcg tcaccaccac gccgtcctgg cgcgagcgct cgccgcccga
1801 cgggaaggac agcgtccgcc accccggatc ggagaccgac ccgtccgcgg tcacccaccg
1861 gtagccgacc tccgcgggca gccgcccgac cgtgaacgtc gccgtgaacg cgggtgcccg
1921 gtcgtgcggc ggcggacagg ccccgagta gtgggtgcgc gagcccacca cggtcacctc
1981 caccgactgc gctgcggggc
//

S.avermitilis\ GDSx 269 aa

SEQ ID No. 67

NP_827753.
1 mrrsritayv tsllLlavgca ltgaataqas paaaatgyva lgdsyssgvg agsylsssgd
61 ckrsskaypy lwqaahspss fsfmacsgar tgdvlanqlg tlnsstglvs ltiggndagf
121 sdvmttcvlq sdsaclsrin takayvdstl pgqldsvyta istkapsahv avlgyprfyk
181 lggsclagls etkrsainda adylnsaiak raadhgftfg dvkstftghe icsssewlhs
241 ldllnigqsy hptaagqsgg ylpvmnsva
//

SEQ ID No. 68

1 ccaccgccgg gtcggcggcg agtctcctgg cctcggtcgc ggagaggttg gccgtgtagc
61 cgttcagcgc ggcgccgaac gtcttcttca ccgtgccgcc gtactcgttg atcaggccct
121 tgcccttgct cgacgcggcc ttgaagccgg tgcccttctt gagcgtgacg atgtagctgc
181 ccttgatcgc ggtgggggag ccggcggcga gcaccgtgcc ctcggccggg gtggcctggg
241 cgggcagtgc ggtgaatccg cccacgaggg cgccggtcgc cacggcggtt atcgcggcga
301 tccggatctt cttgctacgc agctgtgcca tacgagggag tcctcctctg ggcagcggcg
361 cgcctgggtg gggcgcacgg ctgtgggggg tgcgcgcgtc atcacgcaca cggccctgga
421 gcgtcgtgtt ccgccctggg ttgagtaaag cctcggccat ctacgggggt ggctcaaggg
481 agttgagacc ctgtcatgag tctgacatga gcacgcaatc aacggggccg tgagcacccc
541 ggggcgaccc cggaaagtgc cgagaagtct tggcatggac acttcctgtc aacacgcgta
601 gctggtacga cggttacggc agagatcctg ctaaagggag gttccatgag acgttcccga
661 attacggcat acgtgacctc actcctcctc gccgtcggct gcgccctcac cggggcagcg
721 acggcgcagg cgtccccagc cgccgcggcc acgggctatg tggccctcgg cgactcgtac
781 tcgtccggtg tcggcgccgg cagctacctc agctccagcg gcgactgcaa gcgcagttcg
841 aaggcctatc cgtacctctg gcaggccgcg cattcaccct cgtcgttcag tttcatggct
901 tgctcgggcg ctcgtacggg tgatgtcctg gccaatcagc tcggcaccct gaactcgtcc

961 accggcctgg tctccctcac catcggaggc aacgacgcgg gcttctccga cgtcatgacg
1021 acctgtgtgc tccagtccga cagcgcctgc ctctcccgca tcaacacggc gaaggcgtac
1081 gtcgactcca ccctgcccgg ccaactcgac agcgtgtaca cggcgatcag cacgaaggcc
1141 ccgtcggccc atgtggccgt gctgggctac ccccgcttct acaaactggg cggctcctgc
1201 ctcgcgggcc tctcggagac caagcggtcc gccatcaacg acgcggccga ctatctgaac
1261 agcgccatcg ccaagcgcgc cgccgaccac ggcttcacct tcggcgacgt caagagcacc
1321 ttcaccggcc atgagatctg ctccagcagc acctggctgc acagtctcga cctgctgaac
1381 atcggccagt cctaccaccc gaccgcggcc ggccagtccg gcggctatct gccggtcatg
1441 aacagcgtgg cctgagctcc cacggcctga atttttaagg cctgaatttt taaggcgaag
1501 gtgaaccgga agcggaggcc ccgtccgtcg ggtctccgt cgcacaggtc accgagaacg
1561 gcacggagtt ggacgtcgtg cgcaccgggt cgcgcacctc gacggcgatc tcgttcgaga
1621 tcgttccgct cgtgtcgtac gtggtgacga acacctgctt ctgctgggtc tttccgccgc
1681 tcgccgggaa ggacagcgtc ttccagcccg gatccgggac ctcgcccttc ttggtcaccc
1741 agcggtactc cacctcgacc ggcaccggc ccaccgtgaa ggtcgccgtg aacgtgggcg
1801 cctgggcggt gggcggcggg caggcaccgg agtagtcggt gtgcacgccg gtgaccgtca
1861 ccttcacgga ctgggccggc ggggtcgtcg taccgccgcc gccaccgccg cctcccggag
1921 tggagcccga gctgtggtcg cccccgccgt cggcgttgtc gtcctcgggg gttttcgaac

//

*Streptomyces* diglyceride:glycerol acyltransferase

SEQ ID No. 69

ACAGGCCGATGCACGGAACCGTACCTTTCCGCAGTGAAGCGCTCTCCCCCCATCGTTCGC
CGGGACTTCATCCGCGATTTTGGCATGAACACTTCCTTCAACGCGCGTAGCTTGCTACAA
GTGCGGCAGCAGACCCGCTCGTTGGAGGCTCAGTGAGATTGACCCGATCCCTGTCGGCCG
CATCCGTCATCGTCTTCGCCCTGCTGCTCGCGCTGCTGGGCATCAGCCCGGCCCAGGCAG
CCGGCCCGGCCTATGTGGCCCTGGGGGATTCCTATTCCTCGGGCAACGGCGCCGGAAGTT
ACATCGATTCGAGCGGTGACTGTCACCGCAGCAACAACGCGTACCCCGCCCGCTGGGCGG
CGGCCAACGCACCGTCCTCCTTCACCTTCGCGGCCTGCTCGGGAGCGGTGACCACGGATG
TGATCAACAATCAGCTGGGCGCCCTCAACGCGTCCACCGGCCTGGTGAGCATCACCATCG
GCGGCAATGACGCGGGCTTCGCGGACGCGATGACCACCTGCGTCACCAGCTCGGACAGCA
CCTGCCTCAACCGGCTGGCCACCGCCACCAACTACATCAACACCACCCTGCTCGCCCGGC
TCGACGCGGTCTACAGCCAGATCAAGGCCCGTGCCCCCAACGCCCGCGTGGTCGTCCTCG
GCTACCCGCGCATGTACCTGGCCTCGAACCCCTGGTACTGCCTGGGCCTGAGCAACACCA
AGCGCGCGGCCATCAACACCACCGCCGACACCCTCAACTCGGTGATCTCCTCCCGGGCCA
CCGCCCACGGATTCCGATTCGGCGATGTCCGCCCGACCTTCAACAACCACGAACTGTTCT
TCGGCAACGACTGGCTGCACTCACTCACCCTGCCGGTGTGGGAGTCGTACCACCCCACCA
GCACGGGCCATCAGAGCGGCTATCTGCCGGTCCTCAACGCCAACAGCTCGACCTGATCAA
CGCACGGCCGTGCCCGCCCCGCGCGTCACGCTCGGCGCGGGCGCCGCAGCGCGTTGATCA
GCCCACAGTGCCGGTGACGGTCCCACCGTCACGGTCGAGGGTGTACGTCACGGTGGCGCC
GCTCCAGAAGTGGAACGTCAGCAGGACCGTGGAGCCGTCCCTGACCTCGTCGAAGAACTC
CGGGGTCAGCGTGATCACCCCTCCCCCGTAGCCGGGGGCGAAGGCGGCGCCGAACTCCTT
GTAGGACGTCCAGTCGTGCGGCCCGGCGTTGCCACCGTCCGCGTAGACCGCTTCCATGGT
CGCCAGCCGGTCCCCGCGGAACTCGGTGGGGATGTCCGTGCCCAAGGTGGTCCCGGTGGT
GTCCGAGAGCACCGGGGGCTCGTACCGGATGATGTGCAGATCCAAAGAATT

*Streptomyces* diglyceride:glycerol acyltransferase

SEQ ID No. 70

MRLTRSLSAASVIVFALLLALLGISPAQAAGPAYVALGDSYSSGNGAGSYIDSSGDCHRSN
NAYPARWAAANAPSSFTFAACSGAVTTDVINNQLGALNASTGLVSITIGGNDAGFADAMTT
CVTSSDSTCLNRLATATNYINTTLLARLDAVYSQIKARAPNARVVVLGYPRMYLASNPWYC
LGLSNTKRAAINTTADTLNSVISSRATAHGFRFGDVRPTFNNHELFFGNDWLHSLTLPVWE
SYHPTSTGHQSGYLPVLNANSST

IDENTIFICATION OF A DIGLYCERIDE:GLYCEROL ACYLTRANSFERASE ACCORDING TO THE PRESENT INVENTION

Assay for enzymatic reduction of diglyceride in palm oil.

1 gram of palm oil containing 7% diglyceride is scaled in a glass with lid.

50 mg glycerol and 10 μl enzyme solution is added. The reaction mixture is agitated with a magnetic stirrer in a heating chamber at 40°C for 20 hours. The enzyme reaction is stopped by heating to 100°C for 10 minutes. A reference sample added 10μl water instead of enzyme solution is treated in the same way. The samples are analysed by GLC according to standard procedures (see hereinbelow) and the amount of fatty acids, monoglyceride and diglyceride are calculated.

*Calculation:*

From the results of the GLC analyses the increase in free fatty acids and the decrease in diglycerides can be calculated:

Δ % fatty acid = % Fatty acid(enzyme) - % fatty acid(control);
Mv Fa = average molecular weight of the fatty acids;
Δ % diglyceride = % Diglyceride(contol) - % Diglyceride(enzyme);
Mv Di= average molecular weight of diglyceride;

The transferase activity is calculated as a percentage of the total enzymatic activity:

$$\text{\% transferase activity} = = \frac{((\Delta \text{ \% Di}/(\text{Mv Di}) - \Delta \text{ \% Fa}/(\text{Mv FA})) \times 100}{\Delta \text{ \% Di}/(\text{Mv Di})}$$

<u>GLC analysis</u>

Perkin Elmer Autosystem 9000 Capillary Gas Chromatograph equipped with WCOT fused silica column 12.5 m x 025 mm ID x 0.1 μ film thickness 5% phenyl-methyl-silicone (CP Sil 8 CB from Chrompack).

Carrier gas: Helium.

Injector. PSSI cold split injection (initial temp 50°C heated to 385°C), volume 1.0μl Detector FID: 395°C

| | | | |
|---|---|---|---|
| Oven program: | 1 | 2 | 3 |
| Oven temperature, °C. | 90 | 280 | 350 |
| Isothermal, time, min. | 1 | 0 | 10 |
| Temperature rate, °C/min. | 15 | 4 | |

Sample preparation: 30 mg of sample was dissolved in 9 ml Heptane:Pyridin, 2:1 containing, internal standard heptadecane, 0.5 mg/ml. 300μl sample solution was transferred to a crimp vial, 300 μl MSTFA (N-Methyl-N-trimethylsilyltrifluoraceamid) was added and reacted for 20 minutes at 60°C.

ISOLATED

In one aspect, preferably the polypeptide or protein for use in the present invention is in an isolated form. The term "isolated" means that the sequence is at least substantially free from at least one other component with which the sequence is naturally associated in nature and as found in nature.

PURIFIED

In one aspect, preferably the polypeptide or protein for use in the present invention is in a purified form. The term "purified" means that the sequence is in a relatively pure state - e.g. at least about 51% pure, or at least about 75%, or at least about 80%, or at least about 90% pure, or at least about 95% pure or at least about 98% pure.

CLONING A NUCLEOTIDE SEQUENCE ENCODING A POLYPEPTIDE ACCORDING TO THE PRESENT INVENTION

A nucleotide sequence encoding either a polypeptide which has the specific properties as defined herein or a polypeptide which is suitable for modification may be isolated from any cell or organism producing said polypeptide. Various methods are well known within the art for the isolation of nucleotide sequences.

For example, a genomic DNA and/or cDNA library may be constructed using chromosomal DNA or messenger RNA from the organism producing the polypeptide: If the amino acid sequence of the polypeptide is known, labelled oligonucleotide probes may be synthesised and used to identify polypeptide-encoding clones from the genomic library prepared from the organism. Alternatively, a labelled oligonucleotide probe containing sequences homologous to another known polypeptide gene could be used to identity polypeptide-encoding clones. In the latter case, hybridisation and washing conditions of lower stringency are used.

Alternatively, polypeptide-encoding clones could be identified by inserting fragments of genomic DNA into an expression vector, such as a plasmid, transforming enzyme-negative bacteria with the resulting genomic DNA library, and then plating the transformed bacteria onto agar containing an enzyme inhibited by the polypeptide, thereby allowing clones expressing the polypeptide to be identified.

In a yet further alternative, the nucleotide sequence encoding the polypeptide may be prepared synthetically by established standard methods, e.g. the phosphoroamidite method described by Beucage S.L. et al (1981) Tetrahedron Letters 22, p 1859-1869, or the method described by Matthes et al (1984) EMBO J. 3, p 801-805. In the phosphoroamidite method, oligonucleotides are synthesised, e.g. in an automatic DNA synthesiser, purified, annealed, ligated and cloned in appropriate vectors.

The nucleotide sequence may be of mixed genomic and synthetic origin, mixed synthetic and cDNA origin, or mixed genomic and cDNA origin, prepared by ligating fragments of synthetic, genomic or cDNA origin (as appropriate) in accordance with standard techniques. Each ligated fragment corresponds to various parts of the entire nucleotide sequence. The DNA sequence may also be prepared by polymerase chain reaction (PCR) using specific primers, for instance as described in US 4,683,202 or in Saiki R K et al (Science (1988) 239, pp 487-491).

Suitably, the lipid acyltransferase used in the invention may be a variant, i.e. may contain at least one amino acid substitution, deletion or addition, when compared to a parental enzyme. Variant enzymes retain at least 20%, 30%, 40%, 50 %, 60%, 70%, 80%, 90%, 95%, 97%, 99% homology with the parent enzyme. Suitable parent enzymes may include any enzyme with esterase or lipase activity. Preferably, the parent enzyme aligns to the pfam00657 consensus sequence.

In a preferable embodiment a variant lipid acyltransferase enzyme retains or incorporates at least one or more of the pfam00657 consensus sequence amino acid residues found in the GDSx, GANDY and HPT blocks.

Enzymes, such as lipases with no or low lipid acyltransferase activity in an aqueous environment may be mutated using molecular evolution tools to introduce or enhance the transferase activity, thereby producing a lipid acyltransferase enzyme with significant transferase activity suitable for use in the compositions and methods of the present invention.

Suitably, the lipid acyltransferase for use in the invention may be a variant with enhanced enzyme activity on polar lipids, preferably phospholipids and/or glycolipids when compared to the parent enzyme. Preferably, such variants also have low or no activity on lyso polar lipids. The enhanced activity on polar lipids, phospholipids and/or glycolipids may be the result of hydrolysis and/or transferase activity or a combination of both.

Variant lipid acyltransferases for use in the invention may have decreased activity on triglycerides, and/or monoglycerides and/or diglycerides compared with the parent enzyme.

Suitably the variant enzyme may have no activity on triglycerides and/or monoglycerides and/or diglycerides.

Alternatively, the variant enzyme for use in the invention may have increased activity on triglycerides, and/or may also have increased activity on one or more of the following, polar lipids, phospholipids, lecithin, phosphatidylcholine, glycolipids, digalactosyl monoglyceride, monogalactosyl monoglyceride.

Variants of lipid acyltransferases are known, and one or more of such variants may be suitable for use in the methods and uses according to the present invention and/or in the enzyme compositions according to the present invention. By way of example only, variants of lipid acyltransferases are described in the following references may be used in accordance with the present invention: Hilton & Buckley J Biol. Chem. 1991 Jan 15: 266 (2): 997-1000; Robertson et al J. Biol. Chem. 1994 Jan 21; 269(3):2146-50; Brumlik et al J. Bacteriol 1996 Apr; 178 (7): 2060-4; Peelman et al Protein Sci. 1998 Mar; 7(3):587-99.

## AMINO ACID SEQUENCES

As used herein, the term "amino acid sequence" is synonymous with the term "polypeptide" and/or the term "protein". In some instances, the term "amino acid sequence" is synonymous with the term "peptide".

The amino acid sequence may be prepared/isolated from a suitable source, or it may be made synthetically or it may be prepared by use of recombinant DNA techniques.

Suitably, the amino acid sequences may be obtained from the isolated polypeptides taught herein by standard techniques.

One suitable method for determining amino acid sequences from isolated polypeptides is as follows:

Purified polypeptide may be freeze-dried and 100 μg of the freeze-dried material may be dissolved in 50 μl of

a mixture of 8 M urea and 0.4 M ammonium hydrogen carbonate, pH 8.4. The dissolved protein may be denatured and reduced for-15 minutes at 50°C following overlay with nitrogen and addition of 5 μl of 45 mM dithiothreifol After cooling to room temperature, 5 μl of 100 mM iodoacetamide may be added for the cysteine residues to be derivatized for 15 minutes at room temperature in the dark under nitrogen.

135 μl of water and 5 μg of endoproteinase Lys-C in 5 μl of water may be added to the above reaction mixture and the digestion may be carried out at 37°C under nitrogen for 24 hours.

The resulting peptides may be separated by reverse phase HPLC on a VYDAC C18 column (0.46x15cm;10μm; The Separation Group, California, USA) using solvent A: 0.1% TFA in water and solvent B: 0.1% TFA in acetonitrile. Selected peptides may be re-chromatographed on a Develosil C18 column using the same solvent system, prior to N-terminal sequencing. Sequencing may be done using an Applied Biosystems 476A sequencer using pulsed liquid fast cycles according to the manufacturer's instructions (Applied Biosystems, California, USA).

SEQUENCE IDENTITY OR SEQUENCE HOMOLOGY

The present invention also encompasses the use of sequences having a degree of sequence identity or sequence homology with amino acid sequence(s) of a polypeptide having the specific properties defined herein or of any nucleotide sequence encoding such a polypeptide (hereinafter referred to as a "homologous sequence(s)"). Here, the term "homologue" means an entity having a certain homology with the subject amino acid sequences and the subject nucleotide sequences. Here, the term "homology" can be equated with "identity".

The homologous amino acid sequence and/or nucleotide sequence should provide and/or encode a polypeptide which retains the functional activity and/or enhances the activity of the enzyme.

In the present context, a homologous sequence is taken to include an amino acid sequence which may be at least 75, 85 or 90% identical, preferably at least 95 or 98% identical to the subject sequence. Typically, the homologues will comprise the same active sites etc. as the subject amino acid sequence. Although homology can also be considered in terms of similarity (i.e. amino acid residues having similar chemical properties/functions), in the context of the present invention it is preferred to express homology in terms of sequence identity.

In the present context, a homologous sequence is taken to include a nucleotide sequence which may be at least 75, 85 or 90% identical, preferably at least 95 or 98% identical to a nucleotide sequence encoding a polypeptide of the present invention (the subject sequence). Typically, the homologues will comprise the same sequences that code for the active sites etc. as the subject sequence. Although homology can also be considered in terms of similarity.: (i.e. amino acid residues having similar chemical properties/functions), in the context of the present invention it is preferred to express homology in terms of sequence identity.

Homology comparisons can be conducted by eye, or more usually, with the aid of readily available sequence comparison programs. These commercially available computer programs can calculate % homology between two or more sequences.

% homology may be calculated over contiguous sequences, i.e. one sequence is aligned with the other sequence and each amino acid in one sequence is directly compared with the corresponding amino acid in the other sequence, one residue at a time. This is called an "ungapped" alignment. Typically, such untapped alignments are performed only over a relatively short number of residues.

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example when using the GCG Wisconsin Bestfit package the default gap penalty-for amino acid sequences is -12 for a gap and -4 for each extension.

Calculation of maximum % homology therefore firstly requires the production of an optimal alignment, taking into consideration gap penalties. A suitable computer program for carrying out such an alignment is the GCG Wisconsin Bestfit package (Devereux et al 1984 Nuc. Acids Research 12 p387). Examples of other software that can perform sequence comparisons include, but are not limited to, the BLAST package (see Ausubel et al 1999 Short Protocols in

Molecular Biology, 4th Ed - Chapter 18), FASTA (Altschul et al 1990 J. Mol. Biol. 403-410) and the GENEWORKS suite of comparison tools. Both BLAST and FASTA are available for offline and online searching (see Ausubel *et al* 1999, pages 7-58 to 7-60). However, for some applications, it is preferred to use the GCG Bestfit program. A new tool, called BLAST 2 Sequences is also available for comparing protein and nucleotide sequence (see FEMS Microbiol Lett 1999 174(2): 247-50; FEMS Microbiol Lett 1999 177(1): 187-8 and tatiana@ncbi.nlm.nih.gov).

Although the final % homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see user manual for further details). For some applications, it is preferred to use the public default values for the GCG package, or in the case of other software, the default matrix, such as BLOSUM62.

Alternatively, percentage homologies may be calculated using the multiple alignment feature in DNASIS™ (Hitachi Software), based on an algorithm, analogous to CLUSTAL (Higgins DG & Sharp PM (1988), Gene 73(1), 237-244).

Once the software has produced an optimal alignment, it is possible to calculate % homology, preferably % sequence identity. The software typically does this as part of the sequence comparison and generates a numerical result.

The sequences may also have deletions, insertions or substitutions of amino acid residues which produce a silent change and result in a functionally equivalent substance. Deliberate amino acid substitutions may be made on the basis of similarity in polarity, charge, solubility, hydrophobicity, hydrophilicity, and/or the amphipathic nature of the residues as long as the secondary binding activity of the substance is retained. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine.

Conservative substitutions may be made, for example according to the Table below. Amino acids in the same block in the second column and preferably in the same line in the third column may be substituted for each other:

| | | |
|---|---|---|
| ALIPHATIC | Non-polar | G A P |
| | | I L V |
| | Polar-uncharged | C S T M |
| | | N Q |
| | Polar - charged | D E |
| | | K R |
| AROMATIC | | H F W Y |

The present method also encompasses homologous substitution (substitution and replacement are both used herein to mean the interchange of an existing amino acid residue, with an alternative residue) that may occur i.e. like-for-like substitution such as basic for basic, acidic for acidic, polar for polar etc. Non-homologous substitution may also occur i.e. from one class of residue to another or alternatively involving the inclusion of unnatural amino acids such as ornithine (hereinafter referred to as Z), diaminobutyric acid ornithine (hereinafter referred to as B), norleucine ornithine (hereinafter referred to as O), pyriylalanine, thienylalanine, naphthylalanine and phenylglycine.

Replacements may also be made by unnatural amino acids.

Variant amino acid sequences may include suitable spacer groups that may be inserted between any two amino acid residues of the sequence including alkyl groups such as methyl, ethyl or propyl groups in addition to amino acid spacers such as glycine or β-alanine residues. A further form of variation, involves the presence of one or more amino acid residues in peptoid form, will be well understood by those skilled in the art. For the avoidance of doubt, "the peptoid form" is used to refer to variant amino acid residues wherein the α-carbon substituent group is on the residue's nitrogen atom rather than the α-carbon. Processes for preparing peptides in the peptoid form are known in the art, for example Simon RJ et al., PNAS (1992) 89(20), 9367-9371 and Horwell DC, Trends Biotechnol. (1995) 13(4), 132-134.

The invention will now be described, by way of example only, with reference to the following Figures and Examples.

Figure 1 shows a pfam00657 consensus sequence from database version 6 (SEQ ID No.1);

Figure 2 shows an amino acid sequence (SEQ ID No. 2) obtained from the organism *Aeromonas hydrophila* (P10480; GI:121051);

Figure 3 shows an amino acid sequence (SEQ ID No. 3) obtained from the organism *Aeromonas salmonicida* (AAG098404; GI:9964017);

Figure 4 shows an amino acid sequence (SEQ ID No. 4) obtained from the organism *Streptomyces coelicolor* A3 (2) (Genbank accession number NP_631558);

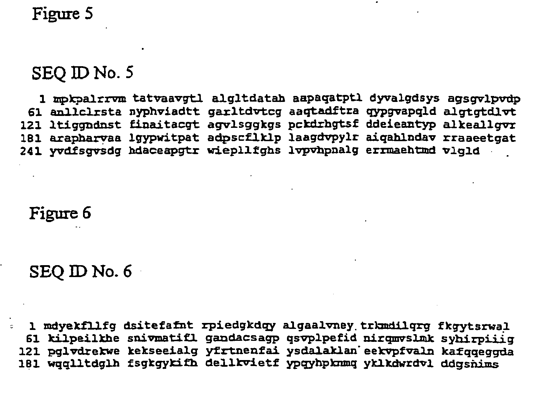

Figure 5 shows an amino acid sequence (SEQ ID No. 5) obtained from the organism *Streptomyces coelicolor* A3 (2) (Genbank accession number: CAC42140);

Figure 6 shows an amino acid sequence (SEQ ID No. 6) obtained from the organism *Saccharomyces cerevisiae* (Genbank accession number P41734);

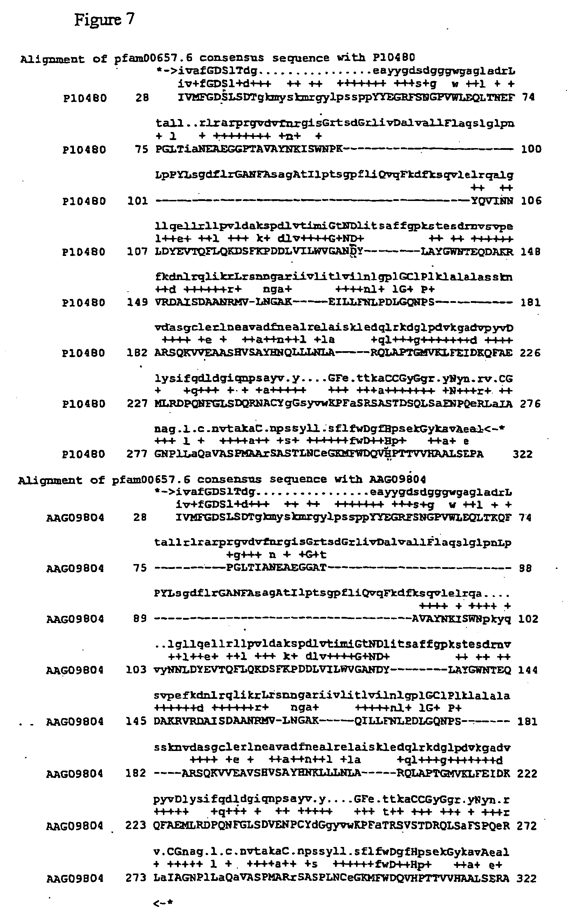

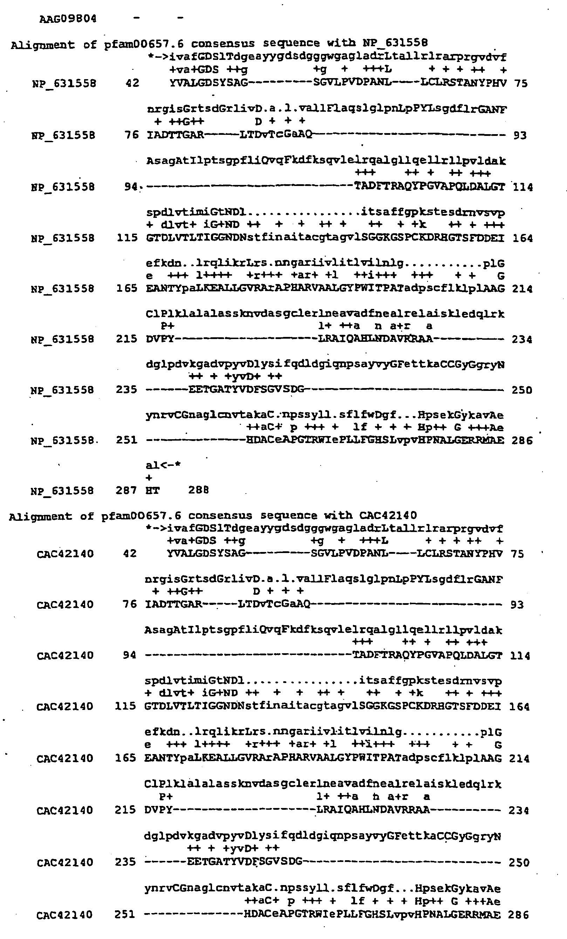

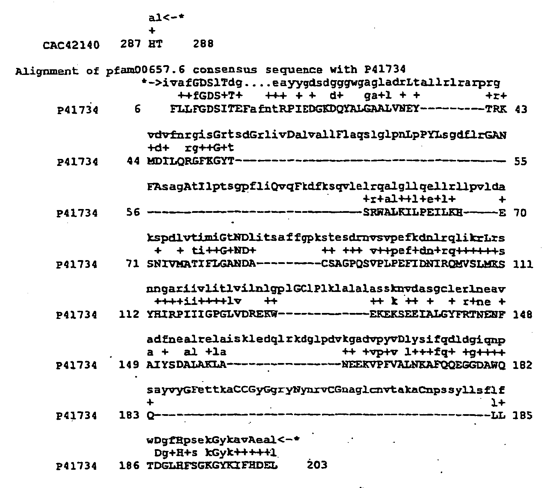

Figure 7 shows an alignment of selected sequences to pfam00657 consensus sequence;

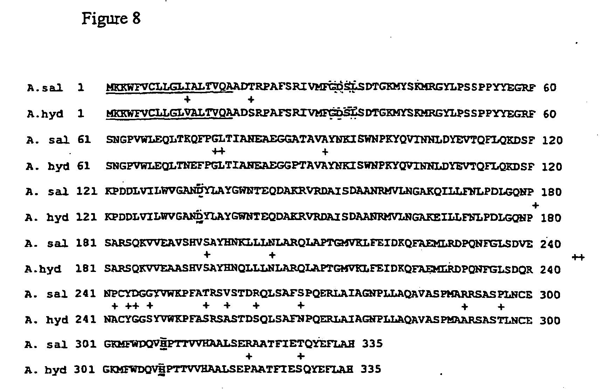

Figure 8 shows a pairwise alignment of SEQ ID No. 3 with SEQ ID No. 2 showing 93% amino acid sequence identity. The signal sequence is underlined. + denotes differences. The GDSX motif containing the active site serine 16, and the active sites aspartic acid 116 and histidine 291 are highlighted (see shaded regions). Numbers after the amino acid is minus the signal sequence;

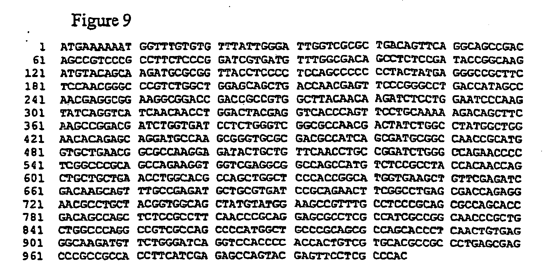

Figure 9 shows a nucleotide sequence (SEQ ID No. 7) encoding a lipid acyl transferase according to the present invention obtained from the organism *Aeromonas hydrophila;*

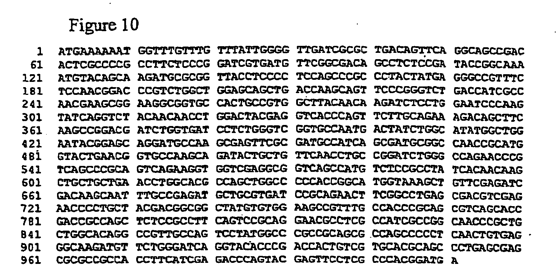

Figure 10 shows a nucleotide sequence (SEQ ID No. 8) encoding a lipid acyl transferase according to the present invention obtained from the organism *Aeromonas salmonicida;*

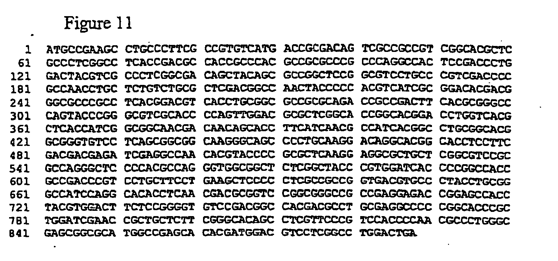

Figure 11 shows a nucleotide sequence (SEQ ID No. 9) encoding a lipid acyl transferase according to the present invention obtained from the organism *Streptomyces coelicolor* A3(2) (Genbank accession number NC_003888.1: 8327480..8328367);

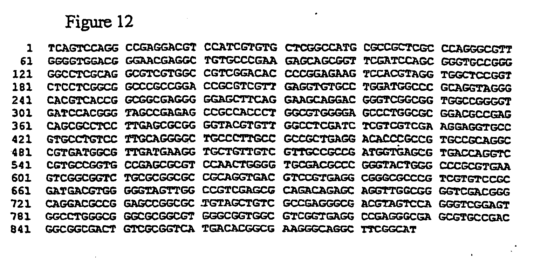

Figure 12 shows a nucleotide sequence (SEQ ID No. 10) encoding a lipid acyl transferase according to the present invention obtained from the organism *Streptomyces coelicolor* A3(2) (Genbank accession number AL939131.1: 266480..266367);

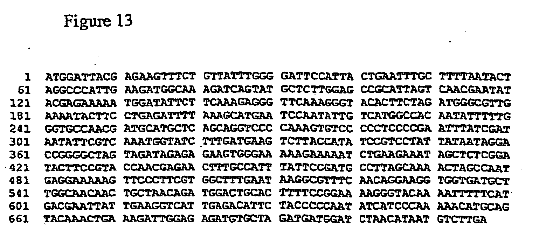

Figure 13 shows a nucleotide sequence (SEQ ID No. 11) encoding a lipid acyl transferase according to the present invention obtained from the organism *Saccharomyces cerevisiae* (Genbank accession number Z75034);

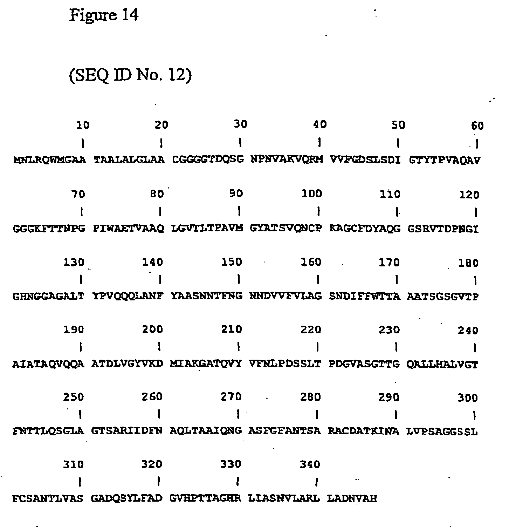

Figure 14 shows an amino acid sequence (SEQ ID No. 12) obtained from the organism *Ralstonia* (Genbank accession number: AL646052);

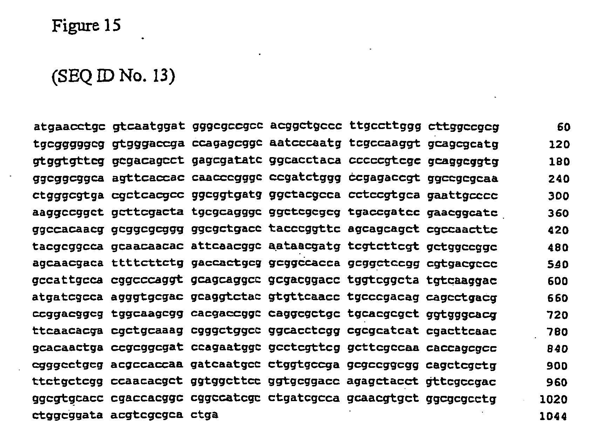

Figure 15 shows a nucleotide sequence (SEQ ID No. 13) encoding a lipid acyl transferase according to the present invention obtained from the organism *Ralstonia;*

Figure 16 shows SEQ ID No. 20. Scoel NCBI protein accession code CAB39707.1 GI:4539178 conserved hypothetical protein [Streptomyces coelicolor A3(2)];

Figure 17 shows a nucleotide sequence shown as SEQ ID No. 21 encoding NCBI protein accession code CAB39707.1 GI:4539178 conserved hypothetical protein [Streptomyces coelicolor A3(2)];

Figure 18 shows an amino acid shown as SEQ ID No.22. Scoe2 NCBI protein accession code CAC01477.1 GI: 9716139 conserved hypothetical protein [Streptomyces coelicolor A3(2)];

Figure 19 shows a nucleotide sequence shown as SEQ ID No. 23 encoding Scoe2 NCBI protein accession code CAC01477.1 GI:9716139 conserved hypothetical protein [Streptomyces coelicolor A3(2)];

Figure 20 shows an amino acid sequence (SEQ ID No.24) Scoe3 NCBI protein accession code CAB88833.1 GI:

7635996 putative secreted protein. [Streptomyces coelicolor A3(2)];

Figure 21 shows a nucleotide sequence shown as SEQ ID No. 25 encoding Scoe3 NCBI protein accession code CAB88833.1 GI:7635996 putative secreted protein. [Streptomyces coelicolor A3(2)];

Figure 22 shows an amino acid sequence (SEQ ID No.26) Scoe4 NCBI protein accession code CAB89450.1 GI: 7672261 putative secreted protein. [Streptomyces coelicolor A3(2)];

Figure 23 shows an nucleotide sequence shown as SEQ ID No. 27 encoding Scoe4 NCBI protein accession code CAB89450.1 GI:7672261 putative secreted protein. [Streptomyces coelicolor A3(2)];

Figure 24 shows an amino acid sequence (SEQ ID No.28) Scoe5 NCBI protein accession code CAB62724.1 GI: 6562793 putative lipoprotein [Streptomyces coelicolor A3(2)];

Figure 25 shows a nucleotide sequence shown as SEQ ID No. 29, encoding Scoe5 NCBI protein accession code CAB62724.1 GI:6562793 putative lipoprotein [Streptomyces coelicolor A3(2)];

Figure 26 shows an amino acid sequence (SEQ ID No.30) Srim1 NCBI protein accession code AAK84028.1 GI: 15082088 GDSL-lipase [Streptomyces rimosus];

Figure 27 shows a nucleotide sequence shown as SEQ ID No. 31 encoding Srim1 NCBI protein accession code AAK84028.1 GI:15082088 GDSL-lipase [Streptomyces rimosus];

Figure 28 shows an amino acid sequence (SEQ ID No.32)A lipid acyl transferase from *Aeromonas hydrophila* (ATCC #7965);

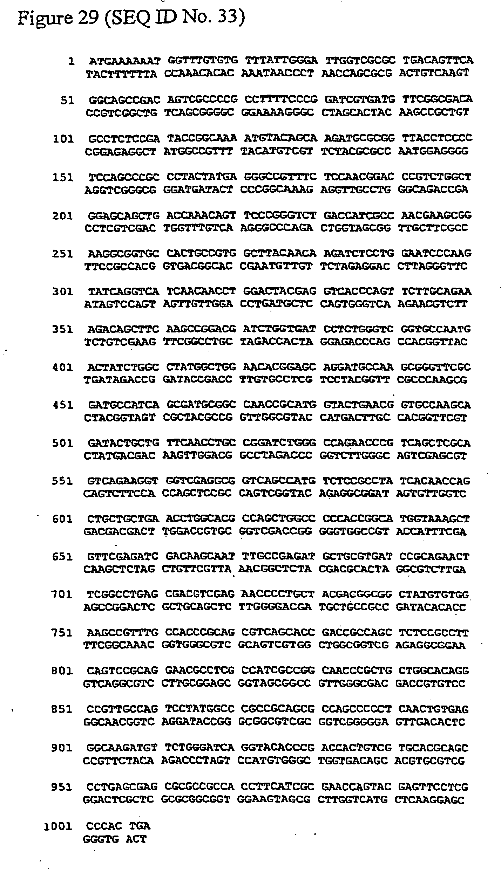

Figure 29 shows a nucleotide sequence (SEQ ID No. 33) encoding a lipid acyltransferase from *Aeromonas hydrophila* (ATCC #7965);

Figure 30 shows an amino acid sequence (SEQ ID No.34) of a lipid acyltransferase from *Aeromonas salmonicida* subsp. *Salmonicida* (ATCC#14174);

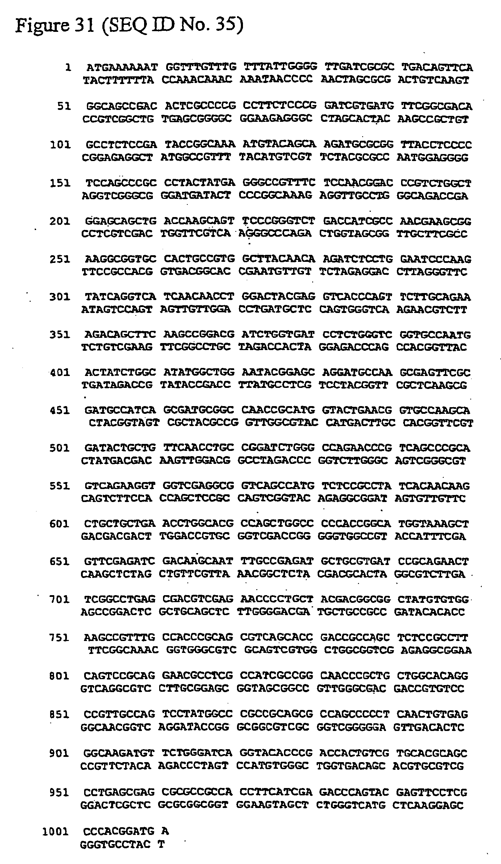

Figure 31 shows a nucleotide sequence (SEQ ID No 35) encoding a lipid acyltransferase from *Aeromonas salmonicida* subsp. *Salmonicida* (ATCC#14174);

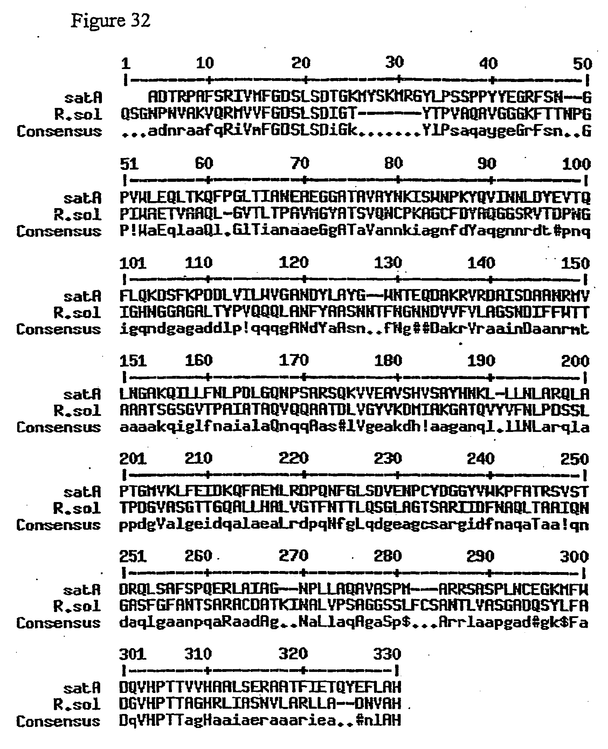

Figure 32 shows that homologues of the *Aeromonas* genes can be identified using the basic local alignment search tool service at the National Center for Biotechnology Information, NIH, MD, USA and the completed genome databases. The GDSX motif was used in the database search and a number of sequences/genes potentially encoding enzymes with lipolytic activity were identified. Genes were identified from the genus Streptomyces, Xanthomonas and Ralstonia. As an example below, the Ralstonia solanacearum was aligned to the Aeromonas salmonicida (satA) gene. Pairwise alignment showed 23% identity. The active site serine is present at the amino terminus and the catalytic residues histidine and aspartic acid can be identified;

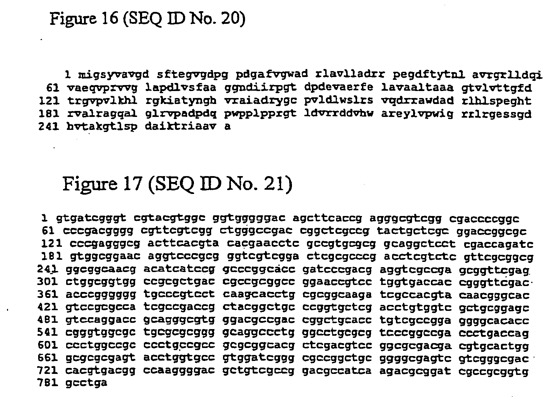

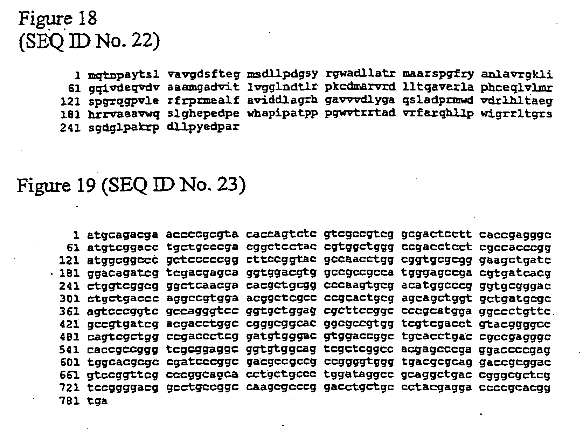

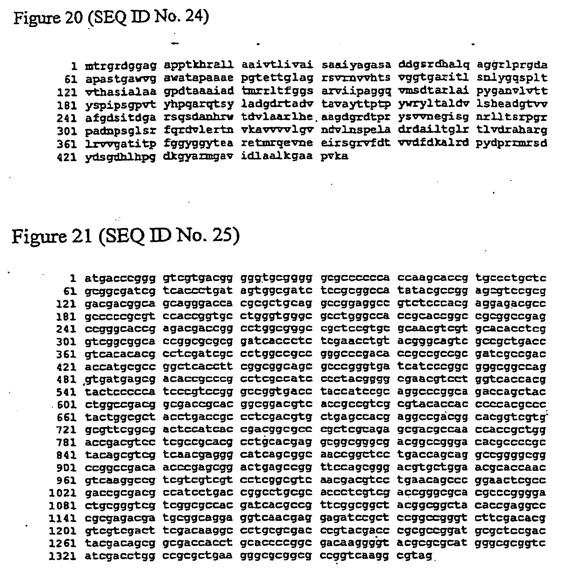

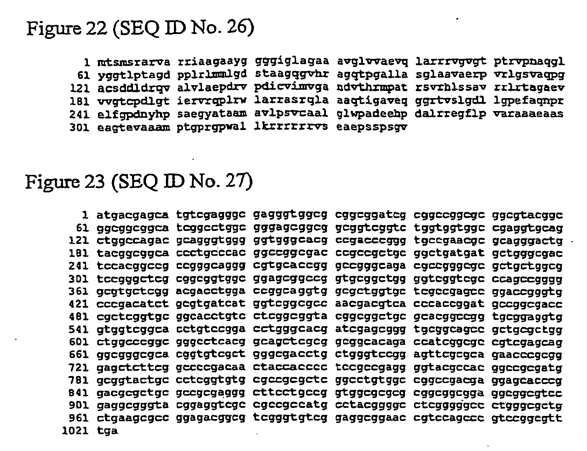

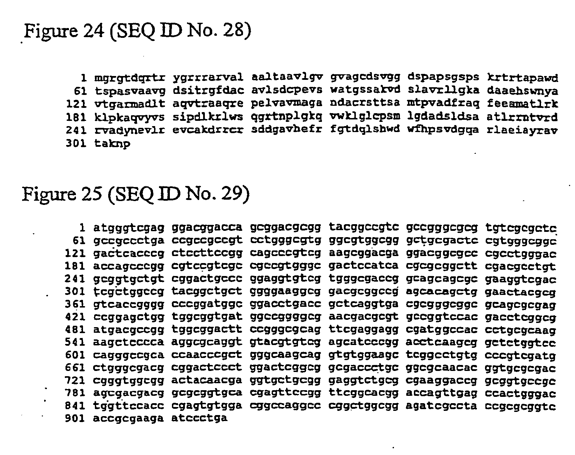

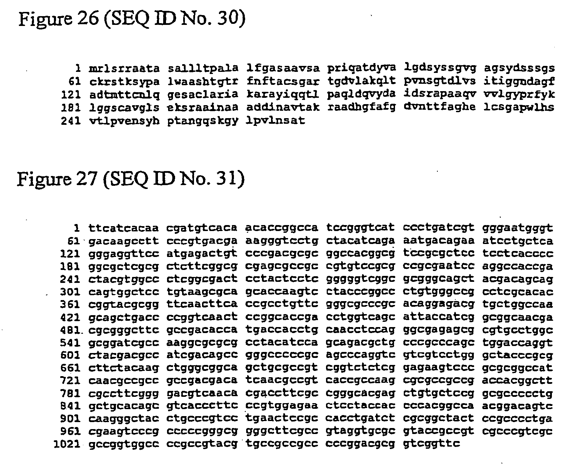

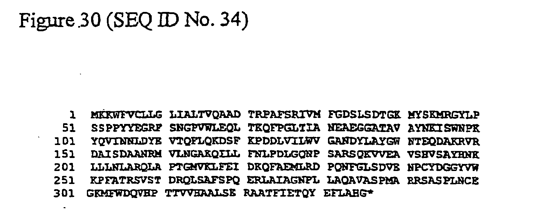

Figure 33 shows the Pfam00657.11 [family 00657, database version 11] consensus sequence (hereafter called Pfam consensus) and the alignment of various sequences to the Pfam consensus sequence. The arrows indicate the active site residues, the underlined boxes indicate three of the homology boxes indicated by [Upton C and Buckley JT (1995) Trends Biochem Sci 20; 179-179]. Capital letters in the Pfam consensus indicate conserved residues in many family members. The - symbol indicates a position where the hidden Markov model of the Pfam consensus expected to find a residue but did not, so a gap is inserted. The . symbol indicates a residue without a corresponding residue in the Pfam consensus. The sequences are the amino acid sequences listed in Figures 16, 18, 20, 22, 24, 26, 28 and 30.

Figure 34 shows the Pfam00657.11 [family 00657, database version 11] consensus sequence (hereafter called Pfam consensus) and the alignment of various sequences to the Pfam consensus sequence. The arrows indicate the active site residues, the underlined boxes indicate three of the homology boxes indicated by [Upton C and Buckley JT (1995) Trends Biochem Sci 20; 179-179]. Capital letters in the Pfam consensus indicate conserved residues in many family members. The - symbol indicates a position where the hidden Markov model of the Pfam consensus expected to find a residue but did not, so a gap is inserted. The . symbol indicates a residue without a

corresponding residue in the Pfam consensus. The sequences are the amino acid sequences listed in Figures 2, 16, 18, 20, 26, 28 and 30. All these proteins were found to be active against lipid substrates.

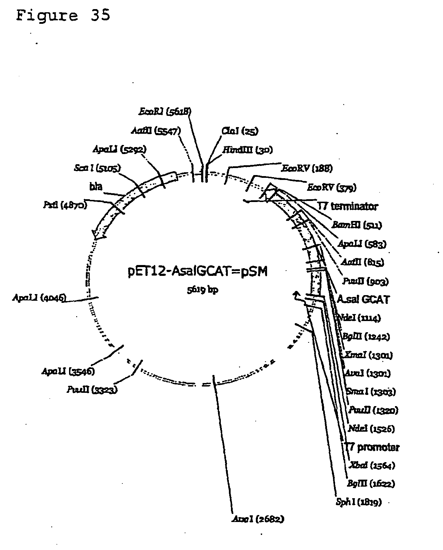

Figure 35 shows a expression vector pet12-AsalGCAT= pSM containing the C-terminal His-tagged *Aeromonas salmonicida* lipid acyltransferase gene;

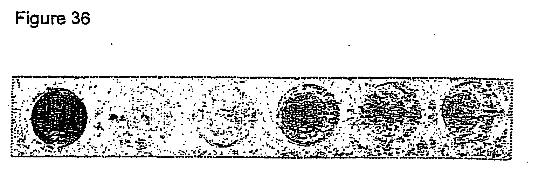

Figure 36 shows the results of testing cell extracts in a NEFA Kit Assay, which depicts the activity of a recombinant, *A. salmonicida* lipid acyltransferase, towards lecithin. The wells from left to right indicate: a positive control, a negative control (i.e. extracts from empty plasmid) and samples collected after 0, 1, 2 and 3 hours cultivation after IPTG induction;

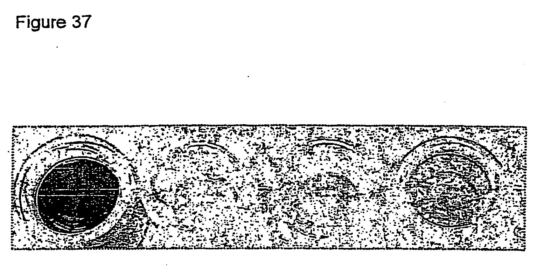

Figure 37 shows growth optimisation of BL21(DE3)pLysS harboring the expression vector pet12-AsalGCAT= pSM showing cultivation at 30°C resulted in the production of enzyme with high activity towards lecithin. Cell extracts were tested for phospholipase activity using the NEFA kit assay. Wells from left to right: positive control; negative control; 20°C; 30°C;

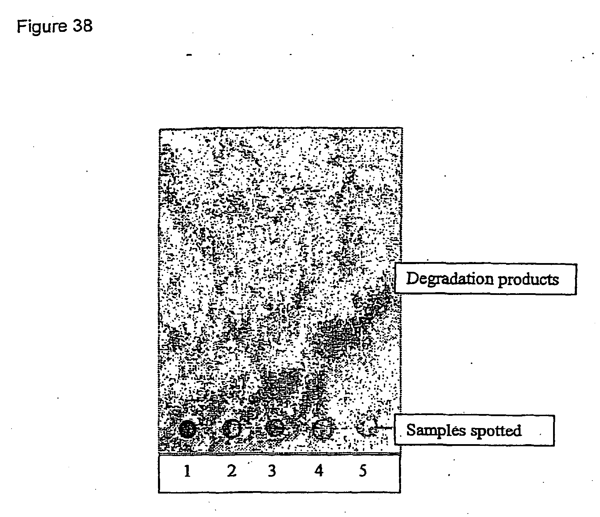

Figure 38 shows crude cell extracts from BL21(DE3)pLysS expressing active lipid acyltransferase incubated with the substrate lecithin and reaction mixture was analyzed using thin layer chromatography showing the presence of degradation products. Lanes: 1. No enzyme; 2. + A.sal-10ul 37°C; 3. + A. sal-20ul 37°C; 4. + A.sal-10ul 24°C;5. +·A. sal-20u 24°C;

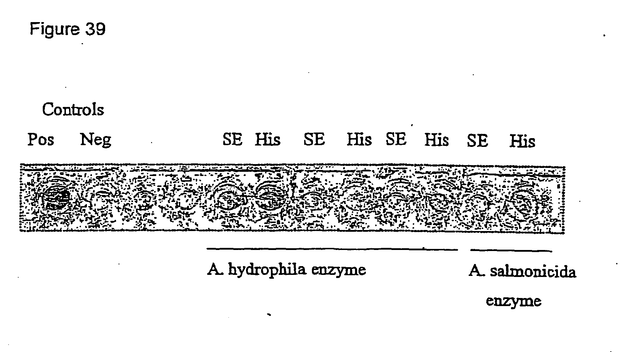

Figure 39 shows partial purification of the *Aeromonas salmonicida* Acyl Transferase showing the phospholipase activity associated with purified His-tag protein. SE = Sonicated extract, His = Purified with Ni-NTA spin-kit from Qiagen;

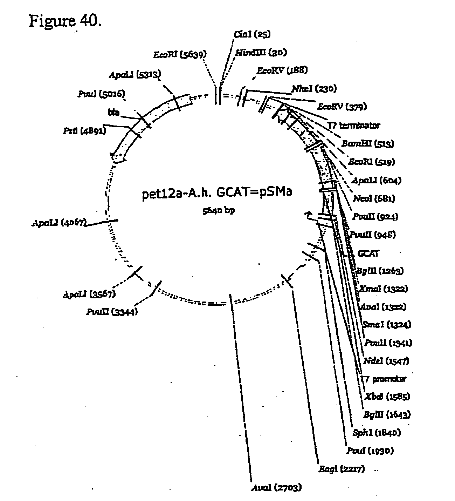

Figure 40 shows the expression vector pet12-A.h. GCAT=pSMa containing the C-terminal His-tagged *Aeromonas hydrophila* Glycerolipid Acyl Transferase (GCAT) gene was used to transform *E. coli* strain BL21(DE3)pLysS;

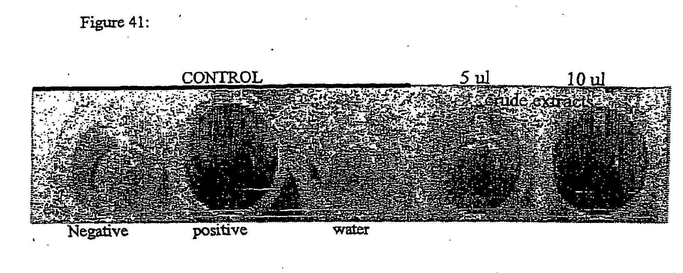

Figure 41 shows the activity of the crude extracts (5 & 10ul) containing the recombinant *Aeromonas hydrophila* GCAT enzyme was tested towards lecithin using Non-Esterified Fatty Acid (NEFA) kit (Roche, Switzerland), showing the presence of active enzyme towards the phospholipid, lecithin;

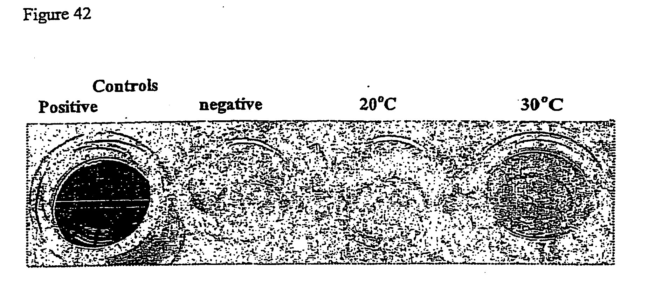

Figure 42 shows growth optimisation of BL21(DE3)pLysS harboring the expression vector pet12-AsalGCAT= pSM showing cultivation at 30°C resulted in the production of enzyme with high activity towards lecithin. Cell extracts were tested for phospholipase activity using the NEFA kit assay;

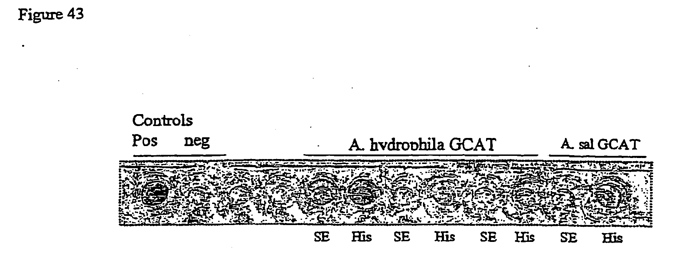

Figure 43 shows the partial purification of the *Aeromonas hydrophila & A. salmonicida* Acyl Transferases showing the phospholipase activity associated with purified His-tag protein. SE = Sonicated extracts, His = Purified with Ni-NTA spin-kit from Qiagen);

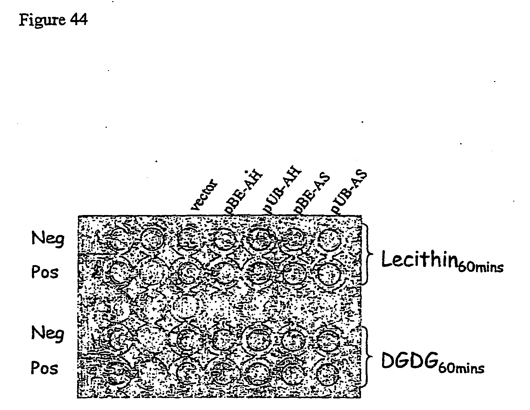

Figure 44 shows the expression of the *Aeromonas* genes in *Bacillus subtilis* 163 showing the production of secreted enzyme with activity towards both lecithin and DGDG. pUB-AH= construct containing the *A. hydrophila* gene and pUB-AS, construct with the *A. salmonicida* gene, Culture filtrate was incubated with the substrates for 60 minutes.

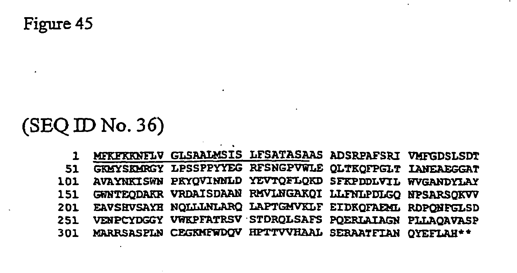

Figure 45 shows an amino acid sequence (SEQ ID No. 36) of the fusion construct used for mutagenesis of the *Aeromonas hydrophila* lipid acyltransferase gene in Example 17. The underlined amino acids is a xylanase signal peptide;

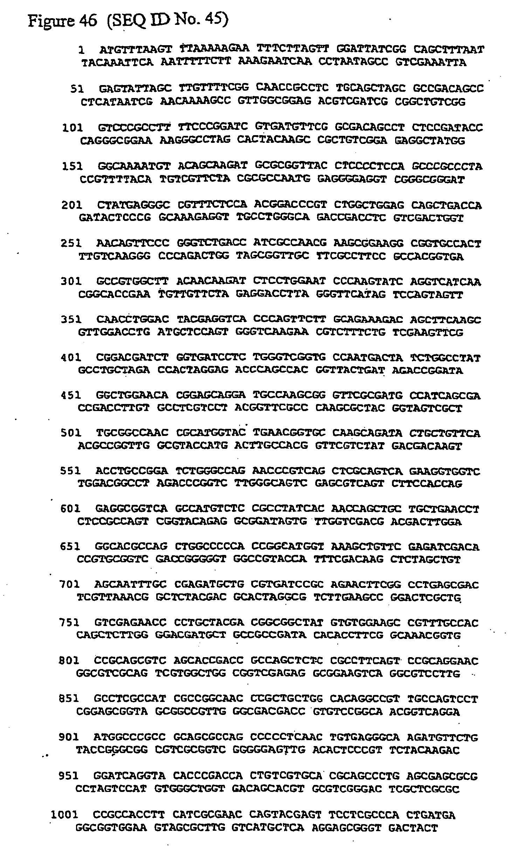

Figure 46 shows a nucleotide sequence (SEQ ID No. 45) encoding an enzyme from *Aeromonas hydrophila* including a xylanase signal peptide;

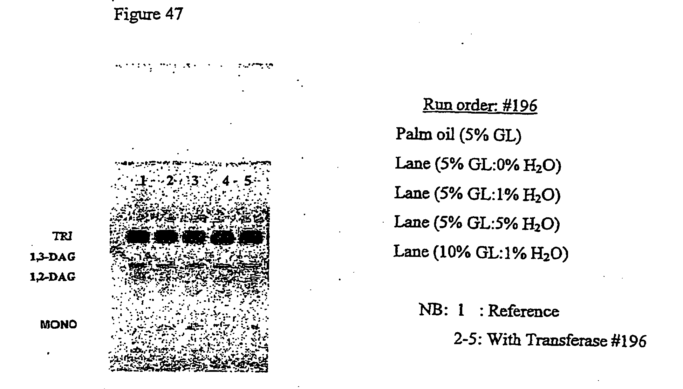

Figure 47 shows the result of the HPTLC analysis in Experiment I;

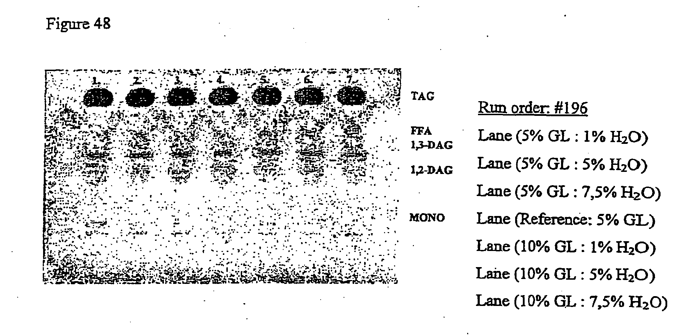

Figure 48 shows the result of the HPTLC analysis in Experiment II;

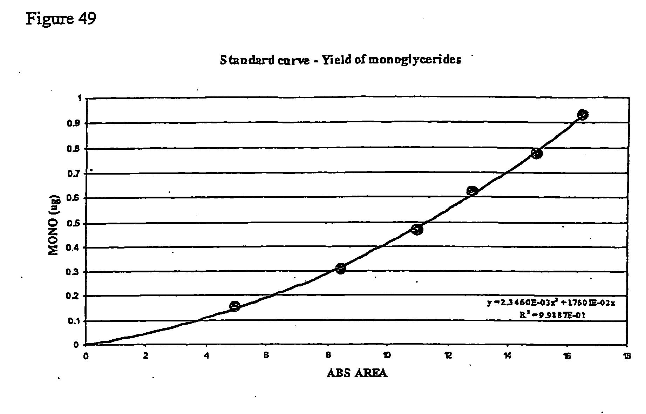

Figure 49 shows a calibration curve for monoglyceride standard solutions;

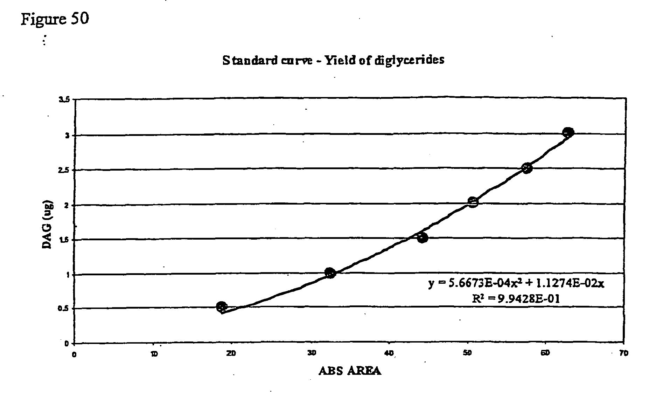

Figure 50 shows a calibration curve for diglyceride standard solutions.

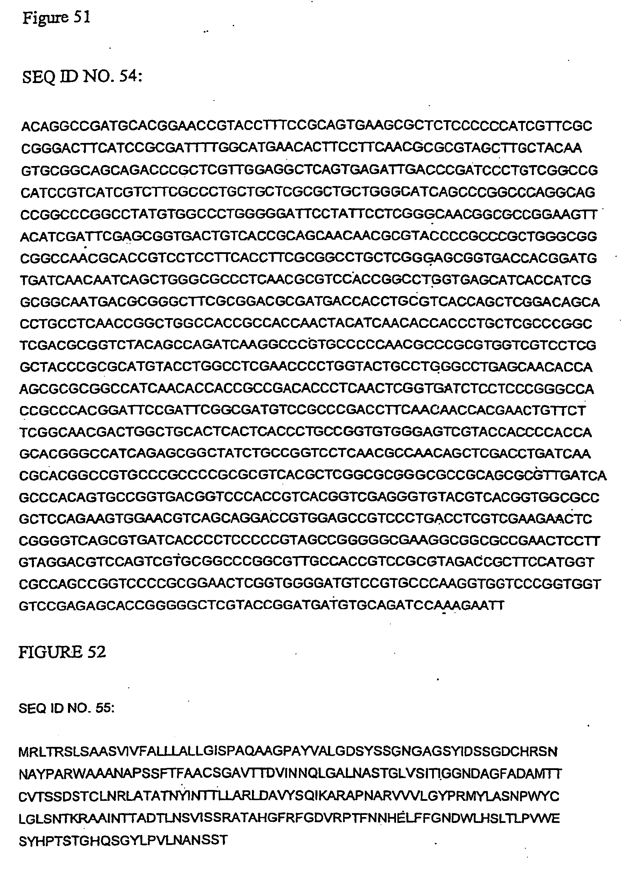

Figure 51 shows a nucleotide sequence encoding a lipid acyltransferase enzyme according to the present invention from *Streptomyces* (SEQ ID No. 54);

Figure 52 shows a polypeptide sequence of a lipid acyltransferase enzyme according to the present invention from *Streptomyces* (SEQ ID No. 55);

EXAMPLES

For the avoidance of doubt, the following abbreviations may be used herein:

MONO = monoglyceride
MAG = monoacylglycerol = monoglyceride
MAG and MONO are interchangeable herein.
DAG = diacylglycerol
FFA = free fatty acid

**EXAMPLE 1: The cloning, sequencing and heterologous expression of a transferase from *Aeromonas salmonicida* subsp. *Salmonicida***

**Strains used:**

*Aeromonas salmonicida subsp. Salmonicida* (ATCC 14174) was obtained from ATCC and grown overnight at 30°C in Luria-Bertani medium (LB). The cells were centrifuged and genomic DNA was isolated using the procedures for genomic DNA isolation from Qiagen Ltd. Genomic DNA buffer set (cat.19060), protease K (cat. 19131) and RNAse A (cat. 19101) were all obtained from Qiagen Ltd. (Boundary court Gatwick Court, West Sussex, RH10 2AX).

Host bacterial strain BL21(DE3)pLysS (Novagen) was used for production of the recombinant *Aeromonas* enzymes. Competent cells of BL21(DE3)pLysS were used as host for transformation with the expression vector pet12-AsalGCAT=pSM.

Transformants containing the appropriate plasmid were grown at 37 °C in LB agar medium containing 100-ug ampicillin/ml.

**Construction of expression vector pet12-AsalGCAT- pSM:**

For all DNA amplifications of the transferase genes from *Aeromonas,* genomic DNA (02-1 ul) was used as template and *pfu* DNA polymerase (2.5 units) was used with 10ul of 10x pfu buffer, 1ul each primer (50pmol/ul), 200 uMdNTP in a total reaction volume of 100ul. PCR reactions were performed in a programmable thermal cycler using the following conditions: 95°C for 30 seconds, 30 cycles of 95 °C for 30 seconds, 60 °C for 1 minute and 68 °C for 2 minutes. An additional extension of 5 minutes at 72 °C was applied.

The PCR amplification of the transferase gene from *A. salmonicida* was carried in 2 separate PCR reactions. PCR reaction 1 was performed using primer pairs, as1USNEW(5'AGCATATGAAAA AATGGTTTGT TTGTTTATTG GGG 3' [SEQ ID No. 56]) and asls950new (5'GTG ATG GTG GGC GAG GAA CTC GTA CTG3' [SEQ ID No. 37]). A second PCR reaction was performed to incorporate a C-terminal Histidine tag using the PCR product from the first reaction and the primers: as1USNEW(5'AGCATATGAAAA AATGGTTTGT TTGTTTATTG GGG 3'[SEQ ID No. 38]) and AHLS1001(5TTGGATCC GAATTCAT CAATG GTG ATG GTG ATG GTG GGC3' [SEQ ID No. 39]). The PCR product from the second reaction was purified and digested with restriction enzymes Nde1 and BamHI. 2 ug of pET 12a vector DNA was also digested with restriction enzymes Nde1 and BamHI and treated with phosphatase. The restriction enzyme-treated pet12a and PCR product from reaction 2 were purified and ligated using the Rapid Ligation Kit (Roche, Switzerland). The ligation mix was used to transform *E. coli* TOP10 cells. Transformants were plated on LB agar medium containing 100ug/ml ampicillin.

The T7 promoter primer (5'TAATACGACTCACTATAG3' [SEQ ID No. 40]) and the T7 terminator primer (5'CTAGTTATTGCTCAGCGG3' [SEQ ID No. 41]) were used to verify the sequences and the orientation of the cloned transferase genes in pET12a vector. DNA sequencing was performed using ABI Prism® BigDye™ Terminators Cycle sequencing kit with 500ng plasmid DNA as template and 3.2pmol T7 promoter and terminator primers.

The construct shown in Figure 35 was used to transform competent bacterial host strain BL21(DE3)pLysS (Novagen) and ampicillin resistant transformants were picked and used for expression analysis.

Expression of the recombinant *Aeromonas salmonicida* lipid acyltransferase

Quantification of enzyme activity towards lecithin was determined on cell extracts using Non-Esterified Fatty Acid (NEFA) kit (Roche, Switzerland).

In Figure 36, BL21(DE3)pLysS harboring the expression vector pet12-AsalGCAT= pSM was grown in LB medium + 100ug/ml ampicillin and incubated with shaking at 37°C until $OD_{600}$ = 0.6 to 1.0 is reached. The cultures are then induced using IPTG (0.4mM) and incubation was continued for the next 3 hours. Samples where taken at 0 hour, 1, 2, and 3 hours after IPTG induction. Enzyme Activity was tested using the NEFA kit and lecithin as substrate.

Growth Optimisation for the production of more active enzymes

BL21(DE3)pLysS harboring the expression vector pet12-AsalGCAT= pSM was grown in LB medium + 100ug/ml ampicillin and incubated with shaking at different growth temperatures (37°C, 30 °C, & 20°C). The optimal condition for the production of active lipid acyltransferase enzyme was when cultures are grown at 30°C as shown in Figure 37.

Partial purification of recombinant *Aeromonas salmonicida* transferase

Strain BL21(DE3)pLysS harboring the expression vector pet12-AsalGCAT=pSM was grown at 37°C & crude cell extracts were prepared by sonication. The recombinant enzyme was further purified from the sonicated crude cell extracts using the Ni-NTA spin kit from Qiagen. Phospholipase activity using the NEFA kit & Lecithin as substrate. Crude cell extracts from BL21(DE3)pLysS expressing active transferase incubated with the substrate lecithin and reaction mixture was analysed using thin layer chromatography showing the presence of degradation products (see Figure 3 8).

Partial Purification of recombinant *Aeromonas salmonicidae* transferase. Strain BL21(DE3)pLysS harbouring the expression vector pet12-AsalGCAT=pSM was grown at 37°C and crude cell extracts were prepared by sonication. The recombinant enzyme ware further purified from the sonicated crude cell extract using the Ni-NTA spin kit from Qiagen. Phospholipase activity using the NEFA kit and lecithin as substrate was tested (see Figure 39).

**EXAMPLE 2 Cloning and Expression of *Aeromonas hydrophila* transferase in *E. coli***

*Aeromonas hydrophila* (ATCC # 7965) was obtained from ATCC and grown overnight at 30°C in Luria-Bertani medium (LB). The cells were centrifuged and genomic DNA was isolated using the procedures for genomic DNA isolation from Qiagen Ltd. Genomic DNA buffer set (cat. 19060), protease K (cat. 19131) and RNAse A (cat. 19101) were all obtained from Qiagen Ltd. (Boundary court Gatwick Court, West Sussex, RH10 2AX).

Host bacterial strain BL21(DE3)pLysS (Novagen) was used for production of the recombinant *Aeromonas* enzymes. Competent cells of BL21(DE3)pLysS were used as host for transformation with the expression vector pet12a-A.h.GCAT=pSMa. Transformants containing the appropriate plasmid were grown at 37 °C in LB agar medium containing 100-ug ampicillin/ml.

**Construction of expression vector pet12a-A.h.GCAT-pSMa:**

For all DNA amplifications of the transferase gene from *Aeromonas,* genomic DNA (02-1 ul) was used as template and *pfu* DNA polymerase (2.5 units) was used with 10ul of 10x pfu buffer, 1ul each primer (50pmol/ul), 200 uMdNTP in a total reaction volume of 100ul. PCR reactions were performed in a programmable thermal cycler using the following conditions: 95 °C for 30 seconds, 30 cycles of 95 °C for 30 seconds, 60 °C for 1 minute and 68 °C for 2 minutes. An additional extension of 5 minutes at 72 °C was applied.

The PCR amplification of the transferase gene from *A. hydrophila* (ATCC # 7965) was carried out in 2 separate PCR reactions.

PCR reaction 1 was performed using primer pairs, AHUS1 (5'GTCATATGAAAAAATGGTTTGTGTGTTTATTGGGATTGGTC3', SEQ ID No. 42) and ahls950 (5'ATGGTGATGGTGGGCGAGGAACTCGTACTG3', SEQ ID No. 43).

A second PCR reaction was performed to incorporate a C-terminal Histidine tag using the PCR product from

the first reaction .and the primer pairs:

AHUS1(5'GTCATATGAAAAAATGGTTTGTGTGTTTATTGGGATTGGTC3' SEQ ID No. 44, ) and AHLS1001(5'TTGGATCCGAATTCATCAATGGTGATGGTGATGGTGGGC3' SEQ ID No. 57).

The PCR product from the second reaction was purified and digested with restriction enzymes Nde1 and BamHI. 2 ug of pET 12a vector DNA was also digested with restriction enzymes Nde1 and BamHI and treated with phosphatase. The restriction enzyme-treated pet12a and PCR product from reaction 2 were purified and ligated using the Rapid Ligation Kit (Roche, Switzerland). The ligation mix was used to transform *E. coli* TOP10 cells. Transformants were plated on LB agar medium containing 100ug/ml ampicillin.

The T7 promoter primer (5'TAATACGACTCACTATAG3') and the T7 terminator primer (5'CTAGTTATTGCTCAGCGG3') were used to verify the sequences and the orientation of the cloned GCAT genes in pET12a vector. DNA sequencing was performed using ABI Prism® BigDye™ Terminators Cycle sequencing kit with 500ng plasmid DNA as template and 3.2pmol T7 promoter and terminator primers.

The construct shown in Figure 40 was used to transform competent bacterial host strain BL21 (DE3)pLysS (Novagen) and ampicillin resistant transformants were picked and used for expression analysis.

Expression of the *Aeromonas hydrophila* transferase in BL21(DE3)pLysS

The *E. coli* strain BL21(DE3)pLysS harboring the expression vector pet12a-A.h.GCAT= pSMa was grown in LB medium + 100ug/ml ampicillin and incubated with shaking at 37°C until $OD_{600}$= 0.6 to 1.0 is reached. The cultures are then induced using IPTG (0.4mM) and incubation was continued for the next 3 hours. Samples where taken at 0hour, 1, 2, and 3 hours after IPTG induction. Enzyme Activity was tested using the NEFA kit and lecithin as substrate (Figure 41).

**Growth Optimisation for the production of more active enzymes**

BL21(DE3)pLysS harboring the expression vector pet12a-A.h.GCAT= pSMa was grown in LB medium + 100ug/ml ampicillin and incubated with shaking at different growth temperatures (37°C, 30°C, & 20 °C). The optimal condition for the production of active GCAT enzyme was when cultures are grown at 30°C as shown in Figure 42.

**Partial purification of recombinant *A.hydrophila* transferase (GCAT)**

Strain BL21(DE3)pLysS harboring the expression vector pet12a-A.h.GCAT=pSMa was grown at 37°C & crude cell extracts were prepared by sonication. The recombinant enzyme was further purified from the sonicated crude cell extracts using the Ni-NTA spin kit from Qiagen. Phospholipase activity assay using the NEFA kit & Lecithin as substrate. (Figure 43).

**EXAMPLE 3: Expression of *Aeromonas* transferases in *Bacillus subtilis* 163**

**Plasmid Construction**

Two different *Bacillus subtilis* expression vectors (pUB 110 & pBE5) were used for the heterologous expression of the *Aeromonas* genes in *Bacillus subtilis.* The pUB110 vector contains the alpha amylase promoter while the pBE vector has the P32 promoter as the regulatory region for the expression of the fused *Aeromonas* genes. In pUB110, the first amino acid of the mature GCAT genes of *Aeromonas* were fused in frame with the last amino acid of the xylanase signal peptide sequence from *Bacillus subtilis* via the restriction site Nhe1, creating an additional 2 amino acids in front of the mature proteins. pBES contains the cgtase signal sequence fusion at the Nco1 site for secretion of the recombinant proteins into the culture filtrate.

PCR reactions were carried out to obtain the *Aeromonas* genes fuse in frame to the signal sequences of the pUB 110 and the pBE5 vectors. PCRs were performed using the following primer pairs for *A. hydrophila* gene:

PCR reaction 1: usAHncol (5'ATGCCATGGCCGACAGCCGTCCCGCC3', SEQ ID No. 46) and lsAH (5'TTGGATCCGAATTCATCAATGGTGATG3', SEQ ID No. 47)

PCR reaction 2: US-AhnheI (5'TTGCTAGCGCCGACAGCCGTCCCGCC3', SEQ ID No. 48.) and lsAH (5'TTGGATCCGAATTCATCAATGGTGATG3, SEQ ID No. 49)

PCRs were performed using the following primer pairs for *A. salmonicida* gene:

PCR reaction 3: US-Asncol (5'TTGCCATGGCCGACACTCGCCCCGCC3', SEQ ID No. 50) and lsAH (5'TTGGATCCGAATTCATCAATGGTGATG3', SEQ ID No. 51)

PCR reaction 4: US-ASnhe1 (5'TTGCTAGCGCCGACACTCGCCCCGCC3', SEQ ID No. 52) and lsAH (5'TTGGATCCGAATTCATCAATGGTGATG3', SEQ ID No. 53).

All the PCR products were cloned into PCR blunt II (TOPO vector) and sequenced with reverse & forward sequencing primers.

Clones from PCR reactions 1 & 3 were cut with Nco1 & Bam HI and used as inserts for ligation to the pBE5 vector cut with Nco1/BamH1/phosphatase. Clones from PCR reactions 2 & 4 were cut with Nhe1 & Bam H1 and used as inserts for ligation to the pUB vector that was cut with Nhe1/BamH1/phosphatase.

Expression of the *Aeromonas* transferase genes in *Bacillus subtilis* and characterization of the enzyme activity.

The acyl transferases from the two *Aeromonas* species have been successfully expressed in *E. coli* (results above). The *Bacillus* pUB 110 & pBE5 gene fusion constructs were used to transform *Bacillus subtilis* and transformants were selected by plating on kanamycin plates. The kanamycin resistant transformants isolated and grown in 2xYT are capable of heterologous expression of the *Aeromonas* genes in *Bacillus.* The culture filtrates have digalactosyldiacylglycerol (DGDG) galactolipase activity, in addition to having both acyl transferase and phospholipase activities. The activity towards digalactosyldiacylglycerol (DGDG) was measured after 60 minutes of incubation of culture supernatant with the substrate, DGDG from wheat flour (obtainable form Sigma) as well as the activity towards lecithin as shown in Figure 44. *Bacillus* produced the enzyme after overnight (20-24 hours) to 48 hours of cultivation in the culture-medium as a secreted protein. In some instances, the expression of the *Aeromonas* genes has been shown to interfere with cell viability and growth in *Bacillus & E. coli,* it is therefore necessary to carefully select expression strains and optimise the growth conditions to ensure expression. For example, several *Bacillus* host strains (B.s 163, DB104 and OS 21) were transformed with the expression vectors for growth comparison. B.s163 is transformable with the 2 *Aeromonas* genes and is capable of expressing active protein. DB104 is transformable with all the constructs but is only able to express *A. salmonicida* transferase.

**EXAMPLE 4: Fermentation and Purification of *Aeromonas* lipid acyltransferases produced in *E.coli***

***E.coli* Fermentations:**

Microorganisms

Two strains of *Eschericia coli, one* containing an *Aeromonas hydrophila* (Example 2) lipid acyltransferase and two containing *Aeromonas salmonicida* lipid acyltransferases, (Example 1) were used in this study.

The *E. coli* strains containing the *A. hydrophila* gene was named DIDK0124, and the *E. coli* strain containing the *A. salmonicida* gene was named DIDK0125. The fermentation with DIDK0124 was named HYDRO0303 and the fermentation with DIDK0125 was named SAL0302. The purified protein from HYDRO025 was named REF#138. The

purified protein from HYDRO0303 was named REF#135.

Growth media and culture conditions

**LB-agar**

The LB agar plates used for maintaining the strains contained: 10 g/L tryptone, 5 g/L yeast extract, 5 g/L NaCl, 15 g/L agar, 100 mg/L ampicillin and 35 mg/L chloramphenicol. The agar plates were incubated at 30°C.

**LB shake flask**

The LB medium (50 mL pr shake flask) used for production of inoculum material for the bioreactor cultivations contained: 10 g/L tryptone, 5 g/L yeast extract, 5 g/L NaCl, 100 mg/L ampicillin and 35 mg/L chloramphenicol. The shake flasks were inoculated from the LB agar plates, and incubated at 30°C and 200 rpm.

**Bioreactor cultivation**

The bioreactor cultivations were carried out in 6 L in-house built bioreactors filled with 4 L medium containing: 10 g/L tryptone, 5 g/L yeast extract, 5 g/L NaCl, 8 g/L $KH_2PO_4$, 0.9 g/L $MgSO_4$,$7H_2O$, 40 g/L glucose monohydrate, 0.4 mL/ ADD APT® Foamstop Sin 260 (ADD APT Chemicals AG, Helmond, The Netherlands), 10 mg/L $(NH_4)_2Fe(SO_4)_2 \cdot 6H_2O$, 0.7 mg/L $CuSO_4 \cdot 5H_2O$, 3 mg/L $ZnSO_4 \cdot 7H_2O$, 3 mg/L $MnSO_4 \cdot H_2O$, 10 mg/L EDTA, 0.1 mg/L $NiSO_4 \cdot 6H_2O$, 0.1 mg/L $CoCl_2$, 0.1 mg/L $H_3BO_4$, 0.1 mg/L KI, 0.1 mg/L $Na_2MoO_4 \cdot 2H_2O$, 1 g/L ampicillin and 35 mg/L chloramphenicol.

The bioreactors were inoculated with an amount of LB culture ensuring end of growth after approximately 20 hours of cultivation (calculated from the maximum specific growth rate of 0.6 $h^{-1}$, the $OD_{600}$ of the LB shake flask and the final $OD_{600}$ in the bioreactor of approximately 20).

SAL0302 was inoculated with IO mL of LB culture, and HYDRO0303 was inoculated with 4 mL of LB culture.

The bioreactors were operated at the following conditions: temperature 30°C, stirring 800-1000 rpm (depending on experiment), aeration 5 L/min, pH 6.9, pH control 8.75% (w/v) $NH_3$-water and 2 M $H_2SO_4$. Induction was achieved by addition of isopropyl β-D-thiogalactoside to a final concentration of 0.6 mM, when 0.4 moles (HYDRO0303) and 0.7 moles $CO_2$ was produced respectively.

Harvest

The following procedure was used for harvest and homogenisation of the biomass:

1) The fermentation broth from the fermentations was centrifuged at 5000 × g and 4°C for 10 minutes, and the supernatant was discharged. The biomass was stored at -20°C until use. The biomass was thawed and resuspended in 500 mL of 20 mM $NaH_2PO_4$, pH 7.4, 500 mM NaCl, 10 mM Imidazole and Complete (EDTA-free) protease inhibitor (Roche, Germany).
2) The suspended biomass was homogenized at 2 kbar and 4°C in a cell disrupter from Constant Systems Ltd (Warwick, UK).
3) The cell debris was removed by centrifugation at 10.000 × g and 4°C for 30 minutes followed by collection of the supernatant
4) The supernatant was clarified further by centrifugation at 13.700× g and 4°C for 60 minutes, followed by collection of the supernatant.
5) The supernatant was filtered through 0.2 μm Vacu Cap filters (Pall Life Sciences, UK) and the filtrate was collected for immediate chromatographic purification.

Chromatographic purification of the Transferases

A column (2.5 x 10 cm) was packed with 50 ml of Chelating Sepharose ff. gel and charged with Ni-sulphate (according to the method described by manufacturer, Amersham Biosciences). The column was equilibrated with 200 ml of 20 mM $NaH_2PO_4$, pH 7.4, 500 mM NaCl, 10 mM Imidazole. 400 ml of crude was applied to the column at a flow rate of 5 ml/min. The column was then washed with 20 mM $NaH_2PO_4$, pH 7.4, 500 mM NaCl, 10 mM Imidazole until the $UV_{280}$ reached the base line. The GCAT was then eluted with 40 ml of 20 mM $NaH_2PO_4$, pH 7.4, 500 mM NaCl and 500 mM Imidazole.

**EXAMPLE 5: Fermentation and Purification of *Aeromonas* lipid acyltransferases produced in *Bacillus subtilis.***

Fermentations

BAC0318-19, BAC0323-24

Microorganism

The microorganisms used in this study originate from transformation of a *Bacillus subtilis* host strain, #163 with a plasmid containing the gene encoding the *Aeromonas salmonicida* transferase inserted in the vector pUB110OIS. The expression of the gene is controlled by an alpha-amylase promoter, and the secretion of the transferase is mediated by the *B. subtilis* xylanase signal sequence (Example 3). The strains were named DIDK0138 (fermentation BAC0318-19) and DIDK0153 (fermentation BAC0323-24).

Growth media and culture conditions

**Pre culture medium**

A shake flask (500 mL total volume, with baffles) was added 100 mL of a medium containing:

| | |
|---|---|
| NaCl | 5 g/L |
| $K_2HPO_4$ | 10 g/L |
| Soy flour | 20 g/L |
| Yeast extract, BioSpringer 106 | 20 g/L |
| Antifoam, SIN260 | 5 mL/L |

pH was adjusted to 7.0 before autoclaving

After autoclaving 6 mL 50% (w/w) Nutriose were added pr flask. Kanamycin was added at a concentration of 50 mg/L after autoclaving.

**Inoculation**

A pre culture shake flask was inoculated with frozen culture directly from a 25% (w/v) glycerol stock. The shake flask was incubated at 33°C and 175 rpm for approximately 16 hours, whereupon 50 mL was used to inoculate the fermentor.

*Fermentations*

The fermentations were carried out in 6 L in house built fermentors.
The batch medium (3 L) contained:

| | |
|---|---|
| Corn steep liquor (50% dw) | 40 g/L |
| Yeast extract BioSpringer 153 (50% dw) | 10 g/L |
| NaCl | 5 g/L |
| $CaCl_2$, $2H_2O$ | 0.25 g/L |
| $Mn(NO_3)_2$, $H_2O$ | 0.2 g/L |
| Antifoam SIN260 | 1 mL/L |
| Kanamycin (filter sterilised to the fermentor after autoclaving | 50 mg/L |

The feed contained:

| | |
|---|---|
| Glucose monohydrate | 540 g/kg |
| $MgSO_4$, $7H_2O$ | 4.8 g/kg |
| Antofoam SIN260 | 4 mL/kg |

(continued)

| | |
|---|---|
| Yeast extract, BioSpringer 153 (50% dw) (autoclaved separately) | 150 g/kg |

The feed in fermentation BAC0318 and BAC0323 was started based on the accumulated $CO_2$, according to the equations below:

$$\text{Feed} - \text{flow}[g/h] = 0, AcCO_2 < 0.15$$

$$\text{Feed} - \text{flow}[g/h] = 2.85 + t \cdot 1.54, AcCO_2 \geq 0.15 \text{ and } t < 12$$

$$\text{Feed} - \text{flow}[g/h] = 21.3, t > 12$$

t time (hours) from the point when the accumulated $CO_2$ ($AcCO_2$) reached 0.15 moles.

The feed in fermentations BAC0319 and BAC0324 was started based on the accumulated $CO_2$, according to the equations below:

$$\text{Feed} - \text{flow}[g/h] = 0, AcCO_2 < 0.15$$

$$\text{Feed} - \text{flow}[g/h] = 2.0 + t \cdot 1.08, AcCO_2 \geq 0.15 \text{ and } t < 12$$

$$\text{Feed} - \text{flow}[g/h] = 15, t > 12$$

t: time (hours) from the point when the accumulated $CO_2$ ($AcCCO_2$) reached 0.15 moles.

The pH was controlled at 7.0 by adding 12.5% (w/v) $NH_3$-water or 2M phosphoric acid.
The aeration was 3 L/min corresponding to 1 wm.
The temperature was 33°C.
The fermentor was equipped with two 8 cm ∅ Rushton impellers placed with a distance of 10 cm.

<u>Harvest</u>

The biomass was removed by centrifugation at 16,000× g for 10 minutes at room temperature. The supernatant was filter sterilized, and the filtrate was used for purification and application tests.

**EXAMPLE 6: Enzymatic removal of DAGs in palm oil catalysed by a lipid acyltransferase from *Aeromonas salmonicidae.***

SUMMARY

The enzymatic glycerolysis experiments were initiated by the addition of the glycerol/transferase solutions to the reactor containing palm oil and glycerol/water in varying concentrations. All the reactions were conducted at 43°C, using magnetic stirring for 24 hours. After reaction, the samples were analyzed using-HPTLC and in some experiments the result was confirmed by GC analysis. The conducted trials showed that there was a good correlation between the water concentration and levels of DAGs and MAGs, respectively: the lowest water concentrations tested gave the highest MAG level and the lowest DAG level.

Based on the trials, it can be concluded that it was possible to reduce the amount of DAGs in palm oil by a transferase-catalysed reaction where the enzyme used DAGs as donor molecules and glycerol as acceptor molecule - in a glycerolysis reaction in which monoglycerides were synthesized. This was in contrast to the glycerolysis reaction with conventional triglyceride hydrolyzing lipases where the amount of DAG increases.

Furthermore, it can be concluded that the water concentration has a significant impact on the synthesized yield of monoglycerides and amount of DAG. The following correlation was found: Low water concentration (<1%) and 5% glycerol gives increased MAG yield and decreased concentration of DAGs. The obtained results also show that primarily 1,2 isomer of diglyceride tend to be reduced.

It is known that diglycerides, especially 1,2 isomers delay the crystallisation of fat. This effect clauses post-crystallization of fat products like margarine and shortenings, which favors formation of large crystals (sandiness). In certain fat blends it was observed that diglycerides improve the stability of β'crystals. A reduction but not a complete removal of diglycerides in palm oil would therefore be preferable. It can thus be concluded that a reduction of diglyceride will result in a better and more uniform oil quality; a fact, which must not be underestimated.

INTRODUCTION

Problems concerning diglycerides depend to some extent on type of product, e.g. whether it is in margarine and shortenings. Thus, depending on the product the presence of diglycerides both have negative and positive effects. For margarine production, only fat product of a required crystal structure can be used in order to obtain proper consistency and plasticity. The β'-crystal form is the most required structure, showing small crystals and a high ability for maintaining the liquid fraction. The most stable crystal form (β-form) is undesirable, as having big crystals causes grained structure of the margarine. According to Hernquist & Anjou (1993) and Wahnelt *et al.* (1991) the presence of diglycerides in fat retard the transition from β' to β-crystals. But as diglycerides retard the transition from β' to β, they also retard the transition from a form to β' form (Walnet *et al.,* 1991). For this reason it can be defined that the presence of diglycerides retards the whole crystallization process. Slow crystallization has a crucial impact on margarine application. The effect of retarding crystallization in margarine blends containing a high portion of palm oil is that after conventional processing the product may be somewhat soft, causing packing difficulties, and subsequent crystallization may lead to a firmer texture than desired (Berger, 1990). In spite of advantages and disadvantages, it may be important to find the right balance between reduced and total removal of diglycerides.

It has surprisingly been found that it is possible to reduce the amount of diglyceride in palm oil by enzymatic glycerolysis of palm oil using a lipid acyltransferase, for example the GDSx lipid acyl transferase from *Aeromonas Salmonicida.* Without wishing to be bound by theory, in this process the enzyme is added to palm oil together with small amount of glycerol and the enzyme catalyses the following reaction:

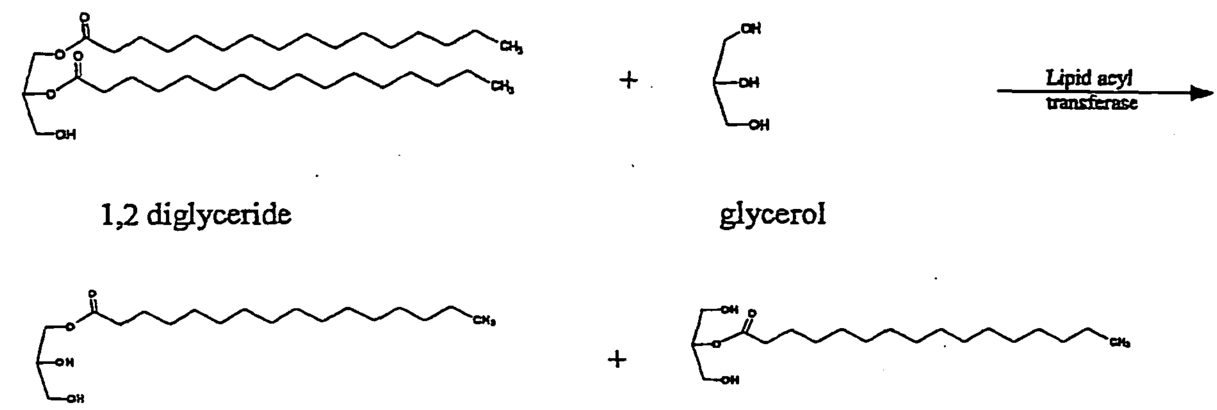


1,2 diglyceride

glycerol

1- monoglyceride

2- monoglyceride

After the reaction the surplus glycerol can; if deemed necessary, easily be separated from the reaction mixture by centrifugation or other processes.

The advantage of this process is that the amount of diglyceride (preferably 1,2 diglyceride) is reduced by a glycerolysis reaction catalysed by an enzyme which is specific for diglyceride without using the triglyceride as donor for the transferase reaction.

The reaction products monoglycerides do not have to be removed from the reaction mixture, but can advantageously be used as an efficient emulsifier for the production of food products like margarine and shortening. The process thus solves two problems. First of all the amount of diglyceride is reduced and preferable 1,2 diglyceride is

removed, which has a negative impact on the crystallisation properties of the triglycerides. Secondly the reaction product monoglyceride can be used as an efficient emulsifier in the production of food products like margarine and shortening.

In one embodiment, it is preferably to remove the monoglycerides produced (if any) by the transferase reaction.

Suitably, if the transferase reaction has been carried out in the crude palm oil, the monoglyceride and/or glycerol and/or residues of water (if any) may be removed by a deodorisation process during the edible oil refining process.

Another very interesting advantage of the use of the lipid acyl transferase is that this enzyme is less dependent of the water content in the reaction mixture and because this enzyme is a transferase low or no hydrolytic activity takes place which means that the amount of free fatty acids do not increase significantly.

It is well known the water content in glycerolysis reactions are very important when lipolytic enzymes like lipases are use in this process (Kristensen, 2004). Even small amount of water, which is necessary for most lipolytic reactions will cause the formation of significant amount of free fatty acids. This problem can be overcome by using the lipid acyl transferase in the glycerolysis reaction.

Another aspect is that lipases known in the art to catalyse glycerolysis reaction, mainly uses triglyceride as donor during formation of diglyceride instead of reducing the amount of diglyceride.

Materials:

| | |
|---|---|
| Palm oil: | Palmotex, Aarhus United, Denmark |
| Glycerol: | J. T. Baker, (7044) |
| DIMODAN® P: | Danisco A/S, Denmark |
| Enzyme: | Lipid acyltransferase GCAT from *Aeromonas salmonicida* expressed in *B. subtilis* and fermented in lab scale (Transferase #196). |

Methods

Lyophilization of enzyme

The enzymes were desalted (PD-10 Desalting columns, Amersham Biosciences) before lyophilization. The desalted enzyme were mixed with glycerol (Enzyme:Glycerol ratio 3,5:1). The sample was lyophilised and added 10% water. The sample contained approximately 20 U (phospholipase units) per gram.

**Determination of phospholipase activity (phospholipase activity assay):**

Substrate

0.6% L-α Phosphatidylcholine 95% Plant (Avanti #4.41601), 0.4% Triton-X 100 (Sigma X-100) and 5 mM $CaCl_2$ was dissolved in 0.05M HEPES buffer pH 7.

Assay procedure:

400 μL substrate was added to an 1.5 mL Eppendorf tube and placed in an Eppendorf Thermomixer at 37°C for 5 minutes. At time t= 0 min, 50 μL enzyme solution was added. Also a blank with water instead of enzyme was analyzed. The sample was mixed at 10* 100 rpm in an Eppendorf Thermomixer at 37°C for 10 minutes. At time t=10 min the Eppendorf tube was placed in another thermomixer at 99°C for 10 minutes to stop the reaction.
Free fatty acid in the samples was analyzed by using the NEFA C kit from WAKO GmbH.
Enzyme activity PLU-7 at pH 7 was calculated as micromole fatty acid produced per minute under assay conditions

Enzyme reaction

Palm oil was reacted with the glycerol-enzyme solution according to the following recipes in Table 1 and Table 2

Table 1: Experiment I

| Enz. no. | Transferase #196 | | | |
|---|---|---|---|---|
| Sample no. | 1 | 2 | 3 | 4 |
| GI (%) | 5 | 5 | 5 | 10 |
| Water(%) | 0 | 1 | 5 | 1 |
| Oil phase (%) | 95 | 94 | 90 | 89 |
| Water in the reaction mixture | 0.5 | 1.5 | 5.5 | 1.5 |

Table 2: Experiment II

| Enz.No. | Transferase #196 | | | | | |
|---|---|---|---|---|---|---|
| Sample no. | 1 | 2 | 3 | 4 | 5 | 6 |
| GI (%) | 5 | 5 | 5 | 10 | 10 | 10 |
| Water (%) | 1 | 5 | 7.5 | 1 | 5 | 7.5 |
| Oil phase (%) | 94 | 90 | 87,5 | 89 | 85 | 82,5 |
| Water in the reaction mixture | 1 | 5 | 7.5. | 1 | 5 | 7.5 |

The palm oil was scaled in a 20 ml Wheaten glass and the glycerol/enzyme and optional water was added. The sample was placed in heating block (Multitherm HP 15 Stheating block heated with differential stirring (15 wells) controlled by a VARIOMAG - Thermomodul 40 ST thermostat) and reacted under the following conditions:

| | | |
|---|---|---|
| Reaction temperature | : | 43 °C |
| Magnetic Stirring. | : | 650 rpm |
| Reaction time | : | 20 hours |

The enzyme in the reaction mixture was inactivated at 97.5°C in 10 min. After reaction the sample were homogenized (Ultra Turrax) for 20 sec. and a homogeneous sample was taken out for further analysis.

HPTLC

Applicator: LINOMAT 5, CAMAG applicator.

HPTLC plate: 10 x 10 cm (Merck no. 1.05633)

The plate was activated before use by drying in an oven at 180°C for 20-30 minutes.

Application: 2,0μl of a 2,0% solution of reacted palm oil dissolved in Chloroform:Methanol (2:1) was applied to the HPTLC plate using LINOMAT 5 applicator.

Running-buffer: P-ether:MTBE:Acetic acid (50:50:1)

Application/Elution time: 8 minutes.

Developing fluid: 6% Cupriacetate in 16% $H_3PO_4$

After elution the plate was dried in an oven at 180°C for 10 minutes, cooled and immersed in the developing fluid and then dried additional in 20 minutes at 180°C. The plate was evaluated visually and scanned (ScanWizard 5) directly.

In Experiment II the components are quantified by Adobe photoshop 6,0 and the amount of MAG and DAG was calculated from calibrations curves of DAG and MAG standard solutions (see Figures 49 & 50)..

Gas chromatography

Perkin Elmer 8420 Capillary Gas Chromatography equipped with WCOT fused silica column 12.5 m x 0,25 mm ID x 0,1μm 5%phenyl-methyl-silicone (CP Sil 8 CB from Crompack).

Carrier: Helium.
Injection: 1.5 μl with split
Detector. FID. 385°C.

| | | | | | |
|---|---|---|---|---|---|
| Oven program | : | 1 | 2 | 3 | 4 |
| Oven temperature, °C | : | 80 | 200 | 240 | 360 |
| Isothermal, time, min. | : | 2 | 0 | 0 | 10 |
| Temperature rate, °C./mm. | : | 20 | 10 | 12 | |

Sample preparation: 50 mg lipid was dissolved in 12ml heptane:pyridine (2:1) containing an internal standard heptadecane, 2 mg/ml. 500 μl of the sample was transferred to a crimp vial. 100 μl MSTFA (N-Methyl-N-trimethylsilyltrifluoracetamid) was added and the reaction was incubated for 15 minutes at 60 °C.

Calculation: Response factors for mono-di-triglycerides, free fatty acids were determined from reference mixtures of these components. Based on these response factors the lipids in the samples were calculated.

Results

Experiment I:

The aim of this experiment was to examine the impact of a diglyceride:glycerol acyltransferase from *Aeromonas salmonicida* on DAGs in palm oil by glycerolysis reaction. It is known that a GCAT is able to transfer fatty acid from lecithin the cholesterol during formation of cholesterolester and lysolecithin. In this study we have investigated the possibility of the enzyme to use DAGs as donor and glycerol as acceptor in order to reduce the amount of DAGs in palm oil and produce monoglyceride.

It is well known in the literature to use enzyme like lipases to catalyze glycerolysis reactions. In these reactions triglycerides is the main substrate and mono-and diglycerides are the reactions products. In these processes the amount of diglycerides increases significantly and the amount of diglycerides produced is on level or high than the amount of monoglyceride produced (Kristensen, 2004).

Four different sample compositions were tested and compared to a reference of palm oil mixed with 5% glycerol but without enzyme and treated in the same way as the samples. Figure 47 shows the result of the HPTLC analysis of the sample from table 1.

The results obtained from the HPTLC analysis shows that the amount of DAGs varies according to the reaction conditions (referring to ratio between GL:$H_2O$). Equal to all the reactions was that the 1,3-isomers of diglyceride were in higher portion than the 1,2-(2,3-) isomers of diglyceride. This observation can be confirmed by theory, which says that the ratio of 1,3-isomer to 1,2- (2,3-) isomer in crude palm oil is 7:3 (Siew & Ng, 1999; Timms, 2004). Furthermore, analyzing the result, the HPTLC plate shows that the transferease successfully reduces the amount of DAGs. The reduction can to some extent correlate to the amount of synthesized MONO.

If we compare the 1,2 isomer with the 1,3 isomer DAGs in figure 1 it appears that the 1,2 DAGs are primarily reduced by the enzyme-catalyzed reaction. This is in agreement with the transferease has a preferred specificity for fatty acids in sn2-position.

In experiments conducted different water concentrations in range of 0.5-5.5% were used. It appears from the result that the water concentration has a significant impact on the synthesized amount of MONO combined to the reduced amount of DAGs. In this instance transferase #196 shows that if the system contains low water concentration increased concentration of MONO is formed and corresponding decreased amount of DAG is observed. The experiment also investigates whether the amount of glycerol has an effect on the equilibrium in the system (Lane 5: 10% GL:1% $H_2O$). Transferease #196 shows that higher concentration of glycerol - also meaning double dosage of enzyme activity - dose not result in higher MONO concentration.

Preferably the present invention is carried out in a low water environment i.e. less than about 1%.

One of the advantages working with transfereases instead of lipases is that the synthesis of MONO dose not

correlates to excessive increased in the concentration of FFA. This fact is confirmed in present experiment Figure 1 shows that none of the reactions shows clear band of FFA (FFA band was expected to be visible between 1,3 DAGs and TAGs).

From this experiment it can be summarized that the optimal concentration of glycerol and water for Transferase #196 is as following:

Transferase #196: 5%GL:0.5% $H_2O$

The sample procedure in HPTLC analysis did not include specific weighing, because of that it was not possible to calculate the concentrations of the different components in the reaction mixtures. Because of that the observation is solely based on visual evaluation.

Result GC

Based on the HPTLC results sample No. 2 was selected for GC analysis. To find out whether the visual evaluation of the HPTLC plates could be confirmed by quantitative analyses of DAG. Before analysis glycerol was removed from the sample by centrifugation and only the lipid phase was exposed to GC analysis The GC results are presented in Table 3 below.

Table 3: Result of GC on selected reaction in Experiment I

| | Ref. | #196 |
|---|---|---|
| Sample No. | 1 | 2 |
| Gl (%)* | 5 | 5 |
| Water (%) | 0 | 0 |
| Oil phase (%) | 95 | 95 |
| GC results | | |
| MONO | <0,01 | 0.34 |
| DAG | 6.21 | 5.77 |
| * Glycerol contains 10% water | | |

Analyzing the result of the GC study it was found that the selected sample differ from the reference in higher MONO content and lower DAG yield. This observation supports the obtained results in the HPTLC analysis.

Conclusion: Experiment I

Concerning content of monoglycerides and diglycerides in the product of glycerolysis, the results of HPTLC analysis indicated that there is a correlation between the concentration of water and the amounts of MONO and DAGs, respectively: The lower concentration of water, the lower DAGs and higher MONO.

GC analysis confirmed degree of reduction in DAG. In this experiment the reduced amount of DAGs counts for 7.1% of the total amount of diglycerides palm oil (Table 4).

Table 4: Degree of reduction of DAGs

| Enz. No. | #196 |
|---|---|
| Sample no. | 2 |
| Reduction of DAGs (%) | 7,1 |

Experiment II:

The aim of this part was to continue the optimization of DAG reduction in palm oil and analyze the transferase-catalysed glycerolysis using Transferease #196. The primarily purpose is to reach a well-balanced reaction in which the amount of DAG has been reduced in time with increase concentration of MONO.

In this part of the experimental work six different sample compositions were tested and compared to a reference of palm oil mixed with 5% glycerol (Table 2). The reference was exposed to the same heating profile as the enzyme reactions, which makes it possible to observe and determine the degree of thermal degradation during reduction. Figure 48 shows the result of the HPTLC analysis.

It appears from the results in figure 48 that especially the amount of MAG is varying. The HPTLC analysis shows that in samples containing 5% glycerol (Lane: 1-3) there is continuous relation between water concentration and MAG yield: Decreased amount of water in the reaction mixture is followed by increased MAG yield. The same tendency is observed in the reactions containing 10% glycerol (Lane: 5-7).

According to the amount of diglycerides, it was not possible to differentiate the samples solely based on visual evaluation of the HPTLC plate. Therefore, to be able to distinguish between the samples, components were quantified by analyzing standard materials with known composition of MAG and DAG. The HPTLC plates were scanned and handle by Adobe Photoshop 6.0 and further calculated by help from a constructed calculation macro (Microsoft Excel 2000). The quantitative analysis of the components analyzed by HPTLC is presented in Table 5 below.

Table 5:

| Enz. No. | Transferase # 196 | | | | | | |
|---|---|---|---|---|---|---|---|
| Run order: HPTLC | 1 | 2 | 3 | 4 | 5 | 6 | 7 |
| MONO (%) | 0,38 | 0.01 | Nm*. | 0.01 | 0.15 | 0.07 | 0.03 |
| DAG (%) | 7.24 | 8.13 | Nm*. | 8.35 | 826 | 7.88 | 7.62 |
| Reduction of DAG (%) | 13,29 | 2,63 | - | - | 1,08 | 5,63 | 8,74 |
| Nm* Not measured<br>- not calculated. | | | | | | | |

The investigation supports the visual evaluation and gives a fine picture of the varying level of diglycerides. It is seen from the results that the highest degree of reduction in the amount of DAGs was achieved in sample no. 1 (5% GL:1.5% $H_2O$). Further it is observed that this sample also contains the highest amount of MAG.

Conclusion: Experiment II

Experiment II confirmed the observations that a lipid acyltransferase GCAT from *Aeromonas salmonicida* was able to reduce the amounts of diglycerides in palm oil. For the experiments with 5% glycerol a correlation was found between the concentration of water in the reaction mixture and the amounts of MAG and DAGs, respectively: The lower concentration of water, the lower DAGs and higher MAG.

HPTLC quantified the degree of reduction (see Table 5). It appears from the results, that the reduced amount of DAGs counts for 13,29% of the total amount of diglycerides in palm oil and the amount of synthesized MONO raised from 0,01% (ref.) to 0.38% (sample No. 1).

From this experiment it can be summarized that the optimal concentration of glycerol and water for Transferase #196 is as following:

Transferase #196: 5%GL:1.5% $H_2O$

Comparing these observations with the result gained in Experiment I, it is confirmed that good consistency in the results is obtained. The optimal concentration of water is presumably between 0-1% $H_2O$ depending on enzyme activity and content of glycerol.

Based on the two experiments conducted, it can be concluded that it is possible to reduce the amount of DAGs in palm oil by a transferase-catalysed reaction, where the enzyme uses DAGs as donor molecules and glycerol as acceptor molecule, in a glycerolysis reaction in which mono glycerides is synthesized. This is in strong contrast to the glycerolysis reaction with conventional triglycerides hydrolysing lipases where the amount of DAG increases due to the partial hydrolysis activity of the triglyceride hydrolyzing lipase.

Based on the fact that the structure of diglycerides is a mixture 1,2- and 1,3-isomers and that the transferase is specific to the sn2-position, even a small reduction in the amount of diglyceride will have a huge physical impact on palm-based products.

**EXAMPLE 7: Immobilisation of a diacylglycerol:glycerol transferase (an lipid acyltransferase according to the present invention) from *Aeromonas salmonicida.***

The diacylglycerol:glycerol transferase is immobilised on Celite by acetone precipitation. 10 ml enzyme solution in 20 mM TEA buffer pH 7 is agitated slowly with 0,1 gram Celite 535 (from Fluka) for 2 hours at room temperature.
50ml cool acetone is added during continued agitation.
The precipitate is isolated by centrifugation 5000 g for 1 minute.
The precipitate is washed 2 times with 20ml cold acetone.
The Celite is tried at ambient temperature for about 1 hour

The immobilised transferase is tested in palm oil (see table below):

**Table**

| | % |
|---|---|
| Palm oil | 92.5 |
| Glycerol | 5 |
| Immobilised diacylglycerol:glycerol On Celite, #178, 45 U/g | 2.0 |
| Water | 0.5 |

Palm oil and glycerol is heated to 42°C. The immobilised transferase was added.

The transferase reaction continued at 42°C during gentle agitation with a magnetic stirrer. Samples are taken out for analyses after ½, 1, 3, 6 and 24 hours and analysed by HPTLC. The reaction is stopped after 24 hours reaction time and the immobilised enzyme was filtered off.

The HPTLC analysis clearly shows the effect of the immobilised diacylglycerol:glycerol from *A. Salmonicida* by the formation of monoglyceride and reduction of diglyceride in palm oil.

REFERENCES

Amano Enzyme Inc. (2004). http://www.amano-enzyme.co.jp/english/productuse/oil_fat.html Dato: 21.06.04

Fødevareministeriet (2003). Bekendtgørelse om indhold af transfedtsyrer i olier og fedtstoffer. Bekendtgørelse nr. 160 af 11/03/2003

Directive 2000/36/EC. http://europa.eu.int/scadplus/leg/en/lvb/121122b.htm. Dato: 16.06.04

Karlshamns, (2004). Press information. http://wvw.karlshamns.com/investor/press_information-show.asp?ID=319. Dato: 16.06.04

Berger, K. G. (1990). Recent developments in palm oil. In Oleagineux 45:437-4.43 Drozdowski, B. (1994). General characteristics of eatable fats. WNT, Warszawa, Poland, pp. 240-243 (In polish)

Hernquist. L. & Anjou, K. (1983). Diglycerides as a stabilizer of the β'-crystal Form in Margarines and Fats. In Fette Seifen Anstrichmittel. 2:64-66

Hernquist, L. Herslof, B. Larsson, K. & Podlaha, O (1981). Polymorphism of rapeseed oil with low content of erucic acid and possibilities to stabilize the β' crystal form in fats. In Journal of Science and Food Agriculture. 32:1197-1202

Jacobsberg, B. & Oh, C.H. (1976). Studies in Palm Oil Crystallisation. In Journal of the American Oil Chemist Society. 53:609-616

Kristensen, A. C. J. (2004). Preparation of margarine and spreads by enzyme-generated emulsifiers. Master thesis, The Royal Veterinary and Agricultural University, Frederiksberg, Copenhagen

McNeill, G. P. & Berger, R. G. (1993). Enzymatic glycerolysis of palm oil fractions and palm oil based model mixture: Relationship between fatty acid composition and monoglyceride yield. In Food Biotechnology 7:75-87

Okiy, D. A. (1977). Partial glycerides and palm oil Crystallisation. In Journal of Science and Food Agriculture. 28:955

Okiy, D. A. (1978). Interaction of triglycerides and diglycerides of palm oil. In Oleagineux. 33:625-628

Qkiy, D. A., Wright, W. B., Berger, K. G. & Morton, I. D. (1978). The physical properties of modified palm oil: In Journal of Science of Food and Agriculture. 29:625-628

Walnett, S. V., Meusel, D. & Tülsner, M. (1991). Zur kenntnis des diglyceride influsses auf das kristallisanonsverhalten von Fetten. In Fat Science Technology. 4:117-121

Siew, N. L. (2001). Understanding the Interactions of Diacylglycerols with oil for better Product performance. Paper presented at the 2001 PIPOC International Palm Oil Congress - Chemistry and Technology Conference. 20-23

August 2001, Kuala Lumpur, Malaysia
Siew, N. L. & Ng, W. L. (2000). Differential scanning thermograms of palm oil diglycerides in the presence of diglycerides. In Journal of Oil Palm Research. 12:1-7 Siew, N. L. & Ng, W. L. (1999). Influence of diglycerides on crystalisation of palm oil. In Journal of Science of Food and Agriculture. 79:722-726
Sonntag, N. O. V. (1982a). Fat splitting, esterification and interesterification. Bailey's Industrial Oils and Fat products. Vol. 2, 4th edn. John Wiley and Sons, New york pp. 97-173
Sonntag, N. O. V. (1982b). Glycerolysis of Fats and methyl esters - status, review and critique. In Journal ofAmerican Oil Chemist Society. 59: 795A-802A
Timms, R. (2004). Oral presentation (Lecture: Trends & delvlopment) at Danisco A/S, Brabrand, Denmark.

BUDAPEST TREATY ON THE INTERNATIONAL RECOGNITION OF THE DEPOSIT OF MICROORGANISMS FOR THE PURPOSES OF PATENT PROCEDURE

Danisco A/S
Langebrogade 1
DK-1001 Copenhagen
Denmark

NAME AND ADDRESS OF DEPOSITOR

INTERNATIONAL FORM

RECEIPT IN THE CASE OF AN ORIGINAL DEPOSIT issued pursuant to Rule 7.1 by the INTERNATIONAL DEPOSITARY AUTHORITY identified at the bottom of this page

**I. IDENTIFICATION OF THE MICROORGANISM**

Identification reference given by the DEPOSITOR:

*Escherichia coli*
TOP10pPet12aAhydro

Accession number given by the INTERNATIONAL DEPOSITARY AUTHORITY:

NCIMB 41204

**II. SCIENTIFIC DESCRIPTION AND/OR PROPOSED TAXONOMIC DESIGNATION**

The microorganism identified under I above was accompanied by:

[ ] a scientific description

[X] a proposed taxonomic designation

(Mark with a cross where applicable)

**III. RECEIPT AND ACCEPTANCE**

This International Depositary Authority accepts the microorganism identified under I above, which was received by it on 22 December 2003 (date of the original deposit)[1]

**IV. RECEIPT OF REQUEST FOR CONVERSION**

The microorganism identified under I above was received by this International Depositary Authority on (date of the original deposit) and a request to convert the original deposit to a deposit under the Budapest Treaty was received by it on (date of receipt of request for conversion)

**V. INTERNATIONAL DEPOSITARY AUTHORITY**

Name: NCIMB Ltd.,

Address: 23 St Machar Drive
Aberdeen
AB24 3RY
Scotland, UK.

Signature(s) of person(s) having the power to represent the International Depositary Authority or of authorised official(s):

Terence [signature]

Date: 9 January 2004

[1] Where Rule 6/4(d) applies, such date is the date on which the status of International Depositary Authority was acquired.

Form BP/4 (sole page)

BUDAPEST TREATY ON THE INTERNATIONAL RECOGNITION OF THE DEPOSIT OF MICROORGANISMS FOR THE PURPOSES OF PATENT PROCEDURE

Danisco A/S
Langebrogade 1
DK-1001 Copenhagen
Denmark

NAME AND ADDRESS OF THE PARTY TO WHOM THE VIABILITY STATEMENT IS ISSUED

INTERNATIONAL FORM

VIABILITY STATEMENT
issued pursuant to Rule 10.2 by the INTERNATIONAL DEPOSITARY AUTHORITY identified on the following page

| I. DEPOSITOR | II. IDENTIFICATION OF THE MICROORGANISM |
|---|---|
| Name: AS ABOVE<br>Address: | Accession number given by the INTERNATIONAL DEPOSITARY AUTHORITY:<br>NCIMB 41204<br>Date of the deposit or of the transfer[1]:<br>22 December 2003 |

**III. VIABILITY STATEMENT**

The viability of the microorganism identified under II above was tested on 22 December 2003 [2]. On that date, the said microorganism was:

[3]

- [X] viable
- [ ] no longer viable

[1] Indicate the date of the original deposit or, where a new deposit or a transfer has been made, the most recent relevant date (date of the new deposit or date of the transfer).

[2] In the cases referred to in Rule 10.2(a)(ii) and (iii), refer to the most recent viability test.

[3] Mark with a cross the applicable box.

| | |
|---|---|
| IV. CONDITIONS UNDER WHICH THE VIABILITY TEST HAS BEEN PERFORMED[4] | |
| | |
| V. INTERNATIONAL DEPOSITARY AUTHORITY | |
| Name: NCIMB Ltd.,<br>Address: 23 St Machar Drive<br>Aberdeen<br>AB24 3RY<br>Scotland | Signature(s) of person(s) having the power to represent the International Depositary Authority or of authorised official(s):<br>Date: 9 January 2004 |

[4] Fill in if the information has been requested and if the results of the test were negative.

BUDAPEST TREATY ON THE INTERNATIONAL RECOGNITION OF THE DEPOSIT OF MICROORGANISMS FOR THE PURPOSES OF PATENT PROCEDURE

INTERNATIONAL FORM

RECEIPT IN THE CASE OF AN ORIGINAL DEPOSIT issued pursuant to Rule 7.1 by the INTERNATIONAL DEPOSITARY AUTHORITY identified at the bottom of this page

Danisco A/S
Langebrogade 1
DK-1001 Copenhagen
Denmark

NAME AND ADDRESS OF DEPOSITOR

| I. IDENTIFICATION OF THE MICROORGANISM | |
|---|---|
| Identification reference given by the DEPOSITOR: | Accession number given by the INTERNATIONAL DEPOSITARY AUTHORITY: |
| *Escherichia coli* TOP10pPet12aAsalmo | NCIMB 41205 |

**II. SCIENTIFIC DESCRIPTION AND/OR PROPOSED TAXONOMIC DESIGNATION**

The microorganism identified under I above was accompanied by:

☐ a scientific description

☒ a proposed taxonomic designation

(Mark with a cross where applicable)

**III. RECEIPT AND ACCEPTANCE**

This International Depositary Authority accepts the microorganism identified under I above, which was received by it on 22 December 2003 (date of the original deposit)[1]

**IV. RECEIPT OF REQUEST FOR CONVERSION**

The microorganism identified under I above was received by this International Depositary Authority on (date of the original deposit) and a request to convert the original deposit to a deposit under the Budapest Treaty was received by it on (date of receipt of request for conversion)

**V. INTERNATIONAL DEPOSITARY AUTHORITY**

Name: NCIMB Ltd.,

Address: 23 St Machar Drive
Aberdeen
AB24 3RY
Scotland, UK.

Signature(s) of person(s) having the power to represent the International Depositary Authority or of authorised official(s):

Date: 9 January 2004

[1] Where Rule 6/4(d) applies, such date is the date on which the status of International Depositary Authority was acquired.

BUDAPEST TREATY ON THE INTERNATIONAL RECOGNITION OF THE DEPOSIT OF MICROORGANISMS FOR THE PURPOSES OF PATENT PROCEDURE

| | |
|---|---|
| Danisco A/S<br>Langebrogade 1<br>DK-1001 Copenhagen<br>Denmark | INTERNATIONAL FORM<br><br>VIABILITY STATEMENT<br>issued pursuant to Rule 10.2 by the<br>INTERNATIONAL DEPOSITARY AUTHORITY<br>identified on the following page |

NAME AND ADDRESS OF THE PARTY TO WHOM THE VIABILITY STATEMENT IS ISSUED

| I. DEPOSITOR | II. IDENTIFICATION OF THE MICROORGANISM |
|---|---|
| Name: AS ABOVE<br>Address: | Accession number given by the INTERNATIONAL DEPOSITARY AUTHORITY:<br>NCIMB 41205<br>Date of the deposit or of the transfer[1]:<br>22 December 2003 |

III. VIABILITY STATEMENT

The viability of the microorganism identified under II above was tested on 22 December 2003 [2]. On that date, the said microorganism was:

[X][3] viable

[ ][3] no longer viable

[1] Indicate the date of the original deposit or, where a new deposit or a transfer has been made, the most recent relevant date (date of the new deposit or date of the transfer).

[2] In the cases referred to in Rule 10.2(a)(ii) and (iii), refer to the most recent viability test.

[3] Mark with a cross the applicable box.

| | |
|---|---|
| IV. CONDITIONS UNDER WHICH THE VIABILITY TEST HAS BEEN PERFORMED[4] | |
| | |
| V. INTERNATIONAL DEPOSITARY AUTHORITY | |
| Name: NCIMB Ltd.,<br>Address: 23 St Machar Drive<br>Aberdeen<br>AB24 3RY<br>Scotland | Signature(s) of person(s) having the power to represent the International Depositary Authority or of authorised official(s):<br>Date: 9 January 2004 |

[4] Fill in if the information has been requested and if the results of the test were negative.

## Claims

1. A method of reducing and/or removing diglyceride from an edible oil, comprising a) admixing an edible oil with an acyl acceptor substrate and a fatty-acid CoA independent diglyceride:glycerol acyltransferase, wherein the fatty-acid CoA independent diglyceride:glycerol acyltransferase is **characterized** as an enzyme of class E.C. 2.3.1.x which in an edible oil transfers an acyl group from a diglyceride to glycerol, wherein x is any subgroup, but which is not an enzyme classified as E.C.2.3.1.20 or E.C.2.3.1.158.

2. A method according to claim 1 wherein the fatty-acid CoA independent diglyceride:glycerol acyltransferase enzyme comprises H-309 or comprises a histidine residue at a position corresponding to His-309 in the amino acid sequence of the *Aeromonas hydrophila* lipolytic enzyme shown as SEQ ID No. 2 or SEQ ID No. 32.

3. A method according to any one of the preceding claims wherein the fatty-acid CoA independent diglyceride:glycerol acyltransferase is obtainable from an organism from one or more of the following genera: *Aeromonas, Streptomyces, Saccharomyces, Lactococcus, Mycobacterium, Streptococcus, Lactobacillus, Desulfitobacterium, Bacillus, Campylobacter, Vibrionaceae, Xylella, Sulfolobus, Aspergillus, Schizosaccharomyces, Listeria, Neisseria, Mesorhizobium, Ralstonia, Xanthomonas and Candida.*

4. A method according to any one of the preceding claims comprising admixing the treated edible oil with one or more food constituents to formulate a foodstuff.

5. Use of a fatty-acid CoA independent diglyceride:glycerol acyltransferase **characterized** as an enzyme of class E.C. 2.3.1.x which in an edible oil transfers an acyl group from a diglyceride to glycerol in the manufacture of an edible oil, for reducing and/or removing diglyceride from said edible oil, wherein x is any subgroup, but which is not an enzyme classified as E.C.2.3.1.20 or E.C.2.3.1.158.

6. Use of a fatty-acid CoA independent diglyceride:glycerol acyltransferase **characterized** as an enzyme of class E.C. 2.3.1.x which in an edible oil transfers an acyl group from a diglyceride to glycerol in the manufacture of a foodstuff comprising an edible oil for improving the crystallization properties of said foodstuff, wherein x is any subgroup, but which is not an enzyme classified as E.C.2.3.1.20 or E.C.2.3.1.158.

7. Use according to any one of claims 5 or 6 wherein the amount of diglyceride in the edible oil is reduced.

8. Use according to any one of claims 5-7 wherein the fatty-acid CoA independent diglyceride:glycerol acyltransferase enzyme comprises H-309 or comprises a histidine residue at a position corresponding to His-309 in the amino acid sequence of the *Aeromonas hydrophila* lipolytic enzyme shown as SEQ ID No. 2 or SEQ ID No. 32.

9. Use according to any one of claims 5-8 wherein the fatty-acid CoA independent diglyceride:glycerol acyltransferase is obtainable from an organism from one or more of the following genera: *Aeromonas, Streptomyces, Saccharomyces, Lactococcus, Mycobacterium, Streptococcus, Lactobacillus, Desulfitobacterium, Bacillus, Campylobacter, Vibrionaceae, Xylella, Sulfolobus, Aspergillus, Schizosaccharomyces, Listeria, Neisseria, Mesorhizobium, Ralstonia, Xanthomonas and Candida.*

10. Use according to any one of claims 6-9 wherein the fatty-acid CoA independent diglyceride:glycerol acyltransferase is used in combination with a crystallization inhibitor.

## Patentansprüche

1. Verfahren zum Reduzieren und/oder Entfernen von Diglycerid in/aus einem Speiseöl, bei dem man ein Speiseöl mit einem Acylakzeptorsubstrat und einer Fettsäure-CoA-unabhängigen Diglycerid:Glycerol-Acyltransferase mischt, wobei die Fettsäure-CoA-unabhängige Diglycerid:Glycerol-Acyltransferase als ein Enzym der Klasse F.C. 2.3.1.x

charakterisiert ist, welches in einem Speiseöl eine Acylgruppe von einem Diglycerid auf ein Glycerol überträgt, wobei x eine beliebige Untergruppe ist, welche jedoch kein Enzym ist, das als Klasse E.C.2.3.1.20 oder E.C.2.3.1.158 klassifiziert ist.

**2.** Verfahren nach Anspruch 1, wobei das Fettsäure-CoA-unabhängige Diglycerid:Glycerol-Acyltransferaseenzym in einer Position, die mit His-309 in der Aminosäuresequenz des lipolytischen Enzyms von *Aeromonas hydrophila,* wie sie in SEQ ID Nr. 2 oder SEQ ID Nr. 32 dargestellt ist, korrespondiert H-309 umfasst oder einen Histidinrest umfasst.

**3.** Verfahren nach einem der vorangehenden Ansprüche, wobei die Fettsäure-CoA-unabhängige Diglycerid:Glycerol-Acyltransferase erhältlich ist aus einem Organismus aus einer oder mehreren der folgenden Gattungen: *Aeromonas, Streptomyces, Saccharomyces, Lactococcus, Mycobacterium, Streptococcus, Lactobacillus, Desulfitobacterium, Bacillus, Campylobacter, Vibrionaceae, Xylella, Sulfolobus, Aspergillus, Schizosaccharomyces, Listeria, Neisseria, Mesorhizobium, Ralstonia, Xanthomonas* und *Candida.*

**4.** Verfahren nach einem der vorangehenden Ansprüche, bei dem man das behandelte Speiseöl mit einem oder mehreren Nahrungsmittelbestandteilen mischt, um so ein Nahrungsmittel zu formulieren.

**5.** Verwendung einer Fettsäure-CoA-unabhängigen Diglycerid:Glycerol-Acyltransferase, die als ein Enzym der Klasse E.C. 2.3.1.x charakterisiert ist, welches in einem Speiseöl eine Acylgruppe von einem Diglycerid auf Glycerol überträgt, bei der Herstellung eines Speiseöls, um in/aus diesem Speiseöl Diglycerid zu reduzieren und/oder zu entfernen, wobei x eine beliebige Untergruppe ist, welche jedoch kein Enzym ist, das als E.C.2.3.1.20 oder E.C.2.3.1.158 klassifiziert ist.

**6.** Verwendung einer Fettsäure-CoA-unabhängigen Diglycerid:Glycerol-Acyltransferase, die als ein Enzym der Klasse E.C. 2.3.1.x charakterisiert ist, welches in einem Speiseöl eine Acylgruppe von einem Diglycerid auf ein Glycerol überträgt, bei der Herstellung eines Nachrungsmittels, umfassend ein Speiseöl, zum Verbessern der Kristallisationseigenschaften des Nahrungsmittels, wobei x eine beliebige Untergruppe ist, welche jedoch kein Enzym ist, das als E.C.2.3.1.20 oder E.C.2.3.1.158 klassifiziert ist.

**7.** Verwendung nach einem der Ansprüche 5 oder 6, wobei die Menge an Diglycerin in dem Speiseöl reduziert wird.

**8.** Verwendung nach einem der Ansprüche 5-7, wobei das Fettsäure-CoA-unabhängige Diglycerid:Glycerol-Acyltransferaseenzym in einer Position, die mit His-309 in der Aminosäuresequenz des lipolytischen Enzyms von *Aeromonas hydrophila,* wie es in SEQ ID Nr. 2 oder SEQ ID Nr. 32 dargestellt ist, korrespondiert H-309 umfasst oder einen Histidinrest umfasst.

**9.** Verwendung nach einem der Ansprüche 5-8, wobei die Fettsäure-CoA-unabhängige Diglycerid:Glycerol-Acyltransferase erhältlich ist aus einem Organismus aus einer oder mehreren der folgenden Gattungen: *Aeromonas, Streptomyces, Saccharomyces, Lactococcus, Mycobacterium, Streptococcus, Lactobacillus, Desulfitobacterium, Bacillus, Campylobacter, Vibrionaceae, Xylella, Sulfolobus, Aspergillus, Schizosaccharomyces, Listeria, Neisseria, Mesorhizobium, Ralstonia, Xanthomonas* und *Candida.*

**10.** Verwendung nach einem der Ansprüche 6-9, wobei die Fettsäure-CoA-unabhängige Diglycerid:Glycerol-Acyltransferase in Kombination mit einem Kristallisationsinhibitor verwendet wird.

## Revendications

**1.** Procédé de réduction et/ou d'élimination de diglycéride d'une huile alimentaire, comprenant l'étape de a) mélanger une huile alimentaire avec un substrat accepteur d'acyle et une diglycéride/glycérol acyltransférase coenzyme A-acide gras-indépendante, dans lequel la diglycéride/glycérol acyltransférase coenzyme A-acide gras-indépendante est **caractérisée** comme étant une enzyme de classe E.C.2.3.1.x qui, dans une huile alimentaire, transfère un groupe acyle d'un diglycéride à un glycérol, dans lequel x est un sous-groupe quelconque, mais qui n'est pas une enzyme classée comme E.C.2.3.1.20 ou E.C.2.3.1.158.

**2.** Procédé selon la revendication 1, dans lequel l'enzyme diglycéride/glycérol acyltransférase coenzyme A-acide gras-indépendante comprend H-309 ou comprend un résidu histidine à une position correspondant à His-309 dans la

séquence d'acides aminés de l'enzyme lipolytique de *Aeromonas hydrophila* décrite dans SEQ ID N° : 2 ou SEQ ID N° : 32.

**3.** Procédé selon l'une quelconque des revendications précédentes, dans lequel la diglycéride/glycérol acyltransférase coenzyme A-acide gras-indépendante est susceptible d'être obtenue à partir d'un organisme d'un ou plusieurs des genres suivants : *Aeromonas, Streptomyces, Saccharomyces, Lactococcus, Mycobacterium, Streptococcus, Lactobacillus, Desulfitobacterium, Bacillus, Campylobacter, Vibrionaceae, Xylella, Sulfolobus, Aspergillus, Schizosaccharomyces, Listera, Neisseria, Mesorhizobium, Ralstonia, Xanthomonas* et *Candida.*

**4.** Procédé selon l'une quelconque des revendications précédentes, comprenant le mélange de l'huile alimentaire traitée avec un ou plusieurs constituants alimentaires pour formuler une denrée alimentaire.

**5.** Utilisation d'une diglycéride/glycérol acyltransférase coenzyme A-acide gras-indépendante **caractérisée** comme étant une enzyme de classe E.C.2.3.1.x qui, dans une huile alimentaire, transfère un groupe acyle d'un diglycéride à un glycérol, dans la fabrication d'une huile alimentaire, pour réduire et/ou éliminer le diglycéride de ladite huile alimentaire, dans laquelle x est un sous-groupe quelconque, mais qui n'est pas une enzyme classée comme E.C. 2.3.1.20 ou E.C.2.3.1.158.

**6.** Utilisation d'une diglycéride/glycérol acyltransférase coenzyme A-acide gras-indépendante **caractérisée** comme étant une enzyme de classe E.C.2.3.1.x qui, dans une huile alimentaire, transfère un groupe acyle d'un diglycéride à un glycérol, dans la fabrication d'une denrée alimentaire comprenant une huile alimentaire pour améliorer les propriétés de cristallisation de ladite denrée alimentaire, dans laquelle x est un sous-groupe quelconque, mais qui n'est pas une enzyme classée comme E.C.2.3.1.20 ou E.C.2.3.1.158.

**7.** Utilisation selon l'une quelconque des revendications 5 ou 6, dans laquelle la quantité de diglycéride dans l'huile alimentaire est réduite.

**8.** Utilisation selon l'une quelconque des revendications 5 à 7, dans laquelle l'enzyme diglycéride/glycérol acyltransférase coenzyme A-acide gras-indépendante comprend H-309 ou comprend un résidu histidine à une position correspondant à His-309 dans la séquence d'acides aminés de l'enzyme lipolytique de *Aeromonas hydrophila* décrite dans SEQ ID N° : 2 ou SEQ ID N° : 32.

**9.** Utilisation selon l'une quelconque des revendications 5 à 8, dans laquelle la diglycéride/glycérol acyltransférase coenzyme A-acide gras-indépendante est susceptible d'être obtenue à partir d'un organisme d'un ou plusieurs des genres suivants : *Aeromonas, Streptomyces, Saccharomyces, Lactococcus, Mycobacterium, Streptococcus, Lactobacillus, Desulfitobacterium, Bacillus, Campylobacter, Vibrionaceae, Xylella, Sulfolobus, Aspergillus, Schizosaccharomyces, Listeria, Neisseria, Mesorhizobium, Ralstonia, Xanthomonas* et *Candida.*

**10.** Utilisation selon l'une quelconque des revendications 6 à 9, dans laquelle la diglycéride/glycérol acyltransférase coenzyme A-acide gras-indépendante est utilisée en combinaison avec un inhibiteur de cristallisation.

Figure 1

SEQ ID No. 1

```
  1 ivafGDSlTd geayygdsdg ggwgagladr Ltallrlrar prgvdvfnrg isGrtsdGrl
 61 ivDalvallF laqslglpnL pPYLsgdflr GANFAsagAt Ilptsgpfli QvqFkdfksq
121 vlelrqalgl lqellrllpv ldakspdlvt imiGtNDlit saffgpkste sdrnvsvpef
181 kdnlrqlikr Lrsnngarii vlitlvilnl gplGClPlkl alalassknv dasgclerln
241 eavadfneal relaiskled qlrkdglpdv kgadvpyvDl ysifqdldgi qnpsayvyGF
301 ettkaCCGyG gryNynrvCG naglcnvtak aCnpssylls flfwDgfHps ekGykavAea
361 l
```

Figure 2

SEQ ID No. 2

```
  1 mkkwfvcllg lvaltvqaad srpafsrivm fgdslsdtgk myskmrgylp ssppyyegrf
 61 sngpvwleql tnefpgltia neaeggptav aynkiswnpk yqvinnldye vtqflqkdsf
121 kpddlvilwv gandylaygw nteqdakrvr daisdaanrm vlngakeill fnlpdlgqnp
181 sarsqkvvea ashvsayhnq lllnlarqla ptgmvklfei dkqfaemlrd pqnfglsdqr
241 nacyggsyvw kpfasrsast dsqlsafnpq erlaiagnpl laqavaspma arsastlnce
301 gkmfwdqvhp ttvvhaalse paatfiesqy eflah
```

Figure 3

SEQ ID No. 3

```
  1 mkkwfvcllg lialtvqaad trpafsrivm fgdslsdtgk myskmrgylp ssppyyegrf
 61 sngpvwleql tkqfpgltia neaeggatav aynkiswnpk yqvynnldye vtqflqkdsf
121 kpddlvilwv gandylaygw nteqdakrvr daisdaanrm vlngakqill fnlpdlgqnp
181 sarsqkvvea vshvsayhnk lllnlarqla ptgmvklfei dkqfaemlrd pqnfglsdve
241 npcydggyvw kpfatrsvst drqlsafspq erlaiagnpl laqavaspma rrsasplnce
301 gkmfwdqvhp ttvvhaalse raatfietqy eflahg
```

Figure 4

SEQ ID No. 4

```
  1 mpkpalrrvm tatvaavgtl algltdatah aapaqatptl dyvalgdsys agsgvlpvdp
 61 anllclrsta nyphviadtt garltdvtcg aaqtadftra qypgvapqld algtgtdlvt
121 ltiggndnst finaitacgt agvlsggkgs pckdrhgtsf ddeieantyp alkeallgvr
181 arapharvaa lgypwitpat adpscflklp laagdvpylr aiqahlndav rraaeetgat
241 yvdfsgvsdg hdaceapgtr wiepllfghs lvpvhpnalg errmaehtmd vlgld
```

Figure 5

SEQ ID No. 5

```
  1 mpkpalrrvm tatvaavgtl algltdatah aapaqatptl dyvalgdsys agsgvlpvdp
 61 anllclrsta nyphviadtt garltdvtcg aaqtadftra qypgvapqld algtgtdlvt
121 ltiggndnst finaitacgt agvlsggkgs pckdrhgtsf ddeieantyp alkeallgvr
181 arapharvaa lgypwitpat adpscflklp laagdvpylr aiqahlndav rraaeetgat
241 yvdfsgvsdg hdaceapgtr wiepllfghs lvpvhpnalg errmaehtmd vlgld
```

Figure 6

SEQ ID No. 6

```
  1 mdyekfllfg dsitefafnt rpiedgkdqy algaalvney trkmdilqrg fkgytsrwal
 61 kilpeilkhe snivmatifl gandacsagp qsvplpefid nirqmvslmk syhirpiiig
121 pglvdrekwe kekseeialg yfrtnenfai ysdalaklan eekvpfvaln kafqqeggda
181 wqqlltdglh fsgkgykifh dellkvietf ypqyhpknmq yklkdwrdvl ddgshims
```

# Figure 7

```
Alignment of pfam00657.6 consensus sequence with P10480
                   *->ivafGDSlTdg................eayygdsdgggwgagladrL
                      iv+fGDSl+d+++  ++ ++  +++++++ +++s+g  w ++l + +
     P10480    28     IVMFGDSLSDTgkmyskmrgylpssppYYEGRFSNGPVWLEQLTNEF 74

                   tall..rlrarprgvdvfnrgisGrtsdGrlivDalvallFlaqslglpn
                   + l    + ++++++++ +n+   +
     P10480    75  PGLTiaNEAEGGPTAVAYNKISWNPK---------------------- 100

                   LpPYLsgdflrGANFAsagAtIlptsgpfliQvqFkdfksqvlelrqalg
                                                                ++  ++
     P10480   101  -------------------------------------------YQVINN 106

                   llqellrllpvldakspdlvtimiGtNDlitsaffgpkstesdrnvsvpe
                   l++e+ ++l +++ k+ dlv++++G+ND+           ++ ++ +++++
     P10480   107  LDYEVTQFLQKDSFKPDDLVILWVGANDY--------LAYGWNTEQDAKR 148

                   fkdnlrqlikrLrsnngariivlitlvilnlgplGClPlklalalasskn
                   ++d +++++r+    nga+       ++++nl+ lG+ P+
     P10480   149  VRDAISDAANRMV-LNGAK-----EILLFNLPDLGQNPS----------- 181

                   vdasgclerlneavadfnealrelaiskledqlrkdglpdvkgadvpyvD
                    ++++ +e +  ++a++n++l +la      +ql+++g++++++++d ++++
     P10480   182  ARSQKVVEAASHVSAYHNQLLLNLA-----RQLAPTGMVKLFEIDKQFAE 226

                   lysifqdldgiqnpsayv.y....GFe.ttkaCCGyGgr.yNyn.rv.CG
                   +    +q+++ +.+ +a+++++   +++ +++a+++++++ +N+++r+ ++
     P10480   227  MLRDPQNFGLSDQRNACYgGsyvwKPFaSRSASTDSQLSaENPQeRLaIA 276

                   nag.l.c.nvtakaC.npssyll.sflfwDgfHpsekGykavAeal<-*
                   +++ l +  ++++a++ +s+ +++++fwD++Hp+   ++a+ e
     P10480   277  GNPlLaQaVASPMAArSASTLNCeGKMFWDQVHPTTVVHAALSEPA    322

Alignment of pfam00657.6 consensus sequence with AAG09804
                   *->ivafGDSlTdg................eayygdsdgggwgagladrL
                      iv+fGDSl+d+++  ++ ++  +++++++ +++s+g  w ++l + +
   AAG09804    28     IVMFGDSLSDTgkmyskmrgylpssppYYEGRFSNGPVWLEQLTKQF 74

                   tallrlrarprgvdvfnrgisGrtsdGrlivDalvallFlaqslglpnLp
                                 +g+++ n + +G+t
   AAG09804    75  ----------PGLTIANEAEGGAT------------------------ 88

                   PYLsgdflrGANFAsagAtIlptsgpfliQvqFkdfksqvlelrqa....
                                                          ++++ + ++++ +
   AAG09804    89  --------------------------------------AVAYNKISWNpkyq 102

                   ..lgllqellrllpvldakspdlvtimiGtNDlitsaffgpkstesdrnv
                    ++l++e+ ++l +++ k+ dlv++++G+ND+           ++ ++ ++
   AAG09804   103  vyNNLDYEVTQFLQKDSFKPDDLVILWVGANDY--------LAYGWNTEQ 144

                   svpefkdnlrqlikrLrsnngariivlitlvilnlgplGClPlklalala
                   ++++++d ++++++r+    nga+      +++++nl+ lG+ P+
 . AAG09804   145  DAKRVRDAISDAANRMV-LNGAK------QILLFNLPDLGQNPS------- 181

                   ssknvdasgclerlneavadfnealrelaiskledqlrkdglpdvkgadv
                        ++++ +e +  ++a++n++l +la      +ql+++g+++++++d
   AAG09804   182  ----ARSQKVVEAVSHVSAYHNKLLLNLA-----RQLAPTGMVKLFEIDK 222

                   pyvDlysifqdldgiqnpsayv.y....GFe.ttkaCCGyGgr.yNyn.r
                   +++++     +q+++ +  ++ +++++    +++ t++ +++ +++ + +++r
   AAG09804   223  QFAEMLRDPQNFGLSDVENPCYdGgyvwKPFaTRSVSTDRQLSaFSPQeR 272

                   v.CGnag.l.c.nvtakaC.npssyll.sflfwDgfHpsekGykavAeal
                   + +++++ l +  ++++a++ +s  +++++fwD++Hp+   ++a+ e+
   AAG09804   273  LaIAGNPlLaQaVASPMARrSASPLNCeGKMFWDQVHPTTVVHAALSERA 322

                   <-*
```

AAG09804 - -

```
Alignment of pfam00657.6 consensus sequence with NP_631558
                    *->ivafGDSlTdgeayygdsdgggwgagladrLtallrlrarprgvdvf
                       +va+GDS ++g          +g  +  +++L      + + + ++  +
   NP_631558     42    YVALGDSYSAG----------SGVLPVDPANL----LCLRSTANYPHV 75

                    nrgisGrtsdGrlivD.a.l.vallFlaqslglpnLpPYLsgdflrGANF
                     + ++G++        D + + +
   NP_631558     76 IADTTGAR------LTDvTcGaAQ------------------------- 93

                    AsagAtIlptsgpfliQvqFkdfksqvlelrqalgllqellrllpvldak
                                                 +++     ++ +  ++ +++
   NP_631558     94 ----------------------------TADFTRAQYPGVAPQLDALGT 114

                    spdlvtimiGtNDl................itsaffgpkstesdrnvsvp
                    + dlvt+ iG+ND ++  +  +  ++ +   ++  + +k   ++ + +++
   NP_631558    115 GTDLVTLTIGGNDNstfinaitacgtagvlSGGKGSPCKDRHGTSFDDEI 164

                    efkdn..lrqlikrLrs.nngariivlitlvilnlg...........plG
                    e  +++ l++++  +r+++ +ar+ +l  ++i+++  +++    + +   G
   NP_631558    165 EANTYpaLKEALLGVRArAPHARVAALGYPWITPATadpscflklplAAG 214

                    ClPlklalalassknvdasgclerlneavadfnealrelaiskledqlrk
                     P+                     l+ ++a  n a+r  a
   NP_631558    215 DVPY-------------------LRAIQAHLNDAVRRAA---------- 234

                    dglpdvkgadvpyvDlysifqdldgiqnpsayvyGFettkaCCGyGgryN
                           ++ + +yvD+ ++
   NP_631558    235 ------EETGATYVDFSGVSDG--------------------------- 250

                    ynrvCGnaglcnvtakaC.npssyll.sflfwDgf...HpsekGykavAe
                                    ++aC+ p +++ +  lf + + + Hp++ G +++Ae
   NP_631558    251 --------------HDACeAPGTRWIePLLFGHSLvpvHPNALGERRMAE 286

                    al<-*
                    +
   NP_631558    287 HT     288

Alignment of pfam00657.6 consensus sequence with CAC42140
                    *->ivafGDSlTdgeayygdsdgggwgagladrLtallrlrarprgvdvf
                       +va+GDS ++g          +g  +  +++L      + + + ++  +
   CAC42140      42    YVALGDSYSAG----------SGVLPVDPANL----LCLRSTANYPHV 75

                    nrgisGrtsdGrlivD.a.l.vallFlaqslglpnLpPYLsgdflrGANF
                     + ++G++        D + + +
   CAC42140      76 IADTTGAR------LTDvTcGaAQ------------------------- 93

                    AsagAtIlptsgpfliQvqFkdfksqvlelrqalgllqellrllpvldak
                                                 +++     ++ +  ++ +++
   CAC42140      94 ----------------------------TADFTRAQYPGVAPQLDALGT 114

                    spdlvtimiGtNDl................itsaffgpkstesdrnvsvp
                    + dlvt+ iG+ND ++  +  +  ++ +   ++  + +k   ++ + +++
   CAC42140     115 GTDLVTLTIGGNDNstfinaitacgtagvlSGGKGSPCKDRHGTSFDDEI 164

                    efkdn..lrqlikrLrs.nngariivlitlvilnlg...........plG
                    e  +++ l++++  +r+++ +ar+ +l  ++i+++  +++    + +   G
   CAC42140     165 EANTYpaLKEALLGVRArAPHARVAALGYPWITPATadpscflklplAAG 214

                    ClPlklalalassknvdasgclerlneavadfnealrelaiskledqlrk
                     P+                     l+ ++a  n a+r  a
   CAC42140     215 DVPY-------------------LRAIQAHLNDAVRRAA---------- 234

                    dglpdvkgadvpyvDlysifqdldgiqnpsayvyGFettkaCCGyGgryN
                           ++ + +yvD+ ++
   CAC42140     235 ------EETGATYVDFSGVSDG--------------------------- 250

                    ynrvCGnaglcnvtakaC.npssyll.sflfwDgf...HpsekGykavAe
                                    ++aC+ p +++ +  lf + + + Hp++ G +++Ae
   CAC42140     251 --------------HDACeAPGTRWIePLLFGHSLvpvHPNALGERRMAE 286
```

```
               al<-*
               +
CAC42140   287 HT     288

Alignment of pfam00657.6 consensus sequence with P41734
              *->ivafGDSlTdg....eayygdsdgggwgagladrLtallrlrarprg
                  ++fGDS+T+ +++ + +  d+     ga+l + +         +r+
P41734       6    FLLFGDSITEFafntRPIEDGKDQYALGAALVNEY---------TRK 43

               vdvfnrgisGrtsdGrlivDalvallFlaqslglpnLpPYLsgdflrGAN
               +d+  rg++G+t
P41734      44 MDILQRGFKGYT-------------------------------------- 55

               FAsagAtIlptsgpfliQvqFkdfksqvlelrqalgllqellrllpvlda
                                             +r+al++l+e+l+      +
P41734      56 ------------------------------SRWALKILPEILKH-----E 70

               kspdlvtimiGtNDlitsaffgpkstesdrnvsvpefkdnlrqlikrLrs
                +  + ti++G+ND+         ++ +++ v++pef+dn+rq++++++s
P41734      71 SNIVMATIFLGANDA---------CSAGPQSVPLPEFIDNIRQMVSLMKS 111

               nngariivlitlvilnlgplGClPlklalalassknvdasgclerlneav
                ++++ii++++lv   ++             ++ k ++ +  + r+ne +
P41734     112 YHIRPIIIGPGLVDREKW-------------EKEKSEEIALGYFRTNENF 148

               adfnealrelaiskledqlrkdglpdvkgadvpyvDlysifqdldgiqnp
               a +  al +la                ++ +vp+v l+++fq+ +g++++
P41734     149 AIYSDALAKLA----------------NEEKVPFVALNKAFQQEGGDAWQ 182

               sayvyGFettkaCCGyGgryNynrvCGnaglcnvtakaCnpssyllsflf
               +                                               l+
P41734     183 Q-----------------------------------------------LL 185

               wDgfHpsekGykavAeal<-*
                Dg+H+s kGyk+++++l
P41734     186 TDGLHFSGKGYKIFHDEL    203
```

Figure 8

```
A.sal   1   MKKWFVCLLGLIALTVQAADTRPAFSRIVMFGDSLSDTGKMYSKMRGYLPSSPPYYEGRF 60
                       +        +
A.hyd   1   MKKWFVCLLGLVALTVQAADSRPAFSRIVMFGDSLSDTGKMYSKMRGYLPSSPPYYEGRF 60
A. sal 61   SNGPVWLEQLTKQFPGLTIANEAEGGATAVAYNKISWNPKYQVINNLDYEVTQFLQKDSF 120
                           ++            +
A. hyd 61   SNGPVWLEQLTNEFPGLTIANEAEGGPTAVAYNKISWNPKYQVINNLDYEVTQFLQKDSF 120
A. sal 121  KPDDLVILWVGANDYLAYGWNTEQDAKRVRDAISDAANRMVLNGAKQILLFNLPDLGQNP 180
                                                                       +
A. hyd 121  KPDDLVILWVGANDYLAYGWNTEQDAKRVRDAISDAANRMVLNGAKEILLFNLPDLGQNP 180
A. sal 181  SARSQKVVEAVSHVSAYHNKLLLNLARQLAPTGMVKLFEIDKQFAEMLRDPQNFGLSDVE 240
                          +        +                                          ++
A.hyd  181  SARSQKVVEAASHVSAYHNQLLLNLARQLAPTGMVKLFEIDKQFAEMLRDPQNFGLSDQR 240
A. sal 241  NPCYDGGYVWKPFATRSVSTDRQLSAFSPQERLAIAGNPLLAQAVASPMARRSASPLNCE 300
             + ++ +       +  +   +     +                      +    +
A. hyd 241  NACYGGSYVWKPFASRSASTDSQLSAFNPQERLAIAGNPLLAQAVASPMAARSASTLNCE 300
A. sal 301  GKMFWDQVHPTTVVHAALSERAATFIETQYEFLAH 335
                                +      +
A. byd 301  GKMFWDQVHPTTVVHAALSEPAATFIESQYEFLAH 335
```

Figure 9

```
  1  ATGAAAAAAT GGTTTGTGTG TTTATTGGGA TTGGTCGCGC TGACAGTTCA GGCAGCCGAC
 61  AGCCGTCCCG CCTTCTCCCG GATCGTGATG TTTGGCGACA GCCTCTCCGA TACCGGCAAG
121  ATGTACAGCA AGATGCGCGG TTACCTCCCC TCCAGCCCCC CCTACTATGA GGGCCGCTTC
181  TCCAACGGGC CCGTCTGGCT GGAGCAGCTG ACCAACGAGT TCCCGGGCCT GACCATAGCC
241  AACGAGGCGG AAGGCGGACC GACCGCCGTG GCTTACAACA AGATCTCCTG GAATCCCAAG
301  TATCAGGTCA TCAACAACCT GGACTACGAG GTCACCCAGT TCCTGCAAAA AGACAGCTTC
361  AAGCCGGACG ATCTGGTGAT CCTCTGGGTC GGCGCCAACG ACTATCTGGC CTATGGCTGG
421  AACACAGAGC AGGATGCCAA GCGGGTGCGC GACGCCATCA GCGATGCGGC CAACCGCATG
481  GTGCTGAACG GCGCCAAGGA GATACTGCTG TTCAACCTGC CGGATCTGGG CCAGAACCCC
541  TCGGCCCGCA GCCAGAAGGT GGTCGAGGCG GCCAGCCATG TCTCCGCCTA CCACAACCAG
601  CTGCTGCTGA ACCTGGCACG CCAGCTGGCT CCCACCGGCA TGGTGAAGCT GTTCGAGATC
661  GACAAGCAGT TTGCCGAGAT GCTGCGTGAT CCGCAGAACT TCGGCCTGAG CGACCAGAGG
721  AACGCCTGCT ACGGTGGCAG CTATGTATGG AAGCCGTTTG CCTCCCGCAG CGCCAGCACC
781  GACAGCCAGC TCTCCGCCTT CAACCCGCAG GAGCGCCTCG CCATCGCCGG CAACCCGCTG
841  CTGGCCCAGG CCGTCGCCAG CCCCATGGCT GCCCGCAGCG CCAGCACCCT CAACTGTGAG
901  GGCAAGATGT TCTGGGATCA GGTCCACCCC ACCACTGTCG TGCACGCCGC CCTGAGCGAG
961  CCCGCCGCCA CCTTCATCGA GAGCCAGTAC GAGTTCCTCG CCCAC
```

Figure 10

```
  1 ATGAAAAAAT GGTTTGTTTG TTTATTGGGG TTGATCGCGC TGACAGTTCA GGCAGCCGAC
 61 ACTCGCCCCG CCTTCTCCCG GATCGTGATG TTCGGCGACA GCCTCTCCGA TACCGGCAAA
121 ATGTACAGCA AGATGCGCGG TTACCTCCCC TCCAGCCCGC CCTACTATGA GGGCCGTTTC
181 TCCAACGGAC CCGTCTGGCT GGAGCAGCTG ACCAAGCAGT TCCCGGGTCT GACCATCGCC
241 AACGAAGCGG AAGGCGGTGC CACTGCCGTG GCTTACAACA AGATCTCCTG GAATCCCAAG
301 TATCAGGTCT ACAACAACCT GGACTACGAG GTCACCCAGT TCTTGCAGAA AGACAGCTTC
361 AAGCCGGACG ATCTGGTGAT CCTCTGGGTC GGTGCCAATG ACTATCTGGC ATATGGCTGG
421 AATACGGAGC AGGATGCCAA GCGAGTTCGC GATGCCATCA GCGATGCGGC CAACCGCATG
481 GTACTGAACG GTGCCAAGCA GATACTGCTG TTCAACCTGC CGGATCTGGG CCAGAACCCG
541 TCAGCCCGCA GTCAGAAGGT GGTCGAGGCG GTCAGCCATG TCTCCGCCTA TCACAACAAG
601 CTGCTGCTGA ACCTGGCACG CCAGCTGGCC CCCACCGGCA TGGTAAAGCT GTTCGAGATC
661 GACAAGCAAT TTGCCGAGAT GCTGCGTGAT CCGCAGAACT TCGGCCTGAG CGACGTCGAG
721 AACCCCTGCT ACGACGGCGG CTATGTGTGG AAGCCGTTTG CCACCCGCAG CGTCAGCACC
781 GACCGCCAGC TCTCCGCCTT CAGTCCGCAG GAACGCCTCG CCATCGCCGG CAACCCGCTG
841 CTGGCACAGG CCGTTGCCAG TCCTATGGCC CGCCGCAGCG CCAGCCCCCT CAACTGTGAG
901 GGCAAGATGT TCTGGGATCA GGTACACCCG ACCACTGTCG TGCACGCAGC CCTGAGCGAG
961 CGCGCCGCCA CCTTCATCGA GACCCAGTAC GAGTTCCTCG CCCACGGATG A
```

Figure 11

```
  1  ATGCCGAAGC CTGCCCTTCG CCGTGTCATG ACCGCGACAG TCGCCGCCGT CGGCACGCTC
 61  GCCCTCGGCC TCACCGACGC CACCGCCCAC GCCGCGCCCG CCCAGGCCAC TCCGACCCTG
121  GACTACGTCG CCCTCGGCGA CAGCTACAGC GCCGGCTCCG GCGTCCTGCC CGTCGACCCC
181  GCCAACCTGC TCTGTCTGCG CTCGACGGCC AACTACCCCC ACGTCATCGC GGACACGACG
241  GGCGCCCGCC TCACGGACGT CACCTGCGGC GCCGCGCAGA CCGCCGACTT CACGCGGGCC
301  CAGTACCCGG GCGTCGCACC CCAGTTGGAC GCGCTCGGCA CCGGCACGGA CCTGGTCACG
361  CTCACCATCG GCGGCAACGA CAACAGCACC TTCATCAACG CCATCACGGC CTGCGGCACG
421  GCGGGTGTCC TCAGCGGCGG CAAGGGCAGC CCCTGCAAGG ACAGGCACGG CACCTCCTTC
481  GACGACGAGA TCGAGGCCAA CACGTACCCC GCGCTCAAGG AGGCGCTGCT CGGCGTCCGC
541  GCCAGGGCTC CCCACGCCAG GGTGGCGGCT CTCGGCTACC CGTGGATCAC CCCGGCCACC
601  GCCGACCCGT CCTGCTTCCT GAAGCTCCCC CTCGCCGCCG GTGACGTGCC CTACCTGCGG
661  GCCATCCAGG CACACCTCAA CGACGCGGTC CGGCGGGCCG CCGAGGAGAC CGGAGCCACC
721  TACGTGGACT TCTCCGGGGT GTCCGACGGC CACGACGCCT GCGAGGCCCC CGGCACCCGC
781  TGGATCGAAC CGCTGCTCTT CGGGCACAGC CTCGTTCCCG TCCACCCCAA CGCCCTGGGC
841  GAGCGGCGCA TGGCCGAGCA CACGATGGAC GTCCTCGGCC TGGACTGA
```

Figure 12

```
  1  TCAGTCCAGG CCGAGGACGT CCATCGTGTG CTCGGCCATG CGCCGCTCGC CCAGGGCGTT
 61  GGGGTGGACG GGAACGAGGC TGTGCCCGAA GAGCAGCGGT TCGATCCAGC GGGTGCCGGG
121  GGCCTCGCAG GCGTCGTGGC CGTCGGACAC CCCGGAGAAG TCCACGTAGG TGGCTCCGGT
181  CTCCTCGGCG GCCCGCCGGA CCGCGTCGTT GAGGTGTGCC TGGATGGCCC GCAGGTAGGG
241  CACGTCACCG GCGGCGAGGG GGAGCTTCAG GAAGCAGGAC GGGTCGGCGG TGGCCGGGGT
301  GATCCACGGG TAGCCGAGAG CCGCCACCCT GGCGTGGGGA GCCCTGGCGC GGACGCCGAG
361  CAGCGCCTCC TTGAGCGCGG GGTACGTGTT GGCCTCGATC TCGTCGTCGA AGGAGGTGCC
421  GTGCCTGTCC TTGCAGGGGC TGCCCTTGCC GCCGCTGAGG ACACCCGCCG TGCCGCAGGC
481  CGTGATGGCG TTGATGAAGG TGCTGTTGTC GTTGCCGCCG ATGGTGAGCG TGACCAGGTC
541  CGTGCCGGTG CCGAGCGCGT CCAACTGGGG TGCGACGCCC GGGTACTGGG CCCGCGTGAA
601  GTCGGCGGTC TGCGCGGCGC CGCAGGTGAC GTCCGTGAGG CGGGCGCCCG TCGTGTCCGC
661  GATGACGTGG GGGTAGTTGG CCGTCGAGCG CAGACAGAGC AGGTTGGCGG GGTCGACGGG
721  CAGGACGCCG GAGCCGGCGC TGTAGCTGTC GCCGAGGGCG ACGTAGTCCA GGGTCGGAGT
781  GGCCTGGGCG GGCGCGGCGT GGGCGGTGGC GTCGGTGAGG CCGAGGGCGA GCGTGCCGAC
841  GGCGGCGACT GTCGCGGTCA TGACACGGCG AAGGGCAGGC TTCGGCAT
```

Figure 13

```
  1 ATGGATTACG AGAAGTTTCT GTTATTTGGG GATTCCATTA CTGAATTTGC TTTTAATACT
 61 AGGCCCATTG AAGATGGCAA AGATCAGTAT GCTCTTGGAG CCGCATTAGT CAACGAATAT
121 ACGAGAAAAA TGGATATTCT TCAAAGAGGG TTCAAAGGGT ACACTTCTAG ATGGGCGTTG
181 AAAATACTTC CTGAGATTTT AAAGCATGAA TCCAATATTG TCATGGCCAC AATATTTTTG
241 GGTGCCAACG ATGCATGCTC AGCAGGTCCC CAAAGTGTCC CCCTCCCCGA ATTTATCGAT
301 AATATTCGTC AAATGGTATC TTTGATGAAG TCTTACCATA TCCGTCCTAT TATAATAGGA
361 CCGGGGCTAG TAGATAGAGA GAAGTGGGAA AAAGAAAAAT CTGAAGAAAT AGCTCTCGGA
421 TACTTCCGTA CCAACGAGAA CTTTGCCATT TATTCCGATG CCTTAGCAAA ACTAGCCAAT
481 GAGGAAAAAG TTCCCTTCGT GGCTTTGAAT AAGGCGTTTC AACAGGAAGG TGGTGATGCT
541 TGGCAACAAC TGCTAACAGA TGGACTGCAC TTTTCCGGAA AAGGGTACAA AATTTTTCAT
601 GACGAATTAT TGAAGGTCAT TGAGACATTC TACCCCCAAT ATCATCCCAA AAACATGCAG
661 TACAAACTGA AAGATTGGAG AGATGTGCTA GATGATGGAT CTAACATAAT GTCTTGA
```

Figure 14

(SEQ ID No. 12)

```
        10         20         30         40         50         60
         |          |          |          |          |          |
MNLRQWMGAA TAALALGLAA CGGGGTDQSG NPNVAKVQRM VVFGDSLSDI GTYTPVAQAV

        70         80         90        100        110        120
         |          |          |          |          |          |
GGGKFTTNPG PIWAETVAAQ LGVTLTPAVM GYATSVQNCP KAGCFDYAQG GSRVTDPNGI

       130        140        150        160        170        180
         |          |          |          |          |          |
GHNGGAGALT YPVQQQLANF YAASNNTFNG NNDVVFVLAG SNDIFFWTTA AATSGSGVTP

       190        200        210        220        230        240
         |          |          |          |          |          |
AIATAQVQQA ATDLVGYVKD MIAKGATQVY VFNLPDSSLT PDGVASGTTG QALLHALVGT

       250        260        270        280        290        300
         |          |          |          |          |          |
FNTTLQSGLA GTSARIIDFN AQLTAAIQNG ASFGFANTSA RACDATKINA LVPSAGGSSL

       310        320        330        340
         |          |          |          |
FCSANTLVAS GADQSYLFAD GVHPTTAGHR LIASNVLARL LADNVAH
```

Figure 15

(SEQ ID No. 13)

```
atgaacctgc gtcaatggat gggcgccgcc acggctgccc ttgccttggg cttggccgcg      60
tgcgggggcg gtgggaccga ccagagcggc aatcccaatg tcgccaaggt gcagcgcatg     120
gtggtgttcg gcgacagcct gagcgatatc ggcacctaca cccccgtcgc gcaggcggtg     180
ggcggcggca agttcaccac caacccgggc ccgatctggg ccgagaccgt ggccgcgcaa     240
ctgggcgtga cgctcacgcc ggcggtgatg ggctacgcca cctccgtgca gaattgcccc     300
aaggccggct gcttcgacta tgcgcagggc ggctcgcgcg tgaccgatcc gaacggcatc     360
ggccacaacg gcggcgcggg ggcgctgacc tacccggttc agcagcagct cgccaacttc     420
tacgcggcca gcaacaacac attcaacggc aataacgatg tcgtcttcgt gctggccggc     480
agcaacgaca ttttcttctg gaccactgcg gcggccacca gcggctccgg cgtgacgccc     540
gccattgcca cggcccaggt gcagcaggcc gcgacggacc tggtcggcta tgtcaaggac     600
atgatcgcca agggtgcgac gcaggtctac gtgttcaacc tgcccgacag cagcctgacg     660
ccggacggcg tggcaagcgg cacgaccggc caggcgctgc tgcacgcgct ggtgggcacg     720
ttcaacacga cgctgcaaag cgggctggcc ggcacctcgg cgcgcatcat cgacttcaac     780
gcacaactga ccgcggcgat ccagaatggc gcctcgttcg gcttcgccaa caccagcgcc     840
cgggcctgcg acgccaccaa gatcaatgcc ctggtgccga gcgccggcgg cagctcgctg     900
ttctgctcgg ccaacacgct ggtggcttcc ggtgcggacc agagctacct gttcgccgac     960
ggcgtgcacc cgaccacggc cggccatcgc ctgatcgcca gcaacgtgct ggcgcgcctg    1020
ctggcggata acgtcgcgca ctga                                          1044
```

Figure 16 (SEQ ID No. 20)

```
  1 migsyvavgd sftegvgdpg pdgafvgwad rlavlladrr pegdftytnl avrgrlldqi
 61 vaeqvprvvg lapdlvsfaa ggndiirpgt dpdevaerfe lavaaltaaa gtvlvttgfd
121 trgvpvlkhl rgkiatyngh vraiadrygc pvldlwslrs vqdrrawdad rlhlspeght
181 rvalragqal glrvpadpdq pwpplpprgt ldvrrddvhw areylvpwig rrlrgessgd
241 hvtakgtlsp daiktriaav a
```

Figure 17 (SEQ ID No. 21)

```
  1 gtgatcgggt cgtacgtggc ggtgggggac agcttcaccg agggcgtcgg cgaccccggc
 61 cccgacgggg cgttcgtcgg ctgggccgac cggctcgccg tactgctcgc ggaccggcgc
121 cccgagggcg acttcacgta cacgaacctc gccgtgcgcg gcaggctcct cgaccagatc
181 gtggcggaac aggtcccgcg ggtcgtcgga ctcgcgcccg acctcgtctc gttcgcggcg
241 ggcggcaacg acatcatccg gcccggcacc gatcccgacg aggtcgccga gcggttcgag
301 ctggcggtgg ccgcgctgac cgccgcggcc ggaaccgtcc tggtgaccac cgggttcgac
361 acccgggggg tgcccgtcct caagcacctg cgcggcaaga tcgccacgta caacgggcac
421 gtccgcgcca tcgccgaccg ctacggctgc ccggtgctcg acctgtggtc gctgcggagc
481 gtccaggacc gcagggcgtg ggacgccgac cggctgcacc tgtcgccgga ggggcacacc
541 cgggtggcgc tgcgcgcggg gcaggccctg ggcctgcgcg tcccggccga ccctgaccag
601 ccctggccgc ccctgccgcc gcgcggcacg ctcgacgtcc ggcgcgacga cgtgcactgg
661 gcgcgcgagt acctggtgcc gtggatcggg cgccggctgc ggggcgagtc gtcgggcgac
721 cacgtgacgg ccaaggggac gctgtcgccg gacgccatca agacgcggat cgccgcggtg
781 gcctga
```

## Figure 18
## (SEQ ID No. 22)

```
  1 mqtnpaytsl vavgdsfteg msdllpdgsy rgwadllatr maarspgfry anlavrgkli
 61 gqivdeqvdv aaamgadvit lvgglndtlr pkcdmarvrd lltqaverla phceqlvlmr
121 spgrqgpvle rfrprmealf aviddlagrh gavvvdlyga qsladprmwd vdrlhltaeg
181 hrrvaeavwq slghepedpe whapipatpp pgwvtrrtad vrferqhllp wigrrltgrs
241 sgdglpakrp dllpyedpar
```

## Figure 19 (SEQ ID No. 23)

```
  1 atgcagacga accccgcgta caccagtctc gtcgccgtcg gcgactcctt caccgagggc
 61 atgtcggacc tgctgcccga cggctcctac cgtggctggg ccgacctcct cgccacccgg
121 atggcggccc gctcccccgg cttccggtac gccaacctgg cggtgcgcgg gaagctgatc
181 ggacagatcg tcgacgagca ggtggacgtg gccgccgcca tgggagccga cgtgatcacg
241 ctggtcggcg ggctcaacga cacgctgcgg cccaagtgcg acatggcccg ggtgcgggac
301 ctgctgaccc aggccgtgga acggctcgcc ccgcactgcg agcagctggt gctgatgcgc
361 agtcccggtc gccagggtcc ggtgctggag cgcttccggc cccgcatgga ggccctgttc
421 gccgtgatcg acgacctggc cggcggcac ggcgccgtgg tcgtcgacct gtacggggcc
481 cagtcgctgg ccgaccctcg gatgtgggac gtggaccggc tgcacctgac cgccgagggc
541 caccgccggg tcgcggaggc ggtgtggcag tcgctcggcc acgagcccga ggaccccgag
601 tggcacgcgc cgatcccggc gacgccgccg ccggggtggg tgacgcgcag gaccgcggac
661 gtccggttcg cccggcagca cctgctgccc tggataggcc gcaggctgac cgggcgctcg
721 tccggggacg gcctgccggc caagcgcccg gacctgctgc cctacgagga ccccgcacgg
781 tga
```

Figure 20 (SEQ ID No. 24)

```
  1 mtrgrdggag apptkhrall aaivtlivai saaiyagasa ddgsrdhalq aggrlprgda
 61 apastgawvg awatapaaae pgtettglag rsvrnvvhts vggtgaritl snlygqsplt
121 vthasialaa gpdtaaaiad tmrrltfggs arviipaggq vmsdtarlai pyganvlvtt
181 yspipsgpvt yhpqarqtsy ladgdrtadv tavayttptp ywryltaldv lsheadgtvv
241 afgdsitdga rsqsdanhrw tdvlaarlhe aagdgrdtpr ysvvnegisg nrlltsrpgr
301 padnpsglsr fqrdvlertn vkavvvvlgv ndvlnspela drdailtglr tlvdraharg
361 lrvvgatitp fggyggytea retmrqevne eirsgrvfdt vvdfdkalrd pydprmrsd
421 ydsgdhlhpg dkgyarmgav idlaalkgaa pvka
```

Figure 21 (SEQ ID No. 25)

```
   1 atgacccggg gtcgtgacgg gggtgcgggg gcgcccccca ccaagcaccg tgccctgctc
  61 gcggcgatcg tcaccctgat agtggcgatc tccgcggcca tatacgccgg agcgtccgcg
 121 gacgacggca gcagggacca cgcgctgcag gccggaggcc gtctcccacg aggagacgcc
 181 gcccccgcgt ccaccggtgc ctgggtgggc gcctgggcca ccgcaccggc cgcggccgag
 241 ccgggcaccg agacgaccgg cctggcgggc cgctccgtgc gcaacgtcgt gcacacctcg
 301 gtcggcggca ccggcgcgcg gatcaccctc tcgaacctgt acgggcagtc gccgctgacc
 361 gtcacacacg cctcgatcgc cctggccgcc gggcccgaca ccgccgccgc gatcgccgac
 421 accatgcgcc ggctcacctt cggcggcagc gcccgggtga tcatcccggc gggcggccag
 481 gtgatgagcg acaccgcccg cctcgccatc ccctacgggg cgaacgtcct ggtcaccacg
 541 tactccccca tcccgtccgg gccggtgacc taccatccgc aggcccggca gaccagctac
 601 ctggccgacg gcgaccgcac ggcggacgtc accgccgtcg cgtacaccac ccccacgccc
 661 tactggcgct acctgaccgc cctcgacgtg ctgagccacg aggccgacgg cacggtcgtg
 721 gcgttcggcg actccatcac cgacggcgcc cgctcgcaga gcgacgccaa ccaccgctgg
 781 accgacgtcc tcgccgcacg cctgcacgag gcggcgggcg acggccggga cacgcccccgc
 841 tacagcgtcg tcaacgaggg catcagcggc aaccggctcc tgaccagcag gccggggcgg
 901 ccggccgaca acccgagcgg actgagccgg ttccagcggg acgtgctgga acgcaccaac
 961 gtcaaggccg tcgtcgtcgt cctcggcgtc aacgacgtcc tgaacagccc ggaactcgcc
1021 gaccgcgacg ccatcctgac cggcctgcgc accctcgtcg accgggcgca cgcccgggga
1081 ctgcgggtcg tcggcgccac gatcacgccg ttcggcggct acggcggcta caccgaggcc
1141 cgcgagacga tgcggcagga ggtcaacgag gagatccgct ccggccgggt cttcgacacg
1201 gtcgtcgact tcgacaaggc cctgcgcgac ccgtacgacc cgcgccggat gcgctccgac
1261 tacgacagcg gcgaccacct gcaccccggc gacaaggggt acgcgcgcat gggcgcggtc
1321 atcgacctgg ccgcgctgaa gggcgcggcg ccggtcaagg cgtag
```

## Figure 22 (SEQ ID No. 26)

```
  1 mtsmsrarva rriaagaayg gggiglagaa avglvvaevq larrrvgvgt ptrvpnaqgl
 61 yggtlptagd pplrlmmlgd staagqgvhr agqtpgalla sglaavaerp vrlgsvaqpg
121 acsddldrqv alvlaepdrv pdicvimvga ndvthrmpat rsvrhlssav rrlrtagaev
181 vvgtcpdlgt iervrqplrw larrasrqla aaqtigaveq ggrtvslgdl lgpefaqnpr
241 elfgpdnyhp saegyataam avlpsvcaal glwpadeehp dalrregflp varaaaeaas
301 eagtevaaam ptgprgpwal lkrrrrrrvs eaepsspsgv
```

## Figure 23 (SEQ ID No. 27)

```
   1 atgacgagca tgtcgagggc gagggtggcg cggcggatcg cggccggcgc ggcgtacggc
  61 ggcggcggca tcggcctggc gggagcggcg gcggtcggtc tggtggtggc cgaggtgcag
 121 ctggccagac gcagggtggg ggtgggcacg ccgacccggg tgccgaacgc gcagggactg
 181 tacggcggca ccctgcccac ggccggcgac ccgccgctgc ggctgatgat gctgggcgac
 241 tccacggccg ccgggcaggg cgtgcaccgg gccgggcaga cgccgggcgc gctgctggcg
 301 tccgggctcg cggcggtggc ggagcggccg gtgcggctgg ggtcggtcgc ccagccgggg
 361 gcgtgctcgg acgacctgga ccggcaggtg gcgctggtgc tcgccgagcc ggaccgggtg
 421 cccgacatct gcgtgatcat ggtcggcgcc aacgacgtca cccaccggat gccggcgacc
 481 cgctcggtgc ggcacctgtc ctcggcggta cggcggctgc gcacggccgg tgcggaggtg
 541 gtggtcggca cctgtccgga cctgggcacg atcgagcggg tgcggcagcc gctgcgctgg
 601 ctggcccggc gggcctcacg gcagctcgcg gcggcacaga ccatcggcgc cgtcgagcag
 661 ggcgggcgca cggtgtcgct gggcgacctg ctgggtccgg agttcgcgca gaacccgcgg
 721 gagctcttcg gccccgacaa ctaccacccc tccgccgagg ggtacgccac ggccgcgatg
 781 gcggtactgc cctcggtgtg cgccgcgctc ggcctgtggc cggccgacga ggagcacccg
 841 gacgcgctgc gccgcgaggg cttcctgccg gtggcgcgcg cggcggcgga ggcggcgtcc
 901 gaggcgggta cggaggtcgc cgccgccatg cctacggggc ctcggggccc ctgggcgctg
 961 ctgaagcgcc ggagacggcg tcgggtgtcg gaggcggaac cgtccagccc gtccggcgtt
1021 tga
```

Figure 24 (SEQ ID No. 28)

```
  1 mgrgtdqrtr ygrrrarval aaltaavlgv gvagcdsvgg dspapsgsps krtrtapawd
 61 tspasvaavg dsitrgfdac avlsdcpevs watgssakvd slavrllgka daaehswnya
121 vtgarmadlt aqvtraaqre pelvavmaga ndacrsttsa mtpvadfraq feeamatlrk
181 klpkaqvyvs sipdlkrlws qgrtnplgkq vwklglcpsm lgdadsldsa atlrrntvrd
241 rvadynevlr evcakdrrcr sddgavhefr fgtdqlshwd wfhpsvdgqa rlaeiayrav
301 taknp
```

Figure 25 (SEQ ID No. 29)

```
  1 atgggtcgag ggacggacca gcggacgcgg tacggccgtc gccgggcgcg tgtcgcgctc
 61 gccgccctga ccgccgccgt cctgggcgtg ggcgtggcgg gctgcgactc cgtgggcggc
121 gactcacccg ctccttccgg cagcccgtcg aagcggacga ggacggcgcc cgcctgggac
181 accagcccgg cgtccgtcgc cgccgtgggc gactccatca cgcgcggctt cgacgcctgt
241 gcggtgctgt cggactgccc ggaggtgtcg tgggcgaccg gcagcagcgc gaaggtcgac
301 tcgctggccg tacggctgct ggggaaggcg gacgcggccg agcacagctg gaactacgcg
361 gtcaccgggg cccggatggc ggacctgacc gctcaggtga cgcgggcggc gcagcgcgag
421 ccggagctgg tggcggtgat ggccggggcg aacgacgcgt gccggtccac gacctcggcg
481 atgacgccgg tggcggactt ccgggcgcag ttcgaggagg cgatggccac cctgcgcaag
541 aagctcccca aggcgcaggt gtacgtgtcg agcatcccgg acctcaagcg gctctggtcc
601 cagggccgca ccaacccgct gggcaagcag gtgtggaagc tcggcctgtg cccgtcgatg
661 ctgggcgacg cggactccct ggactcggcg gcgaccctgc ggcgcaacac ggtgcgcgac
721 cgggtggcgg actacaacga ggtgctgcgg gaggtctgcg cgaaggaccg gcggtgccgc
781 agcgacgacg gcgcggtgca cgagttccgg ttcggcacgg accagttgag ccactgggac
841 tggttccacc cgagtgtgga cggccaggcc cggctggcgg agatcgccta ccgcgcggtc
901 accgcgaaga atccctga
```

Figure 26 (SEQ ID No. 30)

```
  1 mrlsrraata sallltpala lfgasaavsa priqatdyva lgdsyssgvg agsydsssgs
 61 ckrstksypa lwaashtgtr fnftacsgar tgdvlakqlt pvnsgtdlvs itiggndagf
121 adtmttcnlq gesaclaria karayiqqtl paqldqvyda idsrapaaqv vvlgyprfyk
181 lggscavgls eksraainaa addinavtak raadhgfafg dvnttfaghe lcsgapwlhs
241 vtlpvensyh ptangqskgy lpvlnsat
```

Figure 27 (SEQ ID No. 31)

```
   1 ttcatcacaa cgatgtcaca acaccggcca tccgggtcat ccctgatcgt gggaatgggt
  61 gacaagcctt cccgtgacga aagggtcctg ctacatcaga aatgacagaa atcctgctca
 121 gggaggttcc atgagactgt cccgacgcgc ggccacggcg tccgcgctcc tcctcacccc
 181 ggcgctcgcg ctcttcggcg cgagcgccgc cgtgtccgcg ccgcgaatcc aggccaccga
 241 ctacgtggcc ctcggcgact cctactcctc gggggtcggc gcgggcagct acgacagcag
 301 cagtggctcc tgtaagcgca gcaccaagtc ctacccggcc ctgtgggccg cctcgcacac
 361 cggtacgcgg ttcaacttca ccgcctgttc gggcgcccgc acaggagacg tgctggccaa
 421 gcagctgacc ccggtcaact ccggcaccga cctggtcagc attaccatcg gcggcaacga
 481 cgcgggcttc gccgacacca tgaccacctg caacctccag ggcgagagcg cgtgcctggc
 541 gcggatcgcc aaggcgcgcg cctacatcca gcagacgctg cccgcccagc tggaccaggt
 601 ctacgacgcc atcgacagcc gggcccccgc agcccaggtc gtcgtcctgg gctacccgcg
 661 cttctacaag ctgggcggca gctgcgccgt cggtctctcg gagaagtccc gcgcggccat
 721 caacgccgcc gccgacgaca tcaacgccgt caccgccaag cgcgccgccg accacggctt
 781 cgccttcggg gacgtcaaca cgaccttcgc cgggcacgag ctgtgctccg gcgcccccctg
 841 gctgcacagc gtcacccttc ccgtggagaa ctcctaccac cccacggcca acggacagtc
 901 caagggctac ctgcccgtcc tgaactccgc cacctgatct cgcggctact ccgcccctga
 961 cgaagtcccg cccccgggcg gggcttcgcc gtaggtgcgc gtaccgccgt cgcccgtcgc
1021 gccggtggcc ccgccgtacg tgccgccgcc cccggacgcg gtcggttc
```

Figure 28 (SEQ ID No. 32)

```
1    MKKWFVCLLG LVALTVQAAD SRPAFSRIVM FGDSLSDTGK MYSKMRGYLP
51   SSPPYYEGRF SNGPVWLEQL TKQFPGLTIA NEAEGGATAV AYNKISWNPK
101  YQVINNLDYE VTQFLQKDSF KPDDLVILWV GANDYLAYGW NTEQDAKRVR
151  DAISDAANRM VLNGAKQILL FNLPDLGQNP SARSQKVVEA VSHVSAYHNQ
201  LLLNLARQLA PTGMVKLFEI DKQFAEMLRD PQNFGLSDVE NPCYDGGYVW
251  KPFATRSVST DRQLSAFSPQ ERLAIAGNPL LAQAVASPMA RRSASPLNCE
301  GKMFWDQVHP TTVVHAALSE RAATFIANQY EFLAH*
```

# Figure 29 (SEQ ID No. 33)

```
   1  ATGAAAAAAT GGTTTGTGTG TTTATTGGGA TTGGTCGCGC TGACAGTTCA
      TACTTTTTTA CCAAACACAC AAATAACCCT AACCAGCGCG ACTGTCAAGT

  51  GGCAGCCGAC AGTCGCCCCG CCTTTTCCCG GATCGTGATG TTCGGCGACA
      CCGTCGGCTG TCAGCGGGGC GGAAAAGGGC CTAGCACTAC AAGCCGCTGT

 101  GCCTCTCCGA TACCGGCAAA ATGTACAGCA AGATGCGCGG TTACCTCCCC
      CGGAGAGGCT ATGGCCGTTT TACATGTCGT TCTACGCGCC AATGGAGGGG

 151  TCCAGCCCGC CCTACTATGA GGGCCGTTTC TCCAACGGAC CCGTCTGGCT
      AGGTCGGGCG GGATGATACT CCCGGCAAAG AGGTTGCCTG GGCAGACCGA

 201  GGAGCAGCTG ACCAAACAGT TCCCGGGTCT GACCATCGCC AACGAAGCGG
      CCTCGTCGAC TGGTTTGTCA AGGGCCCAGA CTGGTAGCGG TTGCTTCGCC

 251  AAGGCGGTGC CACTGCCGTG GCTTACAACA AGATCTCCTG GAATCCCAAG
      TTCCGCCACG GTGACGGCAC CGAATGTTGT TCTAGAGGAC CTTAGGGTTC

 301  TATCAGGTCA TCAACAACCT GGACTACGAG GTCACCCAGT TCTTGCAGAA
      ATAGTCCAGT AGTTGTTGGA CCTGATGCTC CAGTGGGTCA AGAACGTCTT

 351  AGACAGCTTC AAGCCGGACG ATCTGGTGAT CCTCTGGGTC GGTGCCAATG
      TCTGTCGAAG TTCGGCCTGC TAGACCACTA GGAGACCCAG CCACGGTTAC

 401  ACTATCTGGC CTATGGCTGG AACACGGAGC AGGATGCCAA GCGGGTTCGC
      TGATAGACCG GATACCGACC TTGTGCCTCG TCCTACGGTT CGCCCAAGCG

 451  GATGCCATCA GCGATGCGGC CAACCGCATG GTACTGAACG GTGCCAAGCA
      CTACGGTAGT CGCTACGCCG GTTGGCGTAC CATGACTTGC CACGGTTCGT

 501  GATACTGCTG TTCAACCTGC CGGATCTGGG CCAGAACCCG TCAGCTCGCA
      CTATGACGAC AAGTTGGACG GCCTAGACCC GGTCTTGGGC AGTCGAGCGT

 551  GTCAGAAGGT GGTCGAGGCG GTCAGCCATG TCTCCGCCTA TCACAACCAG
      CAGTCTTCCA CCAGCTCCGC CAGTCGGTAC AGAGGCGGAT AGTGTTGGTC

 601  CTGCTGCTGA ACCTGGCACG CCAGCTGGCC CCCACCGGCA TGGTAAAGCT
      GACGACGACT TGGACCGTGC GGTCGACCGG GGGTGGCCGT ACCATTTCGA

 651  GTTCGAGATC GACAAGCAAT TTGCCGAGAT GCTGCGTGAT CCGCAGAACT
      CAAGCTCTAG CTGTTCGTTA AACGGCTCTA CGACGCACTA GGCGTCTTGA

 701  TCGGCCTGAG CGACGTCGAG AACCCCTGCT ACGACGGCGG CTATGTGTGG
      AGCCGGACTC GCTGCAGCTC TTGGGGACGA TGCTGCCGCC GATACACACC

 751  AAGCCGTTTG CCACCCGCAG CGTCAGCACC GACCGCCAGC TCTCCGCCTT
      TTCGGCAAAC GGTGGGCGTC GCAGTCGTGG CTGGCGGTCG AGAGGCGGAA

 801  CAGTCCGCAG GAACGCCTCG CCATCGCCGG CAACCCGCTG CTGGCACAGG
      GTCAGGCGTC CTTGCGGAGC GGTAGCGGCC GTTGGGCGAC GACCGTGTCC

 851  CCGTTGCCAG TCCTATGGCC CGCCGCAGCG CCAGCCCCCT CAACTGTGAG
      GGCAACGGTC AGGATACCGG GCGGCGTCGC GGTCGGGGGA GTTGACACTC

 901  GGCAAGATGT TCTGGGATCA GGTACACCCG ACCACTGTCG TGCACGCAGC
      CCGTTCTACA AGACCCTAGT CCATGTGGGC TGGTGACAGC ACGTGCGTCG

 951  CCTGAGCGAG CGCGCCGCCA CCTTCATCGC GAACCAGTAC GAGTTCCTCG
      GGACTCGCTC GCGCGGCGGT GGAAGTAGCG CTTGGTCATG CTCAAGGAGC

1001  CCCAC TGA
      GGGTG ACT
```

Figure 30 (SEQ ID No. 34)

```
  1  MKKWFVCLLG LIALTVQAAD TRPAFSRIVM FGDSLSDTGK MYSKMRGYLP
 51  SSPPYYEGRF SNGPVWLEQL TKQFPGLTIA NEAEGGATAV AYNKISWNPK
101  YQVINNLDYE VTQFLQKDSF KPDDLVILWV GANDYLAYGW NTEQDAKRVR
151  DAISDAANRM VLNGAKQILL FNLPDLGQNP SARSQKVVEA VSHVSAYHNK
201  LLLNLARQLA PTGMVKLFEI DKQFAEMLRD PQNFGLSDVE NPCYDGGYVW
251  KPFATRSVST DRQLSAFSPQ ERLAIAGNPL LAQAVASPMA RRSASPLNCE
301  GKMFWDQVHP TTVVHAALSE RAATFIETQY EFLAHG*
```

# Figure 31 (SEQ ID No. 35)

```
   1  ATGAAAAAAT GGTTTGTTTG TTTATTGGGG TTGATCGCGC TGACAGTTCA
      TACTTTTTTA CCAAACAAAC AAATAACCCC AACTAGCGCG ACTGTCAAGT

  51  GGCAGCCGAC ACTCGCCCCG CCTTCTCCCG GATCGTGATG TTCGGCGACA
      CCGTCGGCTG TGAGCGGGGC GGAAGAGGGC CTAGCACTAC AAGCCGCTGT

 101  GCCTCTCCGA TACCGGCAAA ATGTACAGCA AGATGCGCGG TTACCTCCCC
      CGGAGAGGCT ATGGCCGTTT TACATGTCGT TCTACGCGCC AATGGAGGGG

 151  TCCAGCCCGC CCTACTATGA GGGCCGTTTC TCCAACGGAC CCGTCTGGCT
      AGGTCGGGCG GGATGATACT CCCGGCAAAG AGGTTGCCTG GGCAGACCGA

 201  GGAGCAGCTG ACCAAGCAGT TCCCGGGTCT GACCATCGCC AACGAAGCGG
      CCTCGTCGAC TGGTTCGTCA AGGGCCCAGA CTGGTAGCGG TTGCTTCGCC

 251  AAGGCGGTGC CACTGCCGTG GCTTACAACA AGATCTCCTG GAATCCCAAG
      TTCCGCCACG GTGACGGCAC CGAATGTTGT TCTAGAGGAC CTTAGGGTTC

 301  TATCAGGTCA TCAACAACCT GGACTACGAG GTCACCCAGT TCTTGCAGAA
      ATAGTCCAGT AGTTGTTGGA CCTGATGCTC CAGTGGGTCA AGAACGTCTT

 351  AGACAGCTTC AAGCCGGACG ATCTGGTGAT CCTCTGGGTC GGTGCCAATG
      TCTGTCGAAG TTCGGCCTGC TAGACCACTA GGAGACCCAG CCACGGTTAC

 401  ACTATCTGGC ATATGGCTGG AATACGGAGC AGGATGCCAA GCGAGTTCGC
      TGATAGACCG TATACCGACC TTATGCCTCG TCCTACGGTT CGCTCAAGCG

 451  GATGCCATCA GCGATGCGGC CAACCGCATG GTACTGAACG GTGCCAAGCA
      CTACGGTAGT CGCTACGCCG GTTGGCGTAC CATGACTTGC CACGGTTCGT

 501  GATACTGCTG TTCAACCTGC CGGATCTGGG CCAGAACCCG TCAGCCCGCA
      CTATGACGAC AAGTTGGACG GCCTAGACCC GGTCTTGGGC AGTCGGGCGT

 551  GTCAGAAGGT GGTCGAGGCG GTCAGCCATG TCTCCGCCTA TCACAACAAG
      CAGTCTTCCA CCAGCTCCGC CAGTCGGTAC AGAGGCGGAT AGTGTTGTTC

 601  CTGCTGCTGA ACCTGGCACG CCAGCTGGCC CCCACCGGCA TGGTAAAGCT
      GACGACGACT TGGACCGTGC GGTCGACCGG GGGTGGCCGT ACCATTTCGA

 651  GTTCGAGATC GACAAGCAAT TTGCCGAGAT GCTGCGTGAT CCGCAGAACT
      CAAGCTCTAG CTGTTCGTTA AACGGCTCTA CGACGCACTA GGCGTCTTGA

 701  TCGGCCTGAG CGACGTCGAG AACCCCTGCT ACGACGGCGG CTATGTGTGG
      AGCCGGACTC GCTGCAGCTC TTGGGGACGA TGCTGCCGCC GATACACACC

 751  AAGCCGTTTG CCACCCGCAG CGTCAGCACC GACCGCCAGC TCTCCGCCTT
      TTCGGCAAAC GGTGGGCGTC GCAGTCGTGG CTGGCGGTCG AGAGGCGGAA

 801  CAGTCCGCAG GAACGCCTCG CCATCGCCGG CAACCCGCTG CTGGCACAGG
      GTCAGGCGTC CTTGCGGAGC GGTAGCGGCC GTTGGGCGAC GACCGTGTCC

 851  CCGTTGCCAG TCCTATGGCC CGCCGCAGCG CCAGCCCCCT CAACTGTGAG
      GGCAACGGTC AGGATACCGG GCGGCGTCGC GGTCGGGGGA GTTGACACTC

 901  GGCAAGATGT TCTGGGATCA GGTACACCCG ACCACTGTCG TGCACGCAGC
      CCGTTCTACA AGACCCTAGT CCATGTGGGC TGGTGACAGC ACGTGCGTCG

 951  CCTGAGCGAG CGCGCCGCCA CCTTCATCGA GACCCAGTAC GAGTTCCTCG
      GGACTCGCTC GCGCGGCGGT GGAAGTAGCT CTGGGTCATG CTCAAGGAGC

1001  CCCACGGATG A
      GGGTGCCTAC T
```

Figure 32

```
           1       10        20        30        40        50
           |--------+---------+---------+---------+---------|
     satA     ADTRPAFSRIVMFGDSLSDTGKMYSKMRGYLPSSPPYYEGRFSN--G
    R.sol  QSGNPNVAKVQRMVVFGDSLSDIGT-------YTPVAQAVGGGKFTTNPG
Consensus  ...adnraafqRiVmFGDSLSDiGk.......YlPsaqaygeGrFsn..G

           51      60        70        80        90       100
           |--------+---------+---------+---------+---------|
     satA  PVWLEQLTKQFPGLTIANEAEGGATAVAYNKISWNPKYQVINNLDYEVTQ
    R.sol  PIWAETVAAQL-GVTLTPAVMGYATSVQNCPKAGCFDYAQGGSRVTDPNG
Consensus  P!WaEqlaaQl.GlTianaaeGgATaVannkiagnfdYaqgnnrdt#pnq

           101     110       120       130       140       150
           |--------+---------+---------+---------+---------|
     satA  FLQKDSFKPDDLVILWVGANDYLAYG--WNTEQDAKRVRDAISDAANRMV
    R.sol  IGHNGGAGALTYPVQQQLANFYAASNNTFNGNNDVVFVLAGSNDIFFWTT
Consensus  igqndgagaddlp!qqqgANdYaAsn..fNg##DakrVraainDaanrmt

           151     160       170       180       190       200
           |--------+---------+---------+---------+---------|
     satA  LNGAKQILLFNLPDLGQNPSARSQKVVEAVSHVSAYHNKL-LLNLARQLA
    R.sol  AAATSGSGVTPAIATAQVQQAATDLVGYVKDMIAKGATQVYVFNLPDSSL
Consensus  aaaakqiglfnaialaQnqqAas#lVgeakdh!aaganql.llNLarqla

           201     210       220       230       240       250
           |--------+---------+---------+---------+---------|
     satA  PTGMVKLFEIDKQFAEMLRDPQNFGLSDVENPCYDGGYVWKPFATRSVST
    R.sol  TPDGVASGTTGQALLHALVGTFNTTLQSGLAGTSARIIDFNAQLTAAIQN
Consensus  ppdgValgeidqalaeaLrdpqNfgLqdgeagcsargidfnaqaTaa!qn

           251     260       270       280       290       300
           |--------+---------+---------+---------+---------|
     satA  DRQLSAFSPQERLAIAG--NPLLAQAVASPM---ARRSASPLNCEGKMFW
    R.sol  GASFGFANTSARACDATKINALVPSAGGSSLFCSANTLVASGADQSYLFA
Consensus  daqlgaanpqaRaadAg..NaLlaqAgaSp$...Arrlaapgad#gk$Fa

           301     310       320       330
           |--------+---------+---------|
     satA  DQVHPTTVVHAALSERAATFIETQYEFLAH
    R.sol  DGVHPTTAGHRLIASNVLARLLA--DNVAH
Consensus  DqVHPTTagHaaiaeraaariea..#nlAH
```

Figure 33

```
                               ▼
Pfam        *->ivafGDSltdggg..............ayygdsdgggwgagladrltsla..rlrargrgvdv
Sriml  38      YVALGDSYSSGVG............agSYDSSSGSCKRSTKSYPALWAAS..-----HTGTRF 81
Scoe1   5      YVAVGDSFTEG--..............--VGDPGPDGAFVGWADRLAVLL..ADRRPEGDFTY 47
Scoe2  10      LVAVGDSFTEG--..............--MSDLLPDGSYRGWADLLATRM..--AARSPGFRY 50
Scoe3 239      VVAFGDSITDG--..............ARSQSDANHRWTDVLAARLHEAA..GDGRDTPRYSV 283
Scoe4  75      LMMLGDSTAAG--..............------QGVHRAGQTPGALLASG..LAAVAERPVRL 113
Scoe5  66      VAAVGDSITRGFD............acAVLSDCPEVSWATGSSAKVDSLAvrLLGKADAAEHS 116
Ahyd1  28      IVMFGDSLSDTGKmyskmrgylpssppyYEGRFSNGPVWLEQLTNEFPGLTiaNEAEGGPTAVA 91
Asal1  28      IVMFGDSLSDTGKmyskmrgylpssppyYEGRFSNGPVWLEQLTKQF----..------PGLTI 79
Ahyd2  40      IVMFGDSLSDTGKmyskmrgylpssppyYEGRFSNGPVWLEQLTKQFPGLTiaNEAEGGATAVA 103

Pfam          fnrgisGrtsdGrlvvDarlvatllFlaqflGlnlpPYLsgdflrGANFAsagAtIlgtslipflni
Sriml  82 NFTACSGAR---------------------------------------------------------- 90
Scoe1  48 TNLAVRGRL---------------------------------------------------------- 56
Scoe2  51 ANLAVRGKL---------------------------------------------------------- 59
Scoe3 284 VNEGISGNR---------------------------------------------------------- 292
Scoe4 114 GSVAQPGAC---------------------------------------------------------- 122
Scoe5 117 WNYAVTGAR---------------------------------------------------------- 125
Ahyd1  92 YNKISWNPK---------------------------------------------------------- 100
Asal1  80 ANEAEGGAT---------------------------------------------------------- 88
Ahyd2 104 YNKISWNPK---------------------------------------------------------- 112

                                                            ▼
Pfam          QvqFkdfkskvlelrqa.....lgllqellrlvpvldakspdlvtimiGtNDl...itvakfgpks
Sriml  91 -----------------.....---TGDVLAKQLTPVNSGTDLVSITIGGNDAgfaDTMTTCNLQG 131
Scoe1  57 -----------------.....--LDQIVAEQVPRVVGLAPDLVSFAAGGNDI...-----I---- 86
Scoe2  60 -----------------.....--IGQIVDEQVDVAAAMGADVITLVGGLNDT...---------- 88
Scoe3 293 -------LLTSRPGRPA.....DNPSGLSRFQRDVLERTNVKAVVVVLGVNDV...---------- 333
Scoe4 123 -----------------.....SDDLDRQVALVLAEPDRVPDICVIMVGANDV...---------- 153
Scoe5 126 -----------------.....---MADLTAQVTRAAQREPELVAVMAGANDA...--------CR 155
Ahyd1 101 -------------YQVI.....NNLDYEVTQFLQKDSFKPDDLVILWVGANDY...--------LA 137
Asal1  89 -------AVAYNKISWNpkyqvyNNLDYEVTQFLQKDSFKPDDLVILWVGANDY...--------LA 137
Ahyd2 113 -------------YQVI.....NNLDYEVTQFLQKDSFKPDDLVILWVGANDY...--------LA 149

Pfam          ......tksdrnvsvpefrdnlrklikrLrsangariiilitlvllnlpl...........plGCl
Sriml 132 esaclarIAKARAYIQQTLPAQLDQVYDAIDSRAPAA-----QVVVLGYP-...........----- 176
Scoe1  87 ......---RPGTDPDEVAERFELAVAALT-AAAGTVLVTTGFDTRGVP-...........----- 125
Scoe2  89 ......----------LRPKCDMARVRDLLTQAVERLAPHCEQLVLMRSP-...........----- 122
Scoe3 334 ......LNSPELADRDAILTGLRTLVDRAHARGLRVVGATITPFGGYGG-...........----- 376
Scoe4 154 ......---THRMPATRSVRHLSSAVRRLR-TAGAEVVVGTCPDLGTIE-...........----- 192
Scoe5 156 ......STTSAMTPVADFRAQFEEAMATLR-KKLPKAQVYVSSIPDLKRLwsqgrtnplgkQVWKL 214
Ahyd1 138 ......YGWNTEQDAKRVRDAISDAANRMV-LNGAK-----EILLFNLP-...........----- 174
Asal1 138 ......YGWNTEQDAKRVRDAISDAANRMV-LNGAK-----QILLFNLP-...........----- 174
Ahyd2 150 ......YGWNTEQDAKRVRDAISDAANRMV-LNGAK-----QILLFNLP-...........----- 186

Pfam          pq.klalalassknvdatgclerlneavadynealrelaei.ek.l.q.aqlrkdglpdlkeanvpy
Sriml 177 --.RFYKLGGSCAVGLSEKSRAAINAAADDINAVTAKRA--.--.-.-.-----------ADHGFAF 219
Scoe1 126 --.-----------------VLKHLRGKIATYNGHVRAIA--.--.-.-.-----------DRYGCPV 152
Scoe2 123 --.-----------GRQGPVLERFRPRMEALFAVIDDLA--.--.-.-.-----------GRHGAVV 154
Scoe3 377 --.YTEARETMRQEVNEEIRSGRVFDTVVDFDKALRDPY--.--.-.-.------------------ 412
Scoe4 193 --.----------------------------RVRQPLRWLaRRaSrQlAAAQTIGAVEQGGRTVSL 227
Scoe5 215 GLcPSMLGDADSLDSAATLRRNTVRDRVADYNEVLREVC--.--.-.AkDRRCRSDDGAVHEFRFGT 273
Ahyd1 175 --.----DLGQNPSARSQKVVEAASHVSAYHNQLLLNLA--.--.-.-.RQLAPTGMVKLFEIDKQF 224
Asal1 175 --.----DLGQNPSARSQKVVEAVSHVSAYHNKLLLNLA--.--.-.-.RQLAPTGMVKLFEIDKQF 224
Ahyd2 187 --.----DLGQNPSARSQKVVEAVSHVSAYHNQLLLNLA--.--.-.-.RQLAPTGMVKLFEIDKQF 236

Pfam          VDlysifqdldgiqnpsayv.y....GFeet.kaCCGyGgr.yNyn.rv.CGnag.l.ck.vtakaC
Sriml 220 GDVNT---------------.-....-----.---------.-TFAgHElCSGAPwL.HS.VT---- 242
Scoe1 153 LDLWSLRSVQDRRA------.-....-----.---------.----.--.-----.-.--.------ 166
Scoe2 155 VDLYGAQSLADPRM------.-....-----.---------.----.--.-----.-.--.------ 168
Scoe3 413 --------------------.-....-----.---------.----.--.-----.-.--.------ 413
Scoe4 228 GDLLGPEFAQNPREL-----.-....-----.---------.----.--.-----.-.--.------ 242
```

```
Scoe5   274 DQL----------------.-....-----.---------.----.--.-----.-.--.------ 276
Abyd1 225  AEMLRDPQNFGLSDQRNACYgGsyvwKPFASrSASTDSQLSaFNPQeRLaIAGNPlLaQAvASPMAA 291
Asal1 225  AEMLRDPQNFGLSDVENPCYdGgyvwKPFATrSVSTDRQLSaFSPQeRLaIAGNPlLaQAvASPMAR 291
Abyd2 237  AEMLRDPQNFGLSDVENPCYdGgyvwKPFATrSVSTDRQLSaFSPQeRLaIAGNPlLaQAvASPMAR 303

                          ▼
Pfam       .dassyll.atlfwDgf.HpsekGykavAeal<-*
Sriml 243  .-------.--LPVENSyHPTANGQSKGYLPV     263
Scoe1 167  .-------.--WDADRL.HLSPEGHTRVALRA     186
Scoe2 169  .-------.--WDVDRL.HLTAEGHRRVAEAV     188
Scoe3 413  .-DPRRMRsDYDSGDHL.HPGDKGYARMGAVI     441
Scoe4 243  .-------.--FGPDNY.HPSAEGYATAAMAV     262
Scoe5 277  .-------.--SHWDWF.HPSVDGQARLAEIA     296
Abyd1 292  rSASTLNCeGKMFWDQV.HPTTVVHAALSEPA     322
Asal1 292  rSASPLNCeGKMFWDQV.HPTTVVHAALSERA     322
Abyd2 304  rSASPLNCeGKMFWDQV.HPTTVVHAALSERA     334
```

Figure 34

```
Pfam          *->ivafGDSltdggg...............ayygdsdgggwgagladrltsla..rlrargrgvdv
Sriml   38       YVALGDSYSSGVG.............agSYDSSSGSCKRSTKSYPALWAAS..-----HTGTRF 81
Scoe1    5       YVAVGDSFTEG--...............--VGDPGPDGAFVGWADRLAVLL..ADRRPEGDFTY 47
Scoe2   10       LVAVGDSFTEG--...............--MSDLLPDGSYRGWADLLATRM..--AARSPGFRY 50
Ahyd1   28       IVMFGDSLSDTGKmyskmrgylpssppyYEGRFSNGPVWLEQLTNEFPGLTiaNEAEGGPTAVA 91
Asal1   28       IVMFGDSLSDTGKmyskmrgylpssppyYEGRFSNGPVWLEQLTKQF----.------PGLTI 79
Ahyd2   40       IVMFGDSLSDTGKmyskmrgylpssppyYEGRFSNGPVWLEQLTKQFPGLTiaNEAEGGATAVA 103

Pfam          fnrgisGrtsdGrlvvDarlvatllFlaqflGlnlpPYLsgdflrGANFAsagAtIlgtslipflni
Sriml   82 NFTACSGAR---------------------------------------------------------- 90
Scoe1   48 TNLAVRGRL---------------------------------------------------------- 56
Scoe2   51 ANLAVRGKL---------------------------------------------------------- 59
Ahyd1     92 YNKISWNPK-------------------------------------------------------- 100
Asal1     80 ANEAEGGAT-------------------------------------------------------- 88
Ahyd2  104 YNKISWNPK-------------------------------------------------------- 112

Pfam          QvqFkdfkskvlelrqa......lgllqellrlvpvldakspdlvtimiGtNDl...itvakfgpks
Sriml   91 -----------------......---TGDVLAKQLTPVNSGTDLVSITIGGNDAgfaDTMTTCNLQG 131
Scoe1   57 -----------------......--LDQIVAEQVPRVVGLAPDLVSFAAGGNDI...-----I---- 86
Scoe2   60 -----------------......--IGQIVDEQVDVAAAMGADVITLVGGLNDT...---------- 88
Ahyd1  101 -------------YQVI......NNLDYEVTQFLQKDSFKPDDLVILWVGANDY...---------LA 137
Asal1     89 -------AVAYNKISWNpkyqvyNNLDYEVTQFLQKDSFKPDDLVILWVGANDY...---------LA 137
Ahyd2  113 -------------YQVI......NNLDYEVTQFLQKDSFKPDDLVILWVGANDY...---------LA 149

Pfam          .......tksdrnvsvpefrdnlrklikrLrsangariiilitlvllnlplplGCl
Sriml  132 esaclarIAKARAYIQQTLPAQLDQVYDAIDSRAPAA-----QVVVLGYP------ 176
Scoe1   87 .......---RPGTDPDEVAERFELAVAALT-AAAGTVLVTTGFDTRGVP------ 125
Scoe2   89 .......----------LRPKCDMARVRDLLTQAVERLAPHCEQLVLMRSP------ 122
Ahyd1  138 .......YGWNTEQDAKRVRDAISDAANRMV-LNGAK-----EILLFNLP------ 174
Asal1  138 .......YGWNTEQDAKRVRDAISDAANRMV-LNGAK-----QILLFNLP------ 174
Ahyd2  150 .......YGWNTEQDAKRVRDAISDAANRMV-LNGAK------QILLFNLP------ 186

Pfam          pqklalalassknvdatgclerlneavadynealrelaeieklqaqlrkdglpdlkeanvpy
Sriml  177 --RFYKLGGSCAVGLSEKSRAAINAAADDINAVTAKRA-----------------ADHGFAF 219
Scoe1  126 -----------------VLKHLRGKIATYNGHVRAIA------------------DRYGCPV 152
Scoe2  123 -------------GRQGPVLERFRPRMEALFAVIDDLA-----------------GRHGAVV 154
Ahyd1  175 ------DLGQNPSARSQKVVEAASHVSAYHNQLLLNLA------RQLAPTGMVKLFEIDKQF 224
Asal1  175 ------DLGQNPSARSQKVVEAVSHVSAYHNKLLLNLA------RQLAPTGMVKLFEIDKQF 224
Ahyd2  187 ------DLGQNPSARSQKVVEAVSHVSAYHNQLLLNLA------RQLAPTGMVKLFEIDKQF 236

Pfam          VDlysifqdldgiqnpsayv.y....GFeet.kaCCGyGgr.yNyn.rv.CGnag.l.ck.vtakaC
Sriml  220 GDVNT---------------.-....-----.---------.-TFAgHElCSGAPwL.HS.VT---- 242
Scoe1  153 LDLWSLRSVQDRRA------.-....-----.---------.----.--.-----.-.--.------ 166
Scoe2  155 VDLYGAQSLADPRM------.-....-----.---------.----.--.-----.-.--.------ 168
Ahyd1  225 AEMLRDPQNFGLSDQRNACYgGsyvwKPFASrSASTDSQLSaFNPQeRLaIAGNPlLaQAvASPMAA 291
Asal1  225 AEMLRDPQNFGLSDVENPCYdGgyvwKPFATrSVSTDRQLSaFSPQeRLaIAGNPlLaQAvASPMAR 291
Ahyd2  237 AEMLRDPQNFGLSDVENPCYdGgyvwKPFATrSVSTDRQLSaFSPQeRLaIAGNPlLaQAvASPMAR 303

Pfam          .dassyll.atlfwDgf.HpsekGykavAeal<-*
Sriml  243 .-------.--LPVENSyHPTANGQSKGYLPV     263
Scoe1  167 .-------.--WDADRL.HLSPEGHTRVALRA     186
Scoe2  169 .-------.--WDVDRL.HLTAEGHRRVAEAV     188
Ahyd1  292 rSASTLNCeGKMFWDQV.HPTTVVHAALSEPA     322
Asal1  292 rSASPLNCeGKMFWDQV.HPTTVVHAALSERA     322
Ahyd2  304 rSASPLNCeGKMFWDQV.HPTTVVHAALSERA     334
```

Figure 35

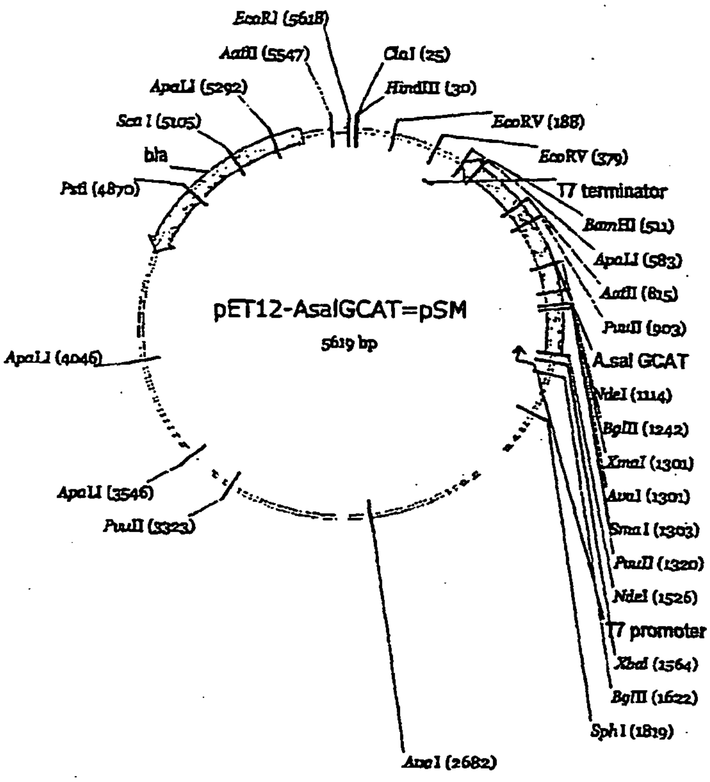
EcoRI (5618)
AatII (5547)
ClaI (25)
HindIII (30)
ApaLI (5292)
ScaI (5105)
EcoRV (188)
EcoRV (379)
bla
PstI (4870)
T7 terminator
BamHI (511)
ApaLI (583)
AatII (815)
PvuII (903)
pET12-AsalGCAT=pSM
5619 bp
ApaLI (4046)
A.sal GCAT
NdeI (1114)
BglII (1242)
XmaI (1301)
AvaI (1301)
SmaI (1303)
PvuII (1320)
ApaLI (3546)
PvuII (3323)
NdeI (1526)
T7 promoter
XbaI (1564)
BglII (1622)
SphI (1819)
AvaI (2682)

Figure 36

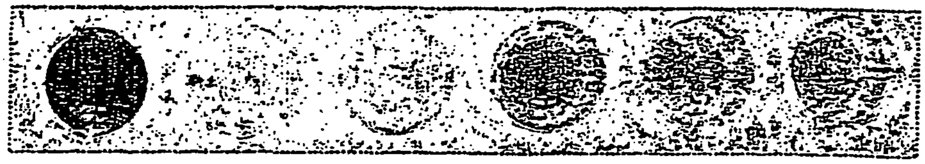

Figure 37

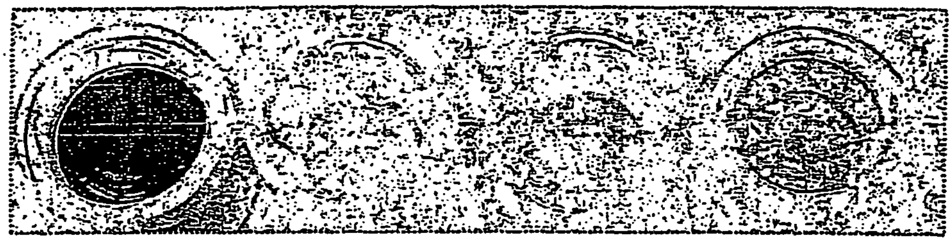

Figure 38

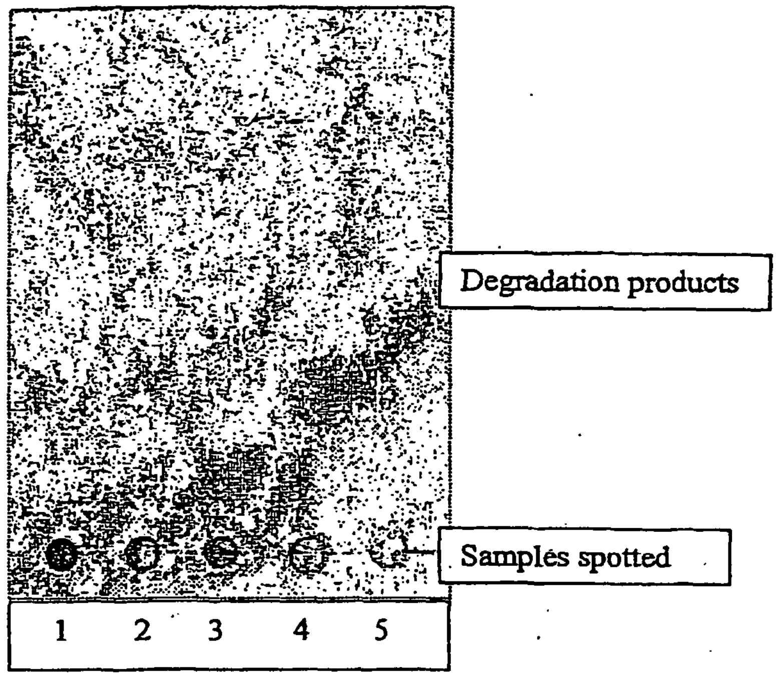

Figure 39

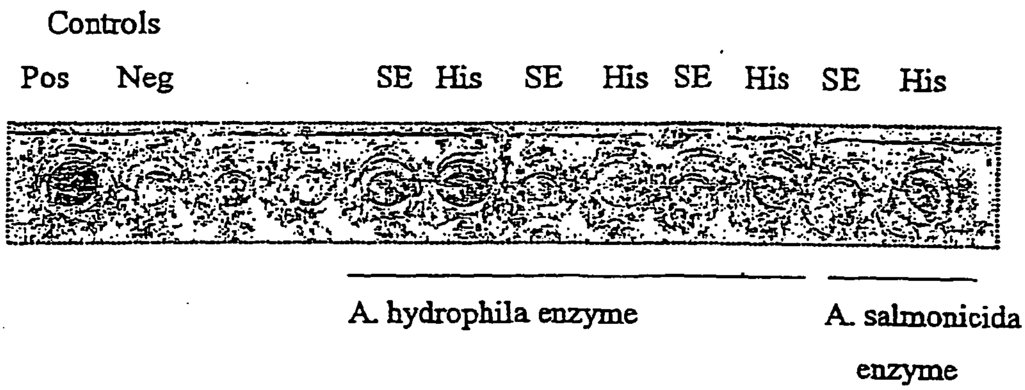
Controls
Pos
Neg
SE
His
SE
His
SE
His
SE
His
A. hydrophila enzyme
A. salmonicida enzyme

Figure 40.

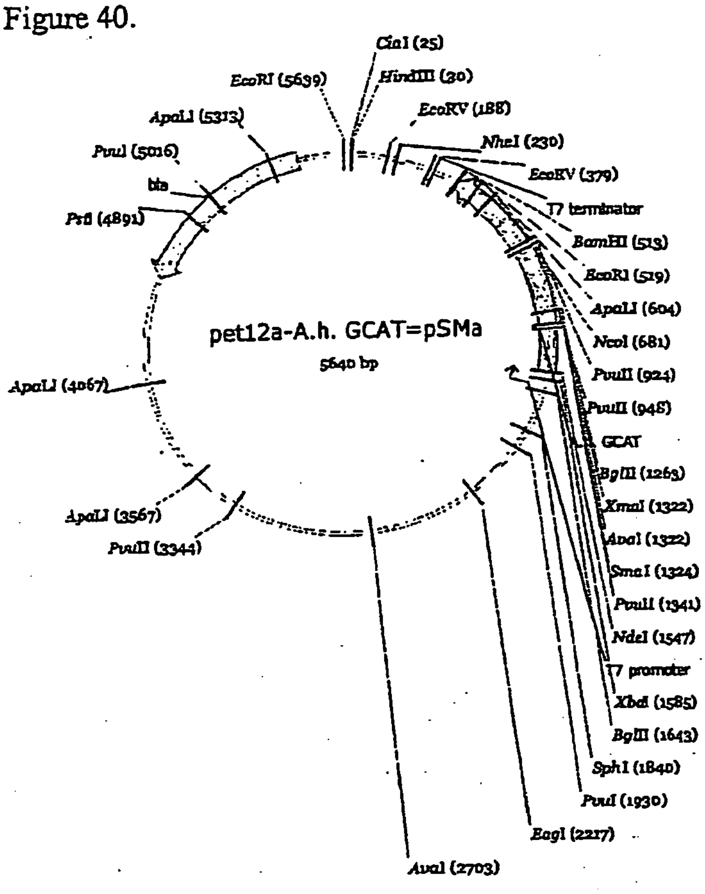
ClaI (25)
HindIII (30)
EcoRV (188)
NheI (230)
EcoRV (379)
T7 terminator
BamHI (513)
EcoRI (519)
ApaLI (604)
NcoI (681)
PvuII (924)
PvuII (948)
GCAT
BglII (1263)
XmaI (1322)
AvaI (1322)
SmaI (1324)
PvuII (1341)
NdeI (1547)
T7 promoter
XbaI (1585)
BglII (1643)
SphI (1840)
PvuI (1930)
EagI (2217)
AvaI (2703)
PvuII (3344)
ApaLI (3567)
ApaLI (4067)
PstI (4891)
bla
PvuI (5016)
ApaLI (5313)
EcoRI (5639)
pet12a-A.h. GCAT=pSMa
5640 bp

Figure 41:

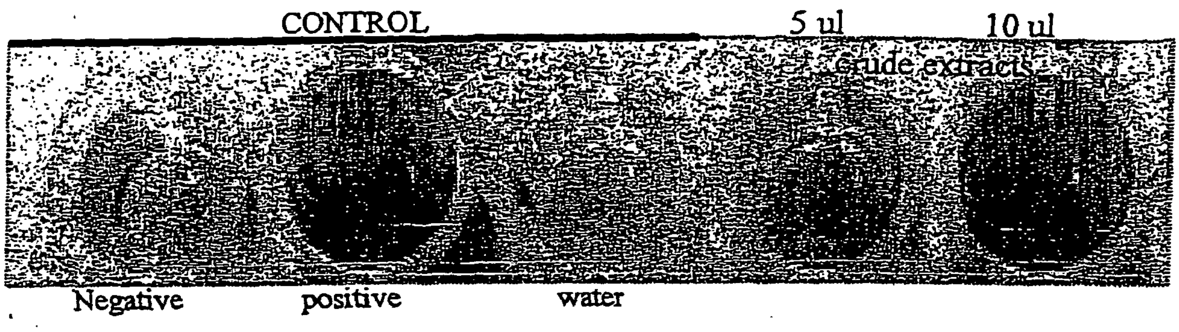

Figure 42

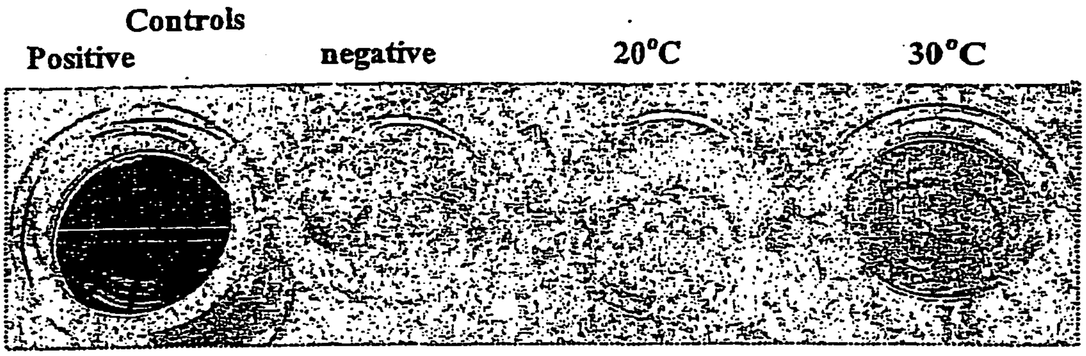

Figure 43

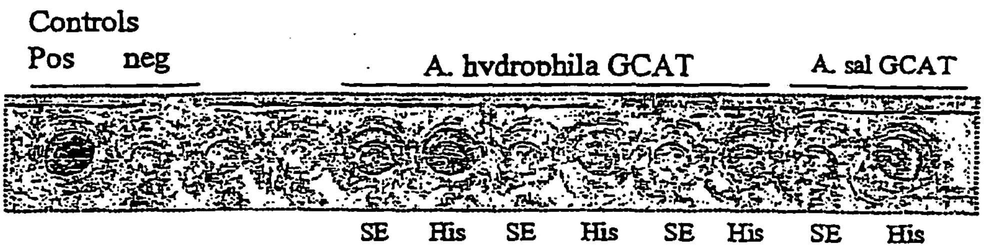
Controls
Pos neg
A. hvdroohila GCAT
A. sal GCAT
SE His SE His SE His SE His

Figure 44

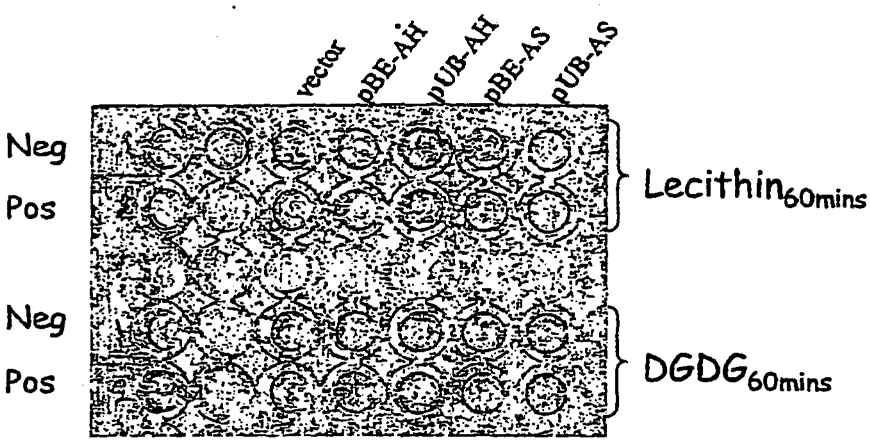
vector
pBE-AH
pUB-AH
pBE-AS
pUB-AS
Neg
Pos
Neg
Pos
Lecithin60mins
DGDG60mins

Figure 45

(SEQ ID No. 36)

```
  1  MFKFKKNFLV GLSAALMSIS LFSATASAAS ADSRPAFSRI VMFGDSLSDT
 51  GKMYSKMRGY LPSSPPYYEG RFSNGPVWLE QLTKQFPGLT IANEAEGGAT
101  AVAYNKISWN PKYQVINNLD YEVTQFLQKD SFKPDDLVIL WVGANDYLAY
151  GWNTEQDAKR VRDAISDAAN RMVLNGAKQI LLFNLPDLGQ NPSARSQKVV
201  EAVSHVSAYH NQLLLNLARQ LAPTGMVKLF EIDKQFAEML RDPQNFGLSD
251  VENPCYDGGY VWKPFATRSV STDRQLSAFS PQERLAIAGN PLLAQAVASP
301  MARRSASPLN CEGKMFWDQV HPTTVVHAAL SERAATFIAN QYEFLAH**
```

Figure 46 (SEQ ID No. 45)

```
   1  ATGTTTAAGT TTAAAAAGAA TTTCTTAGTT GGATTATCGG CAGCTTTAAT
      TACAAATTCA AATTTTTCTT AAAGAATCAA CCTAATAGCC GTCGAAATTA

  51  GAGTATTAGC TTGTTTTCGG CAACCGCCTC TGCAGCTAGC GCCGACAGCC
      CTCATAATCG AACAAAAGCC GTTGGCGGAG ACGTCGATCG CGGCTGTCGG

 101  GTCCCGCCTT TTCCCGGATC GTGATGTTCG GCGACAGCCT CTCCGATACC
      CAGGGCGGAA AAGGGCCTAG CACTACAAGC CGCTGTCGGA GAGGCTATGG

 151  GGCAAAATGT ACAGCAAGAT GCGCGGTTAC CTCCCCTCCA GCCCGCCCTA
      CCGTTTTACA TGTCGTTCTA CGCGCCAATG GAGGGGAGGT CGGGCGGGAT

 201  CTATGAGGGC CGTTTCTCCA ACGGACCCGT CTGGCTGGAG CAGCTGACCA
      GATACTCCCG GCAAAGAGGT TGCCTGGGCA GACCGACCTC GTCGACTGGT

 251  AACAGTTCCC GGGTCTGACC ATCGCCAACG AAGCGGAAGG CGGTGCCACT
      TTGTCAAGGG CCCAGACTGG TAGCGGTTGC TTCGCCTTCC GCCACGGTGA

 301  GCCGTGGCTT ACAACAAGAT CTCCTGGAAT CCCAAGTATC AGGTCATCAA
      CGGCACCGAA TGTTGTTCTA GAGGACCTTA GGGTTCATAG TCCAGTAGTT

 351  CAACCTGGAC TACGAGGTCA CCCAGTTCTT GCAGAAAGAC AGCTTCAAGC
      GTTGGACCTG ATGCTCCAGT GGGTCAAGAA CGTCTTTCTG TCGAAGTTCG

 401  CGGACGATCT GGTGATCCTC TGGGTCGGTG CCAATGACTA TCTGGCCTAT
      GCCTGCTAGA CCACTAGGAG ACCCAGCCAC GGTTACTGAT AGACCGGATA

 451  GGCTGGAACA CGGAGCAGGA TGCCAAGCGG GTTCGCGATG CCATCAGCGA
      CCGACCTTGT GCCTCGTCCT ACGGTTCGCC CAAGCGCTAC GGTAGTCGCT

 501  TGCGGCCAAC CGCATGGTAC TGAACGGTGC CAAGCAGATA CTGCTGTTCA
      ACGCCGGTTG GCGTACCATG ACTTGCCACG GTTCGTCTAT GACGACAAGT

 551  ACCTGCCGGA TCTGGGCCAG AACCCGTCAG CTCGCAGTCA GAAGGTGGTC
      TGGACGGCCT AGACCCGGTC TTGGGCAGTC GAGCGTCAGT CTTCCACCAG

 601  GAGGCGGTCA GCCATGTCTC CGCCTATCAC AACCAGCTGC TGCTGAACCT
      CTCCGCCAGT CGGTACAGAG GCGGATAGTG TTGGTCGACG ACGACTTGGA

 651  GGCACGCCAG CTGGCCCCCA CCGGCATGGT AAAGCTGTTC GAGATCGACA
      CCGTGCGGTC GACCGGGGGT GGCCGTACCA TTTCGACAAG CTCTAGCTGT

 701  AGCAATTTGC CGAGATGCTG CGTGATCCGC AGAACTTCGG CCTGAGCGAC
      TCGTTAAACG GCTCTACGAC GCACTAGGCG TCTTGAAGCC GGACTCGCTG

 751  GTCGAGAACC CCTGCTACGA CGGCGGCTAT GTGTGGAAGC CGTTTGCCAC
      CAGCTCTTGG GGACGATGCT GCCGCCGATA CACACCTTCG GCAAACGGTG

 801  CCGCAGCGTC AGCACCGACC GCCAGCTCTC CGCCTTCAGT CCGCAGGAAC
      GGCGTCGCAG TCGTGGCTGG CGGTCGAGAG GCGGAAGTCA GGCGTCCTTG

 851  GCCTCGCCAT CGCCGGCAAC CCGCTGCTGG CACAGGCCGT TGCCAGTCCT
      CGGAGCGGTA GCGGCCGTTG GGCGACGACC GTGTCCGGCA ACGGTCAGGA

 901  ATGGCCCGCC GCAGCGCCAG CCCCCTCAAC TGTGAGGGCA AGATGTTCTG
      TACCGGGCGG CGTCGCGGTC GGGGGAGTTG ACACTCCCGT TCTACAAGAC

 951  GGATCAGGTA CACCCGACCA CTGTCGTGCA CGCAGCCCTG AGCGAGCGCG
      CCTAGTCCAT GTGGGCTGGT GACAGCACGT GCGTCGGGAC TCGCTCGCGC

1001  CCGCCACCTT CATCGCGAAC CAGTACGAGT TCCTCGCCCA CTGATGA
      GGCGGTGGAA GTAGCGCTTG GTCATGCTCA AGGAGCGGGT GACTACT
```

Figure 47

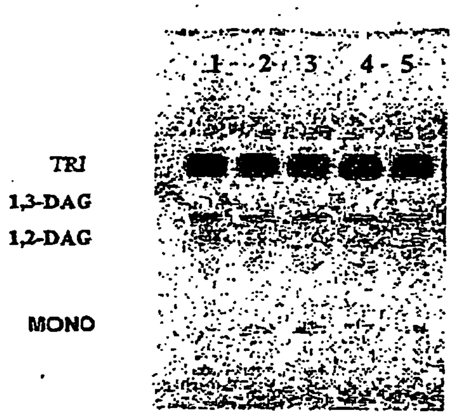


Run order: #196

Palm oil (5% GL)

Lane (5% GL:0% $H_2O$)

Lane (5% GL:1% $H_2O$)

Lane (5% GL:5% $H_2O$)

Lane (10% GL:1% $H_2O$)

NB: 1 : Reference

2-5: With Transferase #196

Figure 48

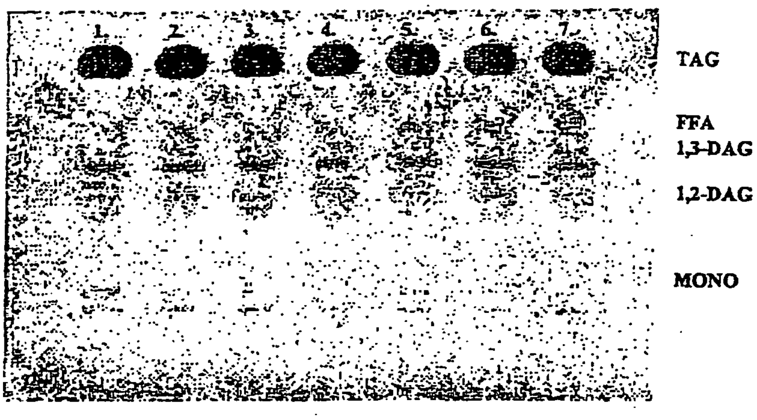


Run order: #196

Lane (5% GL : 1% $H_2O$)

Lane (5% GL : 5% $H_2O$)

Lane (5% GL : 7,5% $H_2O$)

Lane (Reference: 5% GL)

Lane (10% GL : 1% $H_2O$)

Lane (10% GL : 5% $H_2O$)

Lane (10% GL : 7,5% $H_2O$)

Figure 49

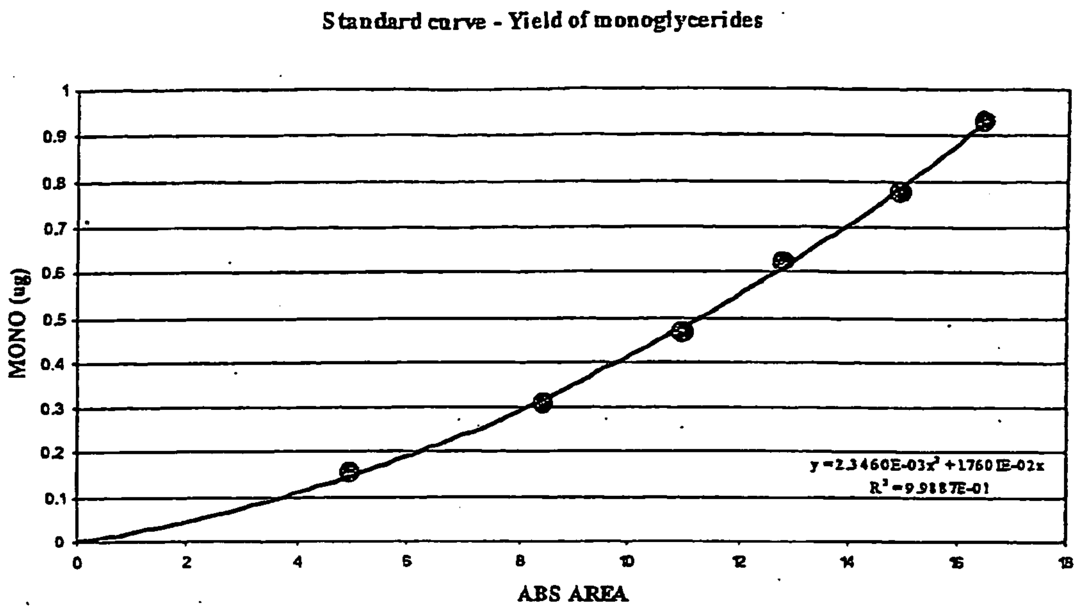
Standard curve - Yield of monoglycerides
MONO (ug)
1
0.9
0.8
0.7
0.6
0.5
0.4
0.3
0.2
0.1
0
0
2
4
6
8
10
12
14
16
18
ABS AREA
y = 2.3460E-03x² + 1.760 E-02x
R² = 9.9887E-01

Figure 50

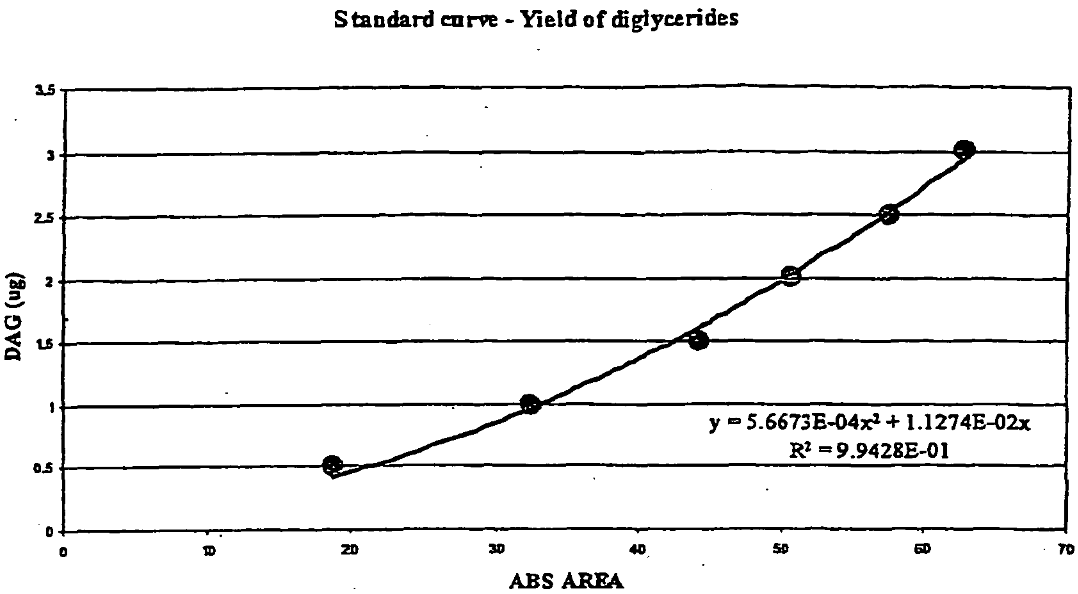
Standard curve - Yield of diglycerides
DAG (ug)
ABS AREA
y = 5.6673E-04x² + 1.1274E-02x
R² = 9.9428E-01
0
0.5
1
1.5
2
2.5
3
3.5
0
10
20
30
40
50
60
70

Figure 51

SEQ ID NO. 54:

ACAGGCCGATGCACGGAACCGTACCTTTCCGCAGTGAAGCGCTCTCCCCCCATCGTTCGC
CGGGACTTCATCCGCGATTTTGGCATGAACACTTCCTTCAACGCGCGTAGCTTGCTACAA
GTGCGGCAGCAGACCCGCTCGTTGGAGGCTCAGTGAGATTGACCCGATCCCTGTCGGCCG
CATCCGTCATCGTCTTCGCCCTGCTGCTCGCGCTGCTGGGCATCAGCCCGGCCCAGGCAG
CCGGCCCGGCCTATGTGGCCCTGGGGGATTCCTATTCCTCGGGCAACGGCGCCGGAAGTT
ACATCGATTCGAGCGGTGACTGTCACCGCAGCAACAACGCGTACCCCGCCCGCTGGGCGG
CGGCCAACGCACCGTCCTCCTTCACCTTCGCGGCCTGCTCGGGAGCGGTGACCACGGATG
TGATCAACAATCAGCTGGGCGCCCTCAACGCGTCCACCGGCCTGGTGAGCATCACCATCG
GCGGCAATGACGCGGGCTTCGCGGACGCGATGACCACCTGCGTCACCAGCTCGGACAGCA
CCTGCCTCAACCGGCTGGCCACCGCCACCAACTACATCAACACCACCCTGCTCGCCCGGC
TCGACGCGGTCTACAGCCAGATCAAGGCCCGTGCCCCCAACGCCCGCGTGGTCGTCCTCG
GCTACCCGCGCATGTACCTGGCCTCGAACCCCTGGTACTGCCTGGGCCTGAGCAACACCA
AGCGCGCGGCCATCAACACCACCGCCGACACCCTCAACTCGGTGATCTCCTCCCGGGCCA
CCGCCCACGGATTCCGATTCGGCGATGTCCGCCCGACCTTCAACAACCACGAACTGTTCT
TCGGCAACGACTGGCTGCACTCACTCACCCTGCCGGTGTGGGAGTCGTACCACCCCACCA
GCACGGGCCATCAGAGCGGCTATCTGCCGGTCCTCAACGCCAACAGCTCGACCTGATCAA
CGCACGGCCGTGCCCGCCCCGCGCGTCACGCTCGGCGCGGGCGCCGCAGCGCGTTGATCA
GCCCACAGTGCCGGTGACGGTCCCACCGTCACGGTCGAGGGTGTACGTCACGGTGGCGCC
GCTCCAGAAGTGGAACGTCAGCAGGACCGTGGAGCCGTCCCTGACCTCGTCGAAGAACTC
CGGGGTCAGCGTGATCACCCCTCCCCCGTAGCCGGGGGCGAAGGCGGCGCCGAACTCCTT
GTAGGACGTCCAGTCGTGCGGCCCGGCGTTGCCACCGTCCGCGTAGACCGCTTCCATGGT
CGCCAGCCGGTCCCCGCGGAACTCGGTGGGGATGTCCGTGCCCAAGGTGGTCCCGGTGGT
GTCCGAGAGCACCGGGGGCTCGTACCGGATGATGTGCAGATCCAAAGAATT

FIGURE 52

SEQ ID NO. 55:

MRLTRSLSAASVIVFALLLALLGISPAQAAGPAYVALGDSYSSGNGAGSYIDSSGDCHRSN
NAYPARWAAANAPSSFTFAACSGAVTTDVINNQLGALNASTGLVSITIGGNDAGFADAMTT
CVTSSDSTCLNRLATATNYINTTLLARLDAVYSQIKARAPNARVVVLGYPRMYLASNPWYC
LGLSNTKRAAINTTADTLNSVISSRATAHGFRFGDVRPTFNNHELFFGNDWLHSLTLPVWE
SYHPTSTGHQSGYLPVLNANSST

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0558112 A
- JP 62061590 B
- EP 0652289 A
- WO 0005396 A
- WO 200036114 A
- US 20030028923 A
- US 20030074695 A
- WO 03100044 A
- EP 0746608 A
- US 5776741 A
- US 4798793 A
- US 5156963 A
- US 6284501 B
- EP 0293194 A
- US 4683202 A
- GB 0330016 A
- WO IB0400655 W
- GB 0416023 A
- US 10898775 B


### Non-patent literature cited in the description


- **Brumlik ; Buckley.** *Journal of Bacteriology,* April 1996, vol. 178 (7), 2060-2064
- **Bateman A et al.** *Nucleic Acids Res.,* 2002, vol. 30, 276-280
- **Bateman A ; Haft DH.** *Brief Bioinform,* 2002, vol. 3, 236-245
- probabilistic models of proteins and nucleic acids. **Durbin R ; Eddy S ; Krogh A.** Biological sequence analysis. Cambridge University Press, 1998
- **Balcao V.M. ; Paiva A.L. ; Malcata F.X.** *Enzyme Microb Technol.,* 01 May 1996, vol. 18 (6), 392-416
- **Retz M.T. ; Jaeger K.E.** *Chem Phys Lipids,* June 1998, vol. 93 (1-2), 3-14
- **Bornscheuer U.T. ; Bessler C ; Srinivas R ; Krishna S.H.** *Trends BiotechnoL,* October 2002, vol. 20 (10), 433-7
- **Plou et al.** *J. Biotechnology,* 2002, vol. 92, 55-66
- **Warmuth et al.** *Bio Forum,* 1992, vol. 9, 282-283
- **Ferrer et al.** *J. Chem. Technol. Biotechnol.,* 2000, vol. 75, 1-8
- **Christensen et al.** *Nachwachsende Rohstoff,* 1998, vol. 10, 98-105
- **Petersen ; Christenen.** Applied Biocatalysis. Harwood Academic Publishers
- **Beucage S.L et al.** *Tetrahedron Letters,* 1981, vol. 22, 1859-1869
- **Matthes et al.** *EMBO J.,* 1984, vol. 3, 801-805
- **Saiki R K et al.** *Science,* 1988, vol. 239, 487-491
- **Hilton ; Buckley.** *J Biol. Chem.,* 15 January 1991, vol. 266 (2), 997-1000
- **Robertson et al.** *J. Biol. Chem.,* 21 January 1994, vol. 269 (3), 2146-50
- **Brumlik et al.** *J. Bacteriol,* April 1996, vol. 178 (7), 2060-4
- **Peelman et al.** *Protein Sci.,* March 1998, vol. 7 (3), 587-99
- **Devereux et al.** *Nuc. Acids Research,* 1984, vol. 12, 387
- **Ausubel et al.** Short Protocols in Molecular Biology. 1999
- **Altschul et al.** *J. Mol. Biol.,* 1990, 403-410
- *FEMS Microbiol Lett,* 1999, vol. 174 (2), 247-50
- *FEMS Microbiol Lett,* 1999, vol. 177 (1), 187-8
- **Higgins DG ; Sharp PM.** *Gene,* 1988, vol. 73 (1), 237-244
- **Simon RJ et al.** *PNAS,* 1992, vol. 89 (20), 9367-9371
- **Horwell DC.** *Trends Biotechnol.,* 1995, vol. 13 (4), 132-134
- **Upton C ; Buckley JT.** *Trends Biochem Sci,* 1995, vol. 20, 179-179
- **Berger, K. G.** Recent developments in palm oil. *Oleagineux,* 1990, vol. 45, 437-4.43
- **Drozdowski, B.** General characteristics of eatable fats. *WNT,* 1994, 240-243
- **Hernquist. L. ; Anjou, K.** Diglycerides as a stabilizer of the β'-crystal Form in Margarines and Fats. *Fette Seifen Anstrichmittel.,* 1983, vol. 2, 64-66
- **Hernquist, L. ; Herslof, B. ; Larsson, K. ; Podlaha, O.** Polymorphism of rapeseed oil with low content of erucic acid and possibilities to stabilize the β' crystal form in fats. *Journal of Science and Food Agriculture,* 1981, vol. 32, 1197-1202

- **Jacobsberg, B. ; Oh, C.H.** Studies in Palm Oil Crystallisation. *Journal of the American Oil Chemist Society,* 1976, vol. 53, 609-616
- **Kristensen, A. C. J.** Preparation of margarine and spreads by enzyme-generated emulsifiers. *Master thesis*
- **McNeill, G. P. ; Berger, R. G.** Enzymatic glycerolysis of palm oil fractions and palm oil based model mixture: Relationship between fatty acid composition and monoglyceride yield. *Food Biotechnology,* 1993, vol. 7, 75-87
- **Okiy, D. A.** Partial glycerides and palm oil Crystallisation. *Journal of Science and Food Agriculture,* 1977, vol. 28, 955
- **Okiy, D. A.** Interaction of triglycerides and diglycerides of palm oil. *Oleagineux,* 1978, vol. 33, 625-628
- **Qkiy, D. A. ; Wright, W. B. ; Berger, K. G. ; Morton, I. D.** The physical properties of modified palm oil. *Journal of Science of Food and Agriculture,* 1978, vol. 29, 625-628
- **Walnett, S. V. ; Meusel, D. ; Tülsner, M.** Zur kenntnis des diglyceride influsses auf das kristallisanonsverhalten von Fetten. *Fat Science Technology,* 1991, vol. 4, 117-121
- **Siew, N. L.** Understanding the Interactions of Diacylglycerols with oil for better Product performance. *2001 PIPOC International Palm Oil Congress - Chemistry and Technology Conference,* 20 August 2001
- **Siew, N. L. ; Ng, W. L.** Differential scanning thermograms of palm oil diglycerides in the presence of diglycerides. *Journal of Oil Palm Research,* 2000, vol. 12, 1-7
- **Siew, N. L. ; Ng, W. L.** Influence of diglycerides on crystalisation of palm oil. *Journal of Science of Food and Agriculture,* 1999, vol. 79, 722-726
- Fat splitting, esterification and interesterification. **Sonntag, N. O. V.** Bailey's Industrial Oils and Fat products. John Wiley and Sons, 1982, vol. 2, 97-173
- **Sonntag, N. O. V.** Glycerolysis of Fats and methyl esters - status, review and critique. *Journal ofAmerican Oil Chemist Society,* 1982, vol. 59, 795A-802A
- **Timms, R.** *Lecture: Trends & delvlopment,* 2004

SEQUENCE LISTING

<110> Danisco A/S

<120> Method

<130> P021904EPA

<150> GB0330016.7
<151> 2003-12-24

<150> PCT/IB04/00655
<151> 2004-01-15

<150> GB0416023.0
<151> 2004-07-16

<150> US 10/898775
<151> 2004-07-26

<160> 73

<170> PatentIn version 3.4

<210> 1
<211> 361
<212> PRT

<213> Artificial

<220>
<223> pfam00567 consensus sequence

<400> 1

```
Ile Val Ala Phe Gly Asp Ser Leu Thr Asp Gly Glu Ala Tyr Tyr Gly
1               5                   10                  15

Asp Ser Asp Gly Gly Gly Trp Gly Ala Gly Leu Ala Asp Arg Leu Thr
            20                  25                  30

Ala Leu Leu Arg Leu Arg Ala Arg Pro Arg Gly Val Asp Val Phe Asn
        35                  40                  45

Arg Gly Ile Ser Gly Arg Thr Ser Asp Gly Arg Leu Ile Val Asp Ala
    50                  55                  60

Leu Val Ala Leu Leu Phe Leu Ala Gln Ser Leu Gly Leu Pro Asn Leu
65                  70                  75                  80

Pro Pro Tyr Leu Ser Gly Asp Phe Leu Arg Gly Ala Asn Phe Ala Ser
                85                  90                  95

Ala Gly Ala Thr Ile Leu Pro Thr Ser Gly Pro Phe Leu Ile Gln Val
            100                 105                 110

Gln Phe Lys Asp Phe Lys Ser Gln Val Leu Glu Leu Arg Gln Ala Leu
        115                 120                 125
```

```
Gly Leu Leu Gln Glu Leu Leu Arg Leu Leu Pro Val Leu Asp Ala Lys
    130                 135                 140

Ser Pro Asp Leu Val Thr Ile Met Ile Gly Thr Asn Asp Leu Ile Thr
145                 150                 155                 160

Ser Ala Phe Phe Gly Pro Lys Ser Thr Glu Ser Asp Arg Asn Val Ser
                165                 170                 175

Val Pro Glu Phe Lys Asp Asn Leu Arg Gln Leu Ile Lys Arg Leu Arg
            180                 185                 190

Ser Asn Asn Gly Ala Arg Ile Ile Val Leu Ile Thr Leu Val Ile Leu
        195                 200                 205

Asn Leu Gly Pro Leu Gly Cys Leu Pro Leu Lys Leu Ala Leu Ala Leu
    210                 215                 220

Ala Ser Ser Lys Asn Val Asp Ala Ser Gly Cys Leu Glu Arg Leu Asn
225                 230                 235                 240

Glu Ala Val Ala Asp Phe Asn Glu Ala Leu Arg Glu Leu Ala Ile Ser
                245                 250                 255

Lys Leu Glu Asp Gln Leu Arg Lys Asp Gly Leu Pro Asp Val Lys Gly
            260                 265                 270

Ala Asp Val Pro Tyr Val Asp Leu Tyr Ser Ile Phe Gln Asp Leu Asp
        275                 280                 285

Gly Ile Gln Asn Pro Ser Ala Tyr Val Tyr Gly Phe Glu Thr Thr Lys
    290                 295                 300

Ala Cys Cys Gly Tyr Gly Gly Arg Tyr Asn Tyr Asn Arg Val Cys Gly
305                 310                 315                 320

Asn Ala Gly Leu Cys Asn Val Thr Ala Lys Ala Cys Asn Pro Ser Ser
                325                 330                 335

Tyr Leu Leu Ser Phe Leu Phe Trp Asp Gly Phe His Pro Ser Glu Lys
            340                 345                 350

Gly Tyr Lys Ala Val Ala Glu Ala Leu
        355                 360
```

<210> 2
<211> 335
<212> PRT

<213> Aeromonas hydrophila

<400> 2

```
Met Lys Lys Trp Phe Val Cys Leu Leu Gly Leu Val Ala Leu Thr Val
1               5                   10                  15

Gln Ala Ala Asp Ser Arg Pro Ala Phe Ser Arg Ile Val Met Phe Gly
            20                  25                  30

Asp Ser Leu Ser Asp Thr Gly Lys Met Tyr Ser Lys Met Arg Gly Tyr
        35                  40                  45

Leu Pro Ser Ser Pro Pro Tyr Tyr Glu Gly Arg Phe Ser Asn Gly Pro
    50                  55                  60

Val Trp Leu Glu Gln Leu Thr Asn Glu Phe Pro Gly Leu Thr Ile Ala
65                  70                  75                  80

Asn Glu Ala Glu Gly Gly Pro Thr Ala Val Ala Tyr Asn Lys Ile Ser
                85                  90                  95

Trp Asn Pro Lys Tyr Gln Val Ile Asn Asn Leu Asp Tyr Glu Val Thr
            100                 105                 110

Gln Phe Leu Gln Lys Asp Ser Phe Lys Pro Asp Asp Leu Val Ile Leu
        115                 120                 125

Trp Val Gly Ala Asn Asp Tyr Leu Ala Tyr Gly Trp Asn Thr Glu Gln
    130                 135                 140

Asp Ala Lys Arg Val Arg Asp Ala Ile Ser Asp Ala Ala Asn Arg Met
145                 150                 155                 160

Val Leu Asn Gly Ala Lys Glu Ile Leu Leu Phe Asn Leu Pro Asp Leu
                165                 170                 175

Gly Gln Asn Pro Ser Ala Arg Ser Gln Lys Val Val Glu Ala Ala Ser
            180                 185                 190

His Val Ser Ala Tyr His Asn Gln Leu Leu Leu Asn Leu Ala Arg Gln
        195                 200                 205

Leu Ala Pro Thr Gly Met Val Lys Leu Phe Glu Ile Asp Lys Gln Phe
    210                 215                 220

Ala Glu Met Leu Arg Asp Pro Gln Asn Phe Gly Leu Ser Asp Gln Arg
225                 230                 235                 240
```

```
Asn Ala Cys Tyr Gly Gly Ser Tyr Val Trp Lys Pro Phe Ala Ser Arg
                245                 250                 255

Ser Ala Ser Thr Asp Ser Gln Leu Ser Ala Phe Asn Pro Gln Glu Arg
            260                 265                 270

Leu Ala Ile Ala Gly Asn Pro Leu Leu Ala Gln Ala Val Ala Ser Pro
        275                 280                 285

Met Ala Ala Arg Ser Ala Ser Thr Leu Asn Cys Glu Gly Lys Met Phe
    290                 295                 300

Trp Asp Gln Val His Pro Thr Thr Val Val His Ala Ala Leu Ser Glu
305                 310                 315                 320

Pro Ala Ala Thr Phe Ile Glu Ser Gln Tyr Glu Phe Leu Ala His
                325                 330                 335
```

<210> 3
<211> 336
<212> PRT
<213> Aeromonas salmonicida

<400> 3

```
Met Lys Lys Trp Phe Val Cys Leu Leu Gly Leu Ile Ala Leu Thr Val
1               5                   10                  15

Gln Ala Ala Asp Thr Arg Pro Ala Phe Ser Arg Ile Val Met Phe Gly
            20                  25                  30

Asp Ser Leu Ser Asp Thr Gly Lys Met Tyr Ser Lys Met Arg Gly Tyr
        35                  40                  45

Leu Pro Ser Ser Pro Pro Tyr Tyr Glu Gly Arg Phe Ser Asn Gly Pro
    50                  55                  60

Val Trp Leu Glu Gln Leu Thr Lys Gln Phe Pro Gly Leu Thr Ile Ala
65                  70                  75                  80

Asn Glu Ala Glu Gly Gly Ala Thr Ala Val Ala Tyr Asn Lys Ile Ser
                85                  90                  95

Trp Asn Pro Lys Tyr Gln Val Tyr Asn Asn Leu Asp Tyr Glu Val Thr
            100                 105                 110

Gln Phe Leu Gln Lys Asp Ser Phe Lys Pro Asp Asp Leu Val Ile Leu
        115                 120                 125
```

```
Trp Val Gly Ala Asn Asp Tyr Leu Ala Tyr Gly Trp Asn Thr Glu Gln
    130                 135                 140

Asp Ala Lys Arg Val Arg Asp Ala Ile Ser Asp Ala Ala Asn Arg Met
145                 150                 155                 160

Val Leu Asn Gly Ala Lys Gln Ile Leu Leu Phe Asn Leu Pro Asp Leu
                165                 170                 175

Gly Gln Asn Pro Ser Ala Arg Ser Gln Lys Val Val Glu Ala Val Ser
            180                 185                 190

His Val Ser Ala Tyr His Asn Lys Leu Leu Leu Asn Leu Ala Arg Gln
        195                 200                 205

Leu Ala Pro Thr Gly Met Val Lys Leu Phe Glu Ile Asp Lys Gln Phe
    210                 215                 220

Ala Glu Met Leu Arg Asp Pro Gln Asn Phe Gly Leu Ser Asp Val Glu
225                 230                 235                 240

Asn Pro Cys Tyr Asp Gly Gly Tyr Val Trp Lys Pro Phe Ala Thr Arg
                245                 250                 255

Ser Val Ser Thr Asp Arg Gln Leu Ser Ala Phe Ser Pro Gln Glu Arg
            260                 265                 270

Leu Ala Ile Ala Gly Asn Pro Leu Leu Ala Gln Ala Val Ala Ser Pro
        275                 280                 285

Met Ala Arg Arg Ser Ala Ser Pro Leu Asn Cys Glu Gly Lys Met Phe
    290                 295                 300

Trp Asp Gln Val His Pro Thr Thr Val Val His Ala Ala Leu Ser Glu
305                 310                 315                 320

Arg Ala Ala Thr Phe Ile Glu Thr Gln Tyr Glu Phe Leu Ala His Gly
                325                 330                 335
```

<210> 4
<211> 295
<212> PRT
<213> Streptomyces coelicolor

<400> 4

```
Met Pro Lys Pro Ala Leu Arg Arg Val Met Thr Ala Thr Val Ala Ala
1               5                   10                  15

Val Gly Thr Leu Ala Leu Gly Leu Thr Asp Ala Thr Ala His Ala Ala
```

```
                20                  25                  30

Pro Ala Gln Ala Thr Pro Thr Leu Asp Tyr Val Ala Leu Gly Asp Ser
        35                  40                  45

Tyr Ser Ala Gly Ser Gly Val Leu Pro Val Asp Pro Ala Asn Leu Leu
    50                  55                  60

Cys Leu Arg Ser Thr Ala Asn Tyr Pro His Val Ile Ala Asp Thr Thr
65                  70                  75                  80

Gly Ala Arg Leu Thr Asp Val Thr Cys Gly Ala Ala Gln Thr Ala Asp
                85                  90                  95

Phe Thr Arg Ala Gln Tyr Pro Gly Val Ala Pro Gln Leu Asp Ala Leu
            100                 105                 110

Gly Thr Gly Thr Asp Leu Val Thr Leu Thr Ile Gly Gly Asn Asp Asn
        115                 120                 125

Ser Thr Phe Ile Asn Ala Ile Thr Ala Cys Gly Thr Ala Gly Val Leu
    130                 135                 140

Ser Gly Gly Lys Gly Ser Pro Cys Lys Asp Arg His Gly Thr Ser Phe
145                 150                 155                 160

Asp Asp Glu Ile Glu Ala Asn Thr Tyr Pro Ala Leu Lys Glu Ala Leu
                165                 170                 175

Leu Gly Val Arg Ala Arg Ala Pro His Ala Arg Val Ala Ala Leu Gly
            180                 185                 190

Tyr Pro Trp Ile Thr Pro Ala Thr Ala Asp Pro Ser Cys Phe Leu Lys
        195                 200                 205

Leu Pro Leu Ala Ala Gly Asp Val Pro Tyr Leu Arg Ala Ile Gln Ala
    210                 215                 220

His Leu Asn Asp Ala Val Arg Arg Ala Ala Glu Glu Thr Gly Ala Thr
225                 230                 235                 240

Tyr Val Asp Phe Ser Gly Val Ser Asp Gly His Asp Ala Cys Glu Ala
                245                 250                 255

Pro Gly Thr Arg Trp Ile Glu Pro Leu Leu Phe Gly His Ser Leu Val
            260                 265                 270

Pro Val His Pro Asn Ala Leu Gly Glu Arg Arg Met Ala Glu His Thr
```

275 280 285

Met Asp Val Leu Gly Leu Asp
290 295

<210> 5
<211> 295
<212> PRT
<213> Streptomyces coelicolor

<400> 5

```
Met Pro Lys Pro Ala Leu Arg Arg Val Met Thr Ala Thr Val Ala Ala
1               5                   10                  15

Val Gly Thr Leu Ala Leu Gly Leu Thr Asp Ala Thr Ala His Ala Ala
            20                  25                  30

Pro Ala Gln Ala Thr Pro Thr Leu Asp Tyr Val Ala Leu Gly Asp Ser
        35                  40                  45

Tyr Ser Ala Gly Ser Gly Val Leu Pro Val Asp Pro Ala Asn Leu Leu
    50                  55                  60

Cys Leu Arg Ser Thr Ala Asn Tyr Pro His Val Ile Ala Asp Thr Thr
65                  70                  75                  80

Gly Ala Arg Leu Thr Asp Val Thr Cys Gly Ala Ala Gln Thr Ala Asp
                85                  90                  95

Phe Thr Arg Ala Gln Tyr Pro Gly Val Ala Pro Gln Leu Asp Ala Leu
            100                 105                 110

Gly Thr Gly Thr Asp Leu Val Thr Leu Thr Ile Gly Gly Asn Asp Asn
        115                 120                 125

Ser Thr Phe Ile Asn Ala Ile Thr Ala Cys Gly Thr Ala Gly Val Leu
    130                 135                 140

Ser Gly Gly Lys Gly Ser Pro Cys Lys Asp Arg His Gly Thr Ser Phe
145                 150                 155                 160

Asp Asp Glu Ile Glu Ala Asn Thr Tyr Pro Ala Leu Lys Glu Ala Leu
                165                 170                 175

Leu Gly Val Arg Ala Arg Ala Pro His Ala Arg Val Ala Ala Leu Gly
            180                 185                 190

Tyr Pro Trp Ile Thr Pro Ala Thr Ala Asp Pro Ser Cys Phe Leu Lys
        195                 200                 205
```

```
Leu Pro Leu Ala Ala Gly Asp Val Pro Tyr Leu Arg Ala Ile Gln Ala
    210                 215                 220

His Leu Asn Asp Ala Val Arg Arg Ala Ala Glu Glu Thr Gly Ala Thr
225                 230                 235                 240

Tyr Val Asp Phe Ser Gly Val Ser Asp Gly His Asp Ala Cys Glu Ala
                245                 250                 255

Pro Gly Thr Arg Trp Ile Glu Pro Leu Leu Phe Gly His Ser Leu Val
            260                 265                 270

Pro Val His Pro Asn Ala Leu Gly Glu Arg Arg Met Ala Glu His Thr
        275                 280                 285

Met Asp Val Leu Gly Leu Asp
    290                 295
```

<210> 6
<211> 238
<212> PRT
<213> Saccharomyces cerevisiae

<400> 6

```
Met Asp Tyr Glu Lys Phe Leu Leu Phe Gly Asp Ser Ile Thr Glu Phe
1               5                   10                  15

Ala Phe Asn Thr Arg Pro Ile Glu Asp Gly Lys Asp Gln Tyr Ala Leu
            20                  25                  30

Gly Ala Ala Leu Val Asn Glu Tyr Thr Arg Lys Met Asp Ile Leu Gln
        35                  40                  45

Arg Gly Phe Lys Gly Tyr Thr Ser Arg Trp Ala Leu Lys Ile Leu Pro
    50                  55                  60

Glu Ile Leu Lys His Glu Ser Asn Ile Val Met Ala Thr Ile Phe Leu
65                  70                  75                  80

Gly Ala Asn Asp Ala Cys Ser Ala Gly Pro Gln Ser Val Pro Leu Pro
                85                  90                  95

Glu Phe Ile Asp Asn Ile Arg Gln Met Val Ser Leu Met Lys Ser Tyr
            100                 105                 110

His Ile Arg Pro Ile Ile Ile Gly Pro Gly Leu Val Asp Arg Glu Lys
        115                 120                 125

Trp Glu Lys Glu Lys Ser Glu Glu Ile Ala Leu Gly Tyr Phe Arg Thr
    130                 135                 140

Asn Glu Asn Phe Ala Ile Tyr Ser Asp Ala Leu Ala Lys Leu Ala Asn
145                 150                 155                 160

Glu Glu Lys Val Pro Phe Val Ala Leu Asn Lys Ala Phe Gln Gln Glu
                165                 170                 175

Gly Gly Asp Ala Trp Gln Gln Leu Leu Thr Asp Gly Leu His Phe Ser
            180                 185                 190

Gly Lys Gly Tyr Lys Ile Phe His Asp Glu Leu Leu Lys Val Ile Glu
        195                 200                 205

Thr Phe Tyr Pro Gln Tyr His Pro Lys Asn Met Gln Tyr Lys Leu Lys
    210                 215                 220

Asp Trp Arg Asp Val Leu Asp Asp Gly Ser Asn Ile Met Ser
225                 230                 235
```

<210> 7

<211> 1005
<212> DNA
<213> Aeromonas hydrophila

<400> 7

```
atgaaaaaat ggtttgtgtg tttattggga ttggtcgcgc tgacagttca ggcagccgac     60
agccgtcccg ccttctcccg gatcgtgatg tttggcgaca gcctctccga taccggcaag    120
atgtacagca agatgcgcgg ttacctcccc tccagccccc cctactatga gggccgcttc    180
tccaacgggc ccgtctggct ggagcagctg accaacgagt tcccgggcct gaccatagcc    240
aacgaggcgg aaggcggacc gaccgccgtg gcttacaaca agatctcctg gaatcccaag    300
tatcaggtca tcaacaacct ggactacgag gtcacccagt tcctgcaaaa agacagcttc    360
aagccggacg atctggtgat cctctgggtc ggcgccaacg actatctggc ctatggctgg    420
aacacagagc aggatgccaa gcgggtgcgc gacgccatca gcgatgcggc caaccgcatg    480
gtgctgaacg gcgccaagga gatactgctg ttcaacctgc cggatctggg ccagaacccc    540
tcggcccgca gccagaaggt ggtcgaggcg gccagccatg tctccgccta ccacaaccag    600
ctgctgctga acctggcacg ccagctggct cccaccggca tggtgaagct gttcgagatc    660
gacaagcagt ttgccgagat gctgcgtgat ccgcagaact tcggcctgag cgaccagagg    720
aacgcctgct acggtggcag ctatgtatgg aagccgtttg cctcccgcag cgccagcacc    780
gacagccagc tctccgcctt caacccgcag gagcgcctcg ccatcgccgg caacccgctg    840
ctggcccagg ccgtcgccag ccccatggct gcccgcagcg ccagcaccct caactgtgag    900

ggcaagatgt tctgggatca ggtccacccc accactgtcg tgcacgccgc cctgagcgag    960
cccgccgcca ccttcatcga gagccagtac gagttcctcg cccac                   1005
```

<210> 8
<211> 1011
<212> DNA
<213> Aeromonas salmonicida

<400> 8

```
atgaaaaaat ggtttgtttg tttattgggg ttgatcgcgc tgacagttca ggcagccgac      60
actcgccccg ccttctcccg gatcgtgatg ttcggcgaca gcctctccga taccggcaaa     120
atgtacagca agatgcgcgg ttacctcccc tccagcccgc cctactatga gggccgtttc     180
tccaacggac ccgtctggct ggagcagctg accaagcagt tcccgggtct gaccatcgcc     240
aacgaagcgg aaggcggtgc cactgccgtg gcttacaaca agatctcctg gaatcccaag     300
tatcaggtct acaacaacct ggactacgag gtcacccagt tcttgcagaa agacagcttc     360
aagccggacg atctggtgat cctctgggtc ggtgccaatg actatctggc atatggctgg     420
aatacggagc aggatgccaa gcgagttcgc gatgccatca gcgatgcggc caaccgcatg     480
gtactgaacg gtgccaagca gatactgctg ttcaacctgc cggatctggg ccagaacccg     540
tcagcccgca gtcagaaggt ggtcgaggcg gtcagccatg tctccgccta tcacaacaag     600
ctgctgctga acctggcacg ccagctggcc cccaccggca tggtaaagct gttcgagatc     660
gacaagcaat ttgccgagat gctgcgtgat ccgcagaact tcggcctgag cgacgtcgag     720
aacccctgct acgacggcgg ctatgtgtgg aagccgtttg ccacccgcag cgtcagcacc     780
gaccgccagc tctccgcctt cagtccgcag gaacgcctcg ccatcgccgg caacccgctg     840
ctggcacagg ccgttgccag tcctatggcc cgccgcagcg ccagccccct caactgtgag     900
ggcaagatgt tctgggatca ggtacacccg accactgtcg tgcacgcagc cctgagcgag     960
cgcgccgcca ccttcatcga gacccagtac gagttcctcg cccacggatg a             1011
```

<210> 9
<211> 888
<212> DNA
<213> Streptomyces coelicolor

<400> 9

```
atgccgaagc ctgcccttcg ccgtgtcatg accgcgacag tcgccgccgt cggcacgctc      60
gccctcggcc tcaccgacgc caccgcccac gccgcgcccg cccaggccac tccgaccctg     120
gactacgtcg ccctcggcga cagctacagc gccggctccg gcgtcctgcc cgtcgacccc     180
gccaacctgc tctgtctgcg ctcgacggcc aactaccccc acgtcatcgc ggacacgacg     240
ggcgcccgcc tcacggacgt cacctgcggc gccgcgcaga ccgccgactt cacgcgggcc     300
cagtacccgg gcgtcgcacc ccagttggac gcgctcggca ccggcacgga cctggtcacg     360
```

ctcaccatcg gcggcaacga caacagcacc ttcatcaacg ccatcacggc ctgcggcacg 420
gcgggtgtcc tcagcggcgg caagggcagc ccctgcaagg acaggcacgg cacctccttc 480
gacgacgaga tcgaggccaa cacgtacccc gcgctcaagg aggcgctgct cggcgtccgc 540
gccagggctc cccacgccag ggtggcggct ctcggctacc cgtggatcac cccggccacc 600
gccgacccgt cctgcttcct gaagctcccc ctcgccgccg gtgacgtgcc ctacctgcgg 660
gccatccagg cacacctcaa cgacgcggtc cggcgggccg ccgaggagac cggagccacc 720
tacgtggact tctccggggt gtccgacggc cacgacgcct gcgaggcccc cggcacccgc 780
tggatcgaac cgctgctctt cgggcacagc ctcgttcccg tccaccccaa cgccctgggc 840
gagcggcgca tggccgagca cacgatggac gtcctcggcc tggactga 888

<210> 10
<211> 888
<212> DNA
<213> Streptomyces coelicolor

<400> 10

tcagtccagg ccgaggacgt ccatcgtgtg ctcggccatg cgccgctcgc ccagggcgtt 60
ggggtggacg ggaacgaggc tgtgcccgaa gagcagcggt tcgatccagc gggtgccggg 120
ggcctcgcag gcgtcgtggc cgtcggacac cccggagaag tccacgtagg tggctccggt 180
ctcctcggcg gcccgccgga ccgcgtcgtt gaggtgtgcc tggatggccc gcaggtaggg 240
cacgtcaccg gcggcgaggg ggagcttcag gaagcaggac gggtcggcgg tggccggggt 300
gatccacggg tagccgagag ccgccaccct ggcgtgggga gccctggcgc ggacgccgag 360
cagcgcctcc ttgagcgcgg ggtacgtgtt ggcctcgatc tcgtcgtcga aggaggtgcc 420
gtgcctgtcc ttgcaggggc tgcccttgcc gccgctgagg acacccgccg tgccgcaggc 480
cgtgatggcg ttgatgaagg tgctgttgtc gttgccgccg atggtgagcg tgaccaggtc 540
cgtgccggtg ccgagcgcgt ccaactgggg tgcgacgccc gggtactggg cccgcgtgaa 600
gtcggcggtc tgcgcggcgc cgcaggtgac gtccgtgagg cgggcgcccg tcgtgtccgc 660
gatgacgtgg gggtagttgg ccgtcgagcg cagacagagc aggttggcgg ggtcgacggg 720
caggacgccg gagccggcgc tgtagctgtc gccgagggcg acgtagtcca gggtcggagt 780
ggcctgggcg ggcgcggcgt gggcggtggc gtcggtgagg ccgagggcga gcgtgccgac 840
ggcggcgact gtcgcggtca tgacacggcg aagggcaggc ttcggcat 888

<210> 11
<211> 717
<212> DNA
<213> Saccharomyces cerevisiae

<400> 11

```
atggattacg agaagtttct gttatttggg gattccatta ctgaatttgc ttttaatact      60
aggcccattg aagatggcaa agatcagtat gctcttggag ccgcattagt caacgaatat     120
acgagaaaaa tggatattct tcaaagaggg ttcaaagggt acacttctag atgggcgttg     180
aaaatacttc ctgagatttt aaagcatgaa tccaatattg tcatggccac aatatttttg     240
ggtgccaacg atgcatgctc agcaggtccc caaagtgtcc ccctccccga atttatcgat     300
aatattcgtc aaatggtatc tttgatgaag tcttaccata tccgtcctat tataatagga     360
ccggggctag tagatagaga gaagtgggaa aaagaaaaat ctgaagaaat agctctcgga     420
tacttccgta ccaacgagaa ctttgccatt tattccgatg ccttagcaaa actagccaat     480
gaggaaaaag ttcccttcgt ggctttgaat aaggcgtttc aacaggaagg tggtgatgct     540
tggcaacaac tgctaacaga tggactgcac ttttccggaa aagggtacaa aatttttcat     600
gacgaattat tgaaggtcat tgagacattc taccccccaat atcatcccaa aaacatgcag     660
tacaaactga aagattggag agatgtgcta gatgatggat ctaacataat gtcttga        717
```

<210> 12
<211> 347
<212> PRT
<213> Ralstonia spp

<400> 12

```
Met Asn Leu Arg Gln Trp Met Gly Ala Ala Thr Ala Ala Leu Ala Leu
1               5                   10                  15

Gly Leu Ala Ala Cys Gly Gly Gly Gly Thr Asp Gln Ser Gly Asn Pro
            20                  25                  30

Asn Val Ala Lys Val Gln Arg Met Val Val Phe Gly Asp Ser Leu Ser
        35                  40                  45

Asp Ile Gly Thr Tyr Thr Pro Val Ala Gln Ala Val Gly Gly Gly Lys
    50                  55                  60

Phe Thr Thr Asn Pro Gly Pro Ile Trp Ala Glu Thr Val Ala Ala Gln
65                  70                  75                  80

Leu Gly Val Thr Leu Thr Pro Ala Val Met Gly Tyr Ala Thr Ser Val
                85                  90                  95

Gln Asn Cys Pro Lys Ala Gly Cys Phe Asp Tyr Ala Gln Gly Gly Ser
            100                 105                 110

Arg Val Thr Asp Pro Asn Gly Ile Gly His Asn Gly Gly Ala Gly Ala
        115                 120                 125

Leu Thr Tyr Pro Val Gln Gln Gln Leu Ala Asn Phe Tyr Ala Ala Ser
```

130 135 140

Asn Asn Thr Phe Asn Gly Asn Asn Asp Val Val Phe Val Leu Ala Gly
145 150 155 160

Ser Asn Asp Ile Phe Phe Trp Thr Thr Ala Ala Ala Thr Ser Gly Ser
165 170 175

Gly Val Thr Pro Ala Ile Ala Thr Ala Gln Val Gln Gln Ala Ala Thr
180 185 190

Asp Leu Val Gly Tyr Val Lys Asp Met Ile Ala Lys Gly Ala Thr Gln
195 200 205

Val Tyr Val Phe Asn Leu Pro Asp Ser Ser Leu Thr Pro Asp Gly Val
210 215 220

Ala Ser Gly Thr Thr Gly Gln Ala Leu Leu His Ala Leu Val Gly Thr
225 230 235 240

Phe Asn Thr Thr Leu Gln Ser Gly Leu Ala Gly Thr Ser Ala Arg Ile
245 250 255

Ile Asp Phe Asn Ala Gln Leu Thr Ala Ala Ile Gln Asn Gly Ala Ser
260 265 270

Phe Gly Phe Ala Asn Thr Ser Ala Arg Ala Cys Asp Ala Thr Lys Ile
275 280 285

Asn Ala Leu Val Pro Ser Ala Gly Gly Ser Ser Leu Phe Cys Ser Ala
290 295 300

Asn Thr Leu Val Ala Ser Gly Ala Asp Gln Ser Tyr Leu Phe Ala Asp
305 310 315 320

Gly Val His Pro Thr Thr Ala Gly His Arg Leu Ile Ala Ser Asn Val
325 330 335

Leu Ala Arg Leu Leu Ala Asp Asn Val Ala His
340 345

<210> 13
<211> 1044
<212> DNA
<213> Ralstonia spp

<400> 13

atgaacctgc gtcaatggat gggcgccgcc acggctgccc ttgccttggg cttggccgcg 60
tgcgggggcg gtgggaccga ccagagcggc aatcccaatg tcgccaaggt gcagcgcatg 120

gtggtgttcg gcgacagcct gagcgatatc ggcacctaca cccccgtcgc gcaggcggtg 180
ggcggcggca agttcaccac caacccgggc ccgatctggg ccgagaccgt ggccgcgcaa 240
ctgggcgtga cgctcacgcc ggcggtgatg ggctacgcca cctccgtgca gaattgcccc 300
aaggccggct gcttcgacta tgcgcagggc ggctcgcgcg tgaccgatcc gaacggcatc 360
ggccacaacg gcggcgcggg ggcgctgacc tacccggttc agcagcagct cgccaacttc 420
tacgcggcca gcaacaacac attcaacggc aataacgatg tcgtcttcgt gctggccggc 480
agcaacgaca ttttcttctg gaccactgcg gcggccacca gcggctccgg cgtgacgccc 540
gccattgcca cggcccaggt gcagcaggcc gcgacggacc tggtcggcta tgtcaaggac 600
atgatcgcca agggtgcgac gcaggtctac gtgttcaacc tgcccgacag cagcctgacg 660
ccggacggcg tggcaagcgg cacgaccggc caggcgctgc tgcacgcgct ggtgggcacg 720
ttcaacacga cgctgcaaag cgggctggcc ggcacctcgg cgcgcatcat cgacttcaac 780
gcacaactga ccgcggcgat ccagaatggc gcctcgttcg gcttcgccaa caccagcgcc 840
cgggcctgcg acgccaccaa gatcaatgcc ctggtgccga gcgccggcgg cagctcgctg 900
ttctgctcgg ccaacacgct ggtggcttcc ggtgcggacc agagctacct gttcgccgac 960
ggcgtgcacc cgaccacggc cggccatcgc ctgatcgcca gcaacgtgct ggcgcgcctg 1020
ctggcggata acgtcgcgca ctga 1044

<210> 14

<400> 14
000

<210> 15

<400> 15
000

<210> 16

<400> 16
000

<210> 17

<400> 17
000

<210> 18

<400> 18
000

<210> 19

<400> 19
000

<210> 20
<211> 261
<212> PRT
<213> Artificial

<220>
<223> conserved hypothetical protein Streptomyces coelicolor

<400> 20

```
Met Ile Gly Ser Tyr Val Ala Val Gly Asp Ser Phe Thr Glu Gly Val
1               5                   10                  15

Gly Asp Pro Gly Pro Asp Gly Ala Phe Val Gly Trp Ala Asp Arg Leu
            20                  25                  30

Ala Val Leu Leu Ala Asp Arg Arg Pro Glu Gly Asp Phe Thr Tyr Thr
        35                  40                  45

Asn Leu Ala Val Arg Gly Arg Leu Leu Asp Gln Ile Val Ala Glu Gln
    50                  55                  60

Val Pro Arg Val Val Gly Leu Ala Pro Asp Leu Val Ser Phe Ala Ala
65                  70                  75                  80

Gly Gly Asn Asp Ile Ile Arg Pro Gly Thr Asp Pro Asp Glu Val Ala
                85                  90                  95

Glu Arg Phe Glu Leu Ala Val Ala Ala Leu Thr Ala Ala Ala Gly Thr
            100                 105                 110

Val Leu Val Thr Thr Gly Phe Asp Thr Arg Gly Val Pro Val Leu Lys
        115                 120                 125

His Leu Arg Gly Lys Ile Ala Thr Tyr Asn Gly His Val Arg Ala Ile
    130                 135                 140

Ala Asp Arg Tyr Gly Cys Pro Val Leu Asp Leu Trp Ser Leu Arg Ser
145                 150                 155                 160

Val Gln Asp Arg Arg Ala Trp Asp Ala Asp Arg Leu His Leu Ser Pro
                165                 170                 175

Glu Gly His Thr Arg Val Ala Leu Arg Ala Gly Gln Ala Leu Gly Leu
            180                 185                 190

Arg Val Pro Ala Asp Pro Asp Gln Pro Trp Pro Pro Leu Pro Pro Arg
        195                 200                 205

Gly Thr Leu Asp Val Arg Arg Asp Asp Val His Trp Ala Arg Glu Tyr
    210                 215                 220
```

```
Leu Val Pro Trp Ile Gly Arg Arg Leu Arg Gly Glu Ser Ser Gly Asp
225                 230                 235                 240

His Val Thr Ala Lys Gly Thr Leu Ser Pro Asp Ala Ile Lys Thr Arg
                245                 250                 255

Ile Ala Ala Val Ala
            260
```

<210> 21
<211> 786
<212> DNA
<213> Streptomyces coelicolor

<400> 21

```
gtgatcgggt cgtacgtggc ggtgggggac agcttcaccg agggcgtcgg cgaccccggc      60
cccgacgggg cgttcgtcgg ctgggccgac cggctcgccg tactgctcgc ggaccggcgc     120
cccgagggcg acttcacgta cacgaacctc gccgtgcgcg gcaggctcct cgaccagatc     180
gtggcggaac aggtcccgcg ggtcgtcgga ctcgcgcccg acctcgtctc gttcgcggcg     240
ggcggcaacg acatcatccg gcccggcacc gatcccgacg aggtcgccga gcggttcgag     300
ctggcggtgg ccgcgctgac cgccgcggcc ggaaccgtcc tggtgaccac cgggttcgac     360
acccgggggg tgcccgtcct caagcacctg cgcggcaaga tcgccacgta caacgggcac     420
gtccgcgcca tcgccgaccg ctacggctgc ccggtgctcg acctgtggtc gctgcggagc     480
gtccaggacc gcagggcgtg ggacgccgac cggctgcacc tgtcgccgga ggggcacacc     540
cgggtggcgc tgcgcgcggg gcaggccctg ggcctgcgcg tcccggccga ccctgaccag     600
ccctggccgc ccctgccgcc gcgcggcacg ctcgacgtcc ggcgcgacga cgtgcactgg     660
gcgcgcgagt acctggtgcc gtggatcggg cgccggctgc ggggcgagtc gtcgggcgac     720
cacgtgacgg ccaaggggac gctgtcgccg gacgccatca agacgcggat cgccgcggtg     780
gcctga                                                                786
```

<210> 22
<211> 260
<212> PRT
<213> Streptomyces coelicolor

<400> 22

```
Met Gln Thr Asn Pro Ala Tyr Thr Ser Leu Val Ala Val Gly Asp Ser
1               5                   10                  15

Phe Thr Glu Gly Met Ser Asp Leu Leu Pro Asp Gly Ser Tyr Arg Gly
            20                  25                  30
```

Trp Ala Asp Leu Leu Ala Thr Arg Met Ala Ala Arg Ser Pro Gly Phe
35 40 45

Arg Tyr Ala Asn Leu Ala Val Arg Gly Lys Leu Ile Gly Gln Ile Val
50 55 60

Asp Glu Gln Val Asp Val Ala Ala Ala Met Gly Ala Asp Val Ile Thr
65 70 75 80

Leu Val Gly Gly Leu Asn Asp Thr Leu Arg Pro Lys Cys Asp Met Ala
85 90 95

Arg Val Arg Asp Leu Leu Thr Gln Ala Val Glu Arg Leu Ala Pro His
100 105 110

Cys Glu Gln Leu Val Leu Met Arg Ser Pro Gly Arg Gln Gly Pro Val
115 120 125

Leu Glu Arg Phe Arg Pro Arg Met Glu Ala Leu Phe Ala Val Ile Asp
130 135 140

Asp Leu Ala Gly Arg His Gly Ala Val Val Val Asp Leu Tyr Gly Ala
145 150 155 160

Gln Ser Leu Ala Asp Pro Arg Met Trp Asp Val Asp Arg Leu His Leu
165 170 175

Thr Ala Glu Gly His Arg Arg Val Ala Glu Ala Val Trp Gln Ser Leu
180 185 190

Gly His Glu Pro Glu Asp Pro Glu Trp His Ala Pro Ile Pro Ala Thr
195 200 205

Pro Pro Pro Gly Trp Val Thr Arg Arg Thr Ala Asp Val Arg Phe Ala
210 215 220

Arg Gln His Leu Leu Pro Trp Ile Gly Arg Arg Leu Thr Gly Arg Ser
225 230 235 240

Ser Gly Asp Gly Leu Pro Ala Lys Arg Pro Asp Leu Leu Pro Tyr Glu
245 250 255

Asp Pro Ala Arg
260

<210> 23
<211> 783
<212> DNA
<213> Streptomyces coelicolor

<400> 23

```
atgcagacga accccgcgta caccagtctc gtcgccgtcg gcgactcctt caccgagggc     60
atgtcggacc tgctgcccga cggctcctac cgtggctggg ccgacctcct cgccacccgg    120
atggcggccc gctcccccgg cttccggtac gccaacctgg cggtgcgcgg gaagctgatc    180
ggacagatcg tcgacgagca ggtggacgtg gccgccgcca tgggagccga cgtgatcacg    240
ctggtcggcg ggctcaacga cacgctgcgg cccaagtgcg acatggcccg ggtgcgggac    300
ctgctgaccc aggccgtgga acggctcgcc ccgcactgcg agcagctggt gctgatgcgc    360
agtcccggtc gccagggtcc ggtgctggag cgcttccggc cccgcatgga ggccctgttc    420
gccgtgatcg acgacctggc cgggcggcac ggcgccgtgg tcgtcgacct gtacggggcc    480
cagtcgctgg ccgaccctcg gatgtgggac gtggaccggc tgcacctgac cgccgagggc    540
caccgccggg tcgcggaggc ggtgtggcag tcgctcggcc acgagcccga ggaccccgag    600
tggcacgcgc cgatcccggc gacgccgccg ccggggtggg tgacgcgcag gaccgcggac    660
gtccggttcg cccggcagca cctgctgccc tggataggcc gcaggctgac cgggcgctcg    720
tccggggacg gcctgccggc caagcgcccg gacctgctgc cctacgagga ccccgcacgg    780
tga                                                                  783
```

<210> 24
<211> 454
<212> PRT
<213> Streptomyces coelicolor

<400> 24

```
Met Thr Arg Gly Arg Asp Gly Gly Ala Gly Ala Pro Pro Thr Lys His
1               5                   10                  15

Arg Ala Leu Leu Ala Ala Ile Val Thr Leu Ile Val Ala Ile Ser Ala
            20                  25                  30

Ala Ile Tyr Ala Gly Ala Ser Ala Asp Asp Gly Ser Arg Asp His Ala
        35                  40                  45

Leu Gln Ala Gly Gly Arg Leu Pro Arg Gly Asp Ala Ala Pro Ala Ser
    50                  55                  60

Thr Gly Ala Trp Val Gly Ala Trp Ala Thr Ala Pro Ala Ala Ala Glu
65                  70                  75                  80

Pro Gly Thr Glu Thr Thr Gly Leu Ala Gly Arg Ser Val Arg Asn Val
                85                  90                  95

Val His Thr Ser Val Gly Gly Thr Gly Ala Arg Ile Thr Leu Ser Asn
            100                 105                 110
```

```
Leu Tyr Gly Gln Ser Pro Leu Thr Val Thr His Ala Ser Ile Ala Leu
        115                 120                 125

Ala Ala Gly Pro Asp Thr Ala Ala Ala Ile Ala Asp Thr Met Arg Arg
    130                 135                 140

Leu Thr Phe Gly Gly Ser Ala Arg Val Ile Ile Pro Ala Gly Gly Gln
145                 150                 155                 160

Val Met Ser Asp Thr Ala Arg Leu Ala Ile Pro Tyr Gly Ala Asn Val
                165                 170                 175

Leu Val Thr Thr Tyr Ser Pro Ile Pro Ser Gly Pro Val Thr Tyr His
            180                 185                 190

Pro Gln Ala Arg Gln Thr Ser Tyr Leu Ala Asp Gly Asp Arg Thr Ala
        195                 200                 205

Asp Val Thr Ala Val Ala Tyr Thr Thr Pro Thr Pro Tyr Trp Arg Tyr
    210                 215                 220

Leu Thr Ala Leu Asp Val Leu Ser His Glu Ala Asp Gly Thr Val Val
225                 230                 235                 240

Ala Phe Gly Asp Ser Ile Thr Asp Gly Ala Arg Ser Gln Ser Asp Ala
                245                 250                 255

Asn His Arg Trp Thr Asp Val Leu Ala Ala Arg Leu His Glu Ala Ala
            260                 265                 270

Gly Asp Gly Arg Asp Thr Pro Arg Tyr Ser Val Val Asn Glu Gly Ile
        275                 280                 285

Ser Gly Asn Arg Leu Leu Thr Ser Arg Pro Gly Arg Pro Ala Asp Asn
    290                 295                 300

Pro Ser Gly Leu Ser Arg Phe Gln Arg Asp Val Leu Glu Arg Thr Asn
305                 310                 315                 320

Val Lys Ala Val Val Val Val Leu Gly Val Asn Asp Val Leu Asn Ser
                325                 330                 335

Pro Glu Leu Ala Asp Arg Asp Ala Ile Leu Thr Gly Leu Arg Thr Leu
            340                 345                 350

Val Asp Arg Ala His Ala Arg Gly Leu Arg Val Val Gly Ala Thr Ile
        355                 360                 365
```

```
Thr Pro Phe Gly Gly Tyr Gly Gly Tyr Thr Glu Ala Arg Glu Thr Met
    370                 375                 380

Arg Gln Glu Val Asn Glu Glu Ile Arg Ser Gly Arg Val Phe Asp Thr
385                 390                 395                 400

Val Val Asp Phe Asp Lys Ala Leu Arg Asp Pro Tyr Asp Pro Arg Arg
                405                 410                 415

Met Arg Ser Asp Tyr Asp Ser Gly Asp His Leu His Pro Gly Asp Lys
            420                 425                 430

Gly Tyr Ala Arg Met Gly Ala Val Ile Asp Leu Ala Ala Leu Lys Gly
        435                 440                 445

Ala Ala Pro Val Lys Ala
    450
```

<210> 25
<211> 1365
<212> DNA
<213> Streptomyces coelicolor

<400> 25

```
atgacccggg gtcgtgacgg gggtgcgggg gcgcccccca ccaagcaccg tgccctgctc      60
gcggcgatcg tcaccctgat agtggcgatc tccgcggcca tatacgccgg agcgtccgcg     120
gacgacggca gcagggacca cgcgctgcag gccggaggcc gtctcccacg aggagacgcc     180
gcccccgcgt ccaccggtgc ctgggtgggc gcctgggcca ccgcaccggc cgcggccgag     240
ccgggcaccg agacgaccgg cctggcgggc cgctccgtgc gcaacgtcgt gcacacctcg     300
gtcggcggca ccggcgcgcg gatcaccctc tcgaacctgt acgggcagtc gccgctgacc     360
gtcacacacg cctcgatcgc cctggccgcc gggcccgaca ccgccgccgc gatcgccgac     420
accatgcgcc ggctcacctt cggcggcagc gcccgggtga tcatcccggc gggcggccag     480
gtgatgagcg acaccgcccg cctcgccatc ccctacgggg cgaacgtcct ggtcaccacg     540
tactccccca tcccgtccgg gccggtgacc taccatccgc aggcccggca gaccagctac     600
ctggccgacg gcgaccgcac ggcggacgtc accgccgtcg cgtacaccac ccccacgccc     660
tactggcgct acctgaccgc cctcgacgtg ctgagccacg aggccgacgg cacggtcgtg     720
gcgttcggcg actccatcac cgacggcgcc cgctcgcaga gcgacgccaa ccaccgctgg     780
accgacgtcc tcgccgcacg cctgcacgag gcggcgggcg acggccggga cacgccccgc     840
tacagcgtcg tcaacgaggg catcagcggc aaccggctcc tgaccagcag gccggggcgg     900
ccggccgaca acccgagcgg actgagccgg ttccagcggg acgtgctgga acgcaccaac     960
```

```
gtcaaggccg tcgtcgtcgt cctcggcgtc aacgacgtcc tgaacagccc ggaactcgcc   1020
gaccgcgacg ccatcctgac cggcctgcgc accctcgtcg accgggcgca cgcccgggga   1080
ctgcgggtcg tcggcgccac gatcacgccg ttcggcggct acggcggcta caccgaggcc   1140
cgcgagacga tgcggcagga ggtcaacgag gagatccgct ccggccgggt cttcgacacg   1200
gtcgtcgact tcgacaaggc cctgcgcgac ccgtacgacc cgcgccggat gcgctccgac   1260
tacgacagcg gcgaccacct gcaccccggc gacaaggggt acgcgcgcat gggcgcggtc   1320
atcgacctgg ccgcgctgaa gggcgcggcg ccggtcaagg cgtag                   1365
```

<210> 26
<211> 340
<212> PRT
<213> Streptomyces coelicolor

<400> 26

```
Met Thr Ser Met Ser Arg Ala Arg Val Ala Arg Arg Ile Ala Ala Gly
1               5                   10                  15

Ala Ala Tyr Gly Gly Gly Gly Ile Gly Leu Ala Gly Ala Ala Ala Val
            20                  25                  30

Gly Leu Val Val Ala Glu Val Gln Leu Ala Arg Arg Arg Val Gly Val
        35                  40                  45

Gly Thr Pro Thr Arg Val Pro Asn Ala Gln Gly Leu Tyr Gly Gly Thr
    50                  55                  60

Leu Pro Thr Ala Gly Asp Pro Pro Leu Arg Leu Met Met Leu Gly Asp
65                  70                  75                  80

Ser Thr Ala Ala Gly Gln Gly Val His Arg Ala Gly Gln Thr Pro Gly
                85                  90                  95

Ala Leu Leu Ala Ser Gly Leu Ala Ala Val Ala Glu Arg Pro Val Arg
            100                 105                 110

Leu Gly Ser Val Ala Gln Pro Gly Ala Cys Ser Asp Asp Leu Asp Arg
        115                 120                 125

Gln Val Ala Leu Val Leu Ala Glu Pro Asp Arg Val Pro Asp Ile Cys
    130                 135                 140

Val Ile Met Val Gly Ala Asn Asp Val Thr His Arg Met Pro Ala Thr
145                 150                 155                 160

Arg Ser Val Arg His Leu Ser Ser Ala Val Arg Arg Leu Arg Thr Ala
                165                 170                 175
```

```
Gly Ala Glu Val Val Val Gly Thr Cys Pro Asp Leu Gly Thr Ile Glu
            180                 185                 190

Arg Val Arg Gln Pro Leu Arg Trp Leu Ala Arg Arg Ala Ser Arg Gln
        195                 200                 205

Leu Ala Ala Ala Gln Thr Ile Gly Ala Val Glu Gln Gly Gly Arg Thr
    210                 215                 220

Val Ser Leu Gly Asp Leu Leu Gly Pro Glu Phe Ala Gln Asn Pro Arg
225                 230                 235                 240

Glu Leu Phe Gly Pro Asp Asn Tyr His Pro Ser Ala Glu Gly Tyr Ala
                245                 250                 255

Thr Ala Ala Met Ala Val Leu Pro Ser Val Cys Ala Ala Leu Gly Leu
            260                 265                 270

Trp Pro Ala Asp Glu Glu His Pro Asp Ala Leu Arg Arg Glu Gly Phe
        275                 280                 285

Leu Pro Val Ala Arg Ala Ala Ala Glu Ala Ala Ser Glu Ala Gly Thr
    290                 295                 300

Glu Val Ala Ala Ala Met Pro Thr Gly Pro Arg Gly Pro Trp Ala Leu
305                 310                 315                 320

Leu Lys Arg Arg Arg Arg Arg Arg Val Ser Glu Ala Glu Pro Ser Ser
                325                 330                 335

Pro Ser Gly Val
            340
```

<210> 27
<211> 1023
<212> DNA
<213> Streptomyces coelicolor

<400> 27

```
atgacgagca tgtcgagggc gagggtggcg cggcggatcg cggccggcgc ggcgtacggc    60
ggcggcggca tcggcctggc gggagcggcg gcggtcggtc tggtggtggc cgaggtgcag   120
ctggccagac gcagggtggg ggtgggcacg ccgacccggg tgccgaacgc gcagggactg   180
tacggcggca ccctgcccac ggccggcgac ccgccgctgc ggctgatgat gctgggcgac   240
tccacggccg ccgggcaggg cgtgcaccgg gccgggcaga cgccgggcgc gctgctggcg   300
tccgggctcg cggcggtggc ggagcggccg gtgcggctgg ggtcggtcgc ccagccgggg   360

gcgtgctcgg acgacctgga ccggcaggtg gcgctggtgc tcgccgagcc ggaccgggtg   420
cccgacatct gcgtgatcat ggtcggcgcc aacgacgtca cccaccggat gccggcgacc   480
cgctcggtgc ggcacctgtc ctcggcggta cggcggctgc gcacggccgg tgcggaggtg   540
gtggtcggca cctgtccgga cctgggcacg atcgagcggg tgcggcagcc gctgcgctgg   600
ctggcccggc gggcctcacg gcagctcgcg gcggcacaga ccatcggcgc cgtcgagcag   660
ggcgggcgca cggtgtcgct gggcgacctg ctgggtccgg agttcgcgca gaacccgcgg   720
gagctcttcg gccccgacaa ctaccacccc tccgccgagg ggtacgccac ggccgcgatg   780
gcggtactgc cctcggtgtg cgccgcgctc ggcctgtggc cggccgacga ggagcacccg   840
gacgcgctgc gccgcgaggg cttcctgccg gtggcgcgcg cggcggcgga ggcggcgtcc   900
gaggcgggta cggaggtcgc cgccgccatg cctacggggc ctcgggggcc ctgggcgctg   960
ctgaagcgcc ggagacggcg tcgggtgtcg gaggcggaac cgtccagccc gtccggcgtt  1020
tga                                                                1023
```

<210> 28
<211> 305
<212> PRT
<213> Streptomyces coelicolor

<400> 28

```
Met Gly Arg Gly Thr Asp Gln Arg Thr Arg Tyr Gly Arg Arg Arg Ala
1               5                   10                  15

Arg Val Ala Leu Ala Ala Leu Thr Ala Ala Val Leu Gly Val Gly Val
            20                  25                  30

Ala Gly Cys Asp Ser Val Gly Gly Asp Ser Pro Ala Pro Ser Gly Ser
        35                  40                  45

Pro Ser Lys Arg Thr Arg Thr Ala Pro Ala Trp Asp Thr Ser Pro Ala
    50                  55                  60

Ser Val Ala Ala Val Gly Asp Ser Ile Thr Arg Gly Phe Asp Ala Cys
65                  70                  75                  80

Ala Val Leu Ser Asp Cys Pro Glu Val Ser Trp Ala Thr Gly Ser Ser
                85                  90                  95

Ala Lys Val Asp Ser Leu Ala Val Arg Leu Leu Gly Lys Ala Asp Ala
            100                 105                 110

Ala Glu His Ser Trp Asn Tyr Ala Val Thr Gly Ala Arg Met Ala Asp
        115                 120                 125
```

```
Leu Thr Ala Gln Val Thr Arg Ala Ala Gln Arg Glu Pro Glu Leu Val
    130                 135                 140

Ala Val Met Ala Gly Ala Asn Asp Ala Cys Arg Ser Thr Thr Ser Ala
145                 150                 155                 160

Met Thr Pro Val Ala Asp Phe Arg Ala Gln Phe Glu Glu Ala Met Ala
                165                 170                 175

Thr Leu Arg Lys Lys Leu Pro Lys Ala Gln Val Tyr Val Ser Ser Ile
            180                 185                 190

Pro Asp Leu Lys Arg Leu Trp Ser Gln Gly Arg Thr Asn Pro Leu Gly
        195                 200                 205

Lys Gln Val Trp Lys Leu Gly Leu Cys Pro Ser Met Leu Gly Asp Ala
    210                 215                 220

Asp Ser Leu Asp Ser Ala Ala Thr Leu Arg Arg Asn Thr Val Arg Asp
225                 230                 235                 240

Arg Val Ala Asp Tyr Asn Glu Val Leu Arg Glu Val Cys Ala Lys Asp
                245                 250                 255

Arg Arg Cys Arg Ser Asp Asp Gly Ala Val His Glu Phe Arg Phe Gly
            260                 265                 270

Thr Asp Gln Leu Ser His Trp Asp Trp Phe His Pro Ser Val Asp Gly
        275                 280                 285

Gln Ala Arg Leu Ala Glu Ile Ala Tyr Arg Ala Val Thr Ala Lys Asn
    290                 295                 300

Pro
305
```

<210> 29
<211> 918
<212> DNA
<213> Streptomyces coelicolor

<400> 29

atgggtcgag ggacggacca gcggacgcgg tacggccgtc gccgggcgcg tgtcgcgctc 60
gccgccctga ccgccgccgt cctgggcgtg ggcgtggcgg gctgcgactc cgtgggcggc 120
gactcacccg ctccttccgg cagcccgtcg aagcggacga ggacggcgcc cgcctgggac 180
accagcccgg cgtccgtcgc cgccgtgggc gactccatca cgcgcggctt cgacgcctgt 240
gcggtgctgt cggactgccc ggaggtgtcg tgggcgaccg gcagcagcgc gaaggtcgac 300

tcgctggccg tacggctgct ggggaaggcg gacgcggccg agcacagctg gaactacgcg 360
gtcaccgggg cccggatggc ggacctgacc gctcaggtga cgcgggcggc gcagcgcgag 420
ccggagctgg tggcggtgat ggccggggcg aacgacgcgt gccggtccac gacctcggcg 480
atgacgccgg tggcggactt ccgggcgcag ttcgaggagg cgatggccac cctgcgcaag 540
aagctcccca aggcgcaggt gtacgtgtcg agcatcccgg acctcaagcg gctctggtcc 600
cagggccgca ccaacccgct gggcaagcag gtgtggaagc tcggcctgtg cccgtcgatg 660
ctgggcgacg cggactccct ggactcggcg gcgaccctgc ggcgcaacac ggtgcgcgac 720
cgggtggcgg actacaacga ggtgctgcgg gaggtctgcg cgaaggaccg gcggtgccgc 780
agcgacgacg gcgcggtgca cgagttccgg ttcggcacgg accagttgag ccactgggac 840
tggttccacc cgagtgtgga cggccaggcc cggctggcgg agatcgccta ccgcgcggtc 900
accgcgaaga atccctga 918

<210> 30
<211> 268
<212> PRT
<213> Streptomyces rimosus

<400> 30

```
Met Arg Leu Ser Arg Arg Ala Ala Thr Ala Ser Ala Leu Leu Leu Thr
1               5                   10                  15

Pro Ala Leu Ala Leu Phe Gly Ala Ser Ala Ala Val Ser Ala Pro Arg
            20                  25                  30

Ile Gln Ala Thr Asp Tyr Val Ala Leu Gly Asp Ser Tyr Ser Ser Gly
        35                  40                  45

Val Gly Ala Gly Ser Tyr Asp Ser Ser Ser Gly Ser Cys Lys Arg Ser
    50                  55                  60

Thr Lys Ser Tyr Pro Ala Leu Trp Ala Ala Ser His Thr Gly Thr Arg
65                  70                  75                  80

Phe Asn Phe Thr Ala Cys Ser Gly Ala Arg Thr Gly Asp Val Leu Ala
                85                  90                  95

Lys Gln Leu Thr Pro Val Asn Ser Gly Thr Asp Leu Val Ser Ile Thr
            100                 105                 110

Ile Gly Gly Asn Asp Ala Gly Phe Ala Asp Thr Met Thr Thr Cys Asn
        115                 120                 125

Leu Gln Gly Glu Ser Ala Cys Leu Ala Arg Ile Ala Lys Ala Arg Ala
    130                 135                 140
```

```
Tyr Ile Gln Gln Thr Leu Pro Ala Gln Leu Asp Gln Val Tyr Asp Ala
145                 150                 155                 160

Ile Asp Ser Arg Ala Pro Ala Ala Gln Val Val Val Leu Gly Tyr Pro
                165                 170                 175

Arg Phe Tyr Lys Leu Gly Gly Ser Cys Ala Val Gly Leu Ser Glu Lys
            180                 185                 190

Ser Arg Ala Ala Ile Asn Ala Ala Ala Asp Asp Ile Asn Ala Val Thr
        195                 200                 205

Ala Lys Arg Ala Ala Asp His Gly Phe Ala Phe Gly Asp Val Asn Thr
    210                 215                 220

Thr Phe Ala Gly His Glu Leu Cys Ser Gly Ala Pro Trp Leu His Ser
225                 230                 235                 240

Val Thr Leu Pro Val Glu Asn Ser Tyr His Pro Thr Ala Asn Gly Gln
                245                 250                 255

Ser Lys Gly Tyr Leu Pro Val Leu Asn Ser Ala Thr
            260                 265
```

<210> 31
<211> 1068
<212> DNA
<213> Streptomyces rimosus

<400> 31

```
ttcatcacaa cgatgtcaca acaccggcca tccgggtcat ccctgatcgt gggaatgggt     60
gacaagcctt cccgtgacga aagggtcctg ctacatcaga aatgacagaa atcctgctca    120
gggaggttcc atgagactgt cccgacgcgc ggccacggcg tccgcgctcc tcctcacccc    180
ggcgctcgcg ctcttcggcg cgagcgccgc cgtgtccgcg ccgcgaatcc aggccaccga    240
ctacgtggcc ctcggcgact cctactcctc gggggtcggc gcgggcagct acgacagcag    300
cagtggctcc tgtaagcgca gcaccaagtc ctacccggcc ctgtgggccg cctcgcacac    360
cggtacgcgg ttcaacttca ccgcctgttc gggcgcccgc acaggagacg tgctggccaa    420
gcagctgacc ccggtcaact ccggcaccga cctggtcagc attaccatcg gcggcaacga    480
cgcgggcttc gccgacacca tgaccacctg caacctccag ggcgagagcg cgtgcctggc    540
gcggatcgcc aaggcgcgcg cctacatcca gcagacgctg cccgcccagc tggaccaggt    600
ctacgacgcc atcgacagcc gggcccccgc agcccaggtc gtcgtcctgg gctacccgcg    660
cttctacaag ctgggcggca gctgcgccgt cggtctctcg gagaagtccc gcgcggccat    720

caacgccgcc gccgacgaca tcaacgccgt caccgccaag cgcgccgccg accacggctt    780
cgccttcggg gacgtcaaca cgaccttcgc cgggcacgag ctgtgctccg gcgcccccctg   840
gctgcacagc gtcacccttc ccgtggagaa ctcctaccac cccacggcca acggacagtc    900
caagggctac ctgcccgtcc tgaactccgc cacctgatct cgcggctact ccgcccctga    960
cgaagtcccg cccccgggcg gggcttcgcc gtaggtgcgc gtaccgccgt cgcccgtcgc   1020
gccggtggcc ccgccgtacg tgccgccgcc cccggacgcg gtcggttc                1068
```

<210> 32
<211> 335
<212> PRT
<213> Aeromonas hydrophila

<400> 32

```
Met Lys Lys Trp Phe Val Cys Leu Leu Gly Leu Val Ala Leu Thr Val
1               5                   10                  15

Gln Ala Ala Asp Ser Arg Pro Ala Phe Ser Arg Ile Val Met Phe Gly
            20                  25                  30

Asp Ser Leu Ser Asp Thr Gly Lys Met Tyr Ser Lys Met Arg Gly Tyr
        35                  40                  45

Leu Pro Ser Ser Pro Pro Tyr Tyr Glu Gly Arg Phe Ser Asn Gly Pro
    50                  55                  60

Val Trp Leu Glu Gln Leu Thr Lys Gln Phe Pro Gly Leu Thr Ile Ala
65                  70                  75                  80

Asn Glu Ala Glu Gly Gly Ala Thr Ala Val Ala Tyr Asn Lys Ile Ser
                85                  90                  95

Trp Asn Pro Lys Tyr Gln Val Ile Asn Asn Leu Asp Tyr Glu Val Thr
            100                 105                 110

Gln Phe Leu Gln Lys Asp Ser Phe Lys Pro Asp Asp Leu Val Ile Leu
        115                 120                 125

Trp Val Gly Ala Asn Asp Tyr Leu Ala Tyr Gly Trp Asn Thr Glu Gln
    130                 135                 140

Asp Ala Lys Arg Val Arg Asp Ala Ile Ser Asp Ala Ala Asn Arg Met
145                 150                 155                 160

Val Leu Asn Gly Ala Lys Gln Ile Leu Leu Phe Asn Leu Pro Asp Leu
                165                 170                 175
```

```
Gly Gln Asn Pro Ser Ala Arg Ser Gln Lys Val Val Glu Ala Val Ser
            180                 185                 190

His Val Ser Ala Tyr His Asn Gln Leu Leu Leu Asn Leu Ala Arg Gln
        195                 200                 205

Leu Ala Pro Thr Gly Met Val Lys Leu Phe Glu Ile Asp Lys Gln Phe
    210                 215                 220

Ala Glu Met Leu Arg Asp Pro Gln Asn Phe Gly Leu Ser Asp Val Glu
225                 230                 235                 240

Asn Pro Cys Tyr Asp Gly Gly Tyr Val Trp Lys Pro Phe Ala Thr Arg
                245                 250                 255

Ser Val Ser Thr Asp Arg Gln Leu Ser Ala Phe Ser Pro Gln Glu Arg
            260                 265                 270

Leu Ala Ile Ala Gly Asn Pro Leu Leu Ala Gln Ala Val Ala Ser Pro
        275                 280                 285

Met Ala Arg Arg Ser Ala Ser Pro Leu Asn Cys Glu Gly Lys Met Phe
    290                 295                 300

Trp Asp Gln Val His Pro Thr Thr Val Val His Ala Ala Leu Ser Glu
305                 310                 315                 320

Arg Ala Ala Thr Phe Ile Ala Asn Gln Tyr Glu Phe Leu Ala His
                325                 330                 335
```

<210> 33
<211> 1008
<212> DNA
<213> Aeromonas hydrophila

<400> 33

```
atgaaaaaat ggtttgtgtg tttattggga ttggtcgcgc tgacagttca ggcagccgac      60
agtcgccccg ccttttcccg gatcgtgatg ttcggcgaca gcctctccga taccggcaaa     120
atgtacagca agatgcgcgg ttacctcccc tccagcccgc cctactatga gggccgtttc     180
tccaacggac ccgtctggct ggagcagctg accaaacagt tcccgggtct gaccatcgcc     240
aacgaagcgg aaggcggtgc cactgccgtg gcttacaaca agatctcctg gaatcccaag     300
tatcaggtca tcaacaacct ggactacgag gtcacccagt tcttgcagaa agacagcttc     360
aagccggacg atctggtgat cctctgggtc ggtgccaatg actatctggc ctatggctgg     420
aacacggagc aggatgccaa gcgggttcgc gatgccatca gcgatgcggc caaccgcatg     480
gtactgaacg gtgccaagca gatactgctg ttcaacctgc cggatctggg ccagaacccg     540

tcagctcgca gtcagaaggt ggtcgaggcg gtcagccatg tctccgccta tcacaaccag     600
ctgctgctga acctggcacg ccagctggcc cccaccggca tggtaaagct gttcgagatc     660
gacaagcaat ttgccgagat gctgcgtgat ccgcagaact tcggcctgag cgacgtcgag     720
aacccctgct acgacggcgg ctatgtgtgg aagccgtttg ccacccgcag cgtcagcacc     780
gaccgccagc tctccgcctt cagtccgcag gaacgcctcg ccatcgccgg caacccgctg     840
ctggcacagg ccgttgccag tcctatggcc cgccgcagcg ccagcccccct caactgtgag     900
ggcaagatgt tctgggatca ggtacacccg accactgtcg tgcacgcagc cctgagcgag     960
cgcgccgcca ccttcatcgc gaaccagtac gagttcctcg cccactga                 1008
```

<210> 34
<211> 336
<212> PRT
<213> Aeromonas salmonicida subsp. Salmonicida

<400> 34

```
Met Lys Lys Trp Phe Val Cys Leu Leu Gly Leu Ile Ala Leu Thr Val
1               5                   10                  15

Gln Ala Ala Asp Thr Arg Pro Ala Phe Ser Arg Ile Val Met Phe Gly
            20                  25                  30

Asp Ser Leu Ser Asp Thr Gly Lys Met Tyr Ser Lys Met Arg Gly Tyr
        35                  40                  45

Leu Pro Ser Ser Pro Pro Tyr Tyr Glu Gly Arg Phe Ser Asn Gly Pro
    50                  55                  60

Val Trp Leu Glu Gln Leu Thr Lys Gln Phe Pro Gly Leu Thr Ile Ala
65                  70                  75                  80

Asn Glu Ala Glu Gly Gly Ala Thr Ala Val Ala Tyr Asn Lys Ile Ser
                85                  90                  95

Trp Asn Pro Lys Tyr Gln Val Ile Asn Asn Leu Asp Tyr Glu Val Thr
            100                 105                 110

Gln Phe Leu Gln Lys Asp Ser Phe Lys Pro Asp Asp Leu Val Ile Leu
        115                 120                 125

Trp Val Gly Ala Asn Asp Tyr Leu Ala Tyr Gly Trp Asn Thr Glu Gln
    130                 135                 140

Asp Ala Lys Arg Val Arg Asp Ala Ile Ser Asp Ala Ala Asn Arg Met
145                 150                 155                 160
```

```
Val Leu Asn Gly Ala Lys Gln Ile Leu Leu Phe Asn Leu Pro Asp Leu
                165                 170                 175

Gly Gln Asn Pro Ser Ala Arg Ser Gln Lys Val Val Glu Ala Val Ser
            180                 185                 190

His Val Ser Ala Tyr His Asn Lys Leu Leu Leu Asn Leu Ala Arg Gln
        195                 200                 205

Leu Ala Pro Thr Gly Met Val Lys Leu Phe Glu Ile Asp Lys Gln Phe
    210                 215                 220

Ala Glu Met Leu Arg Asp Pro Gln Asn Phe Gly Leu Ser Asp Val Glu
225                 230                 235                 240

Asn Pro Cys Tyr Asp Gly Gly Tyr Val Trp Lys Pro Phe Ala Thr Arg
                245                 250                 255

Ser Val Ser Thr Asp Arg Gln Leu Ser Ala Phe Ser Pro Gln Glu Arg
            260                 265                 270

Leu Ala Ile Ala Gly Asn Pro Leu Leu Ala Gln Ala Val Ala Ser Pro
        275                 280                 285

Met Ala Arg Arg Ser Ala Ser Pro Leu Asn Cys Glu Gly Lys Met Phe
    290                 295                 300

Trp Asp Gln Val His Pro Thr Thr Val Val His Ala Ala Leu Ser Glu
305                 310                 315                 320

Arg Ala Ala Thr Phe Ile Glu Thr Gln Tyr Glu Phe Leu Ala His Gly
                325                 330                 335
```

<210> 35
<211> 1011
<212> DNA
<213> Aeromonas salmonicida subsp. Salmonicida

<400> 35

```
atgaaaaaat ggtttgtttg tttattgggg ttgatcgcgc tgacagttca ggcagccgac      60
actcgccccg ccttctcccg gatcgtgatg ttcggcgaca gcctctccga taccggcaaa     120
atgtacagca agatgcgcgg ttacctcccc tccagcccgc cctactatga gggccgtttc     180
tccaacggac ccgtctggct ggagcagctg accaagcagt tcccgggtct gaccatcgcc     240
aacgaagcgg aaggcggtgc cactgccgtg gcttacaaca agatctcctg gaatcccaag     300
tatcaggtca tcaacaacct ggactacgag gtcacccagt tcttgcagaa agacagcttc     360
aagccggacg atctggtgat cctctgggtc ggtgccaatg actatctggc atatggctgg     420

aatacggagc aggatgccaa gcgagttcgc gatgccatca gcgatgcggc caaccgcatg     480
gtactgaacg gtgccaagca gatactgctg ttcaacctgc cggatctggg ccagaacccg     540
tcagcccgca gtcagaaggt ggtcgaggcg gtcagccatg tctccgccta tcacaacaag     600
ctgctgctga acctggcacg ccagctggcc cccaccggca tggtaaagct gttcgagatc     660
gacaagcaat ttgccgagat gctgcgtgat ccgcagaact tcggcctgag cgacgtcgag     720
aacccctgct acgacggcgg ctatgtgtgg aagccgtttg ccacccgcag cgtcagcacc     780
gaccgccagc tctccgcctt cagtccgcag gaacgcctcg ccatcgccgg caacccgctg     840
ctggcacagg ccgttgccag tcctatggcc cgccgcagcg ccagccccct caactgtgag     900
ggcaagatgt tctgggatca ggtacacccg accactgtcg tgcacgcagc cctgagcgag     960
cgcgccgcca ccttcatcga gacccagtac gagttcctcg cccacggatg a             1011
```

<210> 36
<211> 347
<212> PRT
<213> Artificial

<220>
<223> fusion construct

<400> 36

```
Met Phe Lys Phe Lys Lys Asn Phe Leu Val Gly Leu Ser Ala Ala Leu
1               5                   10                  15

Met Ser Ile Ser Leu Phe Ser Ala Thr Ala Ser Ala Ala Ser Ala Asp
            20                  25                  30

Ser Arg Pro Ala Phe Ser Arg Ile Val Met Phe Gly Asp Ser Leu Ser
        35                  40                  45

Asp Thr Gly Lys Met Tyr Ser Lys Met Arg Gly Tyr Leu Pro Ser Ser
    50                  55                  60

Pro Pro Tyr Tyr Glu Gly Arg Phe Ser Asn Gly Pro Val Trp Leu Glu
65                  70                  75                  80

Gln Leu Thr Lys Gln Phe Pro Gly Leu Thr Ile Ala Asn Glu Ala Glu
                85                  90                  95

Gly Gly Ala Thr Ala Val Ala Tyr Asn Lys Ile Ser Trp Asn Pro Lys
            100                 105                 110

Tyr Gln Val Ile Asn Asn Leu Asp Tyr Glu Val Thr Gln Phe Leu Gln
        115                 120                 125

Lys Asp Ser Phe Lys Pro Asp Asp Leu Val Ile Leu Trp Val Gly Ala
```

```
    130                 135                 140

Asn Asp Tyr Leu Ala Tyr Gly Trp Asn Thr Glu Gln Asp Ala Lys Arg
145                 150                 155                 160

Val Arg Asp Ala Ile Ser Asp Ala Ala Asn Arg Met Val Leu Asn Gly
                165                 170                 175

Ala Lys Gln Ile Leu Leu Phe Asn Leu Pro Asp Leu Gly Gln Asn Pro
            180                 185                 190

Ser Ala Arg Ser Gln Lys Val Val Glu Ala Val Ser His Val Ser Ala
        195                 200                 205

Tyr His Asn Gln Leu Leu Leu Asn Leu Ala Arg Gln Leu Ala Pro Thr
    210                 215                 220

Gly Met Val Lys Leu Phe Glu Ile Asp Lys Gln Phe Ala Glu Met Leu
225                 230                 235                 240

Arg Asp Pro Gln Asn Phe Gly Leu Ser Asp Val Glu Asn Pro Cys Tyr
                245                 250                 255

Asp Gly Gly Tyr Val Trp Lys Pro Phe Ala Thr Arg Ser Val Ser Thr
            260                 265                 270

Asp Arg Gln Leu Ser Ala Phe Ser Pro Gln Glu Arg Leu Ala Ile Ala
        275                 280                 285

Gly Asn Pro Leu Leu Ala Gln Ala Val Ala Ser Pro Met Ala Arg Arg
    290                 295                 300

Ser Ala Ser Pro Leu Asn Cys Glu Gly Lys Met Phe Trp Asp Gln Val
305                 310                 315                 320

His Pro Thr Thr Val Val His Ala Ala Leu Ser Glu Arg Ala Ala Thr
                325                 330                 335

Phe Ile Ala Asn Gln Tyr Glu Phe Leu Ala His
            340                 345
```

<210> 37
<211> 27
<212> DNA
<213> Artificial

<220>
<223> Primer asls950new

<400> 37
gtgatggtgg gcgaggaact cgtactg 27

<210> 38
<211> 35
<212> DNA
<213> Artificial

<220>
<223> Primer 1USNEW

<400> 38
agcatatgaa aaaatggttt gtttgtttat tgggg 35

<210> 39
<211> 39
<212> DNA
<213> Artificial

<220>
<223> Primer AHLS1001

<400> 39
ttggatccga attcatcaat ggtgatggtg atggtgggc 39

<210> 40
<211> 18
<212> DNA
<213> Artificial

<220>
<223> T7 promoter primer

<400> 40
taatacgact cactatag 18

<210> 41
<211> 18
<212> DNA
<213> Artificial

<220>
<223> T7 terminator primer

<400> 41
ctagttattg ctcagcgg 18

<210> 42
<211> 41
<212> DNA
<213> Artificial

<220>
<223> Primer AHUS1

<400> 42
gtcatatgaa aaaatggttt gtgtgtttat tgggattggt c 41

<210> 43
<211> 30
<212> DNA
<213> Artificial

<220>
<223> Primer ahls950

<400> 43
atggtgatgg tgggcgagga actcgtactg 30

<210> 44
<211> 41
<212> DNA
<213> Artificial

<220>
<223> Primer AHUS1

<400> 44
gtcatatgaa aaaatggttt gtgtgtttat tgggattggt c 41

<210> 45
<211> 2094
<212> DNA
<213> Aeromonas hydrophila

<400> 45

```
atgtttaagt ttaaaaagaa tttcttagtt ggattatcgg cagctttaat tacaaattca      60
aatttttctt aaagaatcaa cctaatagcc gtcgaaatta gagtattagc ttgttttcgg     120
caaccgcctc tgcagctagc gccgacagcc ctcataatcg aacaaaagcc gttggcggag     180
acgtcgatcg cggctgtcgg gtcccgcctt ttcccggatc gtgatgttcg gcgacagcct     240
ctccgatacc cagggcggaa aagggcctag cactacaagc cgctgtcgga gaggctatgg     300
ggcaaaatgt acagcaagat gcgcggttac ctcccctcca gcccgcccta ccgttttaca     360
tgtcgttcta cgcgccaatg gaggggaggt cgggcgggat ctatgagggc cgtttctcca     420
acggacccgt ctggctggag cagctgacca gatactcccg gcaaagaggt tgcctgggca     480
gaccgacctc gtcgactggt aacagttccc gggtctgacc atcgccaacg aagcggaagg     540
cggtgccact ttgtcaaggg cccagactgg tagcggttgc ttcgccttcc gccacggtga     600
gccgtggctt acaacaagat ctcctggaat cccaagtatc aggtcatcaa cggcaccgaa     660
tgttgttcta gaggacctta gggttcatag tccagtagtt caacctggac tacgaggtca     720
cccagttctt gcagaaagac agcttcaagc gttggacctg atgctccagt gggtcaagaa     780
cgtctttctg tcgaagttcg cggacgatct ggtgatcctc tgggtcggtg ccaatgacta     840
tctggcctat gcctgctaga ccactaggag acccagccac ggttactgat agaccggata     900
ggctggaaca cggagcagga tgccaagcgg gttcgcgatg ccatcagcga ccgaccttgt     960
gcctcgtcct acggttcgcc caagcgctac ggtagtcgct tgcggccaac cgcatggtac    1020
tgaacggtgc caagcagata ctgctgttca acgccggttg gcgtaccatg acttgccacg    1080
```

gttcgtctat gacgacaagt acctgccgga tctgggccag aacccgtcag ctcgcagtca 1140
gaaggtggtc tggacggcct agacccggtc ttgggcagtc gagcgtcagt cttccaccag 1200
gaggcggtca gccatgtctc cgcctatcac aaccagctgc tgctgaacct ctccgccagt 1260
cggtacagag gcggatagtg ttggtcgacg acgacttgga ggcacgccag ctggccccca 1320
ccggcatggt aaagctgttc gagatcgaca ccgtgcggtc gaccgggggt ggccgtacca 1380
tttcgacaag ctctagctgt agcaatttgc cgagatgctg cgtgatccgc agaacttcgg 1440
cctgagcgac tcgttaaacg gctctacgac gcactaggcg tcttgaagcc ggactcgctg 1500
gtcgagaacc cctgctacga cggcggctat gtgtggaagc cgtttgccac cagctcttgg 1560
ggacgatgct gccgccgata cacaccttcg gcaaacggtg ccgcagcgtc agcaccgacc 1620
gccagctctc cgccttcagt ccgcaggaac ggcgtcgcag tcgtggctgg cggtcgagag 1680
gcggaagtca ggcgtccttg gcctcgccat cgccggcaac ccgctgctgg cacaggccgt 1740
tgccagtcct cggagcggta gcggccgttg ggcgacgacc gtgtccggca acggtcagga 1800
atggcccgcc gcagcgccag ccccctcaac tgtgagggca agatgttctg taccgggcgg 1860
cgtcgcggtc gggggagttg acactcccgt tctacaagac ggatcaggta cacccgacca 1920
ctgtcgtgca cgcagccctg agcgagcgcg cctagtccat gtgggctggt gacagcacgt 1980
gcgtcgggac tcgctcgcgc ccgccacctt catcgcgaac cagtacgagt tcctcgccca 2040
ctgatgaggc ggtggaagta gcgcttggtc atgctcaagg agcgggtgac tact 2094

<210> 46
<211> 26
<212> DNA
<213> Artificial

<220>
<223> Primer usAHncol

<400> 46
atgccatggc cgacagccgt cccgcc 26

<210> 47
<211> 27
<212> DNA
<213> Artificial

<220>
<223> Primer lsAH

<400> 47
ttggatccga attcatcaat ggtgatg 27

<210> 48
<211> 26
<212> DNA

<213> Artificial

<220>
<223> Primer US-Ahnhel

<400> 48
ttgctagcgc cgacagccgt cccgcc 26

<210> 49
<211> 27
<212> DNA
<213> Artificial

<220>
<223> Primer lsAH

<400> 49
ttggatccga attcatcaat ggtgatg 27

<210> 50
<211> 26
<212> DNA
<213> Artificial

<220>
<223> Primer US-Asncol

<400> 50
ttgccatggc cgacactcgc cccgcc 26

<210> 51
<211> 27
<212> DNA
<213> Artificial

<220>
<223> Primer lsAH

<400> 51
ttggatccga attcatcaat ggtgatg 27

<210> 52
<211> 26
<212> DNA
<213> Artificial

<220>
<223> Primer US-ASnhe1

<400> 52
ttgctagcgc cgacactcgc cccgcc 26

<210> 53
<211> 27
<212> DNA
<213> Artificial

<220>

<223> Primer IsAH

<400> 53
ttggatccga attcatcaat ggtgatg 27

<210> 54
<211> 1371
<212> DNA
<213> Streptomyces spp

<400> 54

```
acaggccgat gcacggaacc gtacctttcc gcagtgaagc gctctccccc catcgttcgc     60
cgggacttca tccgcgattt tggcatgaac acttccttca acgcgcgtag cttgctacaa    120
gtgcggcagc agacccgctc gttggaggct cagtgagatt gacccgatcc ctgtcggccg    180
catccgtcat cgtcttcgcc ctgctgctcg cgctgctggg catcagcccg gcccaggcag    240
ccggcccggc ctatgtggcc ctgggggatt cctattcctc gggcaacggc gccggaagtt    300
acatcgattc gagcggtgac tgtcaccgca gcaacaacgc gtaccccgcc cgctgggcgg    360
cggccaacgc accgtcctcc ttcaccttcg cggcctgctc gggagcggtg accacggatg    420
tgatcaacaa tcagctgggc gccctcaacg cgtccaccgg cctggtgagc atcaccatcg    480
gcggcaatga cgcgggcttc gcggacgcga tgaccacctg cgtcaccagc tcggacagca    540
cctgcctcaa ccggctggcc accgccacca actacatcaa caccaccctg ctcgcccggc    600
tcgacgcggt ctacagccag atcaaggccc gtgcccccaa cgcccgcgtg gtcgtcctcg    660
gctacccgcg catgtacctg gcctcgaacc cctggtactg cctgggcctg agcaacacca    720
agcgcgcggc catcaacacc accgccgaca ccctcaactc ggtgatctcc tcccgggcca    780
ccgcccacgg attccgattc ggcgatgtcc gcccgacctt caacaaccac gaactgttct    840
tcggcaacga ctggctgcac tcactcaccc tgccggtgtg ggagtcgtac caccccacca    900
gcacgggcca tcagagcggc tatctgccgg tcctcaacgc caacagctcg acctgatcaa    960
cgcacggccg tgcccgcccc gcgcgtcacg ctcggcgcgg gcgccgcagc gcgttgatca   1020
gcccacagtg ccggtgacgg tcccaccgtc acggtcgagg gtgtacgtca cggtggcgcc   1080
gctccagaag tggaacgtca gcaggaccgt ggagccgtcc ctgacctcgt cgaagaactc   1140
cggggtcagc gtgatcaccc ctcccccgta gccgggggcg aaggcggcgc cgaactcctt   1200
gtaggacgtc cagtcgtgcg gcccggcgtt gccaccgtcc gcgtagaccg cttccatggt   1260
cgccagccgg tccccgcgga actcggtggg gatgtccgtg cccaaggtgg tcccggtggt   1320
gtccgagagc accgggggct cgtaccggat gatgtgcaga tccaaagaat t             1371
```

<210> 55
<211> 267
<212> PRT

<213> Streptomyces spp

<400> 55

```
Met Arg Leu Thr Arg Ser Leu Ser Ala Ala Ser Val Ile Val Phe Ala
1               5                   10                  15

Leu Leu Leu Ala Leu Leu Gly Ile Ser Pro Ala Gln Ala Ala Gly Pro
            20                  25                  30

Ala Tyr Val Ala Leu Gly Asp Ser Tyr Ser Ser Gly Asn Gly Ala Gly
        35                  40                  45

Ser Tyr Ile Asp Ser Ser Gly Asp Cys His Arg Ser Asn Asn Ala Tyr
    50                  55                  60

Pro Ala Arg Trp Ala Ala Ala Asn Ala Pro Ser Ser Phe Thr Phe Ala
65                  70                  75                  80

Ala Cys Ser Gly Ala Val Thr Thr Asp Val Ile Asn Asn Gln Leu Gly
                85                  90                  95

Ala Leu Asn Ala Ser Thr Gly Leu Val Ser Ile Thr Ile Gly Gly Asn
            100                 105                 110

Asp Ala Gly Phe Ala Asp Ala Met Thr Thr Cys Val Thr Ser Ser Asp
        115                 120                 125

Ser Thr Cys Leu Asn Arg Leu Ala Thr Ala Thr Asn Tyr Ile Asn Thr
    130                 135                 140

Thr Leu Leu Ala Arg Leu Asp Ala Val Tyr Ser Gln Ile Lys Ala Arg
145                 150                 155                 160

Ala Pro Asn Ala Arg Val Val Val Leu Gly Tyr Pro Arg Met Tyr Leu
                165                 170                 175

Ala Ser Asn Pro Trp Tyr Cys Leu Gly Leu Ser Asn Thr Lys Arg Ala
            180                 185                 190

Ala Ile Asn Thr Thr Ala Asp Thr Leu Asn Ser Val Ile Ser Ser Arg
        195                 200                 205

Ala Thr Ala His Gly Phe Arg Phe Gly Asp Val Arg Pro Thr Phe Asn
    210                 215                 220

Asn His Glu Leu Phe Phe Gly Asn Asp Trp Leu His Ser Leu Thr Leu
225                 230                 235                 240

Pro Val Trp Glu Ser Tyr His Pro Thr Ser Thr Gly His Gln Ser Gly
                245                 250                 255
```

Tyr Leu Pro Val Leu Asn Ala Asn Ser Ser Thr
260 265

<210> 56
<211> 35
<212> DNA
<213> Artificial

<220>
<223> Primer 1USNEW

<400> 56
agcatatgaa aaaatggttt gtttgtttat tgggg 35

<210> 57
<211> 39
<212> DNA
<213> Artificial

<220>
<223> Primer AHLS1001

<400> 57
ttggatccga attcatcaat ggtgatggtg atggtgggc 39

<210> 58
<211> 548
<212> PRT
<213> Thermobifida fusca

<400> 58

```
Met Leu Pro His Pro Ala Gly Glu Arg Gly Glu Val Gly Ala Phe Phe
1               5                   10                  15

Ala Leu Leu Val Gly Thr Pro Gln Asp Arg Arg Leu Arg Leu Glu Cys
            20                  25                  30

His Glu Thr Arg Pro Leu Arg Gly Arg Cys Gly Cys Gly Glu Arg Arg
        35                  40                  45

Val Pro Pro Leu Thr Leu Pro Gly Asp Gly Val Leu Cys Thr Thr Ser
    50                  55                  60

Ser Thr Arg Asp Ala Glu Thr Val Trp Arg Lys His Leu Gln Pro Arg
65                  70                  75                  80

Pro Asp Gly Gly Phe Arg Pro His Leu Gly Val Gly Cys Leu Leu Ala
                85                  90                  95

Gly Gln Gly Ser Pro Gly Val Leu Trp Cys Gly Arg Glu Gly Cys Arg
            100                 105                 110

Phe Glu Val Cys Arg Arg Asp Thr Pro Gly Leu Ser Arg Thr Arg Asn
```

115 120 125

Gly Asp Ser Ser Pro Pro Phe Arg Ala Gly Trp Ser Leu Pro Pro Lys
130 135 140

Cys Gly Glu Ile Ser Gln Ser Ala Arg Lys Thr Pro Ala Val Pro Arg
145 150 155 160

Tyr Ser Leu Leu Arg Thr Asp Arg Pro Asp Gly Pro Arg Gly Arg Phe
165 170 175

Val Gly Ser Gly Pro Arg Ala Ala Thr Arg Arg Arg Leu Phe Leu Gly
180 185 190

Ile Pro Ala Leu Val Leu Val Thr Ala Leu Thr Leu Val Leu Ala Val
195 200 205

Pro Thr Gly Arg Glu Thr Leu Trp Arg Met Trp Cys Glu Ala Thr Gln
210 215 220

Asp Trp Cys Leu Gly Val Pro Val Asp Ser Arg Gly Gln Pro Ala Glu
225 230 235 240

Asp Gly Glu Phe Leu Leu Leu Ser Pro Val Gln Ala Ala Thr Trp Gly
245 250 255

Asn Tyr Tyr Ala Leu Gly Asp Ser Tyr Ser Ser Gly Asp Gly Ala Arg
260 265 270

Asp Tyr Tyr Pro Gly Thr Ala Val Lys Gly Gly Cys Trp Arg Ser Ala
275 280 285

Asn Ala Tyr Pro Glu Leu Val Ala Glu Ala Tyr Asp Phe Ala Gly His
290 295 300

Leu Ser Phe Leu Ala Cys Ser Gly Gln Arg Gly Tyr Ala Met Leu Asp
305 310 315 320

Ala Ile Asp Glu Val Gly Ser Gln Leu Asp Trp Asn Ser Pro His Thr
325 330 335

Ser Leu Val Thr Ile Gly Ile Gly Gly Asn Asp Leu Gly Phe Ser Thr
340 345 350

Val Leu Lys Thr Cys Met Val Arg Val Pro Leu Leu Asp Ser Lys Ala
355 360 365

Cys Thr Asp Gln Glu Asp Ala Ile Arg Lys Arg Met Ala Lys Phe Glu

```
        370                 375                 380

Thr Thr Phe Glu Glu Leu Ile Ser Glu Val Arg Thr Arg Ala Pro Asp
385                 390                 395                 400

Ala Arg Ile Leu Val Val Gly Tyr Pro Arg Ile Phe Pro Glu Glu Pro
                405                 410                 415

Thr Gly Ala Tyr Tyr Thr Leu Thr Ala Ser Asn Gln Arg Trp Leu Asn
            420                 425                 430

Glu Thr Ile Gln Glu Phe Asn Gln Gln Leu Ala Glu Ala Val Ala Val
        435                 440                 445

His Asp Glu Glu Ile Ala Ala Ser Gly Gly Val Gly Ser Val Glu Phe
    450                 455                 460

Val Asp Val Tyr His Ala Leu Asp Gly His Glu Ile Gly Ser Asp Glu
465                 470                 475                 480

Pro Trp Val Asn Gly Val Gln Leu Arg Asp Leu Ala Thr Gly Val Thr
                485                 490                 495

Val Asp Arg Ser Thr Phe His Pro Asn Ala Ala Gly His Arg Ala Val
            500                 505                 510

Gly Glu Arg Val Ile Glu Gln Ile Glu Thr Gly Pro Gly Arg Pro Leu
        515                 520                 525

Tyr Ala Thr Phe Ala Val Val Ala Gly Ala Thr Val Asp Thr Leu Ala
    530                 535                 540

Gly Glu Val Gly
545
```

<210> 59
<211> 3000
<212> DNA
<213> Thermobifida fusca

<400> 59

```
ggtggtgaac cagaacaccc ggtcgtcggc gtgggcgtcc aggtgcaggt gcaggttctt     60
caactgctcc agcaggatgc cgccgtggcc gtgcacgatg gccttgggca ggcctgtggt    120
ccccgacgag tacagcaccc atagcggatg gtcgaacggc agcggggtga actccagttc    180
cgcgccttcg cccgcggctt cgaactccgc ccaggacagg gtgtcggcga cagggccgca    240
gcccaggtac ggcaggacga cggtgtgctg caggctgggc atgccgtcgc gcagggcttt    300
gagcacgtca cggcggtcga agtccttacc gccgtagcgg tagccgtcca cggccagcag    360
```

cactttcggt tcgatctgcg cgaaccggtc gaggacgctg cgcaccccga agtcggggga 420
acaggacgac caggtcgcac cgatcgcggc gcaggcgagg aatgcggccg tcgcctcggc 480
gatgttcggc aggtaggcca cgacccggtc gccggggccc accccgaggc tgcggagggc 540
cgcagcgatc gcggcggtgc gggtccgcag ttctccccag gtccactcgg tcaacggccg 600
gagttcggac gcgtgccgga tcgccacggc tgatgggtca cggtcgcgga agatgtgctc 660
ggcgtagttg agggtggcgc cggggaacca gacggcgccg ggcatggcgt cggaggcgag 720
cactgtggtg tacggggtgg cggcgcgcac ccggtagtac tcccagatcg cggaccagaa 780
tccttcgagg tcggttaccg accagcgcca cagtgcctcg tagtccggtg cgtccacacc 840
gcggtgctcc cgcacccagc gggtgaacgc ggtgaggttg gcgcgttctt tgcgctcctc 900
gtcgggactc cacaggatcg gcggctgcgg cttgagtgtc atgaaacgcg acccccttcgt 960
ggacggtgcg gatgcggtga gcgtcgggtg cctcccctaa cgctccccgg tgacggagtg 1020
ttgtgcacca catctagcac gcgggacgcg gaaaccgtat ggagaaaaca cctacaaccc 1080
cggccggacg gtgggtttcg gccacactta ggggtcgggt gcctgcttgc cgggcagggc 1140
agtcccgggg tgctgtggtg cgggcgggag ggctgtcgct tcgaggtgtg ccggcgggac 1200
actccgggcc tcagccgtac ccgcaacggg gacagttctc ctcccttccg ggctggatgg 1260
tcccttcccc cgaaatgcgg cgagatctcc cagtcagccc ggaaaacacc cgctgtgccc 1320
aggtactctt tgcttcgaac agacaggccg gacggtccac gggggaggtt tgtgggcagc 1380
ggaccacgtg cggcgaccag acgacggttg ttcctcggta tccccgctct tgtacttgtg 1440
acagcgctca cgctggtctt ggctgtcccg acggggcgcg agacgctgtg gcgcatgtgg 1500
tgtgaggcca cccaggactg gtgcctgggg gtgccggtcg actcccgcgg acagcctgcg 1560
gaggacggcg agtttctgct gctttctccg gtccaggcag cgacctgggg gaactattac 1620
gcgctcgggg attcgtactc ttcgggggac ggggcccgcg actactatcc cggcaccgcg 1680
gtgaagggcg gttgctggcg gtccgctaac gcctatccgg agctggtcgc cgaagcctac 1740
gacttcgccg gacacttgtc gttcctggcc tgcagcggcc agcgcggcta cgccatgctt 1800
gacgctatcg acgaggtcgg ctcgcagctg gactggaact cccctcacac gtcgctggtg 1860
acgatcggga tcggcggcaa cgatctgggg ttctccacgg ttttgaagac ctgcatggtg 1920
cgggtgccgc tgctggacag caaggcgtgc acggaccagg aggacgctat ccgcaagcgg 1980
atggcgaaat tcgagacgac gtttgaagag ctcatcagcg aagtgcgcac ccgcgcgccg 2040
gacgcccgga tccttgtcgt gggctacccc cggatttttc cggaggaacc gaccggcgcc 2100
tactacacgc tgaccgcgag caaccagcgg tggctcaacg aaaccattca ggagttcaac 2160
cagcagctcg ccgaggctgt cgcggtccac gacgaggaga ttgccgcgtc gggcggggtg 2220
ggcagcgtgg agttcgtgga cgtctaccac gcgttggacg gccacgagat cggctcggac 2280

```
gagccgtggg tgaacggggt gcagttgcgg gacctcgcca ccggggtgac tgtggaccgc   2340
agtaccttcc accccaacgc cgctgggcac cgggcggtcg gtgagcgggt catcgagcag   2400
atcgaaaccg gcccgggccg tccgctctat gccactttcg cggtggtggc gggggcgacc   2460
gtggacactc tcgcgggcga ggtggggtga cccggcttac cgtccggccc gcaggtctgc   2520
gagcactgcg gcgatctggt ccactgccca gtgcagttcg tcttcggtga tgaccagcgg   2580
cggggagagc cggatcgttg agccgtgcgt gtctttgacg agcacacccc gctgcaggag   2640
ccgttcgcac agttctcttc cggtggccag agtcgggtcg acgtcgatcc cagcccacag   2700
gccgatgctg cgggccgcga ccacgccgtt gccgaccagt tggtcgaggc gggcgcgcag   2760
cacgggggcg agggcgcgga catggtccag gtaagggccg tcgcggacga ggctcaccac   2820
ggcagtgccg accgcgcagg cgagggcgtt gccgccgaag gtgctgccgt gctggccggg   2880
gcggatcacg tcgaagactt ccgcgtcgcc taccgccgcc gccacgggca ggatgccgcc   2940
gcccagcgct ttgccgaaca ggtagatatc ggcgtcgact ccgctgtggt cgcaggcccg   3000
```

<210> 60
<211> 372
<212> PRT
<213> Thermobifida fusca

<400> 60

```
Val Gly Ser Gly Pro Arg Ala Ala Thr Arg Arg Arg Leu Phe Leu Gly
1               5                   10                  15

Ile Pro Ala Leu Val Leu Val Thr Ala Leu Thr Leu Val Leu Ala Val
            20                  25                  30

Pro Thr Gly Arg Glu Thr Leu Trp Arg Met Trp Cys Glu Ala Thr Gln
        35                  40                  45

Asp Trp Cys Leu Gly Val Pro Val Asp Ser Arg Gly Gln Pro Ala Glu
    50                  55                  60

Asp Gly Glu Phe Leu Leu Leu Ser Pro Val Gln Ala Ala Thr Trp Gly
65                  70                  75                  80

Asn Tyr Tyr Ala Leu Gly Asp Ser Tyr Ser Ser Gly Asp Gly Ala Arg
                85                  90                  95

Asp Tyr Tyr Pro Gly Thr Ala Val Lys Gly Gly Cys Trp Arg Ser Ala
            100                 105                 110

Asn Ala Tyr Pro Glu Leu Val Ala Glu Ala Tyr Asp Phe Ala Gly His
        115                 120                 125
```

```
Leu Ser Phe Leu Ala Cys Ser Gly Gln Arg Gly Tyr Ala Met Leu Asp
    130                 135                 140

Ala Ile Asp Glu Val Gly Ser Gln Leu Asp Trp Asn Ser Pro His Thr
145                 150                 155                 160

Ser Leu Val Thr Ile Gly Ile Gly Gly Asn Asp Leu Gly Phe Ser Thr
                165                 170                 175

Val Leu Lys Thr Cys Met Val Arg Val Pro Leu Leu Asp Ser Lys Ala
            180                 185                 190

Cys Thr Asp Gln Glu Asp Ala Ile Arg Lys Arg Met Ala Lys Phe Glu
        195                 200                 205

Thr Thr Phe Glu Glu Leu Ile Ser Glu Val Arg Thr Arg Ala Pro Asp
    210                 215                 220

Ala Arg Ile Leu Val Val Gly Tyr Pro Arg Ile Phe Pro Glu Glu Pro
225                 230                 235                 240

Thr Gly Ala Tyr Tyr Thr Leu Thr Ala Ser Asn Gln Arg Trp Leu Asn
                245                 250                 255

Glu Thr Ile Gln Glu Phe Asn Gln Gln Leu Ala Glu Ala Val Ala Val
            260                 265                 270

His Asp Glu Glu Ile Ala Ala Ser Gly Gly Val Gly Ser Val Glu Phe
        275                 280                 285

Val Asp Val Tyr His Ala Leu Asp Gly His Glu Ile Gly Ser Asp Glu
    290                 295                 300

Pro Trp Val Asn Gly Val Gln Leu Arg Asp Leu Ala Thr Gly Val Thr
305                 310                 315                 320

Val Asp Arg Ser Thr Phe His Pro Asn Ala Ala Gly His Arg Ala Val
                325                 330                 335

Gly Glu Arg Val Ile Glu Gln Ile Glu Thr Gly Pro Gly Arg Pro Leu
            340                 345                 350

Tyr Ala Thr Phe Ala Val Val Ala Gly Ala Thr Val Asp Thr Leu Ala
        355                 360                 365

Gly Glu Val Gly
    370
```

<210> 61
<211> 300
<212> PRT
<213> Corynebacterium efficiens

<400> 61

```
Met Arg Thr Thr Val Ile Ala Ala Ser Ala Leu Leu Leu Leu Ala Gly
1               5                   10                  15

Cys Ala Asp Gly Ala Arg Glu Glu Thr Ala Gly Ala Pro Pro Gly Glu
            20                  25                  30

Ser Ser Gly Gly Ile Arg Glu Glu Gly Ala Glu Ala Ser Thr Ser Ile
        35                  40                  45

Thr Asp Val Tyr Ile Ala Leu Gly Asp Ser Tyr Ala Ala Met Gly Gly
    50                  55                  60

Arg Asp Gln Pro Leu Arg Gly Glu Pro Phe Cys Leu Arg Ser Ser Gly
65                  70                  75                  80

Asn Tyr Pro Glu Leu Leu His Ala Glu Val Thr Asp Leu Thr Cys Gln
                85                  90                  95

Gly Ala Val Thr Gly Asp Leu Leu Glu Pro Arg Thr Leu Gly Glu Arg
            100                 105                 110

Thr Leu Pro Ala Gln Val Asp Ala Leu Thr Glu Asp Thr Thr Leu Val
        115                 120                 125

Thr Leu Ser Ile Gly Gly Asn Asp Leu Gly Phe Gly Glu Val Ala Gly
    130                 135                 140

Cys Ile Arg Glu Arg Ile Ala Gly Glu Asn Ala Asp Asp Cys Val Asp
145                 150                 155                 160

Leu Leu Gly Glu Thr Ile Gly Glu Gln Leu Asp Gln Leu Pro Pro Gln
                165                 170                 175

Leu Asp Arg Val His Glu Ala Ile Arg Asp Arg Ala Gly Asp Ala Gln
            180                 185                 190

Val Val Val Thr Gly Tyr Leu Pro Leu Val Ser Ala Gly Asp Cys Pro
        195                 200                 205

Glu Leu Gly Asp Val Ser Glu Ala Asp Arg Arg Trp Ala Val Glu Leu
    210                 215                 220
```

```
Thr Gly Gln Ile Asn Glu Thr Val Arg Glu Ala Ala Glu Arg His Asp
225                 230                 235                 240

Ala Leu Phe Val Leu Pro Asp Asp Ala Asp Glu His Thr Ser Cys Ala
                245                 250                 255

Pro Pro Gln Gln Arg Trp Ala Asp Ile Gln Gly Gln Gln Thr Asp Ala
            260                 265                 270

Tyr Pro Leu His Pro Thr Ser Ala Gly His Glu Ala Met Ala Ala Ala
        275                 280                 285

Val Arg Asp Ala Leu Gly Leu Glu Pro Val Gln Pro
    290                 295                 300
```

<210> 62
<211> 3000
<212> DNA
<213> Corynebacterium efficiens

<400> 62

```
ttctggggtg ttatggggtt gttatcggct cgtcctgggt ggatcccgcc aggtggggta      60
ttcacggggg acttttgtgt ccaacagccg agaatgagtg ccctgagcgg tgggaatgag     120
gtgggcgggg ctgtgtcgcc atgagggggc ggcgggctct gtggtgcccc gcgacccccg     180
gccccggtga gcggtgaatg aaatccggct gtaatcagca tcccgtgccc accccgtcgg     240
ggaggtcagc gcccggagtg tctacgcagt cggatcctct cggactcggc catgctgtcg     300
gcagcatcgc gctcccgggt cttggcgtcc ctcggctgtt ctgcctgctg tccctggaag     360
gcgaaatgat caccggggag tgatacaccg gtggtctcat cccggatgcc cacttcggcg     420
ccatccggca attcgggcag ctccgggtgg aagtaggtgg catccgatgc gtcggtgacg     480
ccatagtggg cgaagatctc atcctgctcg agggtgctca ggccactctc cggatcgata     540
tcgggggcgt ccttgatggc gtccttgctg aaaccgaggt gcagcttgtg ggcttccaat     600
ttcgcaccac ggagcgggac gaggctggaa tgacggccga agagcccgtg gtggacctca     660
acgaaggtgg gtagtcccgt gtcatcattg aggaacacgc cctccaccgc acccagcttg     720
tggccggagt tgtcgtaggc gctggcatcc agaagggaaa cgatctcata tttgtcggtg     780
tgctcagaca tgatcttcct ttgctgtcgg tgtctggtac taccacggta gggctgaatg     840
caactgttat ttttctgtta ttttaggaat tggtccatat cccacaggct ggctgtggtc     900
aaatcgtcat caagtaatcc ctgtcacaca aaatgggtgg tgggagccct ggtcgcggtt     960
ccgtgggagg cgccgtgccc cgcaggatcg tcggcatcgg cggatctggc cggtaccccg    1020
cggtgaataa aatcattctg taaccttcat cacggttggt tttaggtatc cgcccctttc    1080
gtcctgaccc cgtccccggc gcgcgggagc ccgcgggttg cggtagacag gggagacgtg    1140
```

gacaccatga ggacaacggt catcgcagca agcgcattac tccttctcgc cggatgcgcg 1200
gatggggccc gggaggagac cgccggtgca ccgccgggtg agtcctccgg gggcatccgg 1260
gaggaggggg cggaggcgtc gacaagcatc accgacgtct acatcgccct cggggattcc 1320
tatgcggcga tgggcgggcg ggatcagccg ttacggggtg agccgttctg cctgcgctcg 1380
tccggtaatt acccggaact cctccacgca gaggtcaccg atctcacctg ccaggggcg 1440
gtgaccgggg atctgctcga acccaggacg ctgggggagc gcacgctgcc ggcgcaggtg 1500
gatgcgctga cggaggacac caccctggtc accctctcca tcgggggcaa tgacctcgga 1560
ttcggggagg tggcgggatg catccgggaa cggatcgccg ggagaacgc tgatgattgc 1620
gtggacctgc tgggggaaac catcggggag cagctcgatc agcttccccc gcagctggac 1680
cgcgtgcacg aggctatccg ggaccgcgcc ggggacgcgc aggttgtggt caccggttac 1740
ctgccgctcg tgtctgccgg ggactgcccc gaactggggg atgtctccga ggcggatcgt 1800
cgttgggcgg ttgagctgac cgggcagatc aacgagaccg tgcgcgaggc ggccgaacga 1860
cacgatgccc tctttgtcct gcccgacgat gccgatgagc acaccagttg tgcaccccca 1920
cagcagcgct gggcggatat ccagggccaa cagaccgatg cctatccgct gcacccgacc 1980
tccgccggcc atgaggcgat ggccgccgcc gtccgggacg cgctgggcct ggaaccggtc 2040
cagccgtagc gccgggcgcg cgcttgtcga cgaccaaccc atgccaggct gcagtcacat 2100
ccgcacatag cgcgcgcggg cgatggagta cgcaccatag aggatgagcc cgatgccgac 2160
gatgatgagc agcacactgc cgaagggttg ttccccgagg gtgcgcagag ccgagtccag 2220
acctgcggcc tgctccggat catgggccca accggcgatg acgatcaaca cccccaggat 2280
cccgaaggcg ataccacggg cgacataacc ggctgttccg gtgatgatga tcgcggtccc 2340
gacctgccct gaccccgcac ccgcctccag atcctcccgg aaatcccggg tggccccctt 2400
ccagaggttg tagacacccg cccccagtac caccagcccg gcgaccacaa ccagcaccac 2460
accccagggt tgggatagga cggtggcggt gacatcggtg gcggtctccc catcggaggt 2520
gctgccgccc cgggcgaagg tggaggtggt caccgccagg gagaagtaga ccatggccat 2580
gaccgccccc ttggcccttt ccttgaggtc ctcgcccgcc agcagctggc tcaattgcca 2640
gagtcccagg gccgccaggg cgatgacggc aacccacagg aggaactgcc cacccggagc 2700
ctccgcgatg gtggccaggg cacctgaatt cgaggcctca tcacccgaac cgccggatcc 2760
agtggcgatg cgcaccgcga tccacccgat gaggatgtgc agtatgccca ggacaatgaa 2820
accacctctg gccagggtgg tcagcgcggg gtggtcctcg gcctggtcgg cagcccgttc 2880
gatcgtccgt ttcgcggatc tggtgtcgcc cttatccata gctcccattg aaccgccttg 2940
aggggtgggc ggccactgtc agggcggatt gtgatctgaa ctgtgatgtt ccatcaaccc 3000

<210> 63
<211> 284
<212> PRT
<213> Novosphingobium aromaticivorans

<400> 63

```
Met Gly Gln Val Lys Leu Phe Ala Arg Arg Cys Ala Pro Val Leu Leu
1               5                   10                  15

Ala Leu Ala Gly Leu Ala Pro Ala Ala Thr Val Ala Arg Glu Ala Pro
            20                  25                  30

Leu Ala Glu Gly Ala Arg Tyr Val Ala Leu Gly Ser Ser Phe Ala Ala
        35                  40                  45

Gly Pro Gly Val Gly Pro Asn Ala Pro Gly Ser Pro Glu Arg Cys Gly
    50                  55                  60

Arg Gly Thr Leu Asn Tyr Pro His Leu Leu Ala Glu Ala Leu Lys Leu
65                  70                  75                  80

Asp Leu Val Asp Ala Thr Cys Ser Gly Ala Thr Thr His His Val Leu
                85                  90                  95

Gly Pro Trp Asn Glu Val Pro Pro Gln Ile Asp Ser Val Asn Gly Asp
            100                 105                 110

Thr Arg Leu Val Thr Leu Thr Ile Gly Gly Asn Asp Val Ser Phe Val
        115                 120                 125

Gly Asn Ile Phe Ala Ala Ala Cys Glu Lys Met Ala Ser Pro Asp Pro
    130                 135                 140

Arg Cys Gly Lys Trp Arg Glu Ile Thr Glu Glu Glu Trp Gln Ala Asp
145                 150                 155                 160

Glu Glu Arg Met Arg Ser Ile Val Arg Gln Ile His Ala Arg Ala Pro
                165                 170                 175

Leu Ala Arg Val Val Val Val Asp Tyr Ile Thr Val Leu Pro Pro Ser
            180                 185                 190

Gly Thr Cys Ala Ala Met Ala Ile Ser Pro Asp Arg Leu Ala Gln Ser
        195                 200                 205

Arg Ser Ala Ala Lys Arg Leu Ala Arg Ile Thr Ala Arg Val Ala Arg
    210                 215                 220

Glu Glu Gly Ala Ser Leu Leu Lys Phe Ser His Ile Ser Arg Arg His
225                 230                 235                 240
```

```
His Pro Cys Ser Ala Lys Pro Trp Ser Asn Gly Leu Ser Ala Pro Ala
                245                 250                 255

Asp Asp Gly Ile Pro Val His Pro Asn Arg Leu Gly His Ala Glu Ala
            260                 265                 270

Ala Ala Ala Leu Val Lys Leu Val Lys Leu Met Lys
        275                 280
```

<210> 64
<211> 1500
<212> DNA
<213> Novosphingobium aromaticivorans

<400> 64

tgccggaact caagcggcgt ctagccgaac tcatgcccga aagcgcgtgg cactatcccg 60
aagaccaggt ctcggacgcc agcgagcgcc tgatggccgc cgaaatcacg cgcgaacagc 120
tctaccgcca gctccacgac gagctgccct atgacagtac cgtacgtccc gagaagtacc 180
tccatcgcaa ggacggttcg atcgagatcc accagcagat cgtgattgcc cgcgagacac 240
agcgtccgat cgtgctgggc aagggtggcg cgaagatcaa ggcgatcgga gaggccgcac 300
gcaaggaact ttcgcaattg ctcgacacca aggtgcacct gttcctgcat gtgaaggtcg 360
acgagcgctg ggccgacgcc aaggaaatct acgaggaaat cggcctcgaa tgggtcaagt 420
gaagctcttc gcgcgccgct gcgccccagt acttctcgcc cttgccgggc tggctccggc 480
ggctacggtc gcgcgggaag caccgctggc cgaaggcgcg cgttacgttg cgctgggaag 540
ctccttcgcc gcaggtccgg gcgtggggcc caacgcgccc ggatcgcccg aacgctgcgg 600
ccggggcacg ctcaactacc cgcacctgct cgccgaggcg ctcaagctcg atctcgtcga 660
tgcgacctgc agcggcgcga cgacccacca cgtgctgggc ccctggaacg aggttccccc 720
tcagatcgac agcgtgaatg gcgacacccg cctcgtcacc ctgaccatcg gcggaaacga 780
tgtgtcgttc gtcggcaaca tcttcgccgc cgcttgcgag aagatggcgt cgcccgatcc 840
gcgctgcggc aagtggcggg agatcaccga ggaagagtgg caggccgacg aggagcggat 900
gcgctccatc gtacgccaga tccacgcccg cgcgcctctc gcccgggtgg tggtggtcga 960
ttacatcacg gtcctgccgc catcaggcac ttgcgctgcc atggcgattt cgccggaccg 1020
gctggcccag agccgcagcg ccgcgaaacg gcttgcccgg attaccgcac gggtcgcgcg 1080
agaagagggt gcatcgctgc tcaagttctc gcatatctcg cgccggcacc atccatgctc 1140
tgccaagccc tggagcaacg gcctttccgc cccggccgac gacggcatcc cggtccatcc 1200
gaaccggctc ggacatgctg aagcggcagc ggcgctggtc aagcttgtga aattgatgaa 1260
gtagctactg cactgatttc aaatagtatt gcctgtcagc tttccagccc ggattgttgc 1320

agcgcaacag aaacttgtcc gtaatggatt gatggtttat gtcgctcgca aattgccgtc 1380
gaagggaacg ggcgcgtcgc tcgttaacgt cctgggtgca gcagtgacgg agcgcgtgga 1440
tgagtgatac tggcggtgtc atcggtgtac gcgccgccat tcccatgcct gtacgcgccg 1500

<210> 65
<211> 268
<212> PRT
<213> Streptomyces coelicolor

<400> 65

```
Met Arg Arg Phe Arg Leu Val Gly Phe Leu Ser Ser Leu Val Leu Ala
1               5                   10                  15

Ala Gly Ala Ala Leu Thr Gly Ala Ala Thr Ala Gln Ala Ala Gln Pro
            20                  25                  30

Ala Ala Ala Asp Gly Tyr Val Ala Leu Gly Asp Ser Tyr Ser Ser Gly
        35                  40                  45

Val Gly Ala Gly Ser Tyr Ile Ser Ser Ser Gly Asp Cys Lys Arg Ser
    50                  55                  60

Thr Lys Ala His Pro Tyr Leu Trp Ala Ala Ala His Ser Pro Ser Thr
65                  70                  75                  80

Phe Asp Phe Thr Ala Cys Ser Gly Ala Arg Thr Gly Asp Val Leu Ser
                85                  90                  95

Gly Gln Leu Gly Pro Leu Ser Ser Gly Thr Gly Leu Val Ser Ile Ser
            100                 105                 110

Ile Gly Gly Asn Asp Ala Gly Phe Ala Asp Thr Met Thr Thr Cys Val
        115                 120                 125

Leu Gln Ser Glu Ser Ser Cys Leu Ser Arg Ile Ala Thr Ala Glu Ala
    130                 135                 140

Tyr Val Asp Ser Thr Leu Pro Gly Lys Leu Asp Gly Val Tyr Ser Ala
145                 150                 155                 160

Ile Ser Asp Lys Ala Pro Asn Ala His Val Val Val Ile Gly Tyr Pro
                165                 170                 175

Arg Phe Tyr Lys Leu Gly Thr Thr Cys Ile Gly Leu Ser Glu Thr Lys
            180                 185                 190

Arg Thr Ala Ile Asn Lys Ala Ser Asp His Leu Asn Thr Val Leu Ala
        195                 200                 205
```

```
Gln Arg Ala Ala Ala His Gly Phe Thr Phe Gly Asp Val Arg Thr Thr
    210                 215                 220

Phe Thr Gly His Glu Leu Cys Ser Gly Ser Pro Trp Leu His Ser Val
225                 230                 235                 240

Asn Trp Leu Asn Ile Gly Glu Ser Tyr His Pro Thr Ala Ala Gly Gln
                245                 250                 255

Ser Gly Gly Tyr Leu Pro Val Leu Asn Gly Ala Ala
            260                 265
```

<210> 66
<211> 2000
<212> DNA
<213> Streptomyces coelicolor

<400> 66

```
cccggcggcc cgtgcaggag cagcagccgg cccgcgatgt cctcgggcgt cgtcttcatc      60
aggccgtcca tcgcgtcggc gaccggcgcc gtgtagttgg cccggacctc gtcccaggtg     120
cccgcggcga tctggcgggt ggtgcggtgc gggccgcgcc gaggggagac gtaccagaag     180
cccatcgtca cgttctccgg ctgcggttcg ggctcgtccg ccgctccgtc cgtcgcctcg     240
ccgagcacct tctcggcgag gtcggcgctg gtcgccgtca ccgtgacgtc ggcgccccgg     300
ctccagcgcg agatcagcag cgtccagccg tcgccctccg ccagcgtcgc gctgcggtcg     360
tcgtcgcggg cgatccgcag cacgcgcgcg ccgggcggca gcagcgtggc gccggaccgt     420
acgcggtcga tgttcgccgc gtgcgagtac ggctgctcac ccgtggcgaa acggccgagg     480
aacagcgcgt cgacgacgtc ggacggggag tcgctgtcgt ccacgttgag ccggatcggc     540
agggcttcgt gcgggttcac ggacatgtcg ccatgatcgg gcacccggcc gccgcgtgca     600
cccgctttcc cgggcacgca cgacaggggc tttctcgccg tcttccgtcc gaacttgaac     660
gagtgtcagc catttcttgg catggacact tccagtcaac gcgcgtagct gctaccacgg     720
ttgtggcagc aatcctgcta agggaggttc catgagacgt ttccgacttg tcggcttcct     780
gagttcgctc gtcctcgccg ccggcgccgc cctcaccggg gcagcgaccg cccaggcggc     840
ccaacccgcc gccgccgacg gctatgtggc cctcggcgac tcctactcct ccggggtcgg     900
agcgggcagc tacatcagct cgagcggcga ctgcaagcgc agcacgaagg cccatcccta     960
cctgtgggcg gccgcccact cgccctccac gttcgacttc accgcctgtt ccggcgcccg    1020
tacgggtgat gttctctccg gacagctcgg cccgctcagc tccggcaccg gcctcgtctc    1080
gatcagcatc ggcggcaacg acgccggttt cgccgacacc atgacgacct gtgtgctcca    1140
gtccgagagc tcctgcctgt cgcggatcgc caccgccgag gcgtacgtcg actcgacgct    1200
```

```
gcccggcaag ctcgacggcg tctactcggc aatcagcgac aaggcgccga acgcccacgt   1260
cgtcgtcatc ggctacccgc gcttctacaa gctcggcacc acctgcatcg gcctgtccga   1320
gaccaagcgg acggcgatca acaaggcctc cgaccacctc aacaccgtcc tcgcccagcg   1380
cgccgccgcc cacggcttca ccttcggcga cgtacgcacc accttcaccg gccacgagct   1440
gtgctccggc agcccctggc tgcacagcgt caactggctg aacatcggcg agtcgtacca   1500
ccccaccgcg gccggccagt ccggtggcta cctgccggtc ctcaacggcg ccgcctgacc   1560
tcaggcggaa ggagaagaag aaggagcgga gggagacgag gagtgggagg ccccgcccga   1620
cggggtcccc gtccccgtct ccgtctccgt cccggtcccg caagtcaccg agaacgccac   1680
cgcgtcggac gtggcccgca ccggactccg cacctccacg cgcacggcac tctcgaacgc   1740
gccggtgtcg tcgtgcgtcg tcaccaccac gccgtcctgg cgcgagcgct cgccgcccga   1800
cgggaaggac agcgtccgcc accccggatc ggagaccgac ccgtccgcgg tcacccaccg   1860
gtagccgacc tccgcgggca gccgcccgac cgtgaacgtc gccgtgaacg cgggtgcccg   1920
gtcgtgcggc ggcggacagg cccccgagta gtgggtgcgc gagcccacca cggtcacctc   1980
caccgactgc gctgcggggc                                              2000
```

<210> 67
<211> 269
<212> PRT
<213> Streptomyces avermitilis

<400> 67

```
Met Arg Arg Ser Arg Ile Thr Ala Tyr Val Thr Ser Leu Leu Leu Ala
1               5                   10                  15

Val Gly Cys Ala Leu Thr Gly Ala Ala Thr Ala Gln Ala Ser Pro Ala
            20                  25                  30

Ala Ala Ala Thr Gly Tyr Val Ala Leu Gly Asp Ser Tyr Ser Ser Gly
        35                  40                  45

Val Gly Ala Gly Ser Tyr Leu Ser Ser Ser Gly Asp Cys Lys Arg Ser
    50                  55                  60

Ser Lys Ala Tyr Pro Tyr Leu Trp Gln Ala Ala His Ser Pro Ser Ser
65                  70                  75                  80

Phe Ser Phe Met Ala Cys Ser Gly Ala Arg Thr Gly Asp Val Leu Ala
                85                  90                  95

Asn Gln Leu Gly Thr Leu Asn Ser Ser Thr Gly Leu Val Ser Leu Thr
            100                 105                 110
```

```
Ile Gly Gly Asn Asp Ala Gly Phe Ser Asp Val Met Thr Thr Cys Val
        115                 120                 125

Leu Gln Ser Asp Ser Ala Cys Leu Ser Arg Ile Asn Thr Ala Lys Ala
    130                 135                 140

Tyr Val Asp Ser Thr Leu Pro Gly Gln Leu Asp Ser Val Tyr Thr Ala
145                 150                 155                 160

Ile Ser Thr Lys Ala Pro Ser Ala His Val Ala Val Leu Gly Tyr Pro
                165                 170                 175

Arg Phe Tyr Lys Leu Gly Gly Ser Cys Leu Ala Gly Leu Ser Glu Thr
            180                 185                 190

Lys Arg Ser Ala Ile Asn Asp Ala Ala Asp Tyr Leu Asn Ser Ala Ile
        195                 200                 205

Ala Lys Arg Ala Ala Asp His Gly Phe Thr Phe Gly Asp Val Lys Ser
    210                 215                 220

Thr Phe Thr Gly His Glu Ile Cys Ser Ser Ser Thr Trp Leu His Ser
225                 230                 235                 240

Leu Asp Leu Leu Asn Ile Gly Gln Ser Tyr His Pro Thr Ala Ala Gly
                245                 250                 255

Gln Ser Gly Gly Tyr Leu Pro Val Met Asn Ser Val Ala
            260                 265
```

<210> 68
<211> 1980
<212> DNA
<213> Streptomyces avermitilis

<400> 68

ccaccgccgg gtcggcggcg agtctcctgg cctcggtcgc ggagaggttg gccgtgtagc 60
cgttcagcgc ggcgccgaac gtcttcttca ccgtgccgcc gtactcgttg atcaggccct 120
tgcccttgct cgacgcggcc ttgaagccgg tgcccttctt gagcgtgacg atgtagctgc 180
ccttgatcgc ggtgggggag ccggcggcga gcaccgtgcc ctcggccggg gtggcctggg 240
cgggcagtgc ggtgaatccg cccacgaggg cgccggtcgc cacggcggtt atcgcggcga 300
tccggatctt cttgctacgc agctgtgcca tacgagggag tcctcctctg ggcagcggcg 360
cgcctgggtg gggcgcacgg ctgtgggggg tgcgcgcgtc atcacgcaca cggccctgga 420
gcgtcgtgtt ccgccctggg ttgagtaaag cctcggccat ctacgggggt ggctcaaggg 480
agttgagacc ctgtcatgag tctgacatga gcacgcaatc aacggggccg tgagcacccc 540

```
gggggcgaccc cggaaagtgc cgagaagtct tggcatggac acttcctgtc aacacgcgta     600
gctggtacga cggttacggc agagatcctg ctaaagggag gttccatgag acgttcccga     660
attacggcat acgtgacctc actcctcctc gccgtcggct gcgccctcac cggggcagcg     720
acggcgcagg cgtccccagc cgccgcggcc acgggctatg tggccctcgg cgactcgtac     780
tcgtccggtg tcggcgccgg cagctacctc agctccagcg gcgactgcaa gcgcagttcg     840
aaggcctatc cgtacctctg gcaggccgcg cattcaccct cgtcgttcag tttcatggct     900
tgctcgggcg ctcgtacggg tgatgtcctg gccaatcagc tcggcaccct gaactcgtcc     960
accggcctgg tctccctcac catcggaggc aacgacgcgg gcttctccga cgtcatgacg    1020
acctgtgtgc tccagtccga cagcgcctgc ctctcccgca tcaacacggc gaaggcgtac    1080
gtcgactcca ccctgcccgg ccaactcgac agcgtgtaca cggcgatcag cacgaaggcc    1140
ccgtcggccc atgtggccgt gctgggctac ccccgcttct acaaactggg cggctcctgc    1200
ctcgcgggcc tctcggagac caagcggtcc gccatcaacg acgcggccga ctatctgaac    1260
agcgccatcg ccaagcgcgc cgccgaccac ggcttcacct tcggcgacgt caagagcacc    1320
ttcaccggcc atgagatctg ctccagcagc acctggctgc acagtctcga cctgctgaac    1380
atcggccagt cctaccaccc gaccgcggcc ggccagtccg gcggctatct gccggtcatg    1440
aacagcgtgg cctgagctcc cacggcctga atttttaagg cctgaatttt taaggcgaag    1500
gtgaaccgga agcggaggcc ccgtccgtcg ggtctccgt cgcacaggtc accgagaacg    1560
gcacggagtt ggacgtcgtg cgcaccgggt cgcgcacctc gacggcgatc tcgttcgaga    1620
tcgttccgct cgtgtcgtac gtggtgacga acacctgctt ctgctgggtc tttccgccgc    1680
tcgccgggaa ggacagcgtc ttccagcccg gatccgggac ctcgcccttc ttggtcaccc    1740
agcggtactc cacctcgacc ggcacccggc ccaccgtgaa ggtcgccgtg aacgtgggcg    1800
cctgggcggt gggcggcggg caggcaccgg agtagtcggt gtgcacgccg gtgaccgtca    1860
ccttcacgga ctgggccggc ggggtcgtcg taccgccgcc gccaccgccg cctcccggag    1920
tggagcccga gctgtggtcg cccccgccgt cggcgttgtc gtcctcgggg gttttcgaac    1980
```

<210> 69
<211> 1371
<212> DNA
<213> Streptomyces spp

<400> 69

acaggccgat gcacggaacc gtacctttcc gcagtgaagc gctctccccc catcgttcgc 60
cgggacttca tccgcgattt tggcatgaac acttccttca acgcgcgtag cttgctacaa 120
gtgcggcagc agacccgctc gttggaggct cagtgagatt gacccgatcc ctgtcggccg 180
catccgtcat cgtcttcgcc ctgctgctcg cgctgctggg catcagcccg gcccaggcag 240
ccggcccggc ctatgtggcc ctgggggatt cctattcctc gggcaacggc gccggaagtt 300

acatcgattc gagcggtgac tgtcaccgca gcaacaacgc gtaccccgcc cgctgggcgg 360
cggccaacgc accgtcctcc ttcaccttcg cggcctgctc gggagcggtg accacggatg 420
tgatcaacaa tcagctgggc gccctcaacg cgtccaccgg cctggtgagc atcaccatcg 480
gcggcaatga cgcgggcttc gcggacgcga tgaccacctg cgtcaccagc tcggacagca 540
cctgcctcaa ccggctggcc accgccacca actacatcaa caccaccctg ctcgcccggc 600
tcgacgcggt ctacagccag atcaaggccc gtgcccccaa cgcccgcgtg gtcgtcctcg 660
gctacccgcg catgtacctg gcctcgaacc cctggtactg cctgggcctg agcaacacca 720
agcgcgcggc catcaacacc accgccgaca ccctcaactc ggtgatctcc tcccgggcca 780
ccgcccacgg attccgattc ggcgatgtcc gcccgacctt caacaaccac gaactgttct 840
tcggcaacga ctggctgcac tcactcaccc tgccggtgtg ggagtcgtac caccccacca 900
gcacgggcca tcagagcggc tatctgccgg tcctcaacgc caacagctcg acctgatcaa 960
cgcacggccg tgcccgcccc gcgcgtcacg ctcggcgcgg gcgccgcagc gcgttgatca 1020
gcccacagtg ccggtgacgg tcccaccgtc acggtcgagg gtgtacgtca cggtggcgcc 1080
gctccagaag tggaacgtca gcaggaccgt ggagccgtcc ctgacctcgt cgaagaactc 1140
cggggtcagc gtgatcaccc ctcccccgta gccgggggcg aaggcggcgc cgaactcctt 1200
gtaggacgtc cagtcgtgcg gcccggcgtt gccaccgtcc gcgtagaccg cttccatggt 1260
cgccagccgg tccccgcgga actcggtggg gatgtccgtg cccaaggtgg tcccggtggt 1320
gtccgagagc accgggggct cgtaccggat gatgtgcaga tccaaagaat t 1371

<210> 70
<211> 267
<212> PRT
<213> Streptomyces spp

<400> 70

```
Met Arg Leu Thr Arg Ser Leu Ser Ala Ala Ser Val Ile Val Phe Ala
1               5                   10                  15

Leu Leu Leu Ala Leu Leu Gly Ile Ser Pro Ala Gln Ala Ala Gly Pro
            20                  25                  30

Ala Tyr Val Ala Leu Gly Asp Ser Tyr Ser Ser Gly Asn Gly Ala Gly
        35                  40                  45

Ser Tyr Ile Asp Ser Ser Gly Asp Cys His Arg Ser Asn Asn Ala Tyr
    50                  55                  60

Pro Ala Arg Trp Ala Ala Ala Asn Ala Pro Ser Ser Phe Thr Phe Ala
65                  70                  75                      80
```

```
Ala Cys Ser Gly Ala Val Thr Thr Asp Val Ile Asn Asn Gln Leu Gly
                85                  90                  95

Ala Leu Asn Ala Ser Thr Gly Leu Val Ser Ile Thr Ile Gly Gly Asn
            100                 105                 110

Asp Ala Gly Phe Ala Asp Ala Met Thr Thr Cys Val Thr Ser Ser Asp
        115                 120                 125

Ser Thr Cys Leu Asn Arg Leu Ala Thr Ala Thr Asn Tyr Ile Asn Thr
    130                 135                 140

Thr Leu Leu Ala Arg Leu Asp Ala Val Tyr Ser Gln Ile Lys Ala Arg
145                 150                 155                 160

Ala Pro Asn Ala Arg Val Val Val Leu Gly Tyr Pro Arg Met Tyr Leu
                165                 170                 175

Ala Ser Asn Pro Trp Tyr Cys Leu Gly Leu Ser Asn Thr Lys Arg Ala
            180                 185                 190

Ala Ile Asn Thr Thr Ala Asp Thr Leu Asn Ser Val Ile Ser Ser Arg
        195                 200                 205

Ala Thr Ala His Gly Phe Arg Phe Gly Asp Val Arg Pro Thr Phe Asn
    210                 215                 220

Asn His Glu Leu Phe Phe Gly Asn Asp Trp Leu His Ser Leu Thr Leu
225                 230                 235                 240

Pro Val Trp Glu Ser Tyr His Pro Thr Ser Thr Gly His Gln Ser Gly
                245                 250                 255

Tyr Leu Pro Val Leu Asn Ala Asn Ser Ser Thr
            260                 265
```

<210> 71
<211> 8
<212> PRT
<213> Artificial

<220>
<223> Block 1 - GDSX block

<220>
<221> VARIANT
<222> (1)..(4)
<223> Xaa is a hydrophobic residue selected from Met, Ile, Leu, Val, Ala, Gly, Cys, His, Lys, Trp, Tyr or Phe

<220>
<221> VARIANT
<222> (8)..(8)
<223> Xaa is a hydrophobic residue selected from Met, Ile, Leu, Val, Ala, Gly, Cys, His, Lys, Trp, Tyr or Phe

<400> 71

```
Xaa Xaa Xaa Xaa Gly Asp Ser Xaa
1               5
```

<210> 72
<211> 6
<212> PRT
<213> Artificial

<220>
<223> Block 2 - GANDY block

<220>
<221> VARIANT
<222> (1)..(1)
<223> Xaa is a hydrophobic residue selected from Met, Ile, Leu, Val, Ala, Gly, Cys, His, Lys, Trp, Tyr or Phe

<220>
<221> VARIANT
<222> (3)..(3)
<223> Xaa is a hydrophobic residue selected from Met, Ile, Leu, Val, Ala, Gly, Cys, His, Lys, Trp, Tyr or Phe

<220>
<221> VARIANT
<222> (6)..(6)
<223> Xaa is a hydrophobic residue selected from Met, Ile, Leu, Val, Ala, Gly, Cys, His, Lys, Trp, Tyr or Phe

<400> 72

```
Xaa Gly Xaa Asn Asp Xaa
1               5
```

<210> 73
<211> 4
<212> PRT
<213> Artificial

<220>
<223> Sequence motif

<220>
<221> VARIANT
<222> (4)..(4)
<223> Xaa may be one or more of the following amino acid residues Leu, Ala, Val, Ile, Phe, Tyr, His, Gln, Thr, Asn, Met or Ser.

<400> 73

```
Gly Asp Ser Xaa
1
```